# EUROPEAN PATENT APPLICATION

(11) **EP 4 768 586 A1**
(43) Date of publication of application: **01.07.2026**
(21) Application number: 24870862.0
(22) Date of filing: 26.09.2024
(51) Int. Cl.: C12N 15/113, A61K 31/713, A61K 47/54, A61P 11/00, A61P 25/00, A61P 13/00, A61P 29/00

(54) **NEW SIRNA, COMPOSITION COMPRISING SAME, AND USE**

(30) Priority: 27.09.2023 CN 202311264167; 20.09.2024 CN 202411319454
(71) Applicant: Changchun Genescience Pharmaceutical Co., Ltd., Changchun, Jilin 130012 (CN)
(72) Inventor: ZHOU, Ling, Jilin 130012 (CN); LI, Fu, Jilin 130012 (CN); CHE, Jiuwei, Jilin 130012 (CN); JIA, Pan, Jilin 130012 (CN); YANG, Xue, Jilin 130012 (CN); CHAI, Xiaojuan, Jilin 130012 (CN); DAI, Yan, Jilin 130012 (CN); LI, Meng, Jilin 130012 (CN); WEI, Yong, Jilin 130012 (CN); ZHOU, Ziwen, Jilin 130012 (CN); JIANG, Wenbo, Jilin 130012 (CN); XU, John Liuzhong, Jilin 130012 (CN); WANG, Siqin, Jilin 130012 (CN); JIN, Lei, Jilin 130012 (CN)
(74) Representative: E. Blum & Co. AG
(86) International application number: PCT/CN2024/121385
(87) International publication number: WO 2025/067322

(57) **Abstract**

Provided is an siRNA that inhibits the expression of an RAGE gene, which siRNA comprises a sense strand and an antisense strand. The provided siRNA has good stability and can be used for efficiently and specifically inhibiting the expression of the RAGE gene, thereby providing important clinical value in the treatment of RAGE-related diseases.

## Description

### CROSS-REFERENCE TO RELATED APPLICATIONS

The present application claims priority to Chinese Patent Application No. 202311264167.4 filed on September 27, 2023, and Chinese Patent Application No. 202411319454.5 filed on September 20, 2024, which are incorporated herein by reference in their entirety.

### TECHNICAL FIELD

The present invention provides an siRNA for inhibiting the expression of a receptor for advanced glycation end products (RAGE) and a pharmaceutical composition thereof. The siRNA and the pharmaceutical composition thereof provided by the present invention can treat RAGE-related diseases.

### BACKGROUND ART

The receptor for advanced glycation end products ("RAGE" or "AGER") is a ~43 kDa transmembrane protein of the immunoglobulin superfamily that functions as a pro-inflammatory pattern recognition receptor. In its full-length membrane-bound form, the receptor has three functional domains: an extracellular ligand-binding domain, a hydrophobic transmembrane domain, and a cytoplasmic domain that mediates ligand-dependent signal transduction. The second non-membrane-bound soluble form of the receptor (sRAGE) contains only an extracellular ligand binding domain; formed by the proteolytic cleavage (or alternative splicing) of the full-length membrane-bound RAGE, and sRAGE antagonizes RAGE function because it binds to ligands but lacks the cytoplasmic signaling domain.

RAGE is constitutively expressed at high levels in the lungs, primarily located in type 1 alveolar epithelial cells. Other tissues in the body typically express RAGE at low levels, but its expression is upregulated in the presence of RAGE ligands and chronic inflammation. As a pattern recognition receptor, RAGE binds to a variety of endogenous ligands, including advanced glycation end products (glycomodified proteins or lipids), high mobility group box 1 (HMGB1), β-amyloid Aβ, and S100 protein. Different intermediate signaling pathways can be activated by different RAGE ligands (e.g., ERK1/2, p38, and JAK/STAT), ultimately leading to the production of reactive oxygen species, persistent activation of NF-κB, and alterations in pro-inflammatory genes (e.g., interleukins, interferons, and TNF-α). The transcription of the gene encoding RAGE itself is promoted by NF-κB, forming a positive feedback loop of persistent chronic inflammation.

RAGE is associated with chronic pathological inflammation that leads to many diseases, including: lung diseases (asthma, acute respiratory distress syndrome, idiopathic pulmonary fibrosis, chronic obstructive pulmonary disease, cystic fibrosis, pneumonia, lung cancer, bronchopulmonary dysplasia), cardiovascular diseases (atherosclerosis, myocardial infarction, heart failure, peripheral vascular disease), cancer, diabetes, chronic kidney disease, neurodegenerative diseases, rheumatoid arthritis, non-alcoholic steatohepatitis, damage caused by certain viral infections, including SARS-CoV-2, certain ocular inflammatory conditions, and skeletal muscle atrophy.

In the field of lung diseases, RAGE gene knockout (KO) mice are physiologically and histologically completely protected from allergic asthma caused by house dust mite allergens or ovalbumin. Similarly, RAGE knockout mice are protected from acute lung injury and inflammation induced by hyperoxia or lipopolysaccharide. (See, for example, Oczypok et al., Paediatr Respir Rev., 23: 40-49 (2017); Wang et al., Shock, 50: 472-482 (2018)). Genome-wide association studies (GWAS) have linked the functional gain variant of the RAGE allele (G82S) to increased inflammation, decreased lung function, and asthma risk (see, for example, Hancock et al., Nat Genet., 42: 45-52 (2010); Repapi et al., Nat Genet., 42: 36-44 (2010)).

Despite the potential appeal of RAGE as a drug target, developing effective and selective RAGE inhibitors has proven extremely challenging. A wide range of RAGE ligands interact with multiple binding sites within the antibody-like extracellular domain, rather than with discrete domains (see, for example, Rojas et al., Current Drug Targets, 20: 340-346 (2019)). Therefore, there is a need for RAGE RNAi reagents that are suitable as therapeutic agents for treating RAGE-related diseases and conditions.

### SUMMARY OF THE INVENTION

The present invention provides an siRNA that can effectively inhibit the RAGE gene expression, and thus provides a medicament and method for preventing and/or treating RAGE-related diseases.

### siRNA

In one aspect, the present invention provides a small interfering RNA (siRNA) for inhibiting the expression of an RAGE gene, wherein the siRNA comprises a sense strand and an antisense strand, the antisense strand comprises at least 14 consecutive nucleotides that differ from the nucleotide sequence set forth in any one of SEQ ID NO: 1 to SEQ ID NO: 419 by no more than 4 nucleotides (e.g., 0, 1, 2, 3, or 4 nucleotides), and the sense strand is at least partially complementary to the antisense strand.

The expression "at least partially complementary" means that the two sequences can be completely complementary, or have no more than 4, 3, 2 or 1 mismatched base pairs overall, while retaining the ability to hybridize under relevant conditions. Those skilled in the art will be able to determine the most suitable conditions for testing the complementarity of two sequences depending on the ultimate application of the hybridized nucleotides. Such conditions can, for example, be stringent conditions, such as 400 mM NaCl, 40 mM PIPES pH 6.4, 1 mM EDTA, 50°C or 70°C for 12-16 h, followed by washing. Other conditions, such as physiologically relevant conditions that may be encountered in an organism, may also apply.

In some embodiments, the RAGE mRNA refers to an mRNA having, for example, the sequence set forth in Genbank: NM_001136.5.

In some embodiments, the antisense strand comprises at least 14 consecutive nucleotides (e.g., at least 15, at least 16, or at least 17 consecutive nucleotides) that differ from the nucleotide sequence set forth in any one of SEQ ID NO: 1 to SEQ ID NO: 419 by no more than 4 nucleotides (e.g., 0, 1, 2, 3 or 4 nucleotides). In some embodiments, the antisense strand comprises at least 14 consecutive nucleotides (e.g., at least 15, at least 16, or at least 17 consecutive nucleotides) that differ from the nucleotide sequence set forth in any one of SEQ ID NO: 1 to SEQ ID NO: 419 by no more than 3 nucleotides (e.g., 0, 1, 2, or 3 nucleotides). In some embodiments, the antisense strand comprises at least 14 consecutive nucleotides (e.g., at least 15, at least 16, or at least 17 consecutive nucleotides) that differ from the nucleotide sequence set forth in any one of SEQ ID NO: 1 to SEQ ID NO: 419 by no more than 2 nucleotides (e.g., 0, 1, or 2 nucleotides). In some embodiments, the antisense strand comprises at least 14 consecutive nucleotides (e.g., at least 15, at least 16, or at least 17 consecutive nucleotides) that differ from the nucleotide sequence set forth in any one of SEQ ID NO: 1 to SEQ ID NO: 419 by no more than 1 nucleotide (e.g., 0 or 1 nucleotide). In some embodiments, the antisense strand comprises at least 14 consecutive nucleotides (e.g., at least 15, at least 16, or at least 17 consecutive nucleotides) of the nucleotide sequence set forth in any one of SEQ ID NO: 1 to SEQ ID NO: 419.

In some embodiments, the antisense strand comprises at least 14 consecutive nucleotides that differ from the nucleotide sequence set forth in any one of SEQ ID NO: 1 to SEQ ID NO: 419 by no more than 2 nucleotides.

In some embodiments, the antisense strand comprises at least 17 consecutive nucleotides that differ from the nucleotide sequence set forth in any one of SEQ ID NO: 1 to SEQ ID NO: 419 by no more than 4 nucleotides.

In some embodiments, the antisense strand comprises at least 17 consecutive nucleotides that differ from the nucleotide sequence set forth in any one of SEQ ID NO: 1 to SEQ ID NO: 419 by no more than 2 nucleotides (e.g., 0, 1 or 2 nucleotides). In some embodiments, the antisense strand comprises at least 17 consecutive nucleotides that differ from the nucleotide sequence set forth in any one of SEQ ID NO: 1 to SEQ ID NO: 419 by no more than 1 nucleotide (e.g., 0 or 1 nucleotide). In some embodiments, the antisense strand comprises at least 17 consecutive nucleotides of the nucleotide sequence set forth in any one of SEQ ID NO: 1 to SEQ ID NO: 419.

In some embodiments, the antisense strand is the nucleotide sequence set forth in any one of SEQ ID NO: 1 to SEQ ID NO: 419.

In some embodiments, there are no more than 4 nucleotide mismatches between the sense strand and the antisense strand. There are no more than 3 nucleotide mismatches between the sense strand and the antisense strand. In some embodiments, there are no more than 2 nucleotide mismatches between the sense strand and the antisense strand. In some embodiments, there are no more than 1 nucleotide mismatches between the sense strand and the antisense strand. In some embodiments, the sense strand and the antisense strand are fully complementary.

In some embodiments, the sense strand and the antisense strand have at least 14, 15, 16, 17, 18, 19, 20 or 21 complementary nucleotides. In some embodiments, the sense strand and the antisense strand are complementary to each other with 14 to 19 nucleotides.

In some embodiments, the sense strand and the antisense strand are at least 85% (e.g., at least 90%, at least 95%, at least 99%) complementary or fully complementary over at least 14 consecutive nucleotides.

In some embodiments, the sense strand and the antisense strand have at least 17, 18, 19, 20 or 21 complementary nucleotides. In some embodiments, the sense strand and the antisense strand are complementary to each other with 17 to 19 nucleotides.

In some embodiments, the sense strand and the antisense strand are at least 85% (e.g., at least 90%, at least 95%, at least 99%) complementary or fully complementary over at least 17 consecutive nucleotides.

In some embodiments, the sense strand and the antisense strand form a duplex region of 14-30 nucleotide pairs in length, for example, a duplex region of 14-25 nucleotide pairs, 14-23 nucleotide pairs, 14-21 nucleotide pairs, 15-25 nucleotide pairs, 15-23 nucleotide pairs, 15-21 nucleotide pairs,17-25 nucleotide pairs, 17-23 nucleotide pairs, or 17-21 nucleotide pairs, such as 14, 15, 16, 17, 18, 19, 20 or 21 nucleotide pairs. In some embodiments, the sense strand and the antisense strand form a duplex region with a length of 14 to 19 nucleotide pairs.

In some embodiments, the antisense strand has a length of 14-30 (e.g., 14-29, 14-28, 14-27, 19-30, 19-29, 19-28, 19-27, 19-25, 19-23, 21-25, 21-23) nucleotides, and the sense strand has a length of 14-30 (e.g., 14-29, 14-28, 14-27, 14-26, 14-25, 14-21, 19-21) nucleotides. In some embodiments, the lengths of the antisense strand and the sense strand are each independently 17 to 30 nucleotides.

In some embodiments, the antisense strand has a length of 19-27 nucleotides; and the sense strand has a length of 17-25 nucleotides.

In some embodiments, the antisense strand has a length of 21-23 nucleotides; and the sense strand has a length of 19-21 nucleotides.

In some embodiments, the antisense strand has a length of 21 nucleotides; and the sense strand has a length of 19 nucleotides.

In some embodiments, the siRNA comprises a blunt end and/or an overhang.

In some embodiments, the siRNA comprises an overhang of one or more single-stranded nucleotides, for example, an overhang of 1, 2, 3 or 4 nucleotides. In some embodiments, the overhang can be on the sense strand, the antisense strand, or any combination thereof. In some embodiments, the overhang is present at the 5' end, the 3' end or both ends of the antisense strand or the sense strand of the siRNA.

In some embodiments, the 3' end of the antisense strand of the siRNA has an overhang of 2 nucleotides. In some embodiments, the 3' end of the sense strand of the siRNA is a blunt end.

In some embodiments, the sense strand of the siRNA comprises at least 14 consecutive nucleotides (e.g., at least 15, at least 16, or at least 17 consecutive nucleotides) that differ from the nucleotide sequence set forth in any one of SEQ ID NO: 420 to SEQ ID NO: 838 by no more than 4 nucleotides (e.g., 0, 1, 2, 3 or 4 nucleotides). The at least 14 consecutive nucleotides comprised by the sense strand and antisense strand of the siRNA form a duplex region. In some embodiments, the sense strand comprises at least 14 consecutive nucleotides (e.g., at least 15, at least 16, or at least 17 consecutive nucleotides) that differ from the nucleotide sequence set forth in any one of SEQ ID NO: 420 to SEQ ID NO: 838 by no more than 3 nucleotides (e.g., 0, 1, 2, or 3 nucleotides). In some embodiments, the sense strand comprises at least 14 consecutive nucleotides (e.g., at least 15, at least 16, or at least 17 consecutive nucleotides) that differ from the nucleotide sequence set forth in any one of SEQ ID NO: 420 to SEQ ID NO: 838 by no more than 2 nucleotides (e.g., 0, 1, or 2 nucleotides). In some embodiments, the sense strand comprises at least 14 consecutive nucleotides (e.g., at least 15, at least 16, or at least 17 consecutive nucleotides) that differ from the nucleotide sequence set forth in any one of SEQ ID NO: 420 to SEQ ID NO: 838 by no more than 1 nucleotide (e.g., 0 or 1 nucleotide). In some embodiments, the sense strand has a region within the 14 consecutive nucleotides that is at least 85% (e.g., at least 90%, at least 95%, at least 99%) complementary or fully complementary to the antisense strand. In some embodiments, the sense strand comprises at least 14 consecutive nucleotides (e.g., at least 15, at least 16, or at least 17 consecutive nucleotides) of the nucleotide sequence set forth in any one of SEQ ID NO: 420 to SEQ ID NO: 838. In some embodiments, the sense strand is the nucleotide sequence set forth in any one of SEQ ID NO: 420 to SEQ ID NO: 838.

In some embodiments, the sense strand comprises at least 14 consecutive nucleotides that differ from the nucleotide sequence set forth in any one of SEQ ID NO: 420 to SEQ ID NO: 838 by no more than 2 nucleotides.

In some embodiments, the sense strand of the siRNA comprises at least 17 consecutive nucleotides that differ from the nucleotide sequence set forth in any one of SEQ ID NO: 420 to SEQ ID NO: 838 by no more than 4 nucleotides.

In some embodiments, the sense strand comprises at least 17 consecutive nucleotides that differ from the nucleotide sequence set forth in any one of SEQ ID NO: 420 to SEQ ID NO: 838 by no more than 2 nucleotides (e.g., 0, 1 or 2 nucleotides). In some embodiments, the sense strand comprises at least 17 consecutive nucleotides that differ from the nucleotide sequence set forth in any one of SEQ ID NO: 420 to SEQ ID NO: 838 by no more than 1 nucleotide (e.g., 0 or 1 nucleotide). In some embodiments, the sense strand has a region within the 17 consecutive nucleotides that is at least 85% (e.g., at least 90%, at least 95%, at least 99%) complementary or fully complementary to the antisense strand. In some embodiments, the sense strand comprises at least 17 consecutive nucleotides of the nucleotide sequence set forth in any one of SEQ ID NO: 420 to SEQ ID NO: 838. In some embodiments, the sense strand is the nucleotide sequence set forth in any one of SEQ ID NO: 420 to SEQ ID NO: 838.

In some embodiments, the antisense strand of the siRNA comprises the antisense strand sequence of any of duplexes GPSZRA001 - GPSZRA419 provided in Table 1, or a portion thereof (e.g., at least 14 consecutive nucleotides thereof), and the sense strand of the siRNA comprises the sense strand sequence of the duplex, or a portion thereof (e.g., at least 14 consecutive nucleotides thereof).

In some embodiments, the antisense strand of the siRNA comprises the antisense strand sequence of any one of the duplexes provided in Table 7, or a portion thereof (e.g., at least 14 consecutive nucleotides thereof). Preferably, the sense strand of the siRNA comprises the sense strand sequence of the duplex, or a portion thereof (e.g., at least 14 consecutive nucleotides thereof).

In some embodiments, the antisense strand of the siRNA comprises the antisense strand sequence of any one of the duplexes provided in Table 1: GPSZRA272, GPSZRA267, GPSZRA271, GPSZRA381, GPSZRA284, GPSZRA266, GPSZRA283, GPSZRA308, GPSZRA360, GPSZRA277, GPSZRA276, GPSZRA288, GPSZRA282 or GPSZRA285, or a portion thereof (e.g., at least 14 consecutive nucleotides thereof). Preferably, the sense strand of the siRNA comprises the sense strand sequence of the duplex, or a portion thereof (e.g., at least 14 consecutive nucleotides thereof).

In some embodiments, the antisense strand of the siRNA comprises the antisense sequence of any one of the duplexes provided in Table 1: GPSZRA286, GPSZRA402, GPSZRA092, GPSZRA307, GPSZRA290, GPSZRA410, GPSZRA274, GPSZRA289, GPSZRA382, GPSZRA409, GPSZRA255, GPSZRA087, GPSZRA379, GPSZRA165, GPSZRA275, GPSZRA408, GPSZRA257, GPSZRA318, GPSZRA281, GPSZRA104, GPSZRA254, GPSZRA103, GPSZRA287, GPSZRA252, GPSZRA378, GPSZRA239, GPSZRA088, GPSZRA090, GPSZRA085, GPSZRA380, GPSZRA217, GPSZRA269, GPSZRA212, GPSZRA086, GPSZRA084, GPSZRA214, GPSZRA228, GPSZRA105, GPSZRA292, GPSZRA313, GPSZRA304, GPSZRA237, GPSZRA345, GPSZRA233, GPSZRA215, GPSZRA093, GPSZRA333 or GPSZRA270, or a portion thereof (e.g., at least 14 consecutive nucleotides thereof). Preferably, the sense strand of the siRNA comprises the sense strand sequence of the duplex, or a portion thereof (e.g., at least 14 consecutive nucleotides thereof).

In some embodiments, the siRNA targets a region located within nucleotides 1-400 of the RAGE mRNA, for example, targeting the sequence set forth in SEQ ID NO: 1417. Preferably, the siRNA comprises an antisense strand and/or a sense strand derived from any one of the duplexes GPSZRA001 to GPSZRA118.

In some embodiments, the siRNA targets a region located within nucleotides 351-750 of the RAGE mRNA, for example, targeting the sequence set forth in SEQ ID NO: 1418. Preferably, the siRNA comprises an antisense strand and/or a sense strand derived from any one of the duplexes GPSZRA090 to GPSZRA251.

In some embodiments, the siRNA targets a region located within nucleotides 701-1100 of the RAGE mRNA, for example, targeting the sequence set forth in SEQ ID NO: 1419. Preferably, the siRNA comprises an antisense strand and/or a sense strand derived from any one of the duplexes GPSZRA250 to GPSZRA358.

In some embodiments, the siRNA targets a region located within nucleotides 1021-1420 of the RAGE mRNA, for example, targeting the sequence set forth in SEQ ID NO: 1420. Preferably, the siRNA comprises an antisense strand and/or a sense strand derived from any one of the duplexes GPSZRA359 to GPSZRA419.

### Modification

The siRNA of the present invention can be modified in the nucleobase structure or in the ribose-phosphate backbone structure to reduce off-target effects and/or increase the biological stability of the molecule or increase the physical stability of the duplex formed between the antisense and sense nucleic acids. Therefore, siRNA sequences comprising any modification are also encompassed within the scope of the present invention. The siRNA molecules comprising ribonucleoside analogs or derivatives must retain the ability to form duplexes and allow or mediate specific degradation of the target RNA via the RISC pathway.

In some embodiments, the siRNA at least comprises one modified nucleotide. The modifications need not be identical for each of the multiple modified ribonucleosides in the siRNA.

In some embodiments, all nucleotides in the sense strand and/or antisense strand of the siRNA are modified nucleotides or nucleotide analogs. In some embodiments, all nucleotides in the sense strand of the siRNA are modified nucleotides or nucleotide analogs, and all nucleotides in the antisense strand of the siRNA are modified nucleotides or nucleotide analogs.

In some embodiments, the siRNA comprises 2'-modified nucleotides.

In some embodiments, the modified nucleotides or nucleotide analogs are selected from 2'-methoxynucleotides, 2'-fluoronucleotides, 5'-(E)-vinylphosphonate-modified nucleotides, 2'-deoxyribonucleotides, 2',3'-seco-nucleotide analogs, 2'-fluoroarabinonucleotides, 2'-methoxyethyl nucleotides, 2'-amino-modified nucleotides, 2'-alkyl-modified nucleotides, 3'-methoxynucleotides, 2'-allyl-modified nucleotides, phosphorothioate group-containing nucleotides, methylphosphonate group-containing nucleotides, 5'-phosphate-containing nucleotides, 5'-phosphate mimic-containing nucleotides, diol-modified nucleotides, abasic nucleotides, morpholino nucleotides, locked nucleic acids (LNA), unlocked nucleic acids (UNA), or glycerol nucleotides (GNA), but not limited thereto in the present invention.

In some embodiments, the modified nucleotide or nucleotide analogue is selected from at least one or a combination of 2'-methoxynucleotides, 2'-fluoronucleotides, nucleotides containing a thiophosphate group, locked nucleic acids, 5'-(E)-vinylphosphonate modified nucleotides. In some embodiments, the sense strand comprises at least one or a combination of 2'-methoxynucleotides and 2'-fluoronucleotides. In some embodiments, the sense strand comprises at least one or a combination of 2'-methoxynucleotides, 2'-fluoronucleotides, and locked nucleic acids. In some embodiments, the antisense strand comprises at least one or a combination of 2'-methoxynucleotides, 2'-fluoronucleotides, and 5'-(E)-vinylphosphonate-modified nucleotides. In some embodiments, the sense strand comprises at least locked nucleic acid. In some embodiments, the sense strand comprises at least one nucleotide containing a thiophosphate group. In some embodiments, the antisense strand comprises at least one nucleotide containing a thiophosphate group.

In some embodiments, the first nucleotide at the 5' end of the antisense strand is a 5'-(E)-vinylphosphonate-modified nucleotide. The conventional antisense strand needs to be phosphorylated at the 5' end before it can be loaded with the Ago2 protein to form a RISC complex and exert RNAi activity. By introducing 5'-(E)-vinylphosphonate (5'-(E)-VP), the antisense strand is pre-phosphorylated, eliminating the need for rephosphorylation in vivo. This allows for direct loading to form a RISC complex, improving antisense strand loading efficiency and enhancing the overall activity of siRNA.

In some embodiments, the nucleotides in the sense strand are selected from at least 2 of 2'-methoxynucleotides, 2'-fluoronucleotides or locked nucleic acids, and/or the nucleotides in the antisense strand are selected from 2'-methoxynucleotides and 2'-fluoronucleotides; preferably, the first nucleotide at the 5' end of the antisense strand further comprises a 5'-(E)-vinylphosphonate modification.

In some embodiments, the siRNA of the present invention comprises a modified internucleoside linkage or a modified backbone. The modified internucleoside linkage or backbone includes, but is not limited to, phosphorothioate, 2'-O methoxyethyl (MOE), 2'-fluoro, an alkyl phosphate, a phosphorodithioate, an alkyl phosphorothioate, a phosphoramidate, a carbamate, a carbonate, a phosphotriester, an acetamidate, a carboxymethyl ester, and a combination thereof.

In some embodiments, the modified nucleotide is a nucleotide of which the phosphate group is modified with a phosphorothioate group. That is, a non-bridging oxygen atom in the phosphodiester bond is substituted with a sulfur atom, thereby replacing the phosphodiester bond with a thiophosphodiester bond.

In some embodiments, each of the 5' end and/or 3' end of the sense strand independently comprises 1 or 2 phosphorothioate linkages; and/or each of the 5' end and/or 3' end of the antisense strand independently comprises 1 or 2 phosphorothioate linkages. In some embodiments, the 5' end of the sense strand comprises 1 or 2 phosphorothioate linkages, each of the 5' end and/or 3' end of the antisense strand independently comprises 1 or 2 phosphorothioate linkages.

In some embodiments, the sense strand may include one or more capping residues or moieties, referred to as "capping residues". A "capping residue" is a non-nucleotide compound or other moiety that can be incorporated at one or more ends of the nucleotide sequence of an siRNA. In some embodiments, the capping residue is present at the 5' end, the 3' end, or both the 5' end and the 3' end of the sense strand.

In some embodiments, an inverted abasic residue (iab) is added as a capping residue. Reference can be made to F. Czauderna, Nucleic Acids Res.,2003,31(11),2705-16. In some embodiments, the sense strand may comprise more than one inverted abasic deoxyribose moiety as a capping residue at the 5' end and/or the 3' end.

In some embodiments, one or more inverted abasic residues (iab) are added to the 3' end of the sense strand. In some embodiments, one or more inverted abasic residues (iab) are added to the 5' end of the sense strand. In some embodiments, the sense strand of the siRNA comprises one or more inverted abasic residues at or near one or more ends.

The inverted abasic residues may be linked via phosphate, phosphorothioate, or other internucleoside linkages.

In some embodiments, the antisense strand of the siRNA has a length of 21 nucleotides and comprises the following modification patterns:
(1) 5'-NmsNfsNmNfNmNfNmNfNmNfNmNmNmNfNmNfNmNfNmsNmsNm-3' (SEQ ID NO: 1409);
(2) 5'-NmsNfsNmNmNmNfNmNfNmNmNmNmNmNfNmNfNmNmNmsNmsNm-3' (SEQ ID NO: 1410);
(3) 5'-NmsNfsNmNmNmNfNmNmNmNmNmNmNmNfNmNfNmNmNmsNmsNm-3' (SEQ ID NO: 1411);
(4) 5'-VPNmsNfsNmNfNmNfNmNfNmNfNmNmNmNfNmNfNmNfNmsNmsNm-3' (SEQ ID NO: 1423);
(5) 5'-VPNmsNfsNmNmNmNfNmNfNmNmNmNmNmNfNmNfNmNmNmsNmsNm-3' (SEQ ID NO: 1424); or
(6) 5'-VPNmsNfsNmNmNmNfNmNmNmNmNmNmNmNfNmNfNmNmNmsNmsNm-3' (SEQ ID NO: 1425);
wherein, Nm represents a methoxy-modified nucleotide, Nf represents a fluoro-modified nucleotide, VP indicates that the nucleotide adjacent to its right is a 5'-(E)-vinylphosphonate-modified nucleotide, and s represents a phosphorothioate linkage. Nucleotides can be selected from C, G, U or A.

In some embodiments, the sense strand of the siRNA has a length of 19 nucleotides and comprises the following modification patterns:
(1) 5'-NmsNmNfNmNfNmNfNfNfNmNfNmNfNmNfNmNfNmsNf-3' (SEQ ID NO: 1412);
(2) 5'-NmsNmNfNmNmNmNfNfNfNmNfNmNfNmNfNmNmNmsNm-3' (SEQ ID NO: 1413);
(3) 5'-NmsNmNmNmNmNmNfNfNfNmNmNmNmNmNmNmNmNmsNm-3' (SEQ ID NO: 1414);
(4) 5'-N(LNA)sNmNfNmNmNmNfNfNfNmNfNmNfNmNfNmNmNmsNm-3' (SEQ ID NO: 1415); or
(5) 5'-NmsNmNmNmNfNmNfNfNfNmNmNmNmNmNfNmNmNmsNm-3' (SEQ ID NO: 1416);
wherein, Nm represents a methoxy-modified nucleotide, Nf represents a fluoro-modified nucleotide, N(LNA) represents a locked nucleic acid-modified nucleotide, and s represents a phosphorothioate linkage. Nucleotides can be selected from C, G, U or A.

In some embodiments, the siRNA comprises a modification pattern selected from the following pairs, wherein the first SEQ ID NO corresponds to the antisense strand and the second SEQ ID NO corresponds to the sense strand: SEQ ID NO: 1409 and SEQ ID NO: 1412; SEQ ID NO: 1409 and SEQ ID NO: 1413; SEQ ID NO: 1409 and SEQ ID NO: 1414; SEQ ID NO: 1409 and SEQ ID NO: 1415; SEQ ID NO: 1409 and SEQ ID NO: 1416; SEQ ID NO: 1410 and SEQ ID NO: 1412; SEQ ID NO: 1410 and SEQ ID NO: 1413; SEQ ID NO: 1410 and SEQ ID NO: 1414; SEQ ID NO: 1410 and SEQ ID NO: 1415; SEQ ID NO: 1410 and SEQ ID NO: 1416; SEQ ID NO: 1411 and SEQ ID NO: 1412; SEQ ID NO: 1411 and SEQ ID NO: 1413; SEQ ID NO: 1411 and SEQ ID NO: 1414; SEQ ID NO: 1411 and SEQ ID NO: 1415; SEQ ID NO: 1411 and SEQ ID NO: 1416; SEQ ID NO: 1423 and SEQ ID NO: 1412; SEQ ID NO: 1423 and SEQ ID NO: 1413; SEQ ID NO: 1423 and SEQ ID NO: 1414; SEQ ID NO: 1423 and SEQ ID NO: 1415; SEQ ID NO: 1423 and SEQ ID NO: 1416; SEQ ID NO: 1424 and SEQ ID NO: 1412; SEQ ID NO: 1424 and SEQ ID NO: 1413; SEQ ID NO: 1424 and SEQ ID NO: 1414; SEQ ID NO: 1424 and SEQ ID NO: 1415; SEQ ID NO: 1424 and SEQ ID NO: 1416; SEQ ID NO: 1425 and SEQ ID NO: 1412; SEQ ID NO: 1425 and SEQ ID NO: 1413; SEQ ID NO: 1425 and SEQ ID NO: 1414; SEQ ID NO: 1425 and SEQ ID NO: 1415; SEQ ID NO: 1425 and SEQ ID NO: 1416.

In some embodiments, the antisense strand comprises the nucleotide sequence set forth in any one of SEQ ID NOs: 839-1123, 1426-1438.

In some embodiments, the sense strand comprises the nucleotide sequence set forth in any one of SEQ ID NOs: 1124-1408.

In some embodiments, the sequences of the sense strand and the antisense strand of the siRNA are selected from the sense strand and antisense strand sequences of any one of the duplexes GPSZRA084S1 - GPSZRA402S5-VP provided in Table 4.

In some embodiments, the antisense strand comprises the nucleotide sequence set forth in any one of SEQ ID NOs: 882, 1034 and 1037. In some embodiments, the sense strand comprises the nucleotide sequence set forth in any one of SEQ ID NOs: 1167, 1319 and 1322. In some embodiments, the sequences of the sense strand and the antisense strand of the siRNA are selected from the sense strand and antisense strand sequences of any one of the duplexes GPSZRA318S1, GPSZRA281S4 or GPSZRA284S4 as provided in Table 4.

In some embodiments, the antisense strand comprises the nucleotide sequence set forth in any one of SEQ ID NOs: 846, 850, 852, 858, 860, 861, 863, 866, 869, 872, 878, 890, 891, 894, 904, 907, 909, 913-915, 920, 923, 924, 926, 935, 939, 946, 947, 951, 961, 964, 971, 972, 980, 992, 996, 1004, 1008, 1018, 1021, 1023, 1028, 1029, 1040, 1049, 1053, 1061, 1065, 1075, 1078, 1080, 1085, 1086, 1094, 1097, 1106, 1118. In some embodiments, the sense strand comprises the nucleotide sequence set forth in any one of SEQ ID NOs: 1131, 1135, 1137, 1143, 1145, 1146, 1148, 1151, 1154, 1157, 1163, 1175, 1176, 1179, 1189, 1192, 1194, 1198-1200, 1205, 1208, 1209, 1211, 1220, 1224, 1231, 1232, 1236, 1246, 1249, 1256, 1257, 1265, 1277, 1281, 1289, 1293, 1303, 1306, 1308, 1313, 1314, 1325, 1334, 1338, 1346, 1350, 1360, 1363, 1365, 1370, 1371, 1379, 1382, 1391, 1403. In some embodiments, the sequences of the sense strand and the antisense strand of the siRNA are selected from the sequences of the sense strand and the antisense strand of any one of the duplexes provided in Table 4: GPSZRA233S2, GPSZRA272S2, GPSZRA284S3, GPSZRA402S5, GPSZRA402S3, GPSZRA314S1, GPSZRA314S3, GPSZRA272S5, GPSZRA314S4, GPSZRA271S3, GPSZRA287S4, GPSZRA287S5, GPSZRA314S2, GPSZRA314S5, GPSZRA287S2, GPSZRA233S5, GPSZRA402S4, GPSZRA410S2, GPSZRA231S5, GPSZRA231S1, GPSZRA227S2, GPSZRA402S2, GPSZRA272S4, GPSZRA271S4, GPSZRA410S4, GPSZRA276S1, GPSZRA318S2, GPSZRA271S2, GPSZRA272S1, GPSZRA285S2, GPSZRA231S4, GPSZRA284S1, GPSZRA318S3, GPSZRA233S1, GPSZRA231S2, GPSZRA231S3, GPSZRA402S1, GPSZRA233S4, GPSZRA284S5, GPSZRA271S5, GPSZRA272S3, GPSZRA284S2, GPSZRA267S2, GPSZRA222S1, GPSZRA318S4, GPSZRA292S1, GPSZRA287S1, GPSZRA410S3, GPSZRA281S2, GPSZRA275S1, GPSZRA227S5, GPSZRA407S1, GPSZRA227S4, GPSZRA281S1, GPSZRA227S3, GPSZRA381S2, or GPSZRA410S1.

In some embodiments, the antisense strand comprises the nucleotide sequence set forth in any one of SEQ ID NOs: 846, 960, 1017, 847, 904, 961, 1018, 1075, 1076, 850, 907, 964, 1021, 1078, 852, 909, 966, 1023, 1080, 913, 970, 1084, 857, 914, 971, 1028, 1085, 1035, 923, 980, 1037, 1094, 1040, 1097, 930, 878, 935, 992, 1049, 1106, 939, 996, 1053, 1110, 889, 946, 1003, 890, 947, 1004, 1061, 1118, 891, 948, 1062, 894, 951, 1008, 1065. In some embodiments, the sense strand comprises the nucleotide sequence set forth in any one of SEQ ID NOs: 1131, 1245, 1302, 1132, 1189, 1246, 1303, 1360, 1361, 1135, 1192, 1249, 1306, 1363, 1137, 1194, 1251, 1308, 1365, 1198, 1255, 1369, 1142, 1199, 1256, 1313, 1370, 1320, 1208, 1265, 1322, 1379, 1325, 1382, 1215, 1163, 1220, 1277, 1334, 1391, 1224, 1281, 1338, 1395, 1174, 1231, 1288, 1175, 1232, 1289, 1346, 1403, 1176, 1233, 1347, 1179, 1236, 1293, 1350. In some embodiments, the sequences of the sense strand and the antisense strand of the siRNA are selected from the sequences of the sense strand and the antisense strand of any one of the duplexes provided in Table 4: GPSZRA222S1, GPSZRA222S3, GPSZRA222S4, GPSZRA227S1, GPSZRA227S2, GPSZRA227S3, GPSZRA227S4, GPSZRA227S5, GPSZRA228S5, GPSZRA231S1, GPSZRA231S2, GPSZRA231S3, GPSZRA231S4, GPSZRA231S5, GPSZRA233S1, GPSZRA233S2, GPSZRA233S3, GPSZRA233S4, GPSZRA233S5, GPSZRA267S2, GPSZRA267S3, GPSZRA267S5, GPSZRA271S1, GPSZRA271S2, GPSZRA271S3, GPSZRA271S4, GPSZRA271S5, GPSZRA282S4, GPSZRA284S2, GPSZRA284S3, GPSZRA284S4, GPSZRA284S5, GPSZRA287S4, GPSZRA287S5, GPSZRA301S2, GPSZRA314S1, GPSZRA314S2, GPSZRA314S3, GPSZRA314S4, GPSZRA314S5, GPSZRA318S2, GPSZRA318S3, GPSZRA318S4, GPSZRA318S5, GPSZRA381S1, GPSZRA381S2, GPSZRA381S3, GPSZRA402S1, GPSZRA402S2, GPSZRA402S3, GPSZRA402S4, GPSZRA402S5, GPSZRA407S1, GPSZRA407S2, GPSZRA407S4, GPSZRA410S1, GPSZRA410S2, GPSZRA410S3, or GPSZRA410S4.

In some embodiments, the antisense strand comprises the nucleotide sequence set forth in any one of SEQ ID NOs: 846, 960, 1017, 847, 1018, 1075, 1023, 913, 980, 1040, 878, 935, 992, 1049, 1106, 939, 996, 1053, 946, 1003, 890, 947, 1061, 1118, 948, 1062, 894, 951, 1065. In some embodiments, the sense strand comprises the nucleotide sequence set forth in any one of SEQ ID NOs: 1131, 1245, 1302, 1132, 1303, 1360, 1308, 1198, 1265, 1325, 1163, 1220, 1277, 1334, 1391, 1224, 1281, 1338, 1231, 1288, 1175, 1232, 1346, 1403, 1233, 1347, 1179, 1236, 1350. In some embodiments, the sequences of the sense strand and the antisense strand of the siRNA are selected from the sequences of the sense strand and the antisense strand of any one of the duplexes provided in Table 4: GPSZRA222S1, GPSZRA222S3, GPSZRA222S4, GPSZRA227S1, GPSZRA227S4, GPSZRA227S5, GPSZRA233S4, GPSZRA267S2, GPSZRA284S3, GPSZRA287S4, GPSZRA314S1, GPSZRA314S2, GPSZRA314S3, GPSZRA314S4, GPSZRA314S5, GPSZRA318S2, GPSZRA318S3, GPSZRA318S4, GPSZRA381S2, GPSZRA381S3, GPSZRA402S1, GPSZRA402S2, GPSZRA402S4, GPSZRA402S5, GPSZRA407S2, GPSZRA407S4, GPSZRA410S1, GPSZRA410S2, or GPSZRA410S4.

In some embodiments, the antisense strand comprises the nucleotide sequence set forth in any one of SEQ ID NOs: 846, 960, 1017, 847, 913, 980, 878, 935, 992, 1106, 939, 1061, 1118. In some embodiments, the sense strand comprises the nucleotide sequence set forth in any one of SEQ ID NOs: 1131, 1245, 1302, 1132, 1198, 1265, 1163, 1220, 1277, 1391, 1224, 1346, 1403. In some embodiments, the sequences of the sense strand and the antisense strand of the siRNA are selected from the sequences of the sense strand and the antisense strand of any one of the duplexes provided in Table 4: GPSZRA222S1, GPSZRA222S3, GPSZRA222S4, GPSZRA227S1, GPSZRA267S2, GPSZRA284S3, GPSZRA314S1, GPSZRA314S2, GPSZRA314S3, GPSZRA314S5, GPSZRA318S2, GPSZRA402S4, or GPSZRA402S5.

In some embodiments, the antisense strand comprises the nucleotide sequence set forth in any one of SEQ ID NOs: 1426-1438. In some embodiments, the sense strand comprises the nucleotide sequence set forth in any one of SEQ ID NOs: 1131, 1245, 1302, 1132, 1198, 1265, 1163, 1220, 1277, 1391, 1224, 1346, 1403. In some embodiments, the sequences of the sense strand and the antisense strand of the siRNA are selected from the sequences of the sense strand and the antisense strand of any one of the duplexes provided in Table 4: GPSZRA222S1-VP, GPSZRA222S3-VP, GPSZRA222S4-VP, GPSZRA227S1-VP, GPSZRA267S2-VP, GPSZRA284S3-VP, GPSZRA314S1-VP, GPSZRA314S2-VP, GPSZRA314S3-VP, GPSZRA314S5-VP, GPSZRA318S2-VP, GPSZRA402S4-VP, GPSZRA402S5-VP.

### Delivery

The siRNAs of the present invention can be delivered or introduced by any means known in the art (e.g., delivered or introduced into a cell in vitro, or delivered or introduced into a patient in vivo). For example, for in vivo delivery, the siRNA can be injected into a tissue site, or administered systemically. The in vivo delivery can also be performed via the β-glucan delivery system. Methods for in vitro introduction into cells include those known in the art, such as electroporation and lipofection.

In some embodiments, the delivery method includes, but is not limited to, delivery via a virus (retrovirus, adenovirus, lentivirus, baculovirus, AAV); liposomes (Lipofectamine, cationic DOTAP, neutral DOPC); nanoparticles (cationic polymers, PEI); bacterial delivery (tkRNAi); chemical modification on the siRNA (LNAs) to increase stability; lipid nanoparticles (LNPs); neutral liposomes (NLs); polymeric nanoparticles (low molecular weight polymers or high molecular weight polymers); double-stranded RNA binding motifs (dsRBMs); and other delivery systems known in the art to be suitable for nucleic acid or oligonucleotide delivery.

### Conjugates

In one aspect, the present invention provides a conjugate comprising at least one siRNA of the present invention and a pharmaceutically acceptable targeting molecule. The conjugate of the present invention is obtained by conjugating the siRNA of the present invention with a pharmaceutically acceptable targeting molecule. The conjugate comprises a pharmaceutically acceptable targeting molecule and an optional linker. The siRNA may be non-covalently coupled to the targeting molecule, or covalently coupled to the targeting molecule.

The pharmaceutically acceptable targeting molecule can be a targeting molecule conventionally used in the field of siRNA administration, which typically enhances the pharmacokinetic or biodistribution properties of the siRNA linked thereto, and improves the cell-specific (or organ-specific) distribution and cell-specific (or organ-specific) uptake of the siRNA. Representative targeting molecules include, but are not limited to, compounds having affinity for cell surface molecules, cell receptor ligands, haptens, antibodies or antibody fragments, antibody mimetics, and the like. In some embodiments, the targeting molecules include but are not limited to one or more of the following targeting molecules or their derivatives: integrin family (integrins); lipophilic molecules, such as cholesterol, bile acid, vitamins (such as vitamin E), lipid molecules of different chain lengths; polymers, such as polyethylene glycol; polypeptides, such as membrane-permeable peptides; aptamers; antibodies; quantum dots; carbohydrates, such as lactose, polylactose, mannose, galactose, N-acetylgalactosamine (GalNAc); folate; or receptor ligands expressed by hepatic parenchymal cells, such as desialylated glycoproteins, desialylated sugar residues, lipoproteins (such as high-density lipoproteins, low-density lipoproteins, etc.), glucagon, neurotransmitters (such as adrenaline), growth factors, transferrin, etc.

The linker can be a linker conventionally used in the field of siRNA administration, including but not limited to one or more of the following linkers or their derivatives: amide linker moieties, amino linker moieties, carbonyl linker moieties, carbamate linker moieties, urea linker moieties, ether linker moieties, disulfide linker moieties, succinylamino linker moieties, etc.

In some embodiments, the targeting molecule can be linked directly or indirectly to the siRNA of the present invention via a linker/linking group. In some embodiments, the targeting molecule is linked to the siRNA via an unstable, cleavable or reversible bond or linker. In some embodiments, the targeting molecule is linked to at least one end of the sense strand and/or antisense strand of the siRNA. In some embodiments, the targeting molecule is linked to the 5' end and/or 3' end of the sense strand. In some embodiments, the targeting molecule is linked to the 5' end and/or 3' end of the antisense strand.

### Pharmaceutical composition

In one aspect, the present invention provides a pharmaceutical composition comprising at least one of the siRNAs of the present invention.

In some embodiments, the pharmaceutical composition comprises one siRNA described above.

In other embodiments, the pharmaceutical composition comprises at least 2 (for example, but not limited to, 2, 3, 4, 5, 6, 7, 8, 9, 10 or more) of the siRNAs described above as active ingredients. Preferably, the at least 2 siRNAs, each targeting different target sequences in the RAGE gene, can be expected to simultaneously act on different target sequences and produce a synergistic effect. Here, the expression "different target sequences" refers to target sequences that do not overlap, or target sequences that overlap by less than 5 consecutive nucleotides (e.g., the number of overlapping consecutive nucleotides is 4, 3, 2, 1, or 0). In this case, the at least 2 siRNAs described above may be present in any different ratio. Preferably, the at least 2 siRNAs described above may be present in a molar ratio of 1:100 to 100:1 relative to each other; more preferably, the at least 2 siRNAs described above may be present in a molar ratio of 1:10 to 10:1, 1:5 to 5:1 or 1:2 to 2:1 relative to each other. In some embodiments, the at least 2 siRNAs described above are present in the same molar ratio.

In other embodiments, the pharmaceutical composition comprises at least one siRNA of the present invention and further comprises at least one siRNA targeting another target (e.g., a gene other than RAGE). In this case, the siRNA of the present invention and the siRNA targeting another target can be present in any different ratio, for example, in a molar ratio of 1:100 to 100:1, for example, in a molar ratio of 1:10 to 10:1, 1:5 to 5:1 or 1:2 to 2:1, for example, in the same molar ratio.

In some embodiments, the pharmaceutical composition comprises an effective amount of the siRNA. The expression "effective amount" refers to an amount of the siRNA effective to produce a desired pharmacological, therapeutic or preventive result. For example, if a given clinical treatment is considered effective when there is at least a 10% reduction in a measurable parameter associated with a disease or condition, the therapeutically effective amount of the drug for treating that disease or condition is the amount required to achieve at least a 10% reduction of that parameter. For example, a therapeutically effective amount of an siRNA targeting RAGE can reduce the RAGE mRNA level by at least 10%.

In some embodiments, in the pharmaceutical composition described in any of the above embodiments, the siRNA can be linked to a targeting molecule to form a conjugate. Thus, in some embodiments, the pharmaceutical composition comprises the conjugate of the present invention.

In some embodiments, the pharmaceutical composition further comprises a pharmaceutically acceptable carrier and/or excipient.

In some embodiments, the pharmaceutically acceptable carrier and/or excipient is a delivery vector. The delivery vector is a substance that improves the delivery of a nucleic acid or oligonucleotide to a cell or tissue. Such substances may be any delivery vector known in the art to be suitable for nucleic acid or oligonucleotide delivery, including but not limited to: viruses (retroviruses, adenoviruses, lentiviruses, baculoviruses, AAV); liposomes (Lipofectamine, cationic DOTAP, neutral DOPC); nanoparticles (cationic polymers, PEI); bacteria (tkRNAi); lipid nanoparticles (LNPs); neutral liposomes (NLs); polymeric nanoparticles (low-molecular-weight polymers or high-molecular-weight polymers); double-stranded RNA-binding motifs (dsRBMs), etc.

In some embodiments, the siRNA can be encapsulated by the delivery vector.

In some embodiments, the siRNA can be linked to the delivery vector directly or indirectly via a linker/linking group. In some embodiments, the delivery vector is linked to the siRNA via an unstable, cleavable or reversible bond or linker. In some embodiments, the delivery vector is linked to at least one end of the sense strand and/or antisense strand of the siRNA. In some embodiments, the delivery vector is linked to the 5' end and/or 3' end of the sense strand. In some embodiments, the delivery vector is linked to the 5' end and/or 3' end of the antisense strand.

In some embodiments, the pharmaceutical composition comprises one siRNA provided by the present invention, and the siRNA is encapsulated by the delivery vector. The one siRNA can be encapsulated in the same delivery vector or individually in different delivery vectors.

In other embodiments, the pharmaceutical composition comprises at least 2 (for example, but not limited to, 2, 3, 4, 5, 6, 7, 8, 9, 10 or more) siRNAs provided by the present invention, wherein the siRNA is encapsulated by the delivery vector. The at least 2 siRNA can be encapsulated in the same delivery vector or individually in different delivery vectors. Preferably, the at least 2 siRNAs each target different target sequences in the RAGE gene.

In other embodiments, the pharmaceutical composition comprises at least one siRNA provided by the present invention as well as siRNAs targeting other targets (e.g., genes other than RAGE), wherein the siRNA is encapsulated by the delivery vector. The siRNAs provided by the present invention and those siRNAs targeting other targets (such as genes other than RAGE) can be encapsulated in the same delivery vector, or they can be individually encapsulated in different delivery vectors.

In some embodiments, the pharmaceutical composition of the present invention is formulated into a dosage form compatible with its intended route of administration, for example, it can be administered locally (such as direct injection or implantation), systemically, or subcutaneously, intravenously, intraperitoneally, or parenterally, including intracranial (such as intraventricular, intrameningeal, or intrathecal), intramuscularly, transdermally, or by airway (aerosol), nasal, oral, rectal, or topical (including buccal and sublingual) administration.

In some embodiments, the pharmaceutical composition is administered by inhalation, intranasal administration, intratracheal administration, or oropharyngeal inhalation. A formulation suitable for inhalation administration can be prepared by incorporating a required amount of the active ingredient in an appropriate solvent, followed by sterile filtration. Typically, the formulation for inhalation administration is a sterile solution at physiological pH and has low viscosity. A salt can be added to the formulation to balance the tonicity. In some cases, a surfactant or cosolvent can be added to increase the solubility of the active ingredient and improve aerosol properties. In some cases, an excipient can be added to control viscosity in order to ensure the size and distribution of the aerosolized droplets.

In other embodiments, the pharmaceutical composition can be administered by injection, for example, intravenously, intramuscularly, subcutaneously, intradermally, intraarticularly, intraocularly, intraperitoneally, or topically. The pharmaceutical compositions suitable for injectable use include sterile aqueous solutions or dispersions and sterile powders for the extemporaneous preparation of sterile injectable solutions or dispersions. For intravenous administration, suitable vectors include physiological saline, bacteriostatic water, or phosphate buffered saline (PBS) and the like. Pharmaceutical compositions should be stable under the conditions of manufacture and storage and should be protected against the contaminating action of microorganisms such as bacteria and fungi. For example, the sterile injection solutions can be prepared by incorporating a necessary dose of the active ingredient in an appropriate solvent, and optionally, incorporating other desired ingredients (including but not limited to pH adjusting agents, surfactants, adjuvants, ionic strength enhancers, diluents, osmotic pressure-maintaining reagents, absorption-delaying reagents, preservatives, or any combination thereof), followed by performing filtration sterilization. Additionally, the sterile injectable solutions can be prepared as sterile lyophilized powders (e.g., by vacuum drying or freeze drying) for ease of storage and use.

The siRNA of the present invention can be formulated in dosage unit form for ease of administration. A dosage unit form refers to a physically discrete unit suitable for use as a single dosage for a subject to be treated; each unit contains a predetermined quantity of the active ingredient calculated to produce the desired therapeutic effect in combination with the required pharmaceutical carrier.

In the present invention, dosage regimens may be adjusted to obtain the optimum desired response (e.g., a therapeutic or prophylactic response). For example, a single administration may be performed, multiple administrations may be administered over a period of time, or the dose may be proportionally reduced or increased as indicated by the exigencies of the therapeutic situation.

### Application

### Inhibition of the expression of RAGE

The siRNA of the present invention can be used to inhibit the expression of RAGE in vitro and/or in vivo. The RAGE mRNA refers to an mRNA having, for example, the sequence set forth in Genbank: NM_001136.5.

In one aspect, the present invention provides a method for inhibiting RAGE expression in a cell, comprising: introducing the siRNA, conjugate or pharmaceutical composition of the present invention into the cell. In some embodiments, the method is performed in vitro. The siRNA of the present invention can be introduced by any nucleic acid delivery means known in the art, such as electroporation or lipofection.

The term "inhibition of the expression of RAGE" refers to at least partial suppression of the RAGE gene expression, which can be manifested as a decrease in the level of detectable RAGE mRNA. For example, the degree of inhibition may be expressed as: (mRNA in control cells)-(mRNA in treated cells)/(mRNA in control cells)*100%. Alternatively, the degree of inhibition may be given as a reduction in a parameter functionally associated with RAGE gene expression, such as the level of protein encoded by the RAGE gene. In principle, RAGE gene silencing can be determined in any cell expressing RAGE (expressing constitutively or by genetic engineering) by any suitable assay. Measurements can be made at multiple time points, before, during, and after administration of the siRNA, to determine the effect of the siRNA. The level or expression of RAGE can be measured by evaluation of the mRNA (e.g., by Northern blot or PCR) or protein (e.g., Western blot). For example, the effect of the siRNA on RAGE expression can be determined by measuring the transcription rate of the RAGE gene (e.g., by RT-PCR). For example, the effect of siRNA on RAGE expression can be determined by measuring the expression level of a reporter gene (such as luciferase) fused to the RAGE gene.

In some embodiments, the expression of the RAGE gene is suppressed by at least about 5%, 10%, 15%, 20%, 25%, 30%, 35%, 40%, 45%, or 50% by administering the siRNA of the present invention. In some embodiments, the expression of the RAGE gene is suppressed by at least about 60%, 70%, or 80% by administering the siRNA of the present invention. In some embodiments, the expression of the RAGE gene is suppressed by at least about 85% by administering the siRNA of the present invention. In some embodiments, the expression of the RAGE gene is suppressed by at least about 90% by administering the siRNA of the present invention. In some embodiments, the expression of the RAGE gene is suppressed by at least about 95% by administering the siRNA of the present invention. In some embodiments, the expression of the RAGE gene is suppressed by at least about 96%, 97%, 98%, 99% or 100% by administering the siRNA of the present invention.

In some embodiments, the siRNA, the conjugate, or the pharmaceutical composition is used alone or in combination with an additional pharmaceutically active agent (e.g., an siRNA targeting a different target sequence in the RAGE gene or an siRNA targeting another target).

In some embodiments, one siRNA provided in the present invention is used. The siRNA is optionally encapsulated by a delivery vector. In some embodiments, the one siRNA is encapsulated in the same delivery vector. In some embodiments, the one siRNA is individually encapsulated in different delivery vectors.

In other embodiments, at least 2 siRNAs provided by the present invention (for example, but not limited to, 2, 3, 4, 5, 6, 7, 8, 9, 10 or more) are used, preferably each of the at least 2 siRNAs targets a different target sequence in the RAGE gene. The at least 2 siRNAs are optionally encapsulated by a delivery vector. In some embodiments, the at least 2 siRNAs are encapsulated in the same delivery vector. In some embodiments, the at least 2 siRNAs are individually encapsulated in different delivery vectors.

In other embodiments, at least one siRNAs provided by the present invention and an siRNA targeting another target (e.g., a gene other than RAGE) are used. The siRNAs provided by the present invention and the siRNA targeting another target (e.g., a gene other than RAGE) are optionally encapsulated by a delivery vector. In some embodiments, the siRNAs provided by the present invention and the siRNA targeting another target (e.g., a gene other than RAGE) are encapsulated in the same delivery vector. In other embodiments, the siRNAs provided by the present invention and the siRNA targeting another target (e.g., a gene other than RAGE) are individually encapsulated in different delivery vectors.

### Treatment of RAGE-related diseases

The siRNA of the present invention can be used for the treatment of diseases or conditions that would benefit from a reduction in RAGE levels or inhibition of its expression.

In one aspect, the present invention provides a method for preventing and/or treating an RAGE-related disease in a subject, comprising administering an effective amount of the siRNA, the conjugate, or the pharmaceutical composition of the present invention to a subject in need thereof. The present invention further relates to use of the siRNA or the pharmaceutical composition of the present invention in the manufacture of a medicament for treating and/or preventing an RAGE-related disease.

In some embodiments, the RAGE-related disease involves overexpression of RAGE. Overexpression of RAGE means that RAGE levels (e.g., RAGE levels present in plasma or tissues of a subject, preferably in damaged tissues) are higher than normal RAGE levels (e.g., corresponding levels in healthy controls).

In some embodiments, the RAGE-related disease will benefit from reduced levels or inhibition of RAGE expression.

In some embodiments, the RAGE-related disease is a respiratory disease, such as cystic fibrosis, pneumonia, chronic bronchitis, noncystic fibrotic bronchiectasis, chronic obstructive pulmonary disease (COPD), asthma, respiratory infection, primary ciliary dyskinesia, or lung carcinoma cystic fibrosis.

In some embodiments, the respiratory disease is chronic obstructive pulmonary disease (COPD).

In some embodiments, the RAGE-related disease is a neurological disease, such as Alzheimer's disease or stroke.

In some embodiments, the RAGE-related disease is a urological disease, such as diabetic nephropathy.

In some embodiments, if RAGE expression is elevated for a particular disease, treatment with the siRNA of the present invention may reduce the level or expression of RAGE to a level that is within the range considered normal for individuals without such a disorder.

In some embodiments, the RAGE-related disease involves inflammation, such as chronic inflammation. In some embodiments, the RAGE-related disease is an inflammatory disorder. In some embodiments, the RAGE-related disease will benefit from reduced levels or inhibition of inflammation. In some embodiments, the siRNA of the present invention inhibits or reduces the expression of RAGE and thus inhibits or reduces RAGE-mediated signaling pathways, thereby reducing or suppressing the inflammatory response.

In some embodiments, the RAGE-related disease is an inflammatory respiratory disease. In some embodiments, the RAGE-related disease is an inflammatory neurological disease. In some embodiments, the RAGE-related disease is an inflammatory urological disease. In some embodiments, the RAGE-related disease is a diabetes complication, such as diabetic nephropathy, arteriosclerosis, and retinopathy.

In some embodiments, the subject is a mammal, such as a human.

In some embodiments, the siRNA, the conjugate, or the pharmaceutical composition is used alone, or in combination with an additional pharmaceutically active agent (e.g., an siRNA targeting a different target sequence in the RAGE gene or an siRNA targeting another target), for example, administrated simultaneously or successively.

In some embodiments, one siRNA provided in the present invention is used. The siRNA is optionally encapsulated by a delivery vector. In some embodiments, the one siRNA is encapsulated in the same delivery vector. In some embodiments, the one siRNA is individually encapsulated in different delivery vectors.

In other embodiments, at least 2 siRNAs provided by the present invention (for example, but not limited to, 2, 3, 4, 5, 6, 7, 8, 9, 10 or more) are used, preferably each of the at least 2 siRNAs targets a different target sequence in the RAGE gene. The at least 2 siRNAs are optionally encapsulated by a delivery vector. In some embodiments, the at least 2 siRNAs are encapsulated in the same delivery vector. In some embodiments, the at least 2 siRNAs are individually encapsulated in different delivery vectors.

In other embodiments, at least one siRNAs provided by the present invention and an siRNA targeting another target (e.g., a gene other than RAGE) are used. The siRNAs provided by the present invention and the siRNA targeting another target (e.g., a gene other than RAGE) are optionally encapsulated by a delivery vector. In some embodiments, the siRNAs provided by the present invention and the siRNA targeting another target (e.g., a gene other than RAGE) are encapsulated in the same delivery vector. In other embodiments, the siRNAs provided by the present invention and the siRNA targeting another target (e.g., a gene other than RAGE) are individually encapsulated in different delivery vectors.

### Definitions of terms

In the present invention, unless otherwise specified, the scientific and technical terms used herein have the meanings commonly understood by those skilled in the art. Moreover, in order to better understand the present invention, definitions and explanations of relevant terms are provided below.

In the present disclosure, the mRNA sequence of the receptor for advanced glycation end products (RAGE) is well known to those skilled in the art; for example, reference is made to the mRNA sequence set forth in GenBank Accession No. NM_001136.5. Further, unless otherwise specified, the term "target gene" as used herein refers to the gene that transcribes the aforementioned RAGE mRNA; the term "target mRNA" refers to the aforementioned RAGE mRNA; and the term "inhibiting the RAGE gene" refers to the inhibition of RAGE expression.

In the present disclosure, the uppercase letters C, G, U, A, and T represent the base components of nucleotides, including modified or unmodified nucleotides; the lowercase letter m indicates that the single nucleotide immediately adjacent to the left of m is a methoxy-modified nucleotide; the lowercase letter f indicates that the single nucleotide immediately adjacent to the left of f is a fluoro-modified nucleotide; (LNA) indicates that the single nucleotide immediately adjacent to the left of (LNA) is a locked nucleic acid-modified nucleotide; the lowercase letter s indicates that a phosphorothioate linkage is formed between the two nucleotides immediately adjacent to the left and right of s; the uppercase letters VP indicate that the single nucleotide immediately adjacent to the right of VP is a 5'-(E)-vinylphosphonate-modified nucleotide.

As used herein, the term "modified nucleotide" refers to a nucleotide or nucleotide analog that independently has a modified ribose moiety, a modified internucleoside linkage, or a modified base. Thus, the term "modified nucleotide" encompasses substitutions, additions, or removals (e.g., using functional groups or atoms) of internucleoside linkages, ribose moieties, or bases. "Nucleotide analogs" refer to groups that replace nucleotides in nucleic acids but have a structure different from adenine ribonucleotide, guanine ribonucleotide, cytosine ribonucleotide, uracil ribonucleotide or thymine deoxyribonucleotide. The examples are isonucleotides, bridged nucleic acids (simply called BNA) or acyclic nucleotides. Modifications suitable for use in the present invention include all types of modifications disclosed herein or known in the art. As an example, the modified nucleotide may include a nucleotide or nucleotide analog formed by replacing the 2' hydroxyl group of the ribosome of the nucleotide with another group, or a nucleotide in which the bases on the nucleotide are modified bases, or a nucleotide modified with a 5'-phosphate analog.

The present disclosure provides the structures of the following exemplary modified nucleotides:
the structure of the fluoro-modified nucleotide is as follows: 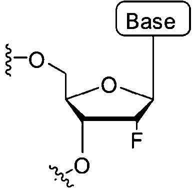
the structure of the methoxy-modified nucleotide is as follows: 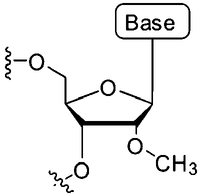
the structure of the locked nucleic acid-modified nucleotide is as follows: 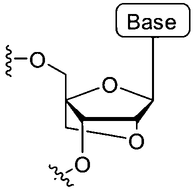
the structure of the 5'-(E)-vinylphosphonate-modified nucleotide is as follows:

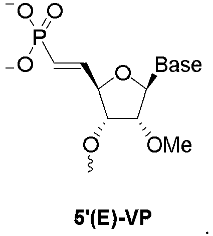

As used herein, the term "siRNA" refers to an RNA molecule that can sequence-specifically induce the RNAi phenomenon, is composed of a sense strand and an antisense strand, and has a partially or completely complementary double-stranded structure. In the siRNAs of the present invention, the length of the complementary double-stranded structure may range from 14 to 30 base pairs, e.g., 14, 15, 16, 17, 18, 19, 20 or 21 base pairs, such as 14 to 19 base pairs. In some embodiments of the present invention, the siRNA can also contain modified nucleotides as needed, and the modified nucleotides will not significantly weaken or lose the function of the siRNA in inhibiting the RAGE gene expression. Currently, there are many ways to modify siRNAs in the art, including, for example, backbone modification (such as phosphate group modification), ribose group modification, base modification and the like (Watts, J.K., G.F. Deleavey, and M.J. Damha, Chemically modified siRNA: tools and applications. Drug Discov Today, 2008. 13(19-20): p. 842-55).

The term "antisense strand" comprises a region that is substantially complementary to the target sequence. The term "sense strand" as used herein refers to the strand whose region is substantially complementary to the region of the antisense strand. The term "complementary region" refers to a region on the antisense strand that is substantially complementary to RAGE mRNA or a region on the sense strand that is substantially complementary to the antisense strand. When the complementary region is not completely complementary to the target sequence, the mismatch may be in internal or terminal regions of the molecule. Typically, the most tolerated mismatches are in the terminal regions, for example, within 5, 4, 3, 2, or 1 nucleotide of the 5' end and/or 3' end.

Herein, unless otherwise specified, the term "complementary" refers to the ability of an oligonucleotide of a first sequence to hybridize with an oligonucleotide of a second sequence under certain conditions and form a double-stranded structure. The expression "at least partially complementary" means that the two sequences can be completely complementary, or have no more than 6, 5, 4, 3, 2, or 1 mismatched base pairs overall, while retaining the ability to hybridize under relevant conditions. Additionally, where two oligonucleotides are designed to form one or more single-stranded overhangs upon hybridization, such overhangs should not be considered mismatches for the purpose of determining complementarity. Herein, the "complementary" sequences may also include or be formed entirely from non-Watson-Crick base pairs and/or base pairs formed from non-natural and modified nucleotides, as long as the above requirements of hybridization capability are met. Such non-Watson-Crick base pairs include, but are not limited to, G:U wobble base pairing or Hoogstein base pairing. Correspondingly, herein, unless otherwise specified, "mismatch" means that the bases at corresponding positions in the siRNA duplex molecule are not paired in a complementary form.

Those skilled in the art will be able to determine the most suitable conditions for testing the complementarity of two sequences depending on the ultimate application of the hybridized nucleotides. Such conditions can, for example, be stringent conditions, such as 400 mM NaCl, 40 mM PIPES pH 6.4, 1 mM EDTA, 50°C or 70°C for 12-16 h, followed by washing. Other conditions, such as physiologically relevant conditions that may be encountered in an organism, may also apply.

Herein, unless otherwise specified, "nucleotide sequence difference" means that the base type of the nucleotide at the same or corresponding position has changed compared to the original nucleotide sequence. For example, when one nucleotide base in the original nucleotide sequence is A, if the nucleotide base at the same or corresponding position is changed to U, C, G or dT, dC, dG, etc., it is considered that there is a difference in the nucleotide sequence at that position. It should be noted here that, when compared to the original nucleotide sequence, the nucleotides at the same or corresponding positions differ only in the presence or absence of modification or the type of modification, it is not considered that there is a difference in the nucleotide sequence at that position.

As used herein, unless otherwise specified, the term "pharmaceutically acceptable carrier and/or excipient" refers to a carrier, delivery vector, diluent, auxiliary material and/or formed salt/ester/hydrate thereof, which are generally chemically or physically compatible with other ingredients constituting a pharmaceutical dosage form (such as the siRNA of the present invention) and physiologically compatible with the subject. The "pharmaceutically acceptable carrier and/or excipient" does not exert, or is not intended to exert, a therapeutic effect at an intended dosage. Such ingredients may serve the following functions: a) assisting in the processing of the drug delivery system during manufacturing; b) protecting, supporting, or enhancing the stability, bioavailability, or patient acceptability of the active ingredient; c) aiding in product identification; and/or d) enhancing any other properties of the active ingredient during storage and use, such as overall safety, efficacy, and delivery. For example, the siRNA of the present invention can be encapsulated by a delivery vector. The "pharmaceutically acceptable carrier and/or excipient" includes, but is not limited to, viruses, liposomes, nanoparticles, bacteria, lipid nanoparticles (LNP), neutral liposomes (NL), polymeric nanoparticles, double-stranded RNA binding motifs (dsRBMs), pH adjusting agents, surfactants, adjuvants, ionic strength enhancers, diluents, osmotic pressure-maintaining reagents, absorption-delaying reagents, and preservatives. For example, viruses include, but are not limited to, retroviruses, adenoviruses, lentiviruses, baculoviruses, and AAV. Liposomes include, but are not limited to, Lipofectamine, cationic DOTAP, and neutral DOPC. Nanoparticles include, but are not limited to, cationic polymers, and PEI. Bacteria include, but are not limited to, tkRNAi. Polymeric nanoparticles include, but are not limited to, low molecular weight polymers or high molecular weight polymers. For example, pH adjusting agents include, but are not limited to, phosphate buffers. Surfactants include, but are not limited to, cationic, anionic or nonionic surfactants, such as Tween-80. Ionic strength enhancers include, but are not limited to, sodium chloride. Preservatives include, but are not limited to, various antibacterial reagents and antifungal reagents such as parabens, chlorobutanol, phenol, sorbic acid, etc. Reagents for maintaining osmotic pressure include, but are not limited to, sugars, NaCl, and analogs thereof. Reagents for delaying absorption include, but are not limited to, monostearates and gelatin. Diluents include, but are not limited to, water, aqueous buffers (e.g. buffered saline), alcohols, polyols (e.g. glycerol), etc.

Herein, unless otherwise specified, the term "inhibition" refers to the situation where the expression of the target gene is down-regulated due to siRNA-mediated degradation of the target gene mRNA. The "downregulation" refers to a decrease in target gene expression level by 5%, 10%, 15%, 20%, 25%, 30%, 35%, 40%, 45%, 50%, 55%, 60%, 65%, 70%, 75%, 80%, 85%, 90%, 95% or 99% or more, or even 100%, relative to the situation in the absence of siRNA treatment. The 100% decrease in target gene expression level means that there is no detectable level of the target gene expression.

Herein, the term "overhang" or "nucleotide overhang" refers to at least one unpaired nucleotide protruding from the siRNA duplex structure. For example, an overhang exists when the 3' end of one strand of an siRNA extends beyond the 5' end of the other strand (or vice versa). The siRNA can comprise an overhang of at least one nucleotide, or the overhang can comprise at least 2 nt, at least 3 nt, at least 4 nt, at least 5 nt, or more. The overhangs may comprise or be composed of nucleotides/nucleoside analogs, including deoxyribonucleotides/nucleosides. The overhang can be on the sense strand, the antisense strand, or any combination thereof. Nucleotides of the overhang may be present at the 5' end, 3' end, or both ends of the antisense or sense strand of the siRNA. Accordingly, the term "blunt end" refers to the absence of nucleotide overhangs.

As used herein, the term "prevention" refers to a method implemented for preventing or delaying the occurrence of a disease or disorder or symptom in a subject; and the term "treatment" refers to a method implemented for achieving beneficial or desired clinical outcomes. For purposes of the present invention, beneficial or desired clinical results include, but are not limited to, alleviation of symptoms, reduction in the extent of diseases, stabilization (i.e., no longer worsening) of disease states, delay or slowing of disease progression, amelioration or palliation of disease states, and relief of symptoms (regardless partial or complete), regardless detectable or undetectable. In addition, the "treatment" may also mean prolonging the survival time as compared to the expected survival time (in the absence of treatment).

Herein, the term "effective amount" refers to an amount sufficient to achieve or at least partially achieve desired effects. For example, an effective amount for preventing a disease refers to an amount sufficient to prevent, inhibit or delay the occurrence of the disease; and an effective amount for treating a disease refers to an amount sufficient to cure or at least partially inhibit the disease and complications thereof in a patient suffering from the disease. Determination of such effective amounts is completely within the capability of those skilled in the art. For example, amounts effective for therapeutic use will depend on the severity of the disease to be treated, the overall state of the immune system of the patient, the general conditions of the patient such as age, weight and sex, the manner of administration of a drug, other therapies administered simultaneously, and the like.

### Beneficial effects of the present invention

The siRNA of the present invention can effectively inhibit RAGE gene expression in vitro and/or in vivo, suppress or reduce RAGE-mediated signaling pathways, and has good stability. Thus, the siRNAs of the present invention can be used to inhibit inflammatory responses and treat diseases or conditions that benefit from the reduction or inhibition of RAGE expression, and therefore hold significant clinical value for the treatment of RAGE-related diseases and the like.

### BRIEF DESCRIPTION OF DRAWINGS

Fig. 1A to Fig. 1G: Gel electrophoresis results of RNase stability assay for modified siRNAs.
Fig. 2A to Fig. 2F: Gel electrophoresis results of serum stability assay for modified siRNAs.
Fig. 3A to Fig. 3G: Gel electrophoresis results of human liver S9 stability assay for modified siRNAs.
Fig. 4A to Fig. 4C: Gel electrophoresis results of RNase stability assay for VP-modified siRNAs.
Fig. 5A to Fig. 5C: Gel electrophoresis results of serum stability assay for VP-modified siRNAs.
Fig. 6A to Fig. 6C: Gel electrophoresis results of human liver S9 stability assay for VP-modified siRNAs.
Fig. 7A to Fig. 7I: Cytotoxicity of VP-modified siRNAs against A549 RAGE LV.
Fig. 8A to Fig. 8I: Cytotoxicity of VP-modified siRNAs against BEAS-2B RAGE LV.
Fig. 9A to Fig. 9K: Results of VP-modified siRNA stimulating human PBMCs to secrete cytokines (TNF-α, IFN-α, IL-1β, IL-2, IL-4, IL-5, IL-6, IL-10, IL-12P70, IL-17, IFN-γ).

The embodiments of the present invention will be described in detail below in conjunction with examples, but those skilled in the art will understand that the following examples are only used for illustrating the present invention rather than limiting the scope of the present invention. Various purposes and advantages of the present invention will become implementable to those skilled in the art according to the following detailed descriptions of the preferred embodiments.

### DETAILED DESCRIPTION OF PREFERRED EMBODIMENTS

The present invention will now be described in the following non-limiting examples.

Those skilled in the art will appreciate that the Examples describe the present invention by way of examples and are not intended to limit the scope of protection claimed for the present application. The experimental methods in the examples are all conventional methods, unless otherwise specified. Examples, in which no specific conditions are specified, are carried out according to conventional conditions or conditions recommended by the manufacturers. The reagents and instruments used without indicating the manufacturer are all conventional products that can be purchased commercially.

Those skilled in the art will appreciate that the siRNA described in the present invention can be obtained by conventional siRNA preparation methods in the art (such as solid-phase synthesis and liquid-phase synthesis), wherein commercial custom services are available for both solid-phase synthesis and liquid-phase synthesis. It is also clear to those skilled in the art that modified nucleotide groups can be introduced into the siRNA described in the present invention by using nucleotide monomers with corresponding modifications. Methods for preparing nucleotide monomers with corresponding modifications are well known to those skilled in the art, and commercial monomers are also available on the market.

### Preparation Example 1: Synthesis of unmodified siRNAs

This preparation example provides an siRNA for inhibiting RAGE expression, the nucleotide sequence of which is designed based on the target mRNA, as shown in Table 1.

**Table 1: siRNA sequences**

| Duplex number | SEQ ID NO: | sense seq (5'-3') | Range in NM_001136.5 | SEQ ID NO: | antisense seq (5'-3') |
|---|---|---|---|---|---|
| GPSZRA001 | 420 | UGGAAGGAAGCAGGAUGGC | (16, 36) | 1 | GCCAUCCUGCUUCCUUCCAGG |
| GPSZRA002 | 421 | GUGCUGGUCCUCAGUCUGU | (60, 80) | 2 | ACAGACUGAGGACCAGCACCC |
| GPSZRA003 | 422 | UGCUGGUCCUCAGUCUGUG | (61, 81) | 3 | CACAGACUGAGGACCAGCACC |
| GPSZRA004 | 423 | AGUAGUAGGUGCUCAAAAC | (86, 106) | 4 | GUUUUGAGCACCUACUACUGC |
| GPSZRA005 | 424 | GUAGUAGGUGCUCAAAACA | (87, 107) | 5 | UGUUUUGAGCACCUACUACUG |
| GPSZRA006 | 425 | UAGUAGGUGCUCAAAACAU | (88, 108) | 6 | AUGUUUUGAGCACCUACUACU |
| GPSZRA007 | 426 | AGUAGGUGCUCAAAACAUC | (89, 109) | 7 | GAUGUUUUGAGCACCUACUAC |
| GPSZRA008 | 427 | GUAGGUGCUCAAAACAUCA | (90, 110) | 8 | UGAUGUUUUGAGCACCUACUA |
| GPSZRA009 | 428 | UAGGUGCUCAAAACAUCAC | (91, 111) | 9 | GUGAUGUUUUGAGCACCUACU |
| GPSZRA010 | 429 | AGGUGCUCAAAACAUCACA | (92, 112) | 10 | UGUGAUGUUUUGAGCACCUAC |
| GPSZRA011 | 430 | GGUGCUCAAAACAUCACAG | (93, 113) | 11 | CUGUGAUGUUUUGAGCACCUA |
| GPSZRA012 | 431 | GUGCUCAAAACAUCACAGC | (94, 114) | 12 | GCUGUGAUGUUUUGAGCACCU |
| GPSZRA013 | 432 | UGCUCAAAACAUCACAGCC | (95, 115) | 13 | GGCUGUGAUGUUUUGAGCACC |
| GPSZRA014 | 433 | GCUCAAAACAUCACAGCCC | (96, 116) | 14 | GGGCUGUGAUGUUUUGAGCAC |
| GPSZRA015 | 434 | CUCAAAACAUCACAGCCCG | (97, 117) | 15 | CGGGCUGUGAUGUUUUGAGCA |
| GPSZRA016 | 435 | UCAAAACAUCACAGCCCGG | (98, 118) | 16 | CCGGGCUGUGAUGUUUUGAGC |
| GPSZRA017 | 436 | CAAAACAUCACAGCCCGGA | (99, 119) | 17 | UCCGGGCUGUGAUGUUUUGAG |
| GPSZRA018 | 437 | AAAACAUCACAGCCCGGAU | (100, 120) | 18 | AUCCGGGCUGUGAUGUUUUGA |
| GPSZRA019 | 438 | GCCACUGGUGCUGAAGUGU | (125, 145) | 19 | ACACUUCAGCACCAGUGGCUC |
| GPSZRA020 | 439 | CCACUGGUGCUGAAGUGUA | (126, 146) | 20 | UACACUUCAGCACCAGUGGCU |
| GPSZRA021 | 440 | CACUGGUGCUGAAGUGUAA | (127, 147) | 21 | UUACACUUCAGCACCAGUGGC |
| GPSZRA022 | 441 | ACUGGUGCUGAAGUGUAAG | (128, 148) | 22 | CUUACACUUCAGCACCAGUGG |
| GPSZRA023 | 442 | CUGGUGCUGAAGUGUAAGG | (129, 149) | 23 | CCUUACACUUCAGCACCAGUG |
| GPSZRA024 | 443 | UGGUGCUGAAGUGUAAGGG | (130, 150) | 24 | CCCUUACACUUCAGCACCAGU |
| GPSZRA025 | 444 | GGUGCUGAAGUGUAAGGGG | (131, 151) | 25 | CCCCUUACACUUCAGCACCAG |
| GPSZRA026 | 445 | GUGCUGAAGUGUAAGGGGG | (132, 152) | 26 | CCCCCUUACACUUCAGCACCA |
| GPSZRA027 | 446 | UGCUGAAGUGUAAGGGGGC | (133, 153) | 27 | GCCCCCUUACACUUCAGCACC |
| GPSZRA028 | 447 | UAAGGGGGCCCCCAAGAAA | (143, 163) | 28 | UUUCUUGGGGGCCCCCUUACA |
| GPSZRA029 | 448 | GCCGGACAGAAGCUUGGAA | (196, 216) | 29 | UUCCAAGCUUCUGUCCGGCCU |
| GPSZRA030 | 449 | CCGGACAGAAGCUUGGAAG | (197, 217) | 30 | CUUCCAAGCUUCUGUCCGGCC |
| GPSZRA031 | 450 | CGGACAGAAGCUUGGAAGG | (198, 218) | 31 | CCUUCCAAGCUUCUGUCCGGC |
| GPSZRA032 | 451 | GGACAGAAGCUUGGAAGGU | (199, 219) | 32 | ACCUUCCAAGCUUCUGUCCGG |
| GPSZRA033 | 452 | GACAGAAGCUUGGAAGGUC | (200, 220) | 33 | GACCUUCCAAGCUUCUGUCCG |
| GPSZRA034 | 453 | ACAGAAGCUUGGAAGGUCC | (201, 221) | 34 | GGACCUUCCAAGCUUCUGUCC |
| GPSZRA035 | 454 | CAGAAGCUUGGAAGGUCCU | (202, 222) | 35 | AGGACCUUCCAAGCUUCUGUC |
| GPSZRA036 | 455 | UGGCUCGUGUCCUUCCCAA | (253, 273) | 36 | UUGGGAAGGACACGAGCCACA |
| GPSZRA037 | 456 | UUCCCAACGGCUCCCUCUU | (265, 285) | 37 | AAGAGGGAGCCGUUGGGAAGG |
| GPSZRA038 | 457 | CAACGGCUCCCUCUUCCUU | (269, 289) | 38 | AAGGAAGAGGGAGCCGUUGGG |
| GPSZRA039 | 458 | AACGGCUCCCUCUUCCUUC | (270, 290) | 39 | GAAGGAAGAGGGAGCCGUUGG |
| GPSZRA040 | 459 | CUGUCGGGAUCCAGGAUGA | (292, 312) | 40 | UCAUCCUGGAUCCCGACAGCC |
| GPSZRA041 | 460 | UGUCGGGAUCCAGGAUGAG | (293, 313) | 41 | CUCAUCCUGGAUCCCGACAGC |
| GPSZRA042 | 461 | GGAUCCAGGAUGAGGGGAU | (298, 318) | 42 | AUCCCCUCAUCCUGGAUCCCG |
| GPSZRA043 | 462 | GAUCCAGGAUGAGGGGAUU | (299, 319) | 43 | AAUCCCCUCAUCCUGGAUCCC |
| GPSZRA044 | 463 | AUCCAGGAUGAGGGGAUUU | (300, 320) | 44 | AAAUCCCCUCAUCCUGGAUCC |
| GPSZRA045 | 464 | UCCAGGAUGAGGGGAUUUU | (301, 321) | 45 | AAAAUCCCCUCAUCCUGGAUC |
| GPSZRA046 | 465 | CCAGGAUGAGGGGAUUUUC | (302, 322) | 46 | GAAAAUCCCCUCAUCCUGGAU |
| GPSZRA047 | 466 | CAGGAUGAGGGGAUUUUCC | (303, 323) | 47 | GGAAAAUCCCCUCAUCCUGGA |
| GPSZRA048 | 467 | AGGAUGAGGGGAUUUUCCG | (304, 324) | 48 | CGGAAAAUCCCCUCAUCCUGG |
| GPSZRA049 | 468 | GGAUGAGGGGAUUUUCCGG | (305, 325) | 49 | CCGGAAAAUCCCCUCAUCCUG |
| GPSZRA050 | 469 | GAUGAGGGGAUUUUCCGGU | (306, 326) | 50 | ACCGGAAAAUCCCCUCAUCCU |
| GPSZRA051 | 470 | AUGAGGGGAUUUUCCGGUG | (307, 327) | 51 | CACCGGAAAAUCCCCUCAUCC |
| GPSZRA052 | 471 | UGAGGGGAUUUUCCGGUGC | (308, 328) | 52 | GCACCGGAAAAUCCCCUCAUC |
| GPSZRA053 | 472 | AGGGGAUUUUCCGGUGCCA | (310, 330) | 53 | UGGCACCGGAAAAUCCCCUCA |
| GPSZRA054 | 473 | GAUUUUCCGGUGCCAGGCA | (314, 334) | 54 | UGCCUGGCACCGGAAAAUCCC |
| GPSZRA055 | 474 | AUUUUCCGGUGCCAGGCAA | (315, 335) | 55 | UUGCCUGGCACCGGAAAAUCC |
| GPSZRA056 | 475 | UUUUCCGGUGCCAGGCAAU | (316, 336) | 56 | AUUGCCUGGCACCGGAAAAUC |
| GPSZRA057 | 476 | UUUCCGGUGCCAGGCAAUG | (317, 337) | 57 | CAUUGCCUGGCACCGGAAAAU |
| GPSZRA058 | 477 | UUCCGGUGCCAGGCAAUGA | (318, 338) | 58 | UCAUUGCCUGGCACCGGAAAA |
| GPSZRA059 | 478 | UCCGGUGCCAGGCAAUGAA | (319, 339) | 59 | UUCAUUGCCUGGCACCGGAAA |
| GPSZRA060 | 479 | CGGUGCCAGGCAAUGAACA | (321, 341) | 60 | UGUUCAUUGCCUGGCACCGGA |
| GPSZRA061 | 480 | GGUGCCAGGCAAUGAACAG | (322, 342) | 61 | CUGUUCAUUGCCUGGCACCGG |
| GPSZRA062 | 481 | GUGCCAGGCAAUGAACAGG | (323, 343) | 62 | CCUGUUCAUUGCCUGGCACCG |
| GPSZRA063 | 482 | UGCCAGGCAAUGAACAGGA | (324, 344) | 63 | UCCUGUUCAUUGCCUGGCACC |
| GPSZRA064 | 483 | GCCAGGCAAUGAACAGGAA | (325, 345) | 64 | UUCCUGUUCAUUGCCUGGCAC |
| GPSZRA065 | 484 | CCAGGCAAUGAACAGGAAU | (326, 346) | 65 | AUUCCUGUUCAUUGCCUGGCA |
| GPSZRA066 | 485 | CAGGCAAUGAACAGGAAUG | (327, 347) | 66 | CAUUCCUGUUCAUUGCCUGGC |
| GPSZRA067 | 486 | AGGCAAUGAACAGGAAUGG | (328, 348) | 67 | CCAUUCCUGUUCAUUGCCUGG |
| GPSZRA068 | 487 | GGCAAUGAACAGGAAUGGA | (329, 349) | 68 | UCCAUUCCUGUUCAUUGCCUG |
| GPSZRA069 | 488 | GCAAUGAACAGGAAUGGAA | (330, 350) | 69 | UUCCAUUCCUGUUCAUUGCCU |
| GPSZRA070 | 489 | CAAUGAACAGGAAUGGAAA | (331, 351) | 70 | UUUCCAUUCCUGUUCAUUGCC |
| GPSZRA071 | 490 | AAUGAACAGGAAUGGAAAG | (332, 352) | 71 | CUUUCCAUUCCUGUUCAUUGC |
| GPSZRA072 | 491 | AUGAACAGGAAUGGAAAGG | (333, 353) | 72 | CCUUUCCAUUCCUGUUCAUUG |
| GPSZRA073 | 492 | UGAACAGGAAUGGAAAGGA | (334, 354) | 73 | UCCUUUCCAUUCCUGUUCAUU |
| GPSZRA074 | 493 | GAACAGGAAUGGAAAGGAG | (335, 355) | 74 | CUCCUUUCCAUUCCUGUUCAU |
| GPSZRA075 | 494 | AACAGGAAUGGAAAGGAGA | (336, 356) | 75 | UCUCCUUUCCAUUCCUGUUCA |
| GPSZRA076 | 495 | ACAGGAAUGGAAAGGAGAC | (337, 357) | 76 | GUCUCCUUUCCAUUCCUGUUC |
| GPSZRA077 | 496 | CAGGAAUGGAAAGGAGACC | (338, 358) | 77 | GGUCUCCUUUCCAUUCCUGUU |
| GPSZRA078 | 497 | AGGAAUGGAAAGGAGACCA | (339, 359) | 78 | UGGUCUCCUUUCCAUUCCUGU |
| GPSZRA079 | 498 | GGAAUGGAAAGGAGACCAA | (340, 360) | 79 | UUGGUCUCCUUUCCAUUCCUG |
| GPSZRA080 | 499 | GAAUGGAAAGGAGACCAAG | (341, 361) | 80 | CUUGGUCUCCUUUCCAUUCCU |
| GPSZRA081 | 500 | AAUGGAAAGGAGACCAAGU | (342, 362) | 81 | ACUUGGUCUCCUUUCCAUUCC |
| GPSZRA082 | 501 | AUGGAAAGGAGACCAAGUC | (343, 363) | 82 | GACUUGGUCUCCUUUCCAUUC |
| GPSZRA083 | 502 | UGGAAAGGAGACCAAGUCC | (344, 364) | 83 | GGACUUGGUCUCCUUUCCAUU |
| GPSZRA084 | 503 | GGAAAGGAGACCAAGUCCA | (345, 365) | 84 | UGGACUUGGUCUCCUUUCCAU |
| GPSZRA085 | 504 | GAAAGGAGACCAAGUCCAA | (346, 366) | 85 | UUGGACUUGGUCUCCUUUCCA |
| GPSZRA086 | 505 | AAAGGAGACCAAGUCCAAC | (347, 367) | 86 | GUUGGACUUGGUCUCCUUUCC |
| GPSZRA087 | 506 | AAGGAGACCAAGUCCAACU | (348, 368) | 87 | AGUUGGACUUGGUCUCCUUUC |
| GPSZRA088 | 507 | AGGAGACCAAGUCCAACUA | (349, 369) | 88 | UAGUUGGACUUGGUCUCCUUU |
| GPSZRA089 | 508 | GGAGACCAAGUCCAACUAC | (350, 370) | 89 | GUAGUUGGACUUGGUCUCCUU |
| GPSZRA090 | 509 | GAGACCAAGUCCAACUACC | (351, 371) | 90 | GGUAGUUGGACUUGGUCUCCU |
| GPSZRA091 | 510 | AGACCAAGUCCAACUACCG | (352, 372) | 91 | CGGUAGUUGGACUUGGUCUCC |
| GPSZRA092 | 511 | GACCAAGUCCAACUACCGA | (353, 373) | 92 | UCGGUAGUUGGACUUGGUCUC |
| GPSZRA093 | 512 | ACCAAGUCCAACUACCGAG | (354, 374) | 93 | CUCGGUAGUUGGACUUGGUCU |
| GPSZRA094 | 513 | CCAAGUCCAACUACCGAGU | (355, 375) | 94 | ACUCGGUAGUUGGACUUGGUC |
| GPSZRA095 | 514 | CAAGUCCAACUACCGAGUC | (356, 376) | 95 | GACUCGGUAGUUGGACUUGGU |
| GPSZRA096 | 515 | AAGUCCAACUACCGAGUCC | (357, 377) | 96 | GGACUCGGUAGUUGGACUUGG |
| GPSZRA097 | 516 | AGUCCAACUACCGAGUCCG | (358, 378) | 97 | CGGACUCGGUAGUUGGACUUG |
| GPSZRA098 | 517 | GUCCAACUACCGAGUCCGU | (359, 379) | 98 | ACGGACUCGGUAGUUGGACUU |
| GPSZRA099 | 518 | UCCAACUACCGAGUCCGUG | (360, 380) | 99 | CACGGACUCGGUAGUUGGACU |
| GPSZRA100 | 519 | CCAACUACCGAGUCCGUGU | (361, 381) | 100 | ACACGGACUCGGUAGUUGGAC |
| GPSZRA101 | 520 | CAACUACCGAGUCCGUGUC | (362, 382) | 101 | GACACGGACUCGGUAGUUGGA |
| GPSZRA102 | 521 | AACUACCGAGUCCGUGUCU | (363, 383) | 102 | AGACACGGACUCGGUAGUUGG |
| GPSZRA103 | 522 | ACUACCGAGUCCGUGUCUA | (364, 384) | 103 | UAGACACGGACUCGGUAGUUG |
| GPSZRA104 | 523 | CUACCGAGUCCGUGUCUAC | (365, 385) | 104 | GUAGACACGGACUCGGUAGUU |
| GPSZRA105 | 524 | UACCGAGUCCGUGUCUACC | (366, 386) | 105 | GGUAGACACGGACUCGGUAGU |
| GPSZRA106 | 525 | ACCGAGUCCGUGUCUACCA | (367, 387) | 106 | UGGUAGACACGGACUCGGUAG |
| GPSZRA107 | 526 | CGAGUCCGUGUCUACCAGA | (369, 389) | 107 | UCUGGUAGACACGGACUCGGU |
| GPSZRA108 | 527 | GAGUCCGUGUCUACCAGAU | (370, 390) | 108 | AUCUGGUAGACACGGACUCGG |
| GPSZRA109 | 528 | AGUCCGUGUCUACCAGAUU | (371, 391) | 109 | AAUCUGGUAGACACGGACUCG |
| GPSZRA110 | 529 | GUCCGUGUCUACCAGAUUC | (372, 392) | 110 | GAAUCUGGUAGACACGGACUC |
| GPSZRA111 | 530 | UCCGUGUCUACCAGAUUCC | (373, 393) | 111 | GGAAUCUGGUAGACACGGACU |
| GPSZRA112 | 531 | CCGUGUCUACCAGAUUCCU | (374, 394) | 112 | AGGAAUCUGGUAGACACGGAC |
| GPSZRA113 | 532 | CGUGUCUACCAGAUUCCUG | (375, 395) | 113 | CAGGAAUCUGGUAGACACGGA |
| GPSZRA114 | 533 | GUGUCUACCAGAUUCCUGG | (376, 396) | 114 | CCAGGAAUCUGGUAGACACGG |
| GPSZRA115 | 534 | UGUCUACCAGAUUCCUGGG | (377, 397) | 115 | CCCAGGAAUCUGGUAGACACG |
| GPSZRA116 | 535 | GUCUACCAGAUUCCUGGGA | (378, 398) | 116 | UCCCAGGAAUCUGGUAGACAC |
| GPSZRA117 | 536 | UCUACCAGAUUCCUGGGAA | (379, 399) | 117 | UUCCCAGGAAUCUGGUAGACA |
| GPSZRA118 | 537 | CUACCAGAUUCCUGGGAAG | (380, 400) | 118 | CUUCCCAGGAAUCUGGUAGAC |
| GPSZRA119 | 538 | UACCAGAUUCCUGGGAAGC | (381, 401) | 119 | GCUUCCCAGGAAUCUGGUAGA |
| GPSZRA120 | 539 | ACCAGAUUCCUGGGAAGCC | (382, 402) | 120 | GGCUUCCCAGGAAUCUGGUAG |
| GPSZRA121 | 540 | GAUUCUGCCUCUGAACUCA | (411, 431) | 121 | UGAGUUCAGAGGCAGAAUCUA |
| GPSZRA122 | 541 | AUUCUGCCUCUGAACUCAC | (412, 432) | 122 | GUGAGUUCAGAGGCAGAAUCU |
| GPSZRA123 | 542 | UUCUGCCUCUGAACUCACG | (413, 433) | 123 | CGUGAGUUCAGAGGCAGAAUC |
| GPSZRA124 | 543 | UCUGCCUCUGAACUCACGG | (414, 434) | 124 | CCGUGAGUUCAGAGGCAGAAU |
| GPSZRA125 | 544 | UGCCUCUGAACUCACGGCU | (416, 436) | 125 | AGCCGUGAGUUCAGAGGCAGA |
| GPSZRA126 | 545 | CUCUGAACUCACGGCUGGU | (419, 439) | 126 | ACCAGCCGUGAGUUCAGAGGC |
| GPSZRA127 | 546 | UCUGAACUCACGGCUGGUG | (420, 440) | 127 | CACCAGCCGUGAGUUCAGAGG |
| GPSZRA128 | 547 | CUGAACUCACGGCUGGUGU | (421, 441) | 128 | ACACCAGCCGUGAGUUCAGAG |
| GPSZRA129 | 548 | UGAACUCACGGCUGGUGUU | (422, 442) | 129 | AACACCAGCCGUGAGUUCAGA |
| GPSZRA130 | 549 | GAACUCACGGCUGGUGUUC | (423, 443) | 130 | GAACACCAGCCGUGAGUUCAG |
| GPSZRA131 | 550 | AACUCACGGCUGGUGUUCC | (424, 444) | 131 | GGAACACCAGCCGUGAGUUCA |
| GPSZRA132 | 551 | UCACGGCUGGUGUUCCCAA | (427, 447) | 132 | UUGGGAACACCAGCCGUGAGU |
| GPSZRA133 | 552 | CACGGCUGGUGUUCCCAAU | (428, 448) | 133 | AUUGGGAACACCAGCCGUGAG |
| GPSZRA134 | 553 | ACGGCUGGUGUUCCCAAUA | (429, 449) | 134 | UAUUGGGAACACCAGCCGUGA |
| GPSZRA135 | 554 | CGGCUGGUGUUCCCAAUAA | (430, 450) | 135 | UUAUUGGGAACACCAGCCGUG |
| GPSZRA136 | 555 | GGCUGGUGUUCCCAAUAAG | (431, 451) | 136 | CUUAUUGGGAACACCAGCCGU |
| GPSZRA137 | 556 | GCUGGUGUUCCCAAUAAGG | (432, 452) | 137 | CCUUAUUGGGAACACCAGCCG |
| GPSZRA138 | 557 | CUGGUGUUCCCAAUAAGGU | (433, 453) | 138 | ACCUUAUUGGGAACACCAGCC |
| GPSZRA139 | 558 | UGGUGUUCCCAAUAAGGUG | (434, 454) | 139 | CACCUUAUUGGGAACACCAGC |
| GPSZRA140 | 559 | GGUGUUCCCAAUAAGGUGG | (435, 455) | 140 | CCACCUUAUUGGGAACACCAG |
| GPSZRA141 | 560 | GUGUUCCCAAUAAGGUGGG | (436, 456) | 141 | CCCACCUUAUUGGGAACACCA |
| GPSZRA142 | 561 | UGUUCCCAAUAAGGUGGGG | (437, 457) | 142 | CCCCACCUUAUUGGGAACACC |
| GPSZRA143 | 562 | GUUCCCAAUAAGGUGGGGA | (438, 458) | 143 | UCCCCACCUUAUUGGGAACAC |
| GPSZRA144 | 563 | UUCCCAAUAAGGUGGGGAC | (439, 459) | 144 | GUCCCCACCUUAUUGGGAACA |
| GPSZRA145 | 564 | UCCCAAUAAGGUGGGGACA | (440, 460) | 145 | UGUCCCCACCUUAUUGGGAAC |
| GPSZRA146 | 565 | CCCAAUAAGGUGGGGACAU | (441, 461) | 146 | AUGUCCCCACCUUAUUGGGAA |
| GPSZRA147 | 566 | CCAAUAAGGUGGGGACAUG | (442, 462) | 147 | CAUGUCCCCACCUUAUUGGGA |
| GPSZRA148 | 567 | CAAUAAGGUGGGGACAUGU | (443, 463) | 148 | ACAUGUCCCCACCUUAUUGGG |
| GPSZRA149 | 568 | AAUAAGGUGGGGACAUGUG | (444, 464) | 149 | CACAUGUCCCCACCUUAUUGG |
| GPSZRA150 | 569 | AUAAGGUGGGGACAUGUGU | (445, 465) | 150 | ACACAUGUCCCCACCUUAUUG |
| GPSZRA151 | 570 | UAAGGUGGGGACAUGUGUG | (446, 466) | 151 | CACACAUGUCCCCACCUUAUU |
| GPSZRA152 | 571 | AAGGUGGGGACAUGUGUGU | (447, 467) | 152 | ACACACAUGUCCCCACCUUAU |
| GPSZRA153 | 572 | AGGUGGGGACAUGUGUGUC | (448, 468) | 153 | GACACACAUGUCCCCACCUUA |
| GPSZRA154 | 573 | GGUGGGGACAUGUGUGUCA | (449, 469) | 154 | UGACACACAUGUCCCCACCUU |
| GPSZRA155 | 574 | GUGGGGACAUGUGUGUCAG | (450, 470) | 155 | CUGACACACAUGUCCCCACCU |
| GPSZRA156 | 575 | UGGGGACAUGUGUGUCAGA | (451, 471) | 156 | UCUGACACACAUGUCCCCACC |
| GPSZRA157 | 576 | GGGGACAUGUGUGUCAGAG | (452, 472) | 157 | CUCUGACACACAUGUCCCCAC |
| GPSZRA158 | 577 | GGGACAUGUGUGUCAGAGG | (453, 473) | 158 | CCUCUGACACACAUGUCCCCA |
| GPSZRA159 | 578 | GGACAUGUGUGUCAGAGGG | (454, 474) | 159 | CCCUCUGACACACAUGUCCCC |
| GPSZRA160 | 579 | GACAUGUGUGUCAGAGGGA | (455, 475) | 160 | UCCCUCUGACACACAUGUCCC |
| GPSZRA161 | 580 | ACAUGUGUGUCAGAGGGAA | (456, 476) | 161 | UUCCCUCUGACACACAUGUCC |
| GPSZRA162 | 581 | CAUGUGUGUCAGAGGGAAG | (457, 477) | 162 | CUUCCCUCUGACACACAUGUC |
| GPSZRA163 | 582 | AUGUGUGUCAGAGGGAAGC | (458, 478) | 163 | GCUUCCCUCUGACACACAUGU |
| GPSZRA164 | 583 | UGUGUGUCAGAGGGAAGCU | (459, 479) | 164 | AGCUUCCCUCUGACACACAUG |
| GPSZRA165 | 584 | GUGUGUCAGAGGGAAGCUA | (460, 480) | 165 | UAGCUUCCCUCUGACACACAU |
| GPSZRA166 | 585 | UGUGUCAGAGGGAAGCUAC | (461, 481) | 166 | GUAGCUUCCCUCUGACACACA |
| GPSZRA167 | 586 | GUGUCAGAGGGAAGCUACC | (462, 482) | 167 | GGUAGCUUCCCUCUGACACAC |
| GPSZRA168 | 587 | UGUCAGAGGGAAGCUACCC | (463, 483) | 168 | GGGUAGCUUCCCUCUGACACA |
| GPSZRA169 | 588 | GUCAGAGGGAAGCUACCCU | (464, 484) | 169 | AGGGUAGCUUCCCUCUGACAC |
| GPSZRA170 | 589 | UCAGAGGGAAGCUACCCUG | (465, 485) | 170 | CAGGGUAGCUUCCCUCUGACA |
| GPSZRA171 | 590 | AGAGGGAAGCUACCCUGCA | (467, 487) | 171 | UGCAGGGUAGCUUCCCUCUGA |
| GPSZRA172 | 591 | AAGCUACCCUGCAGGGACU | (473, 493) | 172 | AGUCCCUGCAGGGUAGCUUCC |
| GPSZRA173 | 592 | CUACCCUGCAGGGACUCUU | (476, 496) | 173 | AAGAGUCCCUGCAGGGUAGCU |
| GPSZRA174 | 593 | UACCCUGCAGGGACUCUUA | (477, 497) | 174 | UAAGAGUCCCUGCAGGGUAGC |
| GPSZRA175 | 594 | ACCCUGCAGGGACUCUUAG | (478, 498) | 175 | CUAAGAGUCCCUGCAGGGUAG |
| GPSZRA176 | 595 | CCUGCAGGGACUCUUAGCU | (480, 500) | 176 | AGCUAAGAGUCCCUGCAGGGU |
| GPSZRA177 | 596 | CUGCAGGGACUCUUAGCUG | (481, 501) | 177 | CAGCUAAGAGUCCCUGCAGGG |
| GPSZRA178 | 597 | UGCAGGGACUCUUAGCUGG | (482, 502) | 178 | CCAGCUAAGAGUCCCUGCAGG |
| GPSZRA179 | 598 | CAGGGACUCUUAGCUGGCA | (484, 504) | 179 | UGCCAGCUAAGAGUCCCUGCA |
| GPSZRA180 | 599 | AGGGACUCUUAGCUGGCAC | (485, 505) | 180 | GUGCCAGCUAAGAGUCCCUGC |
| GPSZRA181 | 600 | GGGACUCUUAGCUGGCACU | (486, 506) | 181 | AGUGCCAGCUAAGAGUCCCUG |
| GPSZRA182 | 601 | GGACUCUUAGCUGGCACUU | (487, 507) | 182 | AAGUGCCAGCUAAGAGUCCCU |
| GPSZRA183 | 602 | GACUCUUAGCUGGCACUUG | (488, 508) | 183 | CAAGUGCCAGCUAAGAGUCCC |
| GPSZRA184 | 603 | ACUCUUAGCUGGCACUUGG | (489, 509) | 184 | CCAAGUGCCAGCUAAGAGUCC |
| GPSZRA185 | 604 | CUCUUAGCUGGCACUUGGA | (490, 510) | 185 | UCCAAGUGCCAGCUAAGAGUC |
| GPSZRA186 | 605 | UCUUAGCUGGCACUUGGAU | (491, 511) | 186 | AUCCAAGUGCCAGCUAAGAGU |
| GPSZRA187 | 606 | CUUAGCUGGCACUUGGAUG | (492, 512) | 187 | CAUCCAAGUGCCAGCUAAGAG |
| GPSZRA188 | 607 | UUAGCUGGCACUUGGAUGG | (493, 513) | 188 | CCAUCCAAGUGCCAGCUAAGA |
| GPSZRA189 | 608 | UAGCUGGCACUUGGAUGGG | (494, 514) | 189 | CCCAUCCAAGUGCCAGCUAAG |
| GPSZRA190 | 609 | AGCUGGCACUUGGAUGGGA | (495, 515) | 190 | UCCCAUCCAAGUGCCAGCUAA |
| GPSZRA191 | 610 | GCUGGCACUUGGAUGGGAA | (496, 516) | 191 | UUCCCAUCCAAGUGCCAGCUA |
| GPSZRA192 | 611 | CUGGCACUUGGAUGGGAAG | (497, 517) | 192 | CUUCCCAUCCAAGUGCCAGCU |
| GPSZRA193 | 612 | UGGCACUUGGAUGGGAAGC | (498, 518) | 193 | GCUUCCCAUCCAAGUGCCAGC |
| GPSZRA194 | 613 | ACUUGGAUGGGAAGCCCCU | (502, 522) | 194 | AGGGGCUUCCCAUCCAAGUGC |
| GPSZRA195 | 614 | GAAGCCCCUGGUGCCUAAU | (512, 532) | 195 | AUUAGGCACCAGGGGCUUCCC |
| GPSZRA196 | 615 | AAGCCCCUGGUGCCUAAUG | (513, 533) | 196 | CAUUAGGCACCAGGGGCUUCC |
| GPSZRA197 | 616 | AGCCCCUGGUGCCUAAUGA | (514, 534) | 197 | UCAUUAGGCACCAGGGGCUUC |
| GPSZRA198 | 617 | CCCCUGGUGCCUAAUGAGA | (516, 536) | 198 | UCUCAUUAGGCACCAGGGGCU |
| GPSZRA199 | 618 | CCCUGGUGCCUAAUGAGAA | (517, 537) | 199 | UUCUCAUUAGGCACCAGGGGC |
| GPSZRA200 | 619 | CCUGGUGCCUAAUGAGAAG | (518, 538) | 200 | CUUCUCAUUAGGCACCAGGGG |
| GPSZRA201 | 620 | CUGGUGCCUAAUGAGAAGG | (519, 539) | 201 | CCUUCUCAUUAGGCACCAGGG |
| GPSZRA202 | 621 | UGGUGCCUAAUGAGAAGGG | (520, 540) | 202 | CCCUUCUCAUUAGGCACCAGG |
| GPSZRA203 | 622 | GGUGCCUAAUGAGAAGGGA | (521, 541) | 203 | UCCCUUCUCAUUAGGCACCAG |
| GPSZRA204 | 623 | GUGCCUAAUGAGAAGGGAG | (522, 542) | 204 | CUCCCUUCUCAUUAGGCACCA |
| GPSZRA205 | 624 | UGCCUAAUGAGAAGGGAGU | (523, 543) | 205 | ACUCCCUUCUCAUUAGGCACC |
| GPSZRA206 | 625 | GCCUAAUGAGAAGGGAGUA | (524, 544) | 206 | UACUCCCUUCUCAUUAGGCAC |
| GPSZRA207 | 626 | CCUAAUGAGAAGGGAGUAU | (525, 545) | 207 | AUACUCCCUUCUCAUUAGGCA |
| GPSZRA208 | 627 | CUAAUGAGAAGGGAGUAUC | (526, 546) | 208 | GAUACUCCCUUCUCAUUAGGC |
| GPSZRA209 | 628 | UAAUGAGAAGGGAGUAUCU | (527, 547) | 209 | AGAUACUCCCUUCUCAUUAGG |
| GPSZRA210 | 629 | AAUGAGAAGGGAGUAUCUG | (528, 548) | 210 | CAGAUACUCCCUUCUCAUUAG |
| GPSZRA211 | 630 | AUGAGAAGGGAGUAUCUGU | (529, 549) | 211 | ACAGAUACUCCCUUCUCAUUA |
| GPSZRA212 | 631 | UGAGAAGGGAGUAUCUGUG | (530, 550) | 212 | CACAGAUACUCCCUUCUCAUU |
| GPSZRA213 | 632 | GAGAAGGGAGUAUCUGUGA | (531, 551) | 213 | UCACAGAUACUCCCUUCUCAU |
| GPSZRA214 | 633 | AGAAGGGAGUAUCUGUGAA | (532, 552) | 214 | UUCACAGAUACUCCCUUCUCA |
| GPSZRA215 | 634 | GAAGGGAGUAUCUGUGAAG | (533, 553) | 215 | CUUCACAGAUACUCCCUUCUC |
| GPSZRA216 | 635 | AAGGGAGUAUCUGUGAAGG | (534, 554) | 216 | CCUUCACAGAUACUCCCUUCU |
| GPSZRA217 | 636 | AGGGAGUAUCUGUGAAGGA | (535, 555) | 217 | UCCUUCACAGAUACUCCCUUC |
| GPSZRA218 | 637 | GGGAGUAUCUGUGAAGGAA | (536, 556) | 218 | UUCCUUCACAGAUACUCCCUU |
| GPSZRA219 | 638 | ACCAGGAGACACCCUGAGA | (558, 578) | 219 | UCUCAGGGUGUCUCCUGGUCU |
| GPSZRA220 | 639 | GCUCUUCACACUGCAGUCG | (581, 601) | 220 | CGACUGCAGUGUGAAGAGCCC |
| GPSZRA221 | 640 | CUCUUCACACUGCAGUCGG | (582, 602) | 221 | CCGACUGCAGUGUGAAGAGCC |
| GPSZRA222 | 641 | UCUUCACACUGCAGUCGGA | (583, 603) | 222 | UCCGACUGCAGUGUGAAGAGC |
| GPSZRA223 | 642 | CUUCACACUGCAGUCGGAG | (584, 604) | 223 | CUCCGACUGCAGUGUGAAGAG |
| GPSZRA224 | 643 | UUCACACUGCAGUCGGAGC | (585, 605) | 224 | GCUCCGACUGCAGUGUGAAGA |
| GPSZRA225 | 644 | UCACACUGCAGUCGGAGCU | (586, 606) | 225 | AGCUCCGACUGCAGUGUGAAG |
| GPSZRA226 | 645 | CACACUGCAGUCGGAGCUA | (587, 607) | 226 | UAGCUCCGACUGCAGUGUGAA |
| GPSZRA227 | 646 | ACACUGCAGUCGGAGCUAA | (588, 608) | 227 | UUAGCUCCGACUGCAGUGUGA |
| GPSZRA228 | 647 | CACUGCAGUCGGAGCUAAU | (589, 609) | 228 | AUUAGCUCCGACUGCAGUGUG |
| GPSZRA229 | 648 | ACUGCAGUCGGAGCUAAUG | (590, 610) | 229 | CAUUAGCUCCGACUGCAGUGU |
| GPSZRA230 | 649 | CUGCAGUCGGAGCUAAUGG | (591, 611) | 230 | CCAUUAGCUCCGACUGCAGUG |
| GPSZRA231 | 650 | UGCAGUCGGAGCUAAUGGU | (592, 612) | 231 | ACCAUUAGCUCCGACUGCAGU |
| GPSZRA232 | 651 | GCAGUCGGAGCUAAUGGUG | (593, 613) | 232 | CACCAUUAGCUCCGACUGCAG |
| GPSZRA233 | 652 | CAGUCGGAGCUAAUGGUGA | (594, 614) | 233 | UCACCAUUAGCUCCGACUGCA |
| GPSZRA234 | 653 | AGUCGGAGCUAAUGGUGAC | (595, 615) | 234 | GUCACCAUUAGCUCCGACUGC |
| GPSZRA235 | 654 | GUCGGAGCUAAUGGUGACC | (596, 616) | 235 | GGUCACCAUUAGCUCCGACUG |
| GPSZRA236 | 655 | UCGGAGCUAAUGGUGACCC | (597, 617) | 236 | GGGUCACCAUUAGCUCCGACU |
| GPSZRA237 | 656 | GGAGCUAAUGGUGACCCCA | (599, 619) | 237 | UGGGGUCACCAUUAGCUCCGA |
| GPSZRA238 | 657 | GAGCUAAUGGUGACCCCAG | (600, 620) | 238 | CUGGGGUCACCAUUAGCUCCG |
| GPSZRA239 | 658 | AGCUAAUGGUGACCCCAGC | (601, 621) | 239 | GCUGGGGUCACCAUUAGCUCC |
| GPSZRA240 | 659 | CCACCUUCUCCUGUAGCUU | (640, 660) | 240 | AAGCUACAGGAGAAGGUGGGA |
| GPSZRA241 | 660 | CACCUUCUCCUGUAGCUUC | (641, 661) | 241 | GAAGCUACAGGAGAAGGUGGG |
| GPSZRA242 | 661 | ACCUUCUCCUGUAGCUUCA | (642, 662) | 242 | UGAAGCUACAGGAGAAGGUGG |
| GPSZRA243 | 662 | CCUUCUCCUGUAGCUUCAG | (643, 663) | 243 | CUGAAGCUACAGGAGAAGGUG |
| GPSZRA244 | 663 | CUUCUCCUGUAGCUUCAGC | (644, 664) | 244 | GCUGAAGCUACAGGAGAAGGU |
| GPSZRA245 | 664 | UUCUCCUGUAGCUUCAGCC | (645, 665) | 245 | GGCUGAAGCUACAGGAGAAGG |
| GPSZRA246 | 665 | UCUCCUGUAGCUUCAGCCC | (646, 666) | 246 | GGGCUGAAGCUACAGGAGAAG |
| GPSZRA247 | 666 | CUCCUGUAGCUUCAGCCCA | (647, 667) | 247 | UGGGCUGAAGCUACAGGAGAA |
| GPSZRA248 | 667 | UCCUGUAGCUUCAGCCCAG | (648, 668) | 248 | CUGGGCUGAAGCUACAGGAGA |
| GPSZRA249 | 668 | UAGCUUCAGCCCAGGCCUU | (653, 673) | 249 | AAGGCCUGGGCUGAAGCUACA |
| GPSZRA250 | 669 | CCUCUGGAGGAGGUCCAAU | (729, 749) | 250 | AUUGGACCUCCUCCAGAGGCA |
| GPSZRA251 | 670 | CUCUGGAGGAGGUCCAAUU | (730, 750) | 251 | AAUUGGACCUCCUCCAGAGGC |
| GPSZRA252 | 671 | UCUGGAGGAGGUCCAAUUG | (731, 751) | 252 | CAAUUGGACCUCCUCCAGAGG |
| GPSZRA253 | 672 | CUGGAGGAGGUCCAAUUGG | (732, 752) | 253 | CCAAUUGGACCUCCUCCAGAG |
| GPSZRA254 | 673 | UGGAGGAGGUCCAAUUGGU | (733, 753) | 254 | ACCAAUUGGACCUCCUCCAGA |
| GPSZRA255 | 674 | GGAGGAGGUCCAAUUGGUG | (734, 754) | 255 | CACCAAUUGGACCUCCUCCAG |
| GPSZRA256 | 675 | GAGGAGGUCCAAUUGGUGG | (735, 755) | 256 | CCACCAAUUGGACCUCCUCCA |
| GPSZRA257 | 676 | AGGAGGUCCAAUUGGUGGU | (736, 756) | 257 | ACCACCAAUUGGACCUCCUCC |
| GPSZRA258 | 677 | GCCAGAAGGUGGAGCAGUA | (758, 778) | 258 | UACUGCUCCACCUUCUGGCUC |
| GPSZRA259 | 678 | CCAGAAGGUGGAGCAGUAG | (759, 779) | 259 | CUACUGCUCCACCUUCUGGCU |
| GPSZRA260 | 679 | CAGAAGGUGGAGCAGUAGC | (760, 780) | 260 | GCUACUGCUCCACCUUCUGGC |
| GPSZRA261 | 680 | AGAAGGUGGAGCAGUAGCU | (761, 781) | 261 | AGCUACUGCUCCACCUUCUGG |
| GPSZRA262 | 681 | GAAGGUGGAGCAGUAGCUC | (762, 782) | 262 | GAGCUACUGCUCCACCUUCUG |
| GPSZRA263 | 682 | AAGGUGGAGCAGUAGCUCC | (763, 783) | 263 | GGAGCUACUGCUCCACCUUCU |
| GPSZRA264 | 683 | AGGUGGAGCAGUAGCUCCU | (764, 784) | 264 | AGGAGCUACUGCUCCACCUUC |
| GPSZRA265 | 684 | UGGAGCAGUAGCUCCUGGU | (767, 787) | 265 | ACCAGGAGCUACUGCUCCACC |
| GPSZRA266 | 685 | AGCAGUAGCUCCUGGUGGA | (770, 790) | 266 | UCCACCAGGAGCUACUGCUCC |
| GPSZRA267 | 686 | GCAGUAGCUCCUGGUGGAA | (771, 791) | 267 | UUCCACCAGGAGCUACUGCUC |
| GPSZRA268 | 687 | CAGUAGCUCCUGGUGGAAC | (772, 792) | 268 | GUUCCACCAGGAGCUACUGCU |
| GPSZRA269 | 688 | AGUAGCUCCUGGUGGAACC | (773, 793) | 269 | GGUUCCACCAGGAGCUACUGC |
| GPSZRA270 | 689 | UAGCUCCUGGUGGAACCGU | (775, 795) | 270 | ACGGUUCCACCAGGAGCUACU |
| GPSZRA271 | 690 | AGCUCCUGGUGGAACCGUA | (776, 796) | 271 | UACGGUUCCACCAGGAGCUAC |
| GPSZRA272 | 691 | GCUCCUGGUGGAACCGUAA | (777, 797) | 272 | UUACGGUUCCACCAGGAGCUA |
| GPSZRA273 | 692 | CUCCUGGUGGAACCGUAAC | (778, 798) | 273 | GUUACGGUUCCACCAGGAGCU |
| GPSZRA274 | 693 | UCCUGGUGGAACCGUAACC | (779, 799) | 274 | GGUUACGGUUCCACCAGGAGC |
| GPSZRA275 | 694 | CUGGUGGAACCGUAACCCU | (781, 801) | 275 | AGGGUUACGGUUCCACCAGGA |
| GPSZRA276 | 695 | UGGUGGAACCGUAACCCUG | (782, 802) | 276 | CAGGGUUACGGUUCCACCAGG |
| GPSZRA277 | 696 | GGUGGAACCGUAACCCUGA | (783, 803) | 277 | UCAGGGUUACGGUUCCACCAG |
| GPSZRA278 | 697 | GUGGAACCGUAACCCUGAC | (784, 804) | 278 | GUCAGGGUUACGGUUCCACCA |
| GPSZRA279 | 698 | UGGAACCGUAACCCUGACC | (785, 805) | 279 | GGUCAGGGUUACGGUUCCACC |
| GPSZRA280 | 699 | GGAACCGUAACCCUGACCU | (786, 806) | 280 | AGGUCAGGGUUACGGUUCCAC |
| GPSZRA281 | 700 | GAACCGUAACCCUGACCUG | (787, 807) | 281 | CAGGUCAGGGUUACGGUUCCA |
| GPSZRA282 | 701 | AACCGUAACCCUGACCUGU | (788, 808) | 282 | ACAGGUCAGGGUUACGGUUCC |
| GPSZRA283 | 702 | ACCGUAACCCUGACCUGUG | (789, 809) | 283 | CACAGGUCAGGGUUACGGUUC |
| GPSZRA284 | 703 | CCGUAACCCUGACCUGUGA | (790, 810) | 284 | UCACAGGUCAGGGUUACGGUU |
| GPSZRA285 | 704 | CGUAACCCUGACCUGUGAA | (791, 811) | 285 | UUCACAGGUCAGGGUUACGGU |
| GPSZRA286 | 705 | GUAACCCUGACCUGUGAAG | (792, 812) | 286 | CUUCACAGGUCAGGGUUACGG |
| GPSZRA287 | 706 | UAACCCUGACCUGUGAAGU | (793, 813) | 287 | ACUUCACAGGUCAGGGUUACG |
| GPSZRA288 | 707 | AACCCUGACCUGUGAAGUC | (794, 814) | 288 | GACUUCACAGGUCAGGGUUAC |
| GPSZRA289 | 708 | ACCCUGACCUGUGAAGUCC | (795, 815) | 289 | GGACUUCACAGGUCAGGGUUA |
| GPSZRA290 | 709 | CCUGACCUGUGAAGUCCCU | (797, 817) | 290 | AGGGACUUCACAGGUCAGGGU |
| GPSZRA291 | 710 | CUGACCUGUGAAGUCCCUG | (798, 818) | 291 | CAGGGACUUCACAGGUCAGGG |
| GPSZRA292 | 711 | UGACCUGUGAAGUCCCUGC | (799, 819) | 292 | GCAGGGACUUCACAGGUCAGG |
| GPSZRA293 | 712 | CCCAGCCCUCUCCUCAAAU | (817, 837) | 293 | AUUUGAGGAGAGGGCUGGGCA |
| GPSZRA294 | 713 | CCAGCCCUCUCCUCAAAUC | (818, 838) | 294 | GAUUUGAGGAGAGGGCUGGGC |
| GPSZRA295 | 714 | CAGCCCUCUCCUCAAAUCC | (819, 839) | 295 | GGAUUUGAGGAGAGGGCUGGG |
| GPSZRA296 | 715 | AGCCCUCUCCUCAAAUCCA | (820, 840) | 296 | UGGAUUUGAGGAGAGGGCUGG |
| GPSZRA297 | 716 | GCCCUCUCCUCAAAUCCAC | (821, 841) | 297 | GUGGAUUUGAGGAGAGGGCUG |
| GPSZRA298 | 717 | CCCUCUCCUCAAAUCCACU | (822, 842) | 298 | AGUGGAUUUGAGGAGAGGGCU |
| GPSZRA299 | 718 | CCUCUCCUCAAAUCCACUG | (823, 843) | 299 | CAGUGGAUUUGAGGAGAGGGC |
| GPSZRA300 | 719 | CUCUCCUCAAAUCCACUGG | (824, 844) | 300 | CCAGUGGAUUUGAGGAGAGGG |
| GPSZRA301 | 720 | UCUCCUCAAAUCCACUGGA | (825, 845) | 301 | UCCAGUGGAUUUGAGGAGAGG |
| GPSZRA302 | 721 | CUCCUCAAAUCCACUGGAU | (826, 846) | 302 | AUCCAGUGGAUUUGAGGAGAG |
| GPSZRA303 | 722 | UCCUCAAAUCCACUGGAUG | (827, 847) | 303 | CAUCCAGUGGAUUUGAGGAGA |
| GPSZRA304 | 723 | CCUCAAAUCCACUGGAUGA | (828, 848) | 304 | UCAUCCAGUGGAUUUGAGGAG |
| GPSZRA305 | 724 | CUCAAAUCCACUGGAUGAA | (829, 849) | 305 | UUCAUCCAGUGGAUUUGAGGA |
| GPSZRA306 | 725 | UCAAAUCCACUGGAUGAAG | (830, 850) | 306 | CUUCAUCCAGUGGAUUUGAGG |
| GPSZRA307 | 726 | CAAAUCCACUGGAUGAAGG | (831, 851) | 307 | CCUUCAUCCAGUGGAUUUGAG |
| GPSZRA308 | 727 | AAAUCCACUGGAUGAAGGA | (832, 852) | 308 | UCCUUCAUCCAGUGGAUUUGA |
| GPSZRA309 | 728 | AAUCCACUGGAUGAAGGAU | (833, 853) | 309 | AUCCUUCAUCCAGUGGAUUUG |
| GPSZRA310 | 729 | AUCCACUGGAUGAAGGAUG | (834, 854) | 310 | CAUCCUUCAUCCAGUGGAUUU |
| GPSZRA311 | 730 | UCCACUGGAUGAAGGAUGG | (835, 855) | 311 | CCAUCCUUCAUCCAGUGGAUU |
| GPSZRA312 | 731 | CCACUGGAUGAAGGAUGGU | (836, 856) | 312 | ACCAUCCUUCAUCCAGUGGAU |
| GPSZRA313 | 732 | CACUGGAUGAAGGAUGGUG | (837, 857) | 313 | CACCAUCCUUCAUCCAGUGGA |
| GPSZRA314 | 733 | ACUGGAUGAAGGAUGGUGU | (838, 858) | 314 | ACACCAUCCUUCAUCCAGUGG |
| GPSZRA315 | 734 | CUGGAUGAAGGAUGGUGUG | (839, 859) | 315 | CACACCAUCCUUCAUCCAGUG |
| GPSZRA316 | 735 | UGGAUGAAGGAUGGUGUGC | (840, 860) | 316 | GCACACCAUCCUUCAUCCAGU |
| GPSZRA317 | 736 | GGAUGAAGGAUGGUGUGCC | (841, 861) | 317 | GGCACACCAUCCUUCAUCCAG |
| GPSZRA318 | 737 | GAUGAAGGAUGGUGUGCCC | (842, 862) | 318 | GGGCACACCAUCCUUCAUCCA |
| GPSZRA319 | 738 | AUGAAGGAUGGUGUGCCCU | (843, 863) | 319 | AGGGCACACCAUCCUUCAUCC |
| GPSZRA320 | 739 | UGAAGGAUGGUGUGCCCUU | (844, 864) | 320 | AAGGGCACACCAUCCUUCAUC |
| GPSZRA321 | 740 | CUGUGCUGAUCCUCCCUGA | (880, 900) | 321 | UCAGGGAGGAUCAGCACAGGG |
| GPSZRA322 | 741 | UGUGCUGAUCCUCCCUGAG | (881, 901) | 322 | CUCAGGGAGGAUCAGCACAGG |
| GPSZRA323 | 742 | GUGCUGAUCCUCCCUGAGA | (882, 902) | 323 | UCUCAGGGAGGAUCAGCACAG |
| GPSZRA324 | 743 | UGCUGAUCCUCCCUGAGAU | (883, 903) | 324 | AUCUCAGGGAGGAUCAGCACA |
| GPSZRA325 | 744 | GCUGAUCCUCCCUGAGAUA | (884, 904) | 325 | UAUCUCAGGGAGGAUCAGCAC |
| GPSZRA326 | 745 | CUGAUCCUCCCUGAGAUAG | (885, 905) | 326 | CUAUCUCAGGGAGGAUCAGCA |
| GPSZRA327 | 746 | UGAUCCUCCCUGAGAUAGG | (886, 906) | 327 | CCUAUCUCAGGGAGGAUCAGC |
| GPSZRA328 | 747 | GAUCCUCCCUGAGAUAGGG | (887, 907) | 328 | CCCUAUCUCAGGGAGGAUCAG |
| GPSZRA329 | 748 | AUCCUCCCUGAGAUAGGGC | (888, 908) | 329 | GCCCUAUCUCAGGGAGGAUCA |
| GPSZRA330 | 749 | AGGACCAGGGAACCUACAG | (910, 930) | 330 | CUGUAGGUUCCCUGGUCCUGA |
| GPSZRA331 | 750 | CAGGAAAGCCGUGCUGUCA | (960, 980) | 331 | UGACAGCACGGCUUUCCUGGG |
| GPSZRA332 | 751 | AGGAAAGCCGUGCUGUCAG | (961, 981) | 332 | CUGACAGCACGGCUUUCCUGG |
| GPSZRA333 | 752 | GAAAGCCGUGCUGUCAGCA | (963, 983) | 333 | UGCUGACAGCACGGCUUUCCU |
| GPSZRA334 | 753 | AAAGCCGUGCUGUCAGCAU | (964, 984) | 334 | AUGCUGACAGCACGGCUUUCC |
| GPSZRA335 | 754 | AAGCCGUGCUGUCAGCAUC | (965, 985) | 335 | GAUGCUGACAGCACGGCUUUC |
| GPSZRA336 | 755 | AGCCGUGCUGUCAGCAUCA | (966, 986) | 336 | UGAUGCUGACAGCACGGCUUU |
| GPSZRA337 | 756 | CGUGCUGUCAGCAUCAGCA | (969, 989) | 337 | UGCUGAUGCUGACAGCACGGC |
| GPSZRA338 | 757 | GUGCUGUCAGCAUCAGCAU | (970, 990) | 338 | AUGCUGAUGCUGACAGCACGG |
| GPSZRA339 | 758 | UGCUGUCAGCAUCAGCAUC | (971, 991) | 339 | GAUGCUGAUGCUGACAGCACG |
| GPSZRA340 | 759 | GCUGUCAGCAUCAGCAUCA | (972, 992) | 340 | UGAUGCUGAUGCUGACAGCAC |
| GPSZRA341 | 760 | CUGUCAGCAUCAGCAUCAU | (973, 993) | 341 | AUGAUGCUGAUGCUGACAGCA |
| GPSZRA342 | 761 | UGUCAGCAUCAGCAUCAUC | (974, 994) | 342 | GAUGAUGCUGAUGCUGACAGC |
| GPSZRA343 | 762 | GUCAGCAUCAGCAUCAUCG | (975, 995) | 343 | CGAUGAUGCUGAUGCUGACAG |
| GPSZRA344 | 763 | UCAGCAUCAGCAUCAUCGA | (976, 996) | 344 | UCGAUGAUGCUGAUGCUGACA |
| GPSZRA345 | 764 | CAGCAUCAGCAUCAUCGAA | (977, 997) | 345 | UUCGAUGAUGCUGAUGCUGAC |
| GPSZRA346 | 765 | AGCAUCAGCAUCAUCGAAC | (978, 998) | 346 | GUUCGAUGAUGCUGAUGCUGA |
| GPSZRA347 | 766 | GCAUCAGCAUCAUCGAACC | (979, 999) | 347 | GGUUCGAUGAUGCUGAUGCUG |
| GPSZRA348 | 767 | CAUCAGCAUCAUCGAACCA | (980, 1000) | 348 | UGGUUCGAUGAUGCUGAUGCU |
| GPSZRA349 | 768 | AUCAGCAUCAUCGAACCAG | (981, 1001) | 349 | CUGGUUCGAUGAUGCUGAUGC |
| GPSZRA350 | 769 | UCAGCAUCAUCGAACCAGG | (982, 1002) | 350 | CCUGGUUCGAUGAUGCUGAUG |
| GPSZRA351 | 770 | CAGCAUCAUCGAACCAGGC | (983, 1003) | 351 | GCCUGGUUCGAUGAUGCUGAU |
| GPSZRA352 | 771 | AGCAUCAUCGAACCAGGCG | (984, 1004) | 352 | CGCCUGGUUCGAUGAUGCUGA |
| GPSZRA353 | 772 | GCAUCAUCGAACCAGGCGA | (985, 1005) | 353 | UCGCCUGGUUCGAUGAUGCUG |
| GPSZRA354 | 773 | CAUCAUCGAACCAGGCGAG | (986, 1006) | 354 | CUCGCCUGGUUCGAUGAUGCU |
| GPSZRA355 | 774 | AUCAUCGAACCAGGCGAGG | (987, 1007) | 355 | CCUCGCCUGGUUCGAUGAUGC |
| GPSZRA356 | 775 | UCAUCGAACCAGGCGAGGA | (988, 1008) | 356 | UCCUCGCCUGGUUCGAUGAUG |
| GPSZRA357 | 776 | AACUGCAGGCUCUGUGGGA | (1013, 1033) | 357 | UCCCACAGAGCCUGCAGUUGG |
| GPSZRA358 | 777 | AGGCUCUGUGGGAGGAUCA | (1019, 1039) | 358 | UGAUCCUCCCACAGAGCCUGC |
| GPSZRA359 | 778 | GCUCAUUGGGGUCAUCUUG | (1097, 1117) | 359 | CAAGAUGACCCCAAUGAGCAG |
| GPSZRA360 | 779 | CUCAUUGGGGUCAUCUUGU | (1098, 1118) | 360 | ACAAGAUGACCCCAAUGAGCA |
| GPSZRA361 | 780 | UCAUUGGGGUCAUCUUGUG | (1099, 1119) | 361 | CACAAGAUGACCCCAAUGAGC |
| GPSZRA362 | 781 | CAUUGGGGUCAUCUUGUGG | (1100, 1120) | 362 | CCACAAGAUGACCCCAAUGAG |
| GPSZRA363 | 782 | AUUGGGGUCAUCUUGUGGC | (1101, 1121) | 363 | GCCACAAGAUGACCCCAAUGA |
| GPSZRA364 | 783 | UUGGGGUCAUCUUGUGGCA | (1102, 1122) | 364 | UGCCACAAGAUGACCCCAAUG |
| GPSZRA365 | 784 | UGGGGUCAUCUUGUGGCAA | (1103, 1123) | 365 | UUGCCACAAGAUGACCCCAAU |
| GPSZRA366 | 785 | GGGGUCAUCUUGUGGCAAA | (1104, 1124) | 366 | UUUGCCACAAGAUGACCCCAA |
| GPSZRA367 | 786 | GGGUCAUCUUGUGGCAAAG | (1105, 1125) | 367 | CUUUGCCACAAGAUGACCCCA |
| GPSZRA368 | 787 | GGUCAUCUUGUGGCAAAGG | (1106, 1126) | 368 | CCUUUGCCACAAGAUGACCCC |
| GPSZRA369 | 788 | GUCAUCUUGUGGCAAAGGC | (1107, 1127) | 369 | GCCUUUGCCACAAGAUGACCC |
| GPSZRA370 | 789 | UCAUCUUGUGGCAAAGGCG | (1108, 1128) | 370 | CGCCUUUGCCACAAGAUGACC |
| GPSZRA371 | 790 | CAUCUUGUGGCAAAGGCGG | (1109, 1129) | 371 | CCGCCUUUGCCACAAGAUGAC |
| GPSZRA372 | 791 | AUCUUGUGGCAAAGGCGGC | (1110, 1130) | 372 | GCCGCCUUUGCCACAAGAUGA |
| GPSZRA373 | 792 | GAAAACCAGGAGGAAGAGG | (1158, 1178) | 373 | CCTCTTCCTCCTGGTTTTCTG |
| GPSZRA374 | 793 | AAAACCAGGAGGAAGAGGA | (1159, 1179) | 374 | TCCTCTTCCTCCTGGTTTTCT |
| GPSZRA375 | 794 | AAACCAGGAGGAAGAGGAG | (1160, 1180) | 375 | CTCCTCTTCCTCCTGGTTTTC |
| GPSZRA376 | 795 | AACCAGGAGGAAGAGGAGG | (1161, 1181) | 376 | CCTCCTCTTCCTCCTGGTTTT |
| GPSZRA377 | 796 | ACCAGGAGGAAGAGGAGGA | (1162, 1182) | 377 | TCCTCCTCTTCCTCCTGGTTT |
| GPSZRA378 | 797 | AAGAGGAGGAGCGUGCAGA | (1171, 1191) | 378 | UCUGCACGCUCCUCCUCUUCC |
| GPSZRA379 | 798 | GAAUCAGUCGGAGGAACCU | (1193, 1213) | 379 | AGGUUCCUCCGACUGAUUCAG |
| GPSZRA380 | 799 | AAUCAGUCGGAGGAACCUG | (1194, 1214) | 380 | CAGGUUCCUCCGACUGAUUCA |
| GPSZRA381 | 800 | AUCAGUCGGAGGAACCUGA | (1195, 1215) | 381 | UCAGGUUCCUCCGACUGAUUC |
| GPSZRA382 | 801 | UCAGUCGGAGGAACCUGAG | (1196, 1216) | 382 | CUCAGGUUCCUCCGACUGAUU |
| GPSZRA383 | 802 | GCUCCCUUUUCUUUUUCCC | (1273, 1293) | 383 | GGGAAAAAGAAAAGGGAGCUG |
| GPSZRA384 | 803 | CUCCCUUUUCUUUUUCCCU | (1274, 1294) | 384 | AGGGAAAAAGAAAAGGGAGCU |
| GPSZRA385 | 804 | UCCCUUUUCUUUUUCCCUU | (1275, 1295) | 385 | AAGGGAAAAAGAAAAGGGAGC |
| GPSZRA386 | 805 | CCCUUUUCUUUUUCCCUUG | (1276, 1296) | 386 | CAAGGGAAAAAGAAAAGGGAG |
| GPSZRA387 | 806 | CCUUUUCUUUUUCCCUUGA | (1277, 1297) | 387 | UCAAGGGAAAAAGAAAAGGGA |
| GPSZRA388 | 807 | CUUUUCUUUUUCCCUUGAA | (1278, 1298) | 388 | UUCAAGGGAAAAAGAAAAGGG |
| GPSZRA389 | 808 | UUUUCUUUUUCCCUUGAAC | (1279, 1299) | 389 | GUUCAAGGGAAAAAGAAAAGG |
| GPSZRA390 | 809 | UUUCUUUUUCCCUUGAACU | (1280, 1300) | 390 | AGUUCAAGGGAAAAAGAAAAG |
| GPSZRA391 | 810 | UUCUUUUUCCCUUGAACUG | (1281, 1301) | 391 | CAGUUCAAGGGAAAAAGAAAA |
| GPSZRA392 | 811 | UCUUUUUCCCUUGAACUGU | (1282, 1302) | 392 | ACAGUUCAAGGGAAAAAGAAA |
| GPSZRA393 | 812 | CUUUUUCCCUUGAACUGUU | (1283, 1303) | 393 | AACAGUUCAAGGGAAAAAGAA |
| GPSZRA394 | 813 | UUUUUCCCUUGAACUGUUC | (1284, 1304) | 394 | GAACAGUUCAAGGGAAAAAGA |
| GPSZRA395 | 814 | UUUUCCCUUGAACUGUUCU | (1285, 1305) | 395 | AGAACAGUUCAAGGGAAAAAG |
| GPSZRA396 | 815 | UUUCCCUUGAACUGUUCUG | (1286, 1306) | 396 | CAGAACAGUUCAAGGGAAAAA |
| GPSZRA397 | 816 | UUCCCUUGAACUGUUCUGG | (1287, 1307) | 397 | CCAGAACAGUUCAAGGGAAAA |
| GPSZRA398 | 817 | UCCCUUGAACUGUUCUGGC | (1288, 1308) | 398 | GCCAGAACAGUUCAAGGGAAA |
| GPSZRA399 | 818 | CCCUUGAACUGUUCUGGCC | (1289, 1309) | 399 | GGCCAGAACAGUUCAAGGGAA |
| GPSZRA400 | 819 | GACCAACUCUCUCCUGUAU | (1311, 1331) | 400 | AUACAGGAGAGAGUUGGUCUG |
| GPSZRA401 | 820 | ACCAACUCUCUCCUGUAUA | (1312, 1332) | 401 | UAUACAGGAGAGAGUUGGUCU |
| GPSZRA402 | 821 | CCAACUCUCUCCUGUAUAA | (1313, 1333) | 402 | UUAUACAGGAGAGAGUUGGUC |
| GPSZRA403 | 822 | CUCUCCUGUAUAACCCCAC | (1335, 1355) | 403 | GUGGGGUUAUACAGGAGAGAG |
| GPSZRA404 | 823 | UCUCCUGUAUAACCCCACC | (1336, 1356) | 404 | GGUGGGGUUAUACAGGAGAGA |
| GPSZRA405 | 824 | CUCCUGUAUAACCCCACCU | (1337, 1357) | 405 | AGGUGGGGUUAUACAGGAGAG |
| GPSZRA406 | 825 | UCCUGUAUAACCCCACCUU | (1338, 1358) | 406 | AAGGUGGGGUUAUACAGGAGA |
| GPSZRA407 | 826 | CCUGUAUAACCCCACCUUG | (1339, 1359) | 407 | CAAGGUGGGGUUAUACAGGAG |
| GPSZRA408 | 827 | CUGUAUAACCCCACCUUGC | (1340, 1360) | 408 | GCAAGGUGGGGUUAUACAGGA |
| GPSZRA409 | 828 | UGUAUAACCCCACCUUGCC | (1341, 1361) | 409 | GGCAAGGUGGGGUUAUACAGG |
| GPSZRA410 | 829 | GUAUAACCCCACCUUGCCA | (1342, 1362) | 410 | UGGCAAGGUGGGGUUAUACAG |
| GPSZRA411 | 830 | UAUAACCCCACCUUGCCAA | (1343, 1363) | 411 | UUGGCAAGGUGGGGUUAUACA |
| GPSZRA412 | 831 | UUCUUCUACAACCAGAGCC | (1365, 1385) | 412 | GGCUCUGGUUGUAGAAGAAAG |
| GPSZRA413 | 832 | UCUUCUACAACCAGAGCCC | (1366, 1386) | 413 | GGGCUCUGGUUGUAGAAGAAA |
| GPSZRA414 | 833 | CUUCUACAACCAGAGCCCC | (1367, 1387) | 414 | GGGGCUCUGGUUGUAGAAGAA |
| GPSZRA415 | 834 | UUCUACAACCAGAGCCCCC | (1368, 1388) | 415 | GGGGGCUCUGGUUGUAGAAGA |
| GPSZRA416 | 835 | AACCAGAGCCCCCCACAAU | (1374, 1394) | 416 | AUUGUGGGGGGCUCUGGUUGU |
| GPSZRA417 | 836 | CAGAGCCCCCCACAAUGAU | (1377, 1397) | 417 | AUCAUUGUGGGGGGCUCUGGU |
| GPSZRA418 | 837 | AGAGCCCCCCACAAUGAUG | (1378, 1398) | 418 | CAUCAUUGUGGGGGGCUCUGG |
| GPSZRA419 | 838 | GAGCCCCCCACAAUGAUGA | (1379, 1399) | 419 | UCAUCAUUGUGGGGGGCUCUG |

Preparation Example 2: Synthesis of modified siRNAs

This preparation example provides a modified siRNA for inhibiting RAGE expression. The modification pattern of the siRNA is shown in Table 2, the overall modification combination of the siRNA is shown in Table 3, and the modified siRNA sequence is shown in Table 4.

### Modification instructions:

In Tables 2-4, Nm represents a methoxy-modified nucleotide, Nf represents a fluoro-modified nucleotide, N(LNA) represents a locked nucleic acid-modified nucleotide, VP indicates that the nucleotide adjacent to its right is a 5'-(E)-vinylphosphonate-modified nucleotide, and s represents a phosphorothioate linkage. In Table 3, the sequence name suffixes S1, S2, S3, S4, S5, S1-VP, S2-VP, S3-VP, S4-VP, and S5-VP represent different modification methods, which are explained below:
S1 represents the sense strand modification pattern SEQ ID NO: 1412 + the antisense strand modification pattern SEQ ID NO: 1409. The sense strand comprises the following chemical modifications: in the 5' to 3' direction, the nucleotides at positions 1 and 2, as well as positions 18 and 19, are linked via phosphorothioate groups; the nucleotides at positions 1, 2, 4, 6, 10, 12, 14, 16 and 18 are methoxy-modified nucleotides; the nucleotides at positions 3, 5, 7, 8, 9, 11, 13, 15, 17 and 19 are fluoro-modified nucleotides; the antisense strand comprises the following chemical modifications: in the 5' to 3' direction, the nucleotides at positions 1 and 2, positions 2 and 3, positions 19 and 20, as well as positions 20 and 21, are linked via phosphorothioate groups; the nucleotides at positions 1, 3, 5, 7, 9, 11, 12, 13, 15, 17, 19, 20 and 21 are methoxy-modified nucleotides; the nucleotides at positions 2, 4, 6, 8, 10, 14, 16 and 18 are fluoro-modified nucleotides.

S2 represents the sense strand modification pattern SEQ ID NO: 1413 + the antisense strand modification pattern SEQ ID NO: 1410. The sense strand comprises the following chemical modifications: in the 5' to 3' direction, the nucleotides at positions 1 and 2, as well as positions 18 and 19, are linked via phosphorothioate groups; the nucleotides at positions 1, 2, 4, 5, 6, 10, 12, 14, 16, 17, 18 and 19 are methoxy-modified nucleotides; the nucleotides at positions 3, 7, 8, 9, 11, 13 and 15 are fluoro-modified nucleotides; the antisense strand comprises the following chemical modifications: in the 5' to 3' direction, the nucleotides at positions 1 and 2, positions 2 and 3, positions 19 and 20, as well as positions 20 and 21, are linked via phosphorothioate groups; the nucleotides at positions 1, 3, 4, 5, 7, 9, 10, 11, 12, 13, 15, 17, 18, 19, 20 and 21 are methoxy-modified nucleotides; the nucleotides at positions 2, 6, 8, 14 and 16 are fluoro-modified nucleotides.

S3 represents the sense strand modification pattern SEQ ID NO: 1414 + the antisense strand modification pattern SEQ ID NO: 1411. The sense strand comprises the following chemical modifications: in the 5' to 3' direction, the nucleotides at positions 1 and 2, as well as positions 18 and 19, are linked via phosphorothioate groups; the nucleotides at positions 1, 2, 3, 4, 5, 6, 10, 11, 12, 13, 14, 15, 16, 17, 18 and 19 are methoxy-modified nucleotides; the nucleotides at positions 7, 8 and 9 are fluoro-modified nucleotides; the antisense strand comprises the following chemical modifications: in the 5' to 3' direction, the nucleotides at positions 1 and 2, positions 2 and 3, positions 19 and 20, as well as positions 20 and 21, are linked via phosphorothioate groups; the nucleotides at positions 1, 3, 4, 5, 7, 8, 9, 10, 11, 12, 13, 15, 17, 18, 19, 20 and 21 are methoxy-modified nucleotides; the nucleotides at positions 2, 6, 14 and 16 are fluoro-modified nucleotides.

S4 represents the sense strand modification pattern SEQ ID NO: 1415 + the antisense strand modification pattern SEQ ID NO: 1410. The sense strand comprises the following chemical modifications: in the 5' to 3' direction, the nucleotides at positions 1 and 2, as well as positions 18 and 19, are linked via phosphorothioate groups; the nucleotide at position 1 is a locked nucleic acid-modified nucleotide; the nucleotides at positions 2, 4, 5, 6, 10, 12, 14, 16, 17, 18 and 19 are methoxy-modified nucleotides; the nucleotides at positions 3, 7, 8, 9, 11, 13 and 15 are fluoro-modified nucleotides; the antisense strand comprises the following chemical modifications: in the 5' to 3' direction, the nucleotides at positions 1 and 2, positions 2 and 3, positions 19 and 20, as well as positions 20 and 21, are linked via phosphorothioate groups; the nucleotides at positions 1, 3, 4, 5, 7, 9, 10, 11, 12, 13, 15, 17, 18, 19, 20 and 21 are methoxy-modified nucleotides; the nucleotides at positions 2, 6, 8, 14 and 16 are fluoro-modified nucleotides.

S5 represents the sense strand modification pattern SEQ ID NO: 1416 + the antisense strand modification pattern SEQ ID NO: 1410. The sense strand comprises the following chemical modifications: in the 5' to 3' direction, the nucleotides at positions 1 and 2, as well as positions 18 and 19, are linked via phosphorothioate groups; the nucleotides at positions 1, 2, 3, 4, 6, 10, 11, 12, 13, 14, 16, 17, 18 and 19 are methoxy-modified nucleotides; the nucleotides at positions 5, 7, 8, 9 and 15 are fluoro-modified nucleotides; the antisense strand comprises the following chemical modifications: in the 5' to 3' direction, the nucleotides at positions 1 and 2, positions 2 and 3, positions 19 and 20, as well as positions 20 and 21, are linked via phosphorothioate groups; the nucleotides at positions 1, 3, 4, 5, 7, 9, 10, 11, 12, 13, 15, 17, 18, 19, 20 and 21 are methoxy-modified nucleotides; the nucleotides at positions 2, 6, 8, 14 and 16 are fluoro-modified nucleotides.

S1-VP represents the sense strand modification pattern SEQ ID NO: 1412 + the antisense strand modification pattern SEQ ID NO: 1423. The sense strand comprises the following chemical modifications: in the 5' to 3' direction, the nucleotides at positions 1 and 2, as well as positions 18 and 19, are linked via phosphorothioate groups; the nucleotides at positions 1, 2, 4, 6, 10, 12, 14, 16 and 18 are methoxy-modified nucleotides; the nucleotides at positions 3, 5, 7, 8, 9, 11, 13, 15, 17 and 19 are fluoro-modified nucleotides; the antisense strand comprises the following chemical modifications: in the 5' to 3' direction, the nucleotides at positions 1 and 2, positions 2 and 3, positions 19 and 20, as well as positions 20 and 21, are linked via phosphorothioate groups; the nucleotides at positions 1, 3, 5, 7, 9, 11, 12, 13, 15, 17, 19, 20 and 21 are methoxy-modified nucleotides; the nucleotides at positions 2, 4, 6, 8, 10, 14, 16 and 18 are fluoro-modified nucleotides, and the nucleotide at position 1 further comprises a 5'-(E)-vinylphosphonate modification.

S2-VP represents the sense strand modification pattern SEQ ID NO: 1413 + the antisense strand modification pattern SEQ ID NO: 1424. The sense strand comprises the following chemical modifications: in the 5' to 3' direction, the nucleotides at positions 1 and 2, as well as positions 18 and 19, are linked via phosphorothioate groups; the nucleotides at positions 1, 2, 4, 5, 6, 10, 12, 14, 16, 17, 18 and 19 are methoxy-modified nucleotides; the nucleotides at positions 3, 7, 8, 9, 11, 13 and 15 are fluoro-modified nucleotides; the antisense strand comprises the following chemical modifications: in the 5' to 3' direction, the nucleotides at positions 1 and 2, positions 2 and 3, positions 19 and 20, as well as positions 20 and 21, are linked via phosphorothioate groups; the nucleotides at positions 1, 3, 4, 5, 7, 9, 10, 11, 12, 13, 15, 17, 18, 19, 20 and 21 are methoxy-modified nucleotides; the nucleotides at positions 2, 6, 8, 14 and 16 are fluoro-modified nucleotides, and the nucleotide at position 1 further comprises a 5'-(E)-vinylphosphonate modification.

S3-VP represents the sense strand modification pattern SEQ ID NO: 1414 + the antisense strand modification pattern SEQ ID NO: 1425. The sense strand comprises the following chemical modifications: in the 5' to 3' direction, the nucleotides at positions 1 and 2, as well as positions 18 and 19, are linked via phosphorothioate groups; the nucleotides at positions 1, 2, 3, 4, 5, 6, 10, 11, 12, 13, 14, 15, 16, 17, 18 and 19 are methoxy-modified nucleotides; the nucleotides at positions 7, 8 and 9 are fluoro-modified nucleotides; the antisense strand comprises the following chemical modifications: in the 5' to 3' direction, the nucleotides at positions 1 and 2, positions 2 and 3, positions 19 and 20, as well as positions 20 and 21, are linked via phosphorothioate groups; the nucleotides at positions 1, 3, 4, 5, 7, 8, 9, 10, 11, 12, 13, 15, 17, 18, 19, 20 and 21 are methoxy-modified nucleotides; the nucleotides at positions 2, 6, 14 and 16 are fluoro-modified nucleotides, and the nucleotide at position 1 further comprises a 5'-(E)-vinylphosphonate modification.

S4-VP represents the sense strand modification pattern SEQ ID NO: 1415 + the antisense strand modification pattern SEQ ID NO: 1424. The sense strand comprises the following chemical modifications: in the 5' to 3' direction, the nucleotides at positions 1 and 2, as well as positions 18 and 19, are linked via phosphorothioate groups; the nucleotide at position 1 is a locked nucleic acid-modified nucleotide, the nucleotides at positions 2, 4, 5, 6, 10, 12, 14, 16, 17, 18 and 19 are methoxy-modified nucleotides; the nucleotides at positions 3, 7, 8, 9, 11, 13 and 15 are fluoro-modified nucleotides; the antisense strand comprises the following chemical modifications: in the 5' to 3' direction, the nucleotides at positions 1 and 2, positions 2 and 3, positions 19 and 20, as well as positions 20 and 21, are linked via phosphorothioate groups; the nucleotides at positions 1, 3, 4, 5, 7, 9, 10, 11, 12, 13, 15, 17, 18, 19, 20 and 21 are methoxy-modified nucleotides; the nucleotides at positions 2, 6, 8, 14 and 16 are fluoro-modified nucleotides, and the nucleotide at position 1 further comprises a 5'-(E)-vinylphosphonate modification.

S5-VP represents the sense strand modification pattern SEQ ID NO: 1416 + the antisense strand modification pattern SEQ ID NO: 1424. The sense strand comprises the following chemical modifications: in the 5' to 3' direction, the nucleotides at positions 1 and 2, as well as positions 18 and 19, are linked via phosphorothioate groups; the nucleotides at positions 1, 2, 3, 4, 6, 10, 11, 12, 13, 14, 16, 17, 18 and 19 are methoxy-modified nucleotides; the nucleotides at positions 5, 7, 8, 9 and 15 are fluoro-modified nucleotides; the antisense strand comprises the following chemical modifications: in the 5' to 3' direction, the nucleotides at positions 1 and 2, positions 2 and 3, positions 19 and 20, as well as positions 20 and 21, are linked via phosphorothioate groups; the nucleotides at positions 1, 3, 4, 5, 7, 9, 10, 11, 12, 13, 15, 17, 18, 19, 20 and 21 are methoxy-modified nucleotides; the nucleotides at positions 2, 6, 8, 14 and 16 are fluoro-modified nucleotides, and the nucleotide at position 1 further comprises a 5'-(E)-vinylphosphonate modification.

**Table 2: Modification patterns of siRNA**

| Name | SEQ ID NO: | 5'-3' |
|---|---|---|
| Antisense strand modificati on pattern 1 | 1409 | NmsNfsNmNfNmNfNmNfNmNfNmNmNmNfNmNfNmNfNmsNmsNm |
| Antisense strand modificati on pattern 2 | 1410 | NmsNfsNmNmNmNfNmNfNmNmNmNmNmNfNmNfNmNmNmsNmsNm |
| Antisense strand modificati on pattern 3 | 1411 | NmsNfsNmNmNmNfNmNmNmNmNmNmNmNfNmNfNmNmNmsNmsNm |
| Antisense strand modificati on pattern 4 | 1423 | VPNmsNfsNmNfNmNfNmNfNmNfNmNmNmNfNmNfNmNfNmsNmsNm |
| Antisense strand modificati on pattern 5 | 1424 | VPNmsNfsNmNmNmNfNmNfNmNmNmNmNmNfNmNfNmNmNmsNmsNm |
| Antisense strand modificati on pattern 6 | 1425 | VPNmsNfsNmNmNmNfNmNmNmNmNmNmNmNfNmNfNmNmNmsNmsNm |
| Sense strand modificati on pattern 1 | 1412 | NmsNmNfNmNfNmNfNfNfNmNfNmNfNmNfNmNfNmsNf |
| Sense strand modificati on pattern 2 | 1413 | NmsNmNfNmNmNmNfNfNfNmNfNmNfNmNfNmNmNmsNm |
| Sense strand modificati on pattern 3 | 1414 | NmsNmNmNmNmNmNfNfNfNmNmNmNmNmNmNmNmNmsNm |
| Sense strand modificati on pattern 4 | 1415 | N(LNA)sNmNfNmNmNmNfNfNfNmNfNmNfNmNfNmNmNmsNm |
| Sense strand modificati on pattern 5 | 1416 | NmsNmNmNmNfNmNfNfNfNmNmNmNmNmNfNmNmNmsNm |

**Table 3: Modification patterns of siRNA - overview**

| | Sense strand (5'-3') | SEQ ID NO: | Antisense strand (5'-3') | SEQ ID NO: |
|---|---|---|---|---|
| S1 modification pattern | NmsNmNfNmNfNmNfNfNfN mNfNmNfNmNfNmNfNmsNf | 1412 | NmsNfsNmNfNmNfNmNfNmNfNmNm NmNfNmNfNmNfNmsNmsNm | 1409 |
| S2 modification pattern | NmsNmNfNmNmNmNfNfNfN mNfNmNfNmNfNmNmNmsN m | 1413 | NmsNfsNmNmNmNfNmNfNmNmNmN mNmNfNmNfNmNmNmsNmsNm | 1410 |
| S3 modification pattern | NmsNmNmNmNmNmNfNfNf NmNmNmNmNmNmNmNmN msNm | 1414 | NmsNfsNmNmNmNfNmNmNmNmNm NmNmNfNmNfNmNmNmsNmsNm | 1411 |
| S4 modification pattern | N(LNA)sNmNfNmNmNmNfN fNfNmNfNmNfNmNfNmNmN msNm | 1415 | NmsNfsNmNmNmNfNmNfNmNmNmN mNmNfNmNfNmNmNmsNmsNm | 1410 |
| S5 modification pattern | NmsNmNmNmNfNmNfNfNfN mNmNmNmNmNfNmNmNms Nm | 1416 | NmsNfsNmNmNmNfNmNfNmNmNmN mNmNfNmNfNmNmNmsNmsNm | 1410 |
| S1-VP modification pattern | NmsNmNfNmNfNmNfNfNfN mNfNmNfNmNfNmNfNmsNf | 1412 | VPNmsNfsNmNfNmNfNmNfNmNfNm NmNmNfNmNfNmNfNmsNmsNm | 1423 |
| S2-VP modification pattern | NmsNmNfNmNmNmNfNfNfN mNfNmNfNmNfNmNmNmsN m | 1413 | VPNmsNfsNmNmNmNfNmNfNmNmN mNmNmNfNmNfNmNmNmsNmsNm | 1424 |
| S3-VP modification pattern | NmsNmNmNmNmNmNfNfNf NmNmNmNmNmNmNmNmN msNm | 1414 | VPNmsNfsNmNmNmNfNmNmNmNm NmNmNmNfNmNfNmNmNmsNmsNm | 1425 |
| S4-VP modification pattern | N(LNA)sNmNfNmNmNmNfN fNfNmNfNmNfNmNfNmNmN msNm | 1415 | VPNmsNfsNmNmNmNfNmNfNmNmN mNmNmNfNmNfNmNmNmsNmsNm | 1424 |
| S5-VP modification pattern | NmsNmNmNmNfNmNfNfNfN mNmNmNmNmNfNmNmNms Nm | 1416 | VPNmsNfsNmNmNmNfNmNfNmNmN mNmNmNfNmNfNmNmNmsNmsNm | 1424 |

**Table 4: Modified siRNA sequences**

| **Name** | **SEQ ID NO** | **sense (5'-3')** | **SEQ ID NO** | **antisense (5'-3')** |
|---|---|---|---|---|
| GPSZRA 084S1 | 1124 | GmsGmAfAmAfGmGfAfGfAmCf CmAfAmGfUmCfCmsAf | 839 | UmsGfsGmAfCmUfUmGfGmUfCmU mCmCfUmUfUmCfCmsAmsUm |
| GPSZRA 087S1 | 1125 | AmsAmGfGmAfGmAfCfCfAmAf GmUfCmCfAmAfCmsUf | 840 | AmsGfsUmUfGmGfAmCfUmUfGmG mUmCfUmCfCmUfUmsUmsCm |
| GPSZRA 092S1 | 1126 | GmsAmCfCmAfAmGfUfCfCmAfA mCfUmAfCmCfGmsAf | 841 | UmsCfsGmGfUmAfGmUfUmGfGmA mCmUfUmGfGmUfCmsUmsCm |
| GPSZRA 093S1 | 1127 | AmsCmCfAmAfGmUfCfCfAmAfC mUfAmCfCmGfAmsGf | 842 | CmsUfsCmGfGmUfAmGfUmUfGmG mAmCfUmUfGmGfUmsCmsUm |
| GPSZRA 097S1 | 1128 | AmsGmUfCmCfAmAfCfUfAmCfC mGfAmGfUmCfCmsGf | 843 | CmsGfsGmAfCmUfCmGfGmUfAmG mUmUfGmGfAmCfUmsUmsGm |
| GPSZRA 118S1 | 1129 | CmsUmAfCmCfAmGfAfUfUmCfC mUfGmGfGmAfAmsGf | 844 | CmsUfsUmCfCmCfAmGfGmAfAmU mCmUfGmGfUmAfGmsAmsCm |
| GPSZRA 217S1 | 1130 | AmsGmGfGmAfGmUfAfUfCmUf GmUfGmAfAmGfGmsAf | 845 | UmsCfsCmUfUmCfAmCfAmGfAmU mAmCfUmCfCmCfUmsUmsCm |
| GPSZRA 222S1 | 1131 | UmsCmUfUmCfAmCfAfCfUmGfC mAfGmUfCmGfGmsAf | 846 | UmsCfsCmGfAmCfUmGfCmAfGmU mGmUfGmAfAmGfAmsGmsCm |
| GPSZRA 227S1 | 1132 | AmsCmAfCmUfGmCfAfGfUmCfG mGfAmGfCmUfAmsAf | 847 | UmsUfsAmGfCmUfCmCfGmAfCmU mGmCfAmGfUmGfUmsGmsAm |
| GPSZRA 228S1 | 1133 | CmsAmCfUmGfCmAfGfUfCmGfG mAfGmCfUmAfAmsUf | 848 | AmsUfsUmAfGmCfUmCfCmGfAmC mUmGfCmAfGmUfGmsUmsGm |
| GPSZRA 229S1 | 1134 | AmsCmUfGmCfAmGfUfCfGmGfA mGfCmUfAmAfUmsGf | 849 | CmsAfsUmUfAmGfCmUfCmCfGmA mCmUfGmCfAmGfUmsGmsUm |
| GPSZRA 231S1 | 1135 | UmsGmCfAmGfUmCfGfGfAmGfC mUfAmAfUmGfGmsUf | 850 | AmsCfsCmAfUmUfAmGfCmUfCmC mGmAfCmUfGmCfAmsGmsUm |
| GPSZRA 232S1 | 1136 | GmsCmAfGmUfCmGfGfAfGmCfU mAfAmUfGmGfUmsGf | 851 | CmsAfsCmCfAmUfUmAfGmCfUmC mCmGfAmCfUmGfCmsAmsGm |
| GPSZRA 233S1 | 1137 | CmsAmGfUmCfGmGfAfGfCmUfA mAfUmGfGmUfGmsAf | 852 | UmsCfsAmCfCmAfUmUfAmGfCmU mCmCfGmAfCmUfGmsCmsAm |
| GPSZRA 252S1 | 1138 | UmsCmUfGmGfAmGfGfAfGmGf UmCfCmAfAmUfUmsGf | 853 | CmsAfsAmUfUmGfGmAfCmCfUmC mCmUfCmCfAmGfAmsGmsGm |
| GPSZRA 257S1 | 1139 | AmsGmGfAmGfGmUfCfCfAmAf UmUfGmGfUmGfGmsUf | 854 | AmsCfsCmAfCmCfAmAfUmUfGmG mAmCfCmUfCmCfUmsCmsCm |
| GPSZRA 266S1 | 1140 | AmsGmCfAmGfUmAfGfCfUmCfC mUfGmGfUmGfGmsAf | 855 | UmsCfsCmAfCmCfAmGfGmAfGmC mUmAfCmUfGmCfUmsCmsCm |
| GPSZRA 267S1 | 1141 | GmsCmAfGmUfAmGfCfUfCmCfU mGfGmUfGmGfAmsAf | 856 | UmsUfsCmCfAmCfCmAfGmGfAmG mCmUfAmCfUmGfCmsUmsCm |
| GPSZRA 271S1 | 1142 | AmsGmCfUmCfCmUfGfGfUmGfG mAfAmCfCmGfUmsAf | 857 | UmsAfsCmGfGmUfUmCfCmAfCmC mAmGfGmAfGmCfUmsAmsCm |
| GPSZRA 272S1 | 1143 | GmsCmUfCmCfUmGfGfUfGmGfA mAfCmCfGmUfAmsAf | 858 | UmsUfsAmCfGmGfUmUfCmCfAmC mCmAfGmGfAmGfCmsUmsAm |
| GPSZRA 274S1 | 1144 | UmsCmCfUmGfGmUfGfGfAmAfC mCfGmUfAmAfCmsCf | 859 | GmsGfsUmUfAmCfGmGfUmUfCmC mAmCfCmAfGmGfAmsGmsCm |
| GPSZRA 275S1 | 1145 | CmsUmGfGmUfGmGfAfAfCmCfG mUfAmAfCmCfCmsUf | 860 | AmsGfsGmGfUmUfAmCfGmGfUmU mCmCfAmCfCmAfGmsGmsAm |
| GPSZRA 276S1 | 1146 | UmsGmGfUmGfGmAfAfCfCmGf UmAfAmCfCmCfUmsGf | 861 | CmsAfsGmGfGmUfUmAfCmGfGmU mUmCfCmAfCmCfAmsGmsGm |
| GPSZRA 27751 | 1147 | GmsGmUfGmGfAmAfCfCfGmUf AmAfCmCfCmUfGmsAf | 862 | UmsCfsAmGfGmGfUmUfAmCfGmG mUmUfCmCfAmCfCmsAmsGm |
| GPSZRA 281S1 | 1148 | GmsAmAfCmCfGmUfAfAfCmCfC mUfGmAfCmCfUmsGf | 863 | CmsAfsGmGfUmCfAmGfGmGfUmU mAmCfGmGfUmUfCmsCmsAm |
| GPSZRA 282S1 | 1149 | AmsAmCfCmGfUmAfAfCfCmCfU mGfAmCfCmUfGmsUf | 864 | AmsCfsAmGfGmUfCmAfGmGfGmU mUmAfCmGfGmUfUmsCmsCm |
| GPSZRA 283S1 | 1150 | AmsCmCfGmUfAmAfCfCfCmUfG mAfCmCfUmGfUmsGf | 865 | CmsAfsCmAfGmGfUmCfAmGfGmG mUmUfAmCfGmGfUmsUmsCm |
| GPSZRA 284S1 | 1151 | CmsCmGfUmAfAmCfCfCfUmGfA mCfCmUfGmUfGmsAf | 866 | UmsCfsAmCfAmGfGmUfCmAfGmG mGmUfUmAfCmGfGmsUmsUm |
| GPSZRA 285S1 | 1152 | CmsGmUfAmAfCmCfCfUfGmAfC mCfUmGfUmGfAmsAf | 867 | UmsUfsCmAfCmAfGmGfUmCfAmG mGmGfUmUfAmCfGmsGmsUm |
| GPSZRA 286S1 | 1153 | GmsUmAfAmCfCmCfUfGfAmCfC mUfGmUfGmAfAmsGf | 868 | CmsUfsUmCfAmCfAmGfGmUfCmA mGmGfGmUfUmAfCmsGmsGm |
| GPSZRA 287S1 | 1154 | UmsAmAfCmCfCmUfGfAfCmCfU mGfUmGfAmAfGmsUf | 869 | AmsCfsUmUfCmAfCmAfGmGfUmC mAmGfGmGfUmUfAmsCmsGm |
| GPSZRA 288S1 | 1155 | AmsAmCfCmCfUmGfAfCfCmUfG mUfGmAfAmGfUmsCf | 870 | GmsAfsCmUfUmCfAmCfAmGfGmU mCmAfGmGfGmUfUmsAmsCm |
| GPSZRA 290S1 | 1156 | CmsCmUfGmAfCmCfUfGfUmGfA mAfGmUfCmCfCmsUf | 871 | AmsGfsGmGfAmCfUmUfCmAfCmA mGmGfUmCfAmGfGmsGmsUm |
| GPSZRA 292S1 | 1157 | UmsGmAfCmCfUmGfUfGfAmAf GmUfCmCfCmUfGmsCf | 872 | GmsCfsAmGfGmGfAmCfUmUfCmA mCmAfGmGfUmCfAmsGmsGm |
| GPSZRA 301S1 | 1158 | UmsCmUfCmCfUmCfAfAfAmUfC mCfAmCfUmGfGmsAf | 873 | UmsCfsCmAfGmUfGmGfAmUfUmU mGmAfGmGfAmGfAmsGmsGm |
| GPSZRA 308S1 | 1159 | AmsAmAfUmCfCmAfCfUfGmGfA mUfGmAfAmGfGmsAf | 874 | UmsCfsCmUfUmCfAmUfCmCfAmG mUmGfGmAfUmUfUmsGmsAm |
| GPSZRA 311S1 | 1160 | UmsCmCfAmCfUmGfGfAfUmGfA mAfGmGfAmUfGmsGf | 875 | CmsCfsAmUfCmCfUmUfCmAfUmCm CmAfGmUfGmGfAmsUmsUm |
| GPSZRA 312S1 | 1161 | CmsCmAfCmUfGmGfAfUfGmAfA mGfGmAfUmGfGmsUf | 876 | AmsCfsCmAfUmCfCmUfUmCfAmU mCmCfAmGfUmGfGmsAmsUm |
| GPSZRA 313S1 | 1162 | CmsAmCfUmGfGmAfUfGfAmAf GmGfAmUfGmGfUmsGf | 877 | CmsAfsCmCfAmUfCmCfUmUfCmAm UmCfCmAfGmUfGmsGmsAm |
| GPSZRA 314S1 | 1163 | AmsCmUfGmGfAmUfGfAfAmGf GmAfUmGfGmUfGmsUf | 878 | AmsCfsAmCfCmAfUmCfCmUfUmCm AmUfCmCfAmGfUmsGmsGm |
| GPSZRA 315S1 | 1164 | CmsUmGfGmAfUmGfAfAfGmGf AmUfGmGfUmGfUmsGf | 879 | CmsAfsCmAfCmCfAmUfCmCfUmUm CmAfUmCfCmAfGmsUmsGm |
| GPSZRA 316S1 | 1165 | UmsGmGfAmUfGmAfAfGfGmAf UmGfGmUfGmUfGmsCf | 880 | GmsCfsAmCfAmCfCmAfUmCfCmUm UmCfAmUfCmCfAmsGmsUm |
| GPSZRA 317S1 | 1166 | GmsGmAfUmGfAmAfGfGfAmUf GmGfUmGfUmGfCmsCf | 881 | GmsGfsCmAfCmAfCmCfAmUfCmCm UmUfCmAfUmCfCmsAmsGm |
| GPSZRA 318S1 | 1167 | GmsAmUfGmAfAmGfGfAfUmGf GmUfGmUfGmCfCmsCf | 882 | GmsGfsGmCfAmCfAmCfCmAfUmC mCmUfUmCfAmUfCmsCmsAm |
| GPSZRA 320S1 | 1168 | UmsGmAfAmGfGmAfUfGfGmUf GmUfGmCfCmCfUmsUf | 883 | AmsAfsGmGfGmCfAmCfAmCfCmA mUmCfCmUfUmCfAmsUmsCm |
| GPSZRA 333S1 | 1169 | GmsAmAfAmGfCmCfGfUfGmCfU mGfUmCfAmGfCmsAf | 884 | UmsGfsCmUfGmAfCmAfGmCfAmC mGmGfCmUfUmUfCmsCmsUm |
| GPSZRA 342S1 | 1170 | UmsGmUfCmAfGmCfAfUfCmAfG mCfAmUfCmAfUmsCf | 885 | GmsAfsUmGfAmUfGmCfUmGfAmU mGmCfUmGfAmCfAmsGmsCm |
| GPSZRA 378S1 | 1171 | AmsAmGfAmGfGmAfGfGfAmGf CmGfUmGfCmAfGmsAf | 886 | UmsCfsUmGfCmAfCmGfCmUfCmCm UmCfCmUfCmUfUmsCmsCm |
| GPSZRA 379S1 | 1172 | GmsAmAfUmCfAmGfUfCfGmGf AmGfGmAfAmCfCmsUf | 887 | AmsGfsGmUfUmCfCmUfCmCfGmA mCmUfGmAfUmUfCmsAmsGm |
| GPSZRA 380S1 | 1173 | AmsAmUfCmAfGmUfCfGfGmAf GmGfAmAfCmCfUmsGf | 888 | CmsAfsGmGfUmUfCmCfUmCfCmG mAmCfUmGfAmUfUmsCmsAm |
| GPSZRA 381S1 | 1174 | AmsUmCfAmGfUmCfGfGfAmGf GmAfAmCfCmUfGmsAf | 889 | UmsCfsAmGfGmUfUmCfCmUfCmC mGmAfCmUfGmAfUmsUmsCm |
| GPSZRA 402S1 | 1175 | CmsCmAfAmCfUmCfUfCfUmCfC mUfGmUfAmUfAmsAf | 890 | UmsUfsAmUfAmCfAmGfGmAfGmA mGmAfGmUfUmGfGmsUmsCm |
| GPSZRA 407S1 | 1176 | CmsCmUfGmUfAmUfAfAfCmCfC mCfAmCfCmUfUmsGf | 891 | CmsAfsAmGfGmUfGmGfGmGfUmU mAmUfAmCfAmGfGmsAmsGm |
| GPSZRA 408S1 | 1177 | CmsUmGfUmAfUmAfAfCfCmCfC mAfCmCfUmUfGmsCf | 892 | GmsCfsAmAfGmGfUmGfGmGfGmU mUmAfUmAfCmAfGmsGmsAm |
| GPSZRA 409S1 | 1178 | UmsGmUfAmUfAmAfCfCfCmCfA mCfCmUfUmGfCmsCf | 893 | GmsGfsCmAfAmGfGmUfGmGfGmG mUmUfAmUfAmCfAmsGmsGm |
| GPSZRA 410S1 | 1179 | GmsUmAfUmAfAmCfCfCfCmAfC mCfUmUfGmCfCmsAf | 894 | UmsGfsGmCfAmAfGmGfUmGfGmG mGmUfUmAfUmAfCmsAmsGm |
| GPSZRA 411S1 | 1180 | UmsAmUfAmAfCmCfCfCfAmCfC mUfUmGfCmCfAmsAf | 895 | UmsUfsGmGfCmAfAmGfGmUfGmG mGmGfUmUfAmUfAmsCmsAm |
| GPSZRA 084S2 | 1181 | GmsGmAfAmAmGmGfAfGfAmCf CmAfAmGfUmCmCmsAm | 896 | UmsGfsGmAmCmUfUmGfGmUmCm UmCmCfUmUfUmCmCmsAmsUm |
| GPSZRA 087S2 | 1182 | AmsAmGfGmAmGmAfCfCfAmAf GmUfCmCfAmAmCmsUm | 897 | AmsGfsUmUmGmGfAmCfUmUmGm GmUmCfUmCfCmUmUmsUmsCm |
| GPSZRA 092S2 | 1183 | GmsAmCfCmAmAmGfUfCfCmAf AmCfUmAfCmCmGmsAm | 898 | UmsCfsGmGmUmAfGmUfUmGmGm AmCmUfUmGfGmUmCmsUmsCm |
| GPSZRA 093S2 | 1184 | AmsCmCfAmAmGmUfCfCfAmAf CmUfAmCfCmGmAmsGm | 899 | CmsUfsCmGmGmUfAmGfUmUmGm GmAmCfUmUfGmGmUmsCmsUm |
| GPSZRA 097S2 | 1185 | AmsGmUfCmCmAmAfCfUfAmCf CmGfAmGfUmCmCmsGm | 900 | CmsGfsGmAmCmUfCmGfGmUmAm GmUmUfGmGfAmCmUmsUmsGm |
| GPSZRA 118S2 | 1186 | CmsUmAfCmCmAmGfAfUfUmCf CmUfGmGfGmAmAmsGm | 901 | CmsUfsUmCmCmCfAmGfGmAmAm UmCmUfGmGfUmAmGmsAmsCm |
| GPSZRA 217S2 | 1187 | AmsGmGfGmAmGmUfAfUfCmUf GmUfGmAfAmGmGmsAm | 902 | UmsCfsCmUmUmCfAmCfAmGmAm UmAmCfUmCfCmCmUmsUmsCm |
| GPSZRA 222S2 | 1188 | UmsCmUfUmCmAmCfAfCfUmGf CmAfGmUfCmGmGmsAm | 903 | UmsCfsCmGmAmCfUmGfCmAmGm UmGmUfGmAfAmGmAmsGmsCm |
| GPSZRA 227S2 | 1189 | AmsCmAfCmUmGmCfAfGfUmCf GmGfAmGfCmUmAmsAm | 904 | UmsUfsAmGmCmUfCmCfGmAmCm UmGmCfAmGfUmGmUmsGmsAm |
| GPSZRA 228S2 | 1190 | CmsAmCfUmGmCmAfGfUfCmGf GmAfGmCfUmAmAmsUm | 905 | AmsUfsUmAmGmCfUmCfCmGmAm CmUmGfCmAfGmUmGmsUmsGm |
| GPSZRA 229S2 | 1191 | AmsCmUfGmCmAmGfUfCfGmGf AmGfCmUfAmAmUmsGm | 906 | CmsAfsUmUmAmGfCmUfCmCmGm AmCmUfGmCfAmGmUmsGmsUm |
| GPSZRA 231S2 | 1192 | UmsGmCfAmGmUmCfGfGfAmGf CmUfAmAfUmGmGmsUm | 907 | AmsCfsCmAmUmUfAmGfCmUmCm CmGmAfCmUfGmCmAmsGmsUm |
| GPSZRA 232S2 | 1193 | GmsCmAfGmUmCmGfGfAfGmCf UmAfAmUfGmGmUmsGm | 908 | CmsAfsCmCmAmUfUmAfGmCmUm CmCmGfAmCfUmGmCmsAmsGm |
| GPSZRA 233S2 | 1194 | CmsAmGfUmCmGmGfAfGfCmUf AmAfUmGfGmUmGmsAm | 909 | UmsCfsAmCmCmAfUmUfAmGmCm UmCmCfGmAfCmUmGmsCmsAm |
| GPSZRA 252S2 | 1195 | UmsCmUfGmGmAmGfGfAfGmGf UmCfCmAfAmUmUmsGm | 910 | CmsAfsAmUmUmGfGmAfCmCmUm CmCmUfCmCfAmGmAmsGmsGm |
| GPSZRA 257S2 | 1196 | AmsGmGfAmGmGmUfCfCfAmAf UmUfGmGfUmGmGmsUm | 911 | AmsCfsCmAmCmCfAmAfUmUmGm GmAmCfCmUfCmCmUmsCmsCm |
| GPSZRA 266S2 | 1197 | AmsGmCfAmGmUmAfGfCfUmCf CmUfGmGfUmGmGmsAm | 912 | UmsCfsCmAmCmCfAmGfGmAmGm CmUmAfCmUfGmCmUmsCmsCm |
| GPSZRA 267S2 | 1198 | GmsCmAfGmUmAmGfCfUfCmCf UmGfGmUfGmGmAmsAm | 913 | UmsUfsCmCmAmCfCmAfGmGmAm GmCmUfAmCfUmGmCmsUmsCm |
| GPSZRA 271S2 | 1199 | AmsGmCfUmCmCmUfGfGfUmGf GmAfAmCfCmGmUmsAm | 914 | UmsAfsCmGmGmUfUmCfCmAmCm CmAmGfGmAfGmCmUmsAmsCm |
| GPSZRA 272S2 | 1200 | GmsCmUfCmCmUmGfGfUfGmGf AmAfCmCfGmUmAmsAm | 915 | UmsUfsAmCmGmGfUmUfCmCmAm CmCmAfGmGfAmGmCmsUmsAm |
| GPSZRA 274S2 | 1201 | UmsCmCfUmGmGmUfGfGfAmAf CmCfGmUfAmAmCmsCm | 916 | GmsGfsUmUmAmCfGmGfUmUmCm CmAmCfCmAfGmGmAmsGmsCm |
| GPSZRA 275S2 | 1202 | CmsUmGfGmUmGmGfAfAfCmCf GmUfAmAfCmCmCmsUm | 917 | AmsGfsGmGmUmUfAmCfGmGmUm UmCmCfAmCfCmAmGmsGmsAm |
| GPSZRA 276S2 | 1203 | UmsGmGfUmGmGmAfAfCfCmGf UmAfAmCfCmCmUmsGm | 918 | CmsAfsGmGmGmUfUmAfCmGmGm UmUmCfCmAfCmCmAmsGmsGm |
| GPSZRA 277S2 | 1204 | GmsGmUfGmGmAmAfCfCfGmUf AmAfCmCfCmUmGmsAm | 919 | UmsCfsAmGmGmGfUmUfAmCmGm GmUmUfCmCfAmCmCmsAmsGm |
| GPSZRA 281S2 | 1205 | GmsAmAfCmCmGmUfAfAfCmCf CmUfGmAfCmCmUmsGm | 920 | CmsAfsGmGmUmCfAmGfGmGmUm UmAmCfGmGfUmUmCmsCmsAm |
| GPSZRA 282S2 | 1206 | AmsAmCfCmGmUmAfAfCfCmCf UmGfAmCfCmUmGmsUm | 921 | AmsCfsAmGmGmUfCmAfGmGmGm UmUmAfCmGfGmUmUmsCmsCm |
| GPSZRA 283S2 | 1207 | AmsCmCfGmUmAmAfCfCfCmUf GmAfCmCfUmGmUmsGm | 922 | CmsAfsCmAmGmGfUmCfAmGmGm GmUmUfAmCfGmGmUmsUmsCm |
| GPSZRA 284S2 | 1208 | CmsCmGfUmAmAmCfCfCfUmGf AmCfCmUfGmUmGmsAm | 923 | UmsCfsAmCmAmGfGmUfCmAmGm GmGmUfUmAfCmGmGmsUmsUm |
| GPSZRA 285S2 | 1209 | CmsGmUfAmAmCmCfCfUfGmAf CmCfUmGfUmGmAmsAm | 924 | UmsUfsCmAmCmAfGmGfUmCmAm GmGmGfUmUfAmCmGmsGmsUm |
| GPSZRA 286S2 | 1210 | GmsUmAfAmCmCmCfUfGfAmCf CmUfGmUfGmAmAmsGm | 925 | CmsUfsUmCmAmCfAmGfGmUmCm AmGmGfGmUfUmAmCmsGmsGm |
| GPSZRA 287S2 | 1211 | UmsAmAfCmCmCmUfGfAfCmCf UmGfUmGfAmAmGmsUm | 926 | AmsCfsUmUmCmAfCmAfGmGmUm CmAmGfGmGfUmUmAmsCmsGm |
| GPSZRA 288S2 | 1212 | AmsAmCfCmCmUmGfAfCfCmUf GmUfGmAfAmGmUmsCm | 927 | GmsAfsCmUmUmCfAmCfAmGmGm UmCmAfGmGfGmUmUmsAmsCm |
| GPSZRA 290S2 | 1213 | CmsCmUfGmAmCmCfUfGfUmGf AmAfGmUfCmCmCmsUm | 928 | AmsGfsGmGmAmCfUmUfCmAmCm AmGmGfUmCfAmGmGmsGmsUm |
| GPSZRA 292S2 | 1214 | UmsGmAfCmCmUmGfUfGfAmAf GmUfCmCfCmUmGmsCm | 929 | GmsCfsAmGmGmGfAmCfUmUmCm AmCmAfGmGfUmCmAmsGmsGm |
| GPSZRA 301S2 | 1215 | UmsCmUfCmCmUmCfAfAfAmUf CmCfAmCfUmGmGmsAm | 930 | UmsCfsCmAmGmUfGmGfAmUmUm UmGmAfGmGfAmGmAmsGmsGm |
| GPSZRA 308S2 | 1216 | AmsAmAfUmCmCmAfCfUfGmGf AmUfGmAfAmGmGmsAm | 931 | UmsCfsCmUmUmCfAmUfCmCmAm GmUmGfGmAfUmUmUmsGmsAm |
| GPSZRA 311S2 | 1217 | UmsCmCfAmCmUmGfGfAfUmGf AmAfGmGfAmUmGmsGm | 932 | CmsCfsAmUmCmCfUmUfCmAmUm CmCmAfGmUfGmGmAmsUmsUm |
| GPSZRA 312S2 | 1218 | CmsCmAfCmUmGmGfAfUfGmAf AmGfGmAfUmGmGmsUm | 933 | AmsCfsCmAmUmCfCmUfUmCmAm UmCmCfAmGfUmGmGmsAmsUm |
| GPSZRA 313S2 | 1219 | CmsAmCfUmGmGmAfUfGfAmAf GmGfAmUfGmGmUmsGm | 934 | CmsAfsCmCmAmUfCmCfUmUmCm AmUmCfCmAfGmUmGmsGmsAm |
| GPSZRA 314S2 | 1220 | AmsCmUfGmGmAmUfGfAfAmGf GmAfUmGfGmUmGmsUm | 935 | AmsCfsAmCmCmAfUmCfCmUmUm CmAmUfCmCfAmGmUmsGmsGm |
| GPSZRA 315S2 | 1221 | CmsUmGfGmAmUmGfAfAfGmGf AmUfGmGfUmGmUmsGm | 936 | CmsAfsCmAmCmCfAmUfCmCmUm UmCmAfUmCfCmAmGmsUmsGm |
| GPSZRA 316S2 | 1222 | UmsGmGfAmUmGmAfAfGfGmAf UmGfGmUfGmUmGmsCm | 937 | GmsCfsAmCmAmCfCmAfUmCmCm UmUmCfAmUfCmCmAmsGmsUm |
| GPSZRA 317S2 | 1223 | GmsGmAfUmGmAmAfGfGfAmUf GmGfUmGfUmGmCmsCm | 938 | GmsGfsCmAmCmAfCmCfAmUmCm CmUmUfCmAfUmCmCmsAmsGm |
| GPSZRA 318S2 | 1224 | GmsAmUfGmAmAmGfGfAfUmGf GmUfGmUfGmCmCmsCm | 939 | GmsGfsGmCmAmCfAmCfCmAmUm CmCmUfUmCfAmUmCmsCmsAm |
| GPSZRA 320S2 | 1225 | UmsGmAfAmGmGmAfUfGfGmUf GmUfGmCfCmCmUmsUm | 940 | AmsAfsGmGmGmCfAmCfAmCmCm AmUmCfCmUfUmCmAmsUmsCm |
| GPSZRA 333S2 | 1226 | GmsAmAfAmGmCmCfGfUfGmCf UmGfUmCfAmGmCmsAm | 941 | UmsGfsCmUmGmAfCmAfGmCmAm CmGmGfCmUfUmUmCmsCmsUm |
| GPSZRA 342S2 | 1227 | UmsGmUfCmAmGmCfAfUfCmAf GmCfAmUfCmAmUmsCm | 942 | GmsAfsUmGmAmUfGmCfUmGmAm UmGmCfUmGfAmCmAmsGmsCm |
| GPSZRA 378S2 | 1228 | AmsAmGfAmGmGmAfGfGfAmGf CmGfUmGfCmAmGmsAm | 943 | UmsCfsUmGmCmAfCmGfCmUmCm CmUmCfCmUfCmUmUmsCmsCm |
| GPSZRA 379S2 | 1229 | GmsAmAfUmCmAmGfUfCfGmGf AmGfGmAfAmCmCmsUm | 944 | AmsGfsGmUmUmCfCmUfCmCmGm AmCmUfGmAfUmUmCmsAmsGm |
| GPSZRA 380S2 | 1230 | AmsAmUfCmAmGmUfCfGfGmAf GmGfAmAfCmCmUmsGm | 945 | CmsAfsGmGmUmUfCmCfUmCmCm GmAmCfUmGfAmUmUmsCmsAm |
| GPSZRA 381S2 | 1231 | AmsUmCfAmGmUmCfGfGfAmGf GmAfAmCfCmUmGmsAm | 946 | UmsCfsAmGmGmUfUmCfCmUmCm CmGmAfCmUfGmAmUmsUmsCm |
| GPSZRA 402S2 | 1232 | CmsCmAfAmCmUmCfUfCfUmCf CmUfGmUfAmUmAmsAm | 947 | UmsUfsAmUmAmCfAmGfGmAmGm AmGmAfGmUfUmGmGmsUmsCm |
| GPSZRA 407S2 | 1233 | CmsCmUfGmUmAmUfAfAfCmCf CmCfAmCfCmUmUmsGm | 948 | CmsAfsAmGmGmUfGmGfGmGmUm UmAmUfAmCfAmGmGmsAmsGm |
| GPSZRA 408S2 | 1234 | CmsUmGfUmAmUmAfAfCfCmCf CmAfCmCfUmUmGmsCm | 949 | GmsCfsAmAmGmGfUmGfGmGmGm UmUmAfUmAfCmAmGmsGmsAm |
| GPSZRA 409S2 | 1235 | UmsGmUfAmUmAmAfCfCfCmCf AmCfCmUfUmGmCmsCm | 950 | GmsGfsCmAmAmGfGmUfGmGmGm GmUmUfAmUfAmCmAmsGmsGm |
| GPSZRA 410S2 | 1236 | GmsUmAfUmAmAmCfCfCfCmAf CmCfUmUfGmCmCmsAm | 951 | UmsGfsGmCmAmAfGmGfUmGmGm GmGmUfUmAfUmAmCmsAmsGm |
| GPSZRA 411S2 | 1237 | UmsAmUfAmAmCmCfCfCfAmCf CmUfUmGfCmCmAmsAm | 952 | UmsUfsGmGmCmA fAmGfGmUmGm GmGmGfUmUfAmUmAmsCmsAm |
| GPSZRA 084S3 | 1238 | GmsGmAmAmAmGmGfAfGfAmC mCmAmAmGmUmCmCmsAm | 953 | UmsGfsGmAmCmUfUmGmGmUmC mUmCmCfUmUfUmCmCmsAmsUm |
| GPSZRA 087S3 | 1239 | AmsAmGmGmAmGmAfCfCfAmA mGmUmCmCmAmAmCmsUm | 954 | AmsGfsUmUmGmGfAmCmUmUmG mGmUmCfUmCfCmUmUmsUmsCm |
| GPSZRA 092S3 | 1240 | GmsAmCmCmAmAmGfUfCfCmA mAmCmUmAmCmCmGmsAm | 955 | UmsCfsGmGmUmAfGmUmUmGmG mAmCmUfUmGfGmUmCmsUmsCm |
| GPSZRA 093S3 | 1241 | AmsCmCmAmAmGmUfCfCfAmA mCmUmAmCmCmGmAmsGm | 956 | CmsUfsCmGmGmUfAmGmUmUmG mGmAmCfUmUfGmGmUmsCmsUm |
| GPSZRA 097S3 | 1242 | AmsGmUmCmCmAmAfCfUfAmC mCmGmAmGmUmCmCmsGm | 957 | CmsGfsGmAmCmUfCmGmGmUmAm GmUmUfGmGfAmCmUmsUmsGm |
| GPSZRA 118S3 | 1243 | CmsUmAmCmCmAmGfAfUfUmC mCmUmGmGmGmAmAmsGm | 958 | CmsUfsUmCmCmCfAmGmGmAmAm UmCmUfGmGfUmAmGmsAmsCm |
| GPSZRA 217S3 | 1244 | AmsGmGmGmAmGmUfAfUfCmU mGmUmGmAmAmGmGmsAm | 959 | UmsCfsCmUmUmCfAmCmAmGmAm UmAmCfUmCfCmCmUmsUmsCm |
| GPSZRA 222S3 | 1245 | UmsCmUmUmCmAmCfAfCfUmG mCmAmGmUmCmGmGmsAm | 960 | UmsCfsCmGmAmCfUmGmCmAmGm UmGmUfGmAfAmGmAmsGmsCm |
| GPSZRA 227S3 | 1246 | AmsCmAmCmUmGmCfAfGfUmC mGmGmAmGmCmUmAmsAm | 961 | UmsUfsAmGmCmUfCmCmGmAmCm UmGmCfAmGfUmGmUmsGmsAm |
| GPSZRA 228S3 | 1247 | CmsAmCmUmGmCmAfGfUfCmG mGmAmGmCmUmAmAmsUm | 962 | AmsUfsUmAmGmCfUmCmCmGmAm CmUmGfCmAfGmUmGmsUmsGm |
| GPSZRA 229S3 | 1248 | AmsCmUmGmCmAmGfUfCfGmG mAmGmCmUmAmAmUmsGm | 963 | CmsAfsUmUmAmGfCmUmCmCmGm AmCmUfGmCfAmGmUmsGmsUm |
| GPSZRA 231S3 | 1249 | UmsGmCmAmGmUmCfGfGfAmG mCmUmAmAmAmUmGmGmsUm | 964 | AmsCfsCmAmUmUfAmGmCmUmCm CmGmAfCmUfGmCmAmsGmsUm |
| GPSZRA 232S3 | 1250 | GmsCmAmGmUmCmGfGfAfGmC mUmAmAmUmGmGmUmsGm | 965 | CmsAfsCmCmAmUfUmAmGmCmUm CmCmGfAmCfUmGmCmsAmsGm |
| GPSZRA 233S3 | 1251 | CmsAmGmUmCmGmGfAfGfCmU mAmAmUmGmGmUmGmsAm | 966 | UmsCfsAmCmCmAfUmUmAmGmCm UmCmCfGmAfCmUmGmsCmsAm |
| GPSZRA 252S3 | 1252 | UmsCmUmGmGmAmGfGfAfGmG mUmCmCmAmAmUmUmsGm | 967 | CmsAfsAmUmUmGfGmAmCmCmUm CmCmUfCmCfAmGmAmsGmsGm |
| GPSZRA 257S3 | 1253 | AmsGmGmAmGmGmUfCfCfAmA mUmUmGmGmUmGmGmsUm | 968 | AmsCfsCmAmCmCfAmAmUmUmGm GmAmCfCmUfCmCmUmsCmsCm |
| GPSZRA 266S3 | 1254 | AmsGmCmAmGmUmAfGfCfUmC mCmUmGmGmUmGmGmsAm | 969 | UmsCfsCmAmCmCfAmGmGmAmGm CmUmAfCmUfGmCmUmsCmsCm |
| GPSZRA 267S3 | 1255 | GmsCmAmGmUmAmGfCfUfCmC mUmGmGmUmGmGmAmsAm | 970 | UmsUfsCmCmAmCfCmAmGmGmAm GmCmUfAmCfUmGmCmsUmsCm |
| GPSZRA 271S3 | 1256 | AmsGmCmUmCmCmUfGfGfUmG mGmAmAmCmCmGmUmsAm | 971 | UmsAfsCmGmGmUfUmCmCmAmCm CmAmGfGmAfGmCmUmsAmsCm |
| GPSZRA 272S3 | 1257 | GmsCmUmCmCmUmGfGfUfGmG mAmAmCmCmGmUmAmsAm | 972 | UmsUfsAmCmGmGfUmUmCmCmAm CmCmAfGmGfAmGmCmsUmsAm |
| GPSZRA 274S3 | 1258 | UmsCmCmUmGmGmUfGfGfAmA mCmCmGmUmAmAmCmsCm | 973 | GmsGfsUmUmAmCfGmGmUmUmC mCmAmCfCmAfGmGmAmsGmsCm |
| GPSZRA 275S3 | 1259 | CmsUmGmGmUmGmGfAfAfCmC mGmUmAmAmCmCmCmsUm | 974 | AmsGfsGmGmUmUfAmCmGmGmU mUmCmCfAmCfCmAmGmsGmsAm |
| GPSZRA 276S3 | 1260 | UmsGmGmUmGmGmAfAfCfCmG mUmAmAmCmCmCmUmsGm | 975 | CmsAfsGmGmGmUfUmAmCmGmG mUmUmCfCmAfCmCmAmsGmsGm |
| GPSZRA 277S3 | 1261 | GmsGmUmGmGmAmAfCfCfGmU mAmAmCmCmCmUmGmsAm | 976 | UmsCfsAmGmGmGfUmUmAmCmG mGmUmUfCmCfAmCmCmsAmsGm |
| GPSZRA 281S3 | 1262 | GmsAmAmCmCmGmUfAfAfCmC mCmUmGmAmCmCmUmsGm | 977 | CmsAfsGmGmUmCfAmGmGmGmU mUmAmCfGmGfUmUmCmsCmsAm |
| GPSZRA 282S3 | 1263 | AmsAmCmCmGmUmAfAfCfCmC mUmGmAmCmCmUmGmsUm | 978 | AmsCfsAmGmGmUfCmAmGmGmG mUmUmAfCmGfGmUmUmsCmsCm |
| GPSZRA 283S3 | 1264 | AmsCmCmGmUmAmAfCfCfCmU mGmAmCmCmCmUmGmUmsGm | 979 | CmsAfsCmAmGmGfUmCmAmGmGm GmUmUfAmCfGmGmUmsUmsCm |
| GPSZRA 284S3 | 1265 | CmsCmGmUmAmAmCfCfCfUmG mAmCmCmUmGmUmGmsAm | 980 | UmsCfsAmCmAmGfGmUmCmAmGm GmGmUfUmAfCmGmGmsUmsUm |
| GPSZRA 285S3 | 1266 | CmsGmUmAmAmCmCfCfUfGmA mCmCmUmGmUmGmAmsAm | 981 | UmsUfsCmAmCmAfGmGmUmCmAm GmGmGfUmUfAmCmGmsGmsUm |
| GPSZRA 286S3 | 1267 | GmsUmAmAmCmCmCfUfGfAmC mCmUmGmUmGmAmAmsGm | 982 | CmsUfsUmCmAmCfAmGmGmUmCm AmGmGfGmUfUmAmCmsGmsGm |
| GPSZRA 287S3 | 1268 | UmsAmAmCmCmCmUfGfAfCmC mUmGmUmGmAmAmGmsUm | 983 | AmsCfsUmUmCmAfCmAmGmGmUm CmAmGfGmGfUmUmAmsCmsGm |
| GPSZRA 288S3 | 1269 | AmsAmCmCmCmUmGfAfCfCfCmU mGmUmGmAmAmGmUmsCm | 984 | GmsAfsCmUmUmCfAmCmAmGmGm UmCmAfGmGfGmUmUmsAmsCm |
| GPSZRA 290S3 | 1270 | CmsCmUmGmAmCmCfUfGfUmG mAmAmGmUmCmCmCmsUm | 985 | AmsGfsGmGmAmCfUmUmCmAmCm AmGmGfUmCfAmGmGmsGmsUm |
| GPSZRA 292S3 | 1271 | UmsGmAmCmCmUmGfUfGfAmA mGmUmCmCmCmUmGmsCm | 986 | GmsCfsAmGmGmGfAmCmUmUmCm AmCmAfGmGfUmCmAmsGmsGm |
| GPSZRA 301S3 | 1272 | UmsCmUmCmCmUmCfAfAfAmU mCmCmAmCmUmGmGmsAm | 987 | UmsCfsCmAmGmUfGmGmAmUmU mUmGmAfGmGfAmGmAmsGmsGm |
| GPSZRA 308S3 | 1273 | AmsAmAmUmCmCmAfCfUfGmG mAmUmGmAmAmGmGmsAm | 988 | UmsCfsCmUmUmCfAmUmCmCmAm GmUmGfGmAfUmUmUmsGmsAm |
| GPSZRA 311S3 | 1274 | UmsCmCmAmCmUmGfGfAfUmG mAmAmGmGmAmUmGmsGm | 989 | CmsCfsAmUmCmCfUmUmCmAmUm CmCmAfGmUfGmGmAmsUmsUm |
| GPSZRA 312S3 | 1275 | CmsCmAmCmUmGmGfAfUfGmA mAmGmGmAmUmGmGmsUm | 990 | AmsCfsCmAmUmCfCmUmUmCmAm UmCmCfAmGfUmGmGmsAmsUm |
| GPSZRA 313S3 | 1276 | CmsAmCmUmGmGmAfUfGfAmA mGmGmAmUmGmGmUmsGm | 991 | CmsAfsCmCmAmUfCmCmUmUmCm AmUmCfCmAfGmUmGmsGmsAm |
| GPSZRA 314S3 | 1277 | AmsCmUmGmGmAmUfGfAfAmG mGmAmUmGmGmUmGmsUm | 992 | AmsCfsAmCmCmAfUmCmCmUmUm CmAmUfCmCfAmGmUmsGmsGm |
| GPSZRA 315S3 | 1278 | CmsUmGmGmAmUmGfAfAfGmG mAmUmGmGmUmGmUmsGm | 993 | CmsAfsCmAmCmCfAmUmCmCmUm UmCmAfUmCfCmAmGmsUmsGm |
| GPSZRA 316S3 | 1279 | UmsGmGmAmUmGmAfAfGfGmA mUmGmGmUmGmUmGmsCm | 994 | GmsCfsAmCmAmCfCmAmUmCmCm UmUmCfAmUfCmCmAmsGmsUm |
| GPSZRA 317S3 | 1280 | GmsGmAmUmGmAmAfGfGfAmU mGmGmUmGmUmGmUmGmCmsCm | 995 | GmsGfsCmAmCmAfCmCmAmUmCm CmUmUfCmAfUmCmCmsAmsGm |
| GPSZRA 318S3 | 1281 | GmsAmUmGmAmAmGfGfAfUmG mGmUmGmUmGmCmCmsCm | 996 | GmsGfsGmCmAmCfAmCmCmAmUm CmCmUfUmCfAmUmCmsCmsAm |
| GPSZRA 320S3 | 1282 | UmsGmAmAmGmGmAfUfGfGmU mGmUmGmCmCmCmUmsUm | 997 | AmsAfsGmGmGmCfAmCmAmCmCm AmUmCfCmUfUmCmAmsUmsCm |
| GPSZRA 333S3 | 1283 | GmsAmAmAmGmCmCfGfUfGmC mUmGmUmCmAmGmCmsAm | 998 | UmsGfsCmUmGmAfCmAmGmCmAm CmGmGfCmUfUmUmCmsCmsUm |
| GPSZRA 342S3 | 1284 | UmsGmUmCmAmGmCfAfUfCmA mGmCmAmUmCmAmUmsCm | 999 | GmsAfsUmGmAmUfGmCmUmGmA mUmGmCfUmGfAmCmAmsGmsCm |
| GPSZRA 378S3 | 1285 | AmsAmGmAmGmGmAfGfGfAmG mCmGmUmGmCmAmGmsAm | 1000 | UmsCfsUmGmCmAfCmGmCmUmCm CmUmCfCmUfCmUmUmsCmsCm |
| GPSZRA 379S3 | 1286 | GmsAmAmUmCmAmGfUfCfGmG mAmGmGmAmAmCmCmsUm | 1001 | AmsGfsGmUmUmCfCmUmCmCmGm AmCmUfGmAfUmUmCmsAmsGm |
| GPSZRA 380S3 | 1287 | AmsAmUmCmAmGmUfCfGfGmA mGmGmAmAmCmCmUmsGm | 1002 | CmsAfsGmGmUmUfCmCmUmCmCm GmAmCfUmGfAmUmUmsCmsAm |
| GPSZRA 381S3 | 1288 | AmsUmCmAmGmUmCfGfGfAmG mGmAmAmCmCmUmGmsAm | 1003 | UmsCfsAmGmGmUfUmCmCmUmCm CmGmAfCmUfGmAmUmsUmsCm |
| GPSZRA 402S3 | 1289 | CmsCmAmAmCmUmCfUfCfUmC mCmUmGmUmAmUmAmsAm | 1004 | UmsUfsAmUmAmCfAmGmGmAmG mAmGmAfGmUfUmGmGmsUmsCm |
| GPSZRA 407S3 | 1290 | CmsCmUmGmUmAmUfAfAfCmC mCmCmAmCmCmUmUmsGm | 1005 | CmsAfsAmGmGmUfGmGmGmGmU mUmAmUfAmCfAmGmGmsAmsGm |
| GPSZRA 408S3 | 1291 | CmsUmGmUmAmUmAfAfCfCmC mCmAmCmCmUmUmGmsCm | 1006 | GmsCfsAmAmGmGfUmGmGmGmG mUmUmAfUmAfCmAmGmsGmsAm |
| GPSZRA 409S3 | 1292 | UmsGmUmAmUmAmAfCfCfCmC mAmCmCmUmUmGmCmsCm | 1007 | GmsGfsCmAmAmGfGmUmGmGmG mGmUmUfAmUfAmCmAmsGmsGm |
| GPSZRA 410S3 | 1293 | GmsUmAmUmAmAmCfCfCfCmA mCmCmUmUmGmCmCmsAm | 1008 | UmsGfsGmCmAmAfGmGmUmGmG mGmGmUfUmAfUmAmCmsAmsGm |
| GPSZRA 411S3 | 1294 | UmsAmUmAmAmCmCfCfCfAmC mCmUmUmGmCmCmAmsAm | 1009 | UmsUfsGmGmCmAfAmGmGmUmG mGmGmGfUmUfAmUmAmsCmsAm |
| GPSZRA 084S4 | 1295 | G(LNA)sGmAfAmAmGmGfAfGfA mCfCmAfAmGfUmCmCmsAm | 1010 | UmsGfsGmAmCmUfUmGfGmUmCm UmCmCfUmUfUmCmCmsAmsUm |
| GPSZRA 087S4 | 1296 | A(LNA)sAmGfGmAmGmAfCfCfA mAfGmUfCmCfAmAmCmsUm | 1011 | AmsGfsUmUmGmGfAmCfUmUmGm GmUmCfUmCfCmUmUmsUmsCm |
| GPSZRA 092S4 | 1297 | G(LNA)sAmCfCmAmAmGfUfCfC mAfAmCfUmAfCmCmGmsAm | 1012 | UmsCfsGmGmUmAfGmUfUmGmGm AmCmUfUmGfGmUmCmsUmsCm |
| GPSZRA 093S4 | 1298 | A(LNA)sCmCfAmAmGmUfCfCfA mAfCmUfAmCfCmGmAmsGm | 1013 | CmsUfsCmGmGmUfAmGfUmUmGm GmAmCfUmUfGmGmUmsCmsUm |
| GPSZRA 097S4 | 1299 | A(LNA)sGmUfCmCmAmAfCfUfA mCfCmGfAmGfUmCmCmsGm | 1014 | CmsGfsGmAmCmUfCmGfGmUmAm GmUmUfGmGfAmCmUmsUmsGm |
| GPSZRA 118S4 | 1300 | C(LNA)sUmAfCmCmAmGfAfUfU mCfCmUfGmGfGmAmAmsGm | 1015 | CmsUfsUmCmCmCfAmGfGmAmAm UmCmUfGmGfUmAmGmsAmsCm |
| GPSZRA 217S4 | 1301 | A(LNA)sGmGfGmAmGmUfAfUfC mUfGmUfGmAfAmGmGmsAm | 1016 | UmsCfsCmUmUmCfAmCfAmGmAm UmAmCfUmCfCmCmUmsUmsCm |
| GPSZRA 222S4 | 1302 | U(LNA)sCmUfUmCmAmCfAfCfU mGfCmAfGmUfCmGmGmsAm | 1017 | UmsCfsCmGmAmCfUmGfCmAmGm UmGmUfGmAfAmGmAmsGmsCm |
| GPSZRA 227S4 | 1303 | A(LNA)sCmAfCmUmGmCfAfGfU mCfGmGfAmGfCmUmAmsAm | 1018 | UmsUfsAmGmCmUfCmCfGmAmCm UmGmCfAmGfUmGmUmsGmsAm |
| GPSZRA 228S4 | 1304 | C(LNA)sAmCfUmGmCmAfGfUfC mGfGmAfGmCfUmAmAmsUm | 1019 | AmsUfsUmAmGmCfUmCfCmGmAm CmUmGfCmAfGmUmGmsUmsGm |
| GPSZRA 229S4 | 1305 | A(LNA)sCmUfGmCmAmGfUfCfG mGfAmGfCmUfAmAmUmsGm | 1020 | CmsAfsUmUmAmGfCmUfCmCmGm AmCmUfGmCfAmGmUmsGmsUm |
| GPSZRA 231S4 | 1306 | U(LNA)sGmCfAmGmUmCfGfGfA mGfCmUfAmAfUmGmGmsUm | 1021 | AmsCfsCmAmUmUfAmGfCmUmCm CmGmAfCmUfGmCmAmsGmsUm |
| GPSZRA 232S4 | 1307 | G(LNA)sCmAfGmUmCmGfGfAfG mCfUmAfAmUfGmGmUmsGm | 1022 | CmsAfsCmCmAmUfUmAfGmCmUm CmCmGfAmCfUmGmCmsAmsGm |
| GPSZRA 233S4 | 1308 | C(LNA)sAmGfUmCmGmGfAfGfC mUfAmAfUmGfGmUmGmsAm | 1023 | UmsCfsAmCmCmAfUmUfAmGmCm UmCmCfGmAfCmUmGmsCmsAm |
| GPSZRA 252S4 | 1309 | U(LNA)sCmUfGmGmAmGfGfAfG mGfUmCfCmAfAmUmUmsGm | 1024 | CmsAfsAmUmUmGfGmAfCmCmUm CmCmUfCmCfAmGmAmsGmsGm |
| GPSZRA 257S4 | 1310 | A(LNA)sGmGfAmGmGmUfCfCfA mAfUmUfGmGfUmGmGmsUm | 1025 | AmsCfsCmAmCmCfAmAfUmUmGm GmAmCfCmUfCmCmUmsCmsCm |
| GPSZRA 266S4 | 1311 | A(LNA)sGmCfAmGmUmAfGfCfU mCfCmUfGmGfUmGmGmsAm | 1026 | UmsCfsCmAmCmCfAmGfGmAmGm CmUmAfCmUfGmCmUmsCmsCm |
| GPSZRA 267S4 | 1312 | G(LNA)sCmAfGmUmAmGfCfUfC mCfUmGfGmUfGmGmAmsAm | 1027 | UmsUfsCmCmAmCfCmAfGmGmAm GmCmUfAmCfUmGmCmsUmsCm |
| GPSZRA 271S4 | 1313 | A(LNA)sGmCfUmCmCmUfGfGfU mGfGmAfAmCfCmGmUmsAm | 1028 | UmsAfsCmGmGmUfUmCfCmAmCm CmAmGfGmAfGmCmUmsAmsCm |
| GPSZRA 272S4 | 1314 | G(LNA)sCmUfCmCmUmGfGfUfG mGfAmAfCmCfGmUmAmsAm | 1029 | UmsUfsAmCmGmGfUmUfCmCmAm CmCmAfGmGfAmGmCmsUmsAm |
| GPSZRA 274S4 | 1315 | U(LNA)sCmCfUmGmGmUfGfGfA mAfCmCfGmUfAmAmCmsCm | 1030 | GmsGfsUmUmAmCfGmGfUmUmCm CmAmCfCmAfGmGmAmsGmsCm |
| GPSZRA 275S4 | 1316 | C(LNA)sUmGfGmUmGmGfAfAfC mCfGmUfAmAfCmCmCmsUm | 1031 | AmsGfsGmGmUmUfAmCfGmGmUm UmCmCfAmCfCmAmGmsGmsAm |
| GPSZRA 276S4 | 1317 | U(LNA)sGmGfUmGmGmAfAfCfC mGfUmAfAmCfCmCmUmsGm | 1032 | CmsAfsGmGmGmUfUmAfCmGmGm UmUmCfCmAfCmCmAmsGmsGm |
| GPSZRA 277S4 | 1318 | G(LNA)sGmUfGmGmAmAfCfCfG mUfAmAfCmCfCmUmGmsAm | 1033 | UmsCfsAmGmGmGfUmUfAmCmGm GmUmUfCmCfAmCmCmsAmsGm |
| GPSZRA 281S4 | 1319 | G(LNA)sAmAfCmCmGmUfAfAfC mCfCmUfGmAfCmCmUmsGm | 1034 | CmsAfsGmGmUmCfAmGfGmGmUm UmAmCfGmGfUmUmCmsCmsAm |
| GPSZRA 282S4 | 1320 | A(LNA)sAmCfCmGmUmAfAfCfC mCfUmGfAmCfCmUmGmsUm | 1035 | AmsCfsAmGmGmUfCmAfGmGmGm UmUmAfCmGfGmUmUmsCmsCm |
| GPSZRA 283S4 | 1321 | A(LNA)sCmCfGmUmAmAfCfCfC mUfGmAfCmCfUmGmUmsGm | 1036 | CmsAfsCmAmGmGfUmCfAmGmGm GmUmUfAmCfGmGmUmsUmsCm |
| GPSZRA 284S4 | 1322 | C(LNA)sCmGfUmAmAmCfCfCfU mGfAmCfCmUfGmUmGmsAm | 1037 | UmsCfsAmCmAmGfGmUfCmAmGm GmGmUfUmAfCmGmGmsUmsUm |
| GPSZRA 285S4 | 1323 | C(LNA)sGmUfAmAmCmCfCfUfG mAfCmCfUmGfUmGmAmsAm | 1038 | UmsUfsCmAmCmAfGmGfUmCmAm GmGmGfUmUfAmCmGmsGmsUm |
| GPSZRA 286S4 | 1324 | G(LNA)sUmAfAmCmCmCfUfGfA mCfCmUfGmUfGmAmAmsGm | 1039 | CmsUfsUmCmAmCfAmGfGmUmCm AmGmGfGmUfUmAmCmsGmsGm |
| GPSZRA 287S4 | 1325 | U(LNA)sAmAfCmCmCmUfGfAfC mCfUmGfUmGfAmAmGmsUm | 1040 | AmsCfsUmUmCmAfCmAfGmGmUm CmAmGfGmGfUmUmAmsCmsGm |
| GPSZRA 288S4 | 1326 | A(LNA)sAmCfCmCmUmGfAfCfC mUfGmUfGmAfAmGmUmsCm | 1041 | GmsAfsCmUmUmCfAmCfAmGmGm UmCmAfGmGfGmUmUmsAmsCm |
| GPSZRA 290S4 | 1327 | C(LNA)sCmUfGmAmCmCfUfGfU mGfAmAfGmUfCmCmCmsUm | 1042 | AmsGfsGmGmAmCfUmUfCmAmCm AmGmGfUmCfAmGmGmsGmsUm |
| GPSZRA 292S4 | 1328 | U(LNA)sGmAfCmCmUmGfUfGfA mAfGmUfCmCfCmUmGmsCm | 1043 | GmsCfsAmGmGmGfAmCfUmUmCm AmCmAfGmGfUmCmAmsGmsGm |
| GPSZRA 301S4 | 1329 | U(LNA)sCmUfCmCmUmCfAfAfA mUfCmCfAmCfUmGmGmsAm | 1044 | UmsCfsCmAmGmUfGmGfAmUmUm UmGmAfGmGfAmGmAmsGmsGm |
| GPSZRA 308S4 | 1330 | A(LNA)sAmAfUmCmCmAfCfUfG mGfAmUfGmAfAmGmGmsAm | 1045 | UmsCfsCmUmUmCfAmUfCmCmAm GmUmGfGmAfUmUmUmsGmsAm |
| GPSZRA 311S4 | 1331 | U(LNA)sCmCfAmCmUmGfGfAfU mGfAmAfGmGfAmUmGmsGm | 1046 | CmsCfsAmUmCmCfUmUfCmAmUm CmCmAfGmUfGmGmAmsUmsUm |
| GPSZRA 312S4 | 1332 | C(LNA)sCmAfCmUmGmGfAfUfG mAfAmGfGmAfUmGmGmsUm | 1047 | AmsCfsCmAmUmCfCmUfUmCmAm UmCmCfAmGfUmGmGmsAmsUm |
| GPSZRA 313S4 | 1333 | C(LNA)sAmCfUmGmGmAfUfGfA mAfGmGfAmUfGmGmUmsGm | 1048 | CmsAfsCmCmAmUfCmCfUmUmCm AmUmCfCmAfGmUmGmsGmsAm |
| GPSZRA 314S4 | 1334 | A(LNA)sCmUfGmGmAmUfGfAfA mGfGmAfUmGfGmUmGmsUm | 1049 | AmsCfsAmCmCmAfUmCfCmUmUm CmAmUfCmCfAmGmUmsGmsGm |
| GPSZRA 315S4 | 1335 | C(LNA)sUmGfGmAmUmGfAfAfG mGfAmUfGmGfUmGmUmsGm | 1050 | CmsAfsCmAmCmCfAmUfCmCmUm UmCmAfUmCfCmAmGmsUmsGm |
| GPSZRA 316S4 | 1336 | U(LNA)sGmGfAmUmGmAfAfGfG mAfUmGfGmUfGmUmGmsCm | 1051 | GmsCfsAmCmAmCfCmAfUmCmCm UmUmCfAmUfCmCmAmsGmsUm |
| GPSZRA 317S4 | 1337 | G(LNA)sGmAfUmGmAmAfGfGfA mUfGmGfUmGfUmGmCmsCm | 1052 | GmsGfsCmAmCmAfCmCfAmUmCm CmUmUfCmAfUmCmCmsAmsGm |
| GPSZRA 318S4 | 1338 | G(LNA)sAmUfGmAmAmGfGfAfU mGfGmUfGmUfGmCmCmsCm | 1053 | GmsGfsGmCmAmCfAmCfCmAmUm CmCmUfUmCfAmUmCmsCmsAm |
| GPSZRA 320S4 | 1339 | U(LNA)sGmAfAmGmGmAfUfGfG mUfGmUfGmCfCmCmUmsUm | 1054 | AmsAfsGmGmGmCfAmCfAmCmCm AmUmCfCmUfUmCmAmsUmsCm |
| GPSZRA 333S4 | 1340 | G(LNA)sAmAfAmGmCmCfGfUfG mCfUmGfUmCfAmGmCmsAm | 1055 | UmsGfsCmUmGmAfCmAfGmCmAm CmGmGfCmUfUmUmCmsCmsUm |
| GPSZRA 342S4 | 1341 | U(LNA)sGmUfCmAmGmCfAfUfC mAfGmCfAmUfCmAmUmsCm | 1056 | GmsAfsUmGmAmUfGmCfUmGmAm UmGmCfUmGfAmCmAmsGmsCm |
| GPSZRA 378S4 | 1342 | A(LNA)sAmGfAmGmGmAfGfGfA mGfCmGfUmGfCmAmGmsAm | 1057 | UmsCfsUmGmCmAfCmGfCmUmCm CmUmCfCmUfCmUmUmsCmsCm |
| GPSZRA 379S4 | 1343 | G(LNA)sAmAfUmCmAmGfUfCfG mGfAmGfGmAfAmCmCmsUm | 1058 | AmsGfsGmUmUmCfCmUfCmCmGm AmCmUfGmAfUmUmCmsAmsGm |
| GPSZRA 380S4 | 1344 | A(LNA)sAmUfCmAmGmUfCfGfG mAfGmGfAmAfCmCmUmsGm | 1059 | CmsAfsGmGmUmUfCmCfUmCmCm GmAmCfUmGfAmUmUmsCmsAm |
| GPSZRA 381S4 | 1345 | A(LNA)sUmCfAmGmUmCfGfGfA mGfGmAfAmCfCmUmGmsAm | 1060 | UmsCfsAmGmGmUfUmCfCmUmCm CmGmAfCmUfGmAmUmsUmsCm |
| GPSZRA 402S4 | 1346 | C(LNA)sCmAfAmCmUmCfUfCfU mCfCmUfGmUfAmUmAmsAm | 1061 | UmsUfsAmUmAmCfAmGfGmAmGm AmGmAfGmUfUmGmGmsUmsCm |
| GPSZRA 407S4 | 1347 | C(LNA)sCmUfGmUmAmUfAfAfC mCfCmCfAmCfCmUmUmsGm | 1062 | CmsAfsAmGmGmUfGmGfGmGmUm UmAmUfAmCfAmGmGmsAmsGm |
| GPSZRA 408S4 | 1348 | C(LNA)sUmGfUmAmUmAfAfCfC mCfCmAfCmCfUmUmGmsCm | 1063 | GmsCfsAmAmGmGfUmGfGmGmGm UmUmAfUmAfCmAmGmsGmsAm |
| GPSZRA 409S4 | 1349 | U(LNA)sGmUfAmUmAmAfCfCfC mCfAmCfCmUfUmGmCmsCm | 1064 | GmsGfsCmAmAmGfGmUfGmGmGm GmUmUfAmUfAmCmAmsGmsGm |
| GPSZRA 410S4 | 1350 | G(LNA)sUmAfUmAmAmCfCfCfC mAfCmCfUmUfGmCmCmsAm | 1065 | UmsGfsGmCmAmAfGmGfUmGmGm GmGmUfUmAfUmAmCmsAmsGm |
| GPSZRA 411S4 | 1351 | U(LNA)sAmUfAmAmCmCfCfCfA mCfCmUfUmGfCmCmAmsAm | 1066 | UmsUfsGmGmCmAfAmGfGmUmGm GmGmGfUmUfAmUmAmsCmsAm |
| GPSZRA 084S5 | 1352 | GmsGmAmAmAfGmGfAfGfAmC mCmAmAmGfUmCmCmsAm | 1067 | UmsGfsGmAmCmUfUmGfGmUmCm UmCmCfUmUfUmCmCmsAmsUm |
| GPSZRA 087S5 | 1353 | AmsAmGmGmAfGmAfCfCfAmA mGmUmCmCfAmAmCmsUm | 1068 | AmsGfsUmUmGmGfAmCfUmUmGm GmUmCfUmCfCmUmUmsUmsCm |
| GPSZRA 092S5 | 1354 | GmsAmCmCmAfAmGfUfCfCmA mAmCmUmAfCmCmGmsAm | 1069 | UmsCfsGmGmUmAfGmUfUmGmGm AmCmUfUmGfGmUmCmsUmsCm |
| GPSZRA 093S5 | 1355 | AmsCmCmAmAfGmUfCfCfAmA mCmUmAmCfCmGmAmsGm | 1070 | CmsUfsCmGmGmUfAmGfUmUmGm GmAmCfUmUfGmGmUmsCmsUm |
| GPSZRA 097S5 | 1356 | AmsGmUmCmCfAmAfCfUfAmC mCmGmAmGfUmCmCmsGm | 1071 | CmsGfsGmAmCmUfCmGfGmUmAm GmUmUfGmGfAmCmUmsUmsGm |
| GPSZRA 118S5 | 1357 | CmsUmAmCmCfAmGfAfUfUmC mCmUmGmGfGmAmAmsGm | 1072 | CmsUfsUmCmCmCfAmGfGmAmAm UmCmUfGmGfUmAmGmsAmsCm |
| GPSZRA 217S5 | 1358 | AmsGmGmGmAfGmUfAfUfCmU mGmUmGmAfAmGmGmsAm | 1073 | UmsCfsCmUmUmCfAmCfAmGmAm UmAmCfUmCfCmCmUmsUmsCm |
| GPSZRA 222S5 | 1359 | UmsCmUmUmCfAmCfAfCfUmG mCmAmGmUfCmGmGmsAm | 1074 | UmsCfsCmGmAmCfUmGfCmAmGm UmGmUfGmAfAmGmAmsGmsCm |
| GPSZRA 227S5 | 1360 | AmsCmAmCmUfGmCfAfGfUmC mGmGmAmGfCmUmAmsAm | 1075 | UmsUfsAmGmCmUfCmCfGmAmCm UmGmCfAmGfUmGmUmsGmsAm |
| GPSZRA 228S5 | 1361 | CmsAmCmUmGfCmAfGfUfCmG mGmAmGmCfUmAmAmsUm | 1076 | AmsUfsUmAmGmCfUmCfCmGmAm CmUmGfCmAfGmUmGmsUmsGm |
| GPSZRA 229S5 | 1362 | AmsCmUmGmCfAmGfUfCfGmG mAmGmCmUfAmAmUmsGm | 1077 | CmsAfsUmUmAmGfCmUfCmCmGm AmCmUfGmCfAmGmUmsGmsUm |
| GPSZRA 231S5 | 1363 | UmsGmCmAmGfUmCfGfGfAmG mCmUmAmAfUmGmGmsUm | 1078 | AmsCfsCmAmUmUfAmGfCmUmCm CmGmAfCmUfGmCmAmsGmsUm |
| GPSZRA 232S5 | 1364 | GmsCmAmGmUfCmGfGfAfGmC mUmAmAmUfGmGmUmsGm | 1079 | CmsAfsCmCmAmUfUmAfGmCmUm CmCmGfAmCfUmGmCmsAmsGm |
| GPSZRA 233S5 | 1365 | CmsAmGmUmCfGmGfAfGfCmU mAmAmUmGfGmUmGmsAm | 1080 | UmsCfsAmCmCmAfUmUfAmGmCm UmCmCfGmAfCmUmGmsCmsAm |
| GPSZRA 252S5 | 1366 | UmsCmUmGmGfAmGfGfAfGmG mUmCmCmAfAmUmUmsGm | 1081 | CmsAfsAmUmUmGfGmAfCmCmUm CmCmUfCmCfAmGmAmsGmsGm |
| GPSZRA 257S5 | 1367 | AmsGmGmAmGfGmUfCfCfAmA mUmUmGmGfUmGmGmsUm | 1082 | AmsCfsCmAmCmCfAmAfUmUmGm GmAmCfCmUfCmCmUmsCmsCm |
| GPSZRA 266S5 | 1368 | AmsGmCmAmGfUmAfGfCfUmC mCmUmGmGfUmGmGmsAm | 1083 | UmsCfsCmAmCmCfAmGfGmAmGm CmUmAfCmUfGmCmUmsCmsCm |
| GPSZRA 267S5 | 1369 | GmsCmAmGmUfAmGfCfUfCmC mUmGmGmUfGmGmAmsAm | 1084 | UmsUfsCmCmAmCfCmAfGmGmAm GmCmUfAmCfUmGmCmsUmsCm |
| GPSZRA 271S5 | 1370 | AmsGmCmUmCfCmUfGfGfUmG mGmAmAmCfCmGmUmsAm | 1085 | UmsAfsCmGmGmUfUmCfCmAmCm CmAmGfGmAfGmCmUmsAmsCm |
| GPSZRA 272S5 | 1371 | GmsCmUmCmCfUmGfGfUfGmG mAmAmCmCfGmUmAmsAm | 1086 | UmsUfsAmCmGmGfUmUfCmCmAm CmCmAfGmGfAmGmCmsUmsAm |
| GPSZRA 274S5 | 1372 | UmsCmCmUmGfGmUfGfGfAmA mCmCmGmUfAmAmCmsCm | 1087 | GmsGfsUmUmAmCfGmGfUmUmCm CmAmCfCmAfGmGmAmsGmsCm |
| GPSZRA 275S5 | 1373 | CmsUmGmGmUfGmGfAfAfCmC mGmUmAmAfCmCmCmsUm | 1088 | AmsGfsGmGmUmUfAmCfGmGmUm UmCmCfAmCfCmAmGmsGmsAm |
| GPSZRA 276S5 | 1374 | UmsGmGmUmGfGmAfAfCfCmG mUmAmAmCfCmCmUmsGm | 1089 | CmsAfsGmGmGmUfUmAfCmGmGm UmUmCfCmAfCmCmAmsGmsGm |
| GPSZRA 277S5 | 1375 | GmsGmUmGmGfAmAfCfCfGmU mAmAmCmCfCmUmGmsAm | 1090 | UmsCfsAmGmGmGfUmUfAmCmGm GmUmUfCmCfAmCmCmsAmsGm |
| GPSZRA 281S5 | 1376 | GmsAmAmCmCfGmUfAfAfCmC mCmUmGmAfCmCmUmsGm | 1091 | CmsAfsGmGmUmCfAmGfGmGmUm UmAmCfGmGfUmUmCmsCmsAm |
| GPSZRA 282S5 | 1377 | AmsAmCmCmGfUmAfAfCfCmC mUmGmAmCfCmUmGmsUm | 1092 | AmsCfsAmGmGmUfCmAfGmGmGm UmUmAfCmGfGmUmUmsCmsCm |
| GPSZRA 283S5 | 1378 | AmsCmCmGmUfAmAfCfCfCmU mGmAmCmCfUmGmUmsGm | 1093 | CmsAfsCmAmGmGfUmCfAmGmGm GmUmUfAmCfGmGmUmsUmsCm |
| GPSZRA 284S5 | 1379 | CmsCmGmUmAfAmCfCfCfUmG mAmCmCmUfGmUmGmsAm | 1094 | UmsCfsAmCmAmGfGmUfCmAmGm GmGmUfUmAfCmGmGmsUmsUm |
| GPSZRA 285S5 | 1380 | CmsGmUmAmAfCmCfCfUfGmA mCmCmUmGfUmGmAmsAm | 1095 | UmsUfsCmAmCmAfGmGfUmCmAm GmGmGfUmUfAmCmGmsGmsUm |
| GPSZRA 286S5 | 1381 | GmsUmAmAmCfCmCfUfGfAmC mCmUmGmUfGmAmAmsGm | 1096 | CmsUfsUmCmAmCfAmGfGmUmCm AmGmGfGmUfUmAmCmsGmsGm |
| GPSZRA 287S5 | 1392 | UmsAmAmCmCfCmUfGfAfCmC mUmGmUmGfAmAmGmsUm | 1097 | AmsCfsUmUmCmAfCmAfGmGmUm CmAmGfGmGfUmUmAmsCmsGm |
| GPSZRA 288S5 | 1383 | AmsAmCmCmCfUmGfAfCfCmU mGmUmGmAfAmGmUmsCm | 1098 | GmsAfsCmUmUmCfAmCfAmGmGm UmCmAfGmGfGmUmUmsAmsCm |
| GPSZRA 290S5 | 1384 | CmsCmUmGmAfCmCfUfGfUmG mAmAmGmUfCmCmCmsUm | 1099 | AmsGfsGmGmAmCfUmUfCmAmCm AmGmGfUmCfAmGmGmsGmsUm |
| GPSZRA 292S5 | 1385 | UmsGmAmCmCfUmGfUfGfAmA mGmUmCmCfCmUmGmsCm | 1100 | GmsCfsAmGmGmGfAmCfUmUmCm AmCmAfGmGfUmCmAmsGmsGm |
| GPSZRA 301S5 | 1386 | UmsCmUmCmCfUmCfAfAfAmU mCmCmAmCfUmGmGmsAm | 1101 | UmsCfsCmAmGmUfGmGfAmUmUm UmGmAfGmGfAmGmAmsGmsGm |
| GPSZRA 308S5 | 1387 | AmsAmAmUmCfCmAfCfUfGmG mAmUmGmAfAmGmGmsAm | 1102 | UmsCfsCmUmUmCfAmUfCmCmAm GmUmGfGmAfUmUmUmsGmsAm |
| GPSZRA 311S5 | 1388 | UmsCmCmAmCfUmGfGfAfUmG mAmAmGmGfAmUmGmsGm | 1103 | CmsCfsAmUmCmCfUmUfCmAmUm CmCmAfGmUfGmGmAmsUmsUm |
| GPSZRA 312S5 | 1389 | CmsCmAmCmUfGmGfAfUfGmA mAmGmGmAfUmGmGmsUm | 1104 | AmsCfsCmAmUmCfCmUfUmCmAm UmCmCfAmGfUmGmGmsAmsUm |
| GPSZRA 313S5 | 1390 | CmsAmCmUmGfGmAfUfGfAmA mGmGmAmUfGmGmUmsGm | 1105 | CmsAfsCmCmAmUfCmCfUmUmCm AmUmCfCmAfGmUmGmsGmsAm |
| GPSZRA 314S5 | 1391 | AmsCmUmGmGfAmUfGfAfAmG mGmAmUmGfGmUmGmsUm | 1106 | AmsCfsAmCmCmAfUmCfCmUmUm CmAmUfCmCfAmGmUmsGmsGm |
| GPSZRA 315S5 | 1392 | CmsUmGmGmAfUmGfAfAfGmG mAmUmGmGfUmGmUmsGm | 1107 | CmsAfsCmAmCmCfAmUfCmCmUm UmCmAfUmCfCmAmGmsUmsGm |
| GPSZRA 316S5 | 1393 | UmsGmGmAmUfGmAfAfGfGmA mUmGmGmUfGmUmGmsCm | 1108 | GmsCfsAmCmAmCfCmAfUmCmCm UmUmCfAmUfCmCmAmsGmsUm |
| GPSZRA 317S5 | 1394 | GmsGmAmUmGfAmAfGfGfAmU mGmGmUmGfUmGmCmsCm | 1109 | GmsGfsCmAmCmAfCmCfAmUmCm CmUmUfCmAfUmCmCmsAmsGm |
| GPSZRA 318S5 | 1395 | GmsAmUmGmAfAmGfGfAfUmG mGmUmGmUfGmCmCmsCm | 1110 | GmsGfsGmCmAmCfAmCfCmAmUm CmCmUfUmCfAmUmCmsCmsAm |
| GPSZRA 320S5 | 1396 | UmsGmAmAmGfGmAfUfGfGmU mGmUmGmCfCmCmUmsUm | 1111 | AmsAfsGmGmGmCfAmCfAmCmCm AmUmCfCmUfUmCmAmsUmsCm |
| GPSZRA 333S5 | 1397 | GmsAmAmAmGfCmCfGfUfGmC mUmGmUmCfAmGmCmsAm | 1112 | UmsGfsCmUmGmAfCmAfGmCmAm CmGmGfCmUfUmUmCmsCmsUm |
| GPSZRA 342S5 | 1398 | UmsGmUmCmAfGmCfAfUfCmA mGmCmAmUfCmAmUmsCm | 1113 | GmsAfsUmGmAmUfGmCfUmGmAm UmGmCfUmGfAmCmAmsGmsCm |
| GPSZRA 378S5 | 1399 | AmsAmGmAmGfGmAfGfGfAmG mCmGmUmGfCmAmGmsAm | 1114 | UmsCfsUmGmCmAfCmGfCmUmCm CmUmCfCmUfCmUmUmsCmsCm |
| GPSZRA 379S5 | 1400 | GmsAmAmUmCfAmGfUfCfGmG mAmGmGmAfAmCmCmsUm | 1115 | AmsGfsGmUmUmCfCmUfCmCmGm AmCmUfGmAfUmUmCmsAmsGm |
| GPSZRA 380S5 | 1401 | AmsAmUmCmAfGmUfCfGfGmA mGmGmAmAfCmCmUmsGm | 1116 | CmsAfsGmGmUmUfCmCfUmCmCm GmAmCfUmGfAmUmUmsCmsAm |
| GPSZRA 381S5 | 1402 | AmsUmCmAmGfUmCfGfGfAmG mGmAmAmCfCmUmGmsAm | 1117 | UmsCfsAmGmGmUfUmCfCmUmCm CmGmAfCmUfGmAmUmsUmsCm |
| GPSZRA 402S5 | 1403 | CmsCmAmAmCfUmCfUfCfUmCm CmUmGmUfAmUmAmsAm | 1118 | UmsUfsAmUmAmCfAmGfGmAmGm AmGmAfGmUfUmGmGmsUmsCm |
| GPSZRA 407S5 | 1404 | CmsCmUmGmUfAmUfAfAfCmC mCmCmAmCfCmUmUmsGm | 1119 | CmsAfsAmGmGmUfGmGfGmGmUm UmAmUfAmCfAmGmGmsAmsGm |
| GPSZRA 408S5 | 1405 | CmsUmGmUmAfUmAfAfCfCmC mCmAmCmCfUmUmGmsCm | 1120 | GmsCfsAmAmGmGfUmGfGmGmGm UmUmAfUmAfCmAmGmsGmsAm |
| GPSZRA 409S5 | 1406 | UmsGmUmAmUfAmAfCfCfCmC mAmCmCmUfUmGmCmsCm | 1121 | GmsGfsCmAmAmGfGmUfGmGmGm GmUmUfAmUfAmCmAmsGmsGm |
| GPSZRA 410S5 | 1407 | GmsUmAmUmAfAmCfCfCfCmA mCmCmUmUfGmCmCmsAm | 1122 | UmsGfsGmCmAmAfGmGfUmGmGm GmGmUfUmAfUmAmCmsAmsGm |
| GPSZRA 411S5 | 1408 | UmsAmUmAmAfCmCfCfCfAmC mCmUmUmGfCmCmAmsAm | 1123 | UmsUfsGmGmCmAfAmGfGmUmGm GmGmGfUmUfAmUmAmsCmsAm |
| GPSZRA 222S1-VP | 1131 | UmsCmUfUmCfAmCfAfCfUmGfC mAfGmUfCmGfGmsAf | 1426 | VPUmsCfsCmGfAmCfUmGfCmAfGm UmGmUfGmAfAmGfAmsGmsCm |
| GPSZRA 222S3-VP | 1245 | UmsCmUmUmCmAmCfAfCfUmG mCmAmGmUmCmGmGmsAm | 1427 | VPUmsCfsCmGmAmCfUmGmCmAm GmUmGmUfGmAfAmGmAmsGmsC m |
| GPSZRA 222S4-VP | 1302 | U(LNA)sCmUfUmCmAmCfAfCfU mGfCmAfGmUfCmGmGmsAm | 1428 | VPUmsCfsCmGmAmCfUmGfCmAmG mUmGmUfGmAfAmGmAmsGmsCm |
| GPSZRA 227S1-VP | 1132 | AmsCmAfCmUfGmCfAfGfUmCfG mGfAmGfCmUfAmsAf | 1429 | VPUmsUfsAmGfCmUfCmCfGmAfCm UmGmCfAmGfUmGfUmsGmsAm |
| GPSZRA 267S2-VP | 1198 | GmsCmAfGmUmAmGfCfUfCmCf UmGfGmUfGmGmAmsAm | 1430 | VPUmsUfsCmCmAmCfCmAfGmGmA mGmCmUfAmCfUmGmCmsUmsCm |
| GPSZRA 284S3-VP | 1265 | CmsCmGmUmAmAmCfCfCfUmG mAmCmCmCmUmGmUmGmsAm | 1431 | VPUmsCfsAmCmAmGfGmUmCmAm GmGmGmUfUmAfCmGmGmsUmsU m |
| GPSZRA 31451-VP | 1163 | AmsCmUfGmGfAmUfGfAfAmGf GmAfUmGfGmUfGmsUf | 1432 | VPAmsCfsAmCfCmAfUmCfCmUfUm CmAmUfCmCfAmGfUmsGmsGm |
| GPSZRA 314S2-VP | 1220 | AmsCmUfGmGmAmUfGfAfAmGf GmAfUmGfGmUmGmsUm | 1433 | VPAmsCfsAmCmCmAfUmCfCmUmU mCmAmUfCmCfAmGmUmsGmsGm |
| GPSZRA 314S3-VP | 1277 | AmsCmUmGmGmAmUfGfAfAmG mGmAmUmGmGmUmGmsUm | 1434 | VPAmsCfsAmCmCmAfUmCmCmUm UmCmAmUfCmCfAmGmUmsGmsGm |
| GPSZRA 314S5-VP | 1391 | AmsCmUmGmGfAmUfGfAfAmG mGmAmUmGfGmUmGmsUm | 1435 | VPAmsCfsAmCmCmAfUmCfCmUmU mCmAmUfCmCfAmGmUmsGmsGm |
| GPSZRA 318S2-VP | 1224 | GmsAmUfGmAmAmGfGfAfUmGf GmUfGmUfGmCmCmsCm | 1436 | VPGmsGfsGmCmAmCfAmCfCmAmU mCmCmUfUmCfAmUmCmsCmsAm |
| GPSZRA 402S4-VP | 1346 | C(LNA)sCmAfAmCmUmCfUfCfU mCfCmUfGmUfAmUmAmsAm | 1437 | VPUmsUfsAmUmAmCfAmGfGmAm GmAmGmAfGmUfUmGmGmsUmsC m |
| GPSZRA 402S5-VP | 1403 | CmsCmAmAmCfUmCfUfCfUmCm CmUmGmUfAmUmAmsAm | 1438 | VPUmsUfsAmUmAmCfAmGfGmAm GmAmGmAfGmUfUmGmGmsUmsC m |

The following examples relate to the detection of siRNA bioactivity. The experimental methods in the examples are all conventional methods, unless otherwise specified. Examples, in which no specific conditions are specified, are carried out according to conventional conditions or conditions recommended by the manufacturers. The reagents and instruments used without indicating the manufacturer are all conventional products that can be purchased commercially. The experimental cells used in the following examples are 293T cells, purchased from the Cell Bank of the Chinese Academy of Sciences.

### Example 1: Detection of the on-target activity of unmodified siRNA for inhibiting RAGE

This example provides an on-target activity assay for unmodified siRNA for RAGE inhibition, which is performed using a plasmid vector constructed with the psiCHECK2 vector. The psiCHECK2 vector is a plasmid vector designed to monitor changes in the expression of a target gene fused to a reporter gene. It employs Renilla luciferase as the primary reporter gene; the target fragment is cloned into the multiple cloning site downstream of the translation stop codon of Renilla luciferase. The RNAi process targeting the target gene, triggered by the synthetic siRNA, results in the cleavage and subsequent degradation of the fused mRNA. By measuring changes in Renilla luciferase activity, the targeting relationship between the siRNA and the target gene fragment can be determined. The experimental procedure is described as follows:

### (1) Construction of detection plasmid RAGE-psiCHECK2

Detection plasmids were constructed using the psiCHECK^{™}-2 (Promega^{™}) plasmid. The detection plasmids contain the insertion sequence shown below, which is derived from the mRNA of the sequence set forth in Genbank Accession No. NM_001136.5. A single copy of the insertion sequence was cloned into the Xho I/Not I site of the psiCHECK^{™}-2 plasmid to obtain the detection plasmids GPRAGE-1-psiCHECK2, GPRAGE-2-psiCHECK2, GPRAGE-3-psiCHECK2, and GPRAGE-4-psiCHECK2.
GPRAGE-1-psiCHECK2 insert sequence is RAGE mRNA1: 5'-agacagagccaggaccctggaaggaagcaggatggctgccggaacagcagttggagcctgggtgctggtcctcagtctgtggggggcagtagtaggtgctcaaaac atcacagcccggattggcgagccactggtgctgaagtgtaagggggcccccaagaaaccaccccagcggctggaatggaaactgaacacaggccggacagaagctt ggaaggtcctgtctccccagggaggaggcccctgggacagtgtggctcgtgtccttcccaacggctccctcttccttccggctgtcgggatccaggatgaggggattttc cggtgccaggcaatgaacaggaatggaaaggagaccaagtccaactaccgagtccgtgtctaccagattcctgggaag-3' (SEQ ID NO: 1417);
GPRAGE-2-psiCHECK2 insert sequence is RAGE mRNA2: 5'-aggagaccaagtccaactaccgagtccgtgtctaccagattcctgggaagccagaaattgtagattctgcctctgaactcacggctggtgttcccaataaggtggggacat gtgtgtcagagggaagctaccctgcagggactcttagctggcacttggatgggaagcccctggtgcctaatgagaagggagtatctgtgaaggaacagaccaggagac accctgagacagggctcttcacactgcagtcggagctaatggtgaccccagcccggggaggagatccccgtcccaccttctcctgtagcttcagcccaggccttccccg acaccgggccttgcgcacagcccccatccagccccgtgtctgggagcctgtgcctctggaggaggtccaatt-3' (SEQ ID NO: 1418);
GPRAGE-3-psiCHECK2 insert sequence is RAGE mRNA3: 5'-cccatccagccccgtgtctgggagcctgtgcctctggaggaggtccaattggtggtggagccagaaggtggagcagtagctcctggtggaaccgtaaccctgacctgt gaagtccctgcccagccctctcctcaaatccactggatgaaggatggtgtgcccttgccccttccccccagccctgtgctgatcctccctgagatagggcctcaggacca gggaacctacagctgtgtggccacccattccagccacgggccccaggaaagccgtgctgtcagcatcagcatcatcgaaccaggcgaggaggggccaactgcaggc tctgtgggaggatcagggctgggaactctagccctggccctggggatcctgggaggcctggggacagccgccctgc-3' (seq ID NO: 1419);
GPRAGE-4-psiCHECK2 insert sequence is RAGE mRNA4: 5'-aggctctgtgggaggatcagggctgggaactctagccctggccctggggatcctgggaggcctggggacagccgccctgctcattggggtcatcttgtggcaaaggc ggcaacgccgaggagaggagaggaaggccccagaaaaccaggaggaagaggaggagcgtgcagaactgaatcagtcggaggaacctgaggcaggcgagagta gtactggagggccttgaggggcccacagacagatcccatccatcagctcccttttctttttcccttgaactgttctggcctcagaccaactctctcctgtataatctctctcctgt ataaccccaccttgccaagctttcttctacaaccagagccccccacaatgatgattaaacacctgacacatcttg-3' (SEQ ID NO: 1420);
Grouped assays were performed on the synthesized siRNA sequences described above, as detailed in Table 5. Among them, GPSZRA001-GPSZRA118 were assayed using the detection plasmid GPSZRA-1-psiCHECK2; GPSZRA090-GPSZRA251 were assayed using the detection plasmid GPSZRA-2-psiCHECK2; GPSZRA250-GPSZRA358 were assayed using the detection plasmid GPSZRA-3-psiCHECK2; and GPSZRA359-GPSZRA419 were assayed using the detection plasmid GPSZRA-4-psiCHECK2.

**Table 5: Grouped assays of siRNA**

| No. | siRNA name | Corresponding vector | Corresponding negative control |
|---|---|---|---|
| 1 | GPSZRA001-GPSZRA118 | GPSZRA-1-psiCHECK2 | NC1 |
| 2 | GPSZRA090-GPSZRA251 | GPSZRA-2-psiCHECK2 | NC1 |
| 3 | GPSZRA250-GPSZRA358 | GPSZRA-3-psiCHECK2 | NC1 |
| 4 | GPSZRA359-GPSZRA419 | GPSZRA-4-psiCHECK2 | NC1 |

### (2) Cell culture and transfection

In a 96-well plate, 5 µl/well of siRNA was mixed with 12.5 µl/well of Opti-MEM containing 20 ng of GPSZRA-1-psiCHECK2 detection plasmid (the corresponding detection plasmid was used based on the siRNA), followed by the addition of 32.5 µl of Opti-MEM and 0.3 µl of Lipofectamine 2000 (purchased from Invitrogen, Cat. No. 11668-019). The mixture was incubated at room temperature for 15 min. Then, 50 µl of DMEM complete medium containing 1×104 293T cells (purchased from Transgen Biotech, Cat. No. FI101-01) was added to the above mixture per well, followed by incubation at 37°C for 24 h for subsequent dual-luciferase assay. The experiment was performed at a final siRNA concentration of 1 nM.

### (3) Dual-luciferase assay

The 5× lysis buffer in the Dual Luciferase Assay Kit (purchased from Promega, Cat. No. E2940) was diluted with water to make 1× lysis buffer. The cells cultured in step 2 were harvested, and the supernatant was discarded. Each well was rinsed twice with PBS buffer (purchased from Hyclone, Cat. No. SH30256.01). Then, 50 µL of 1× lysis buffer was added to each well of the cell culture plate, followed by lysis at room temperature for 20 min to obtain the lysed cell plate. Subsequently, 30 µL of the lysate was aspirated from each well of the lysed cell plate and transferred to an opaque 96-well assay plate. Two types of substrates were prepared using the dual-luciferase assay kit in accordance with the manufacturer's instructions. The two substrates, substrate 1 and substrate 2, were then added to a new 96-well plate at a volume of 30 µL/well, respectively. After each substrate addition, detection was performed using a multifunctional microplate reader, and the luminescence intensity values of Firefly luciferase and Renilla luciferase were obtained separately.

Renilla luciferase was detected using a microplate reader. The luminescence ratio Renilla/Firefly of each well in the microplate was calculated. The luminescence ratio of each test group or control group was defined as the mean value of the luminescence ratios from three replicate wells. Using the luminescence ratio of the control group as the reference, the luminescence ratios of each test group were normalized to obtain the ratio R = luminescence ratio (test group) / luminescence ratio (control group), which represents the expression level of the Renilla reporter gene, namely the relative residual activity. The inhibition rate of siRNA is (1-R)×100%.

The on-target activity results are shown in Table 6 below. The results show that these duplexes have inhibitory activity against RAGE expression.

**Table 6: On-target activity assay of unmodified siRNA**

| Sequence ID | Residual activity | Inhibition rate | Sequence ID | Residual activity | Inhibition rate |
|---|---|---|---|---|---|
| GPSZRA001 | 0.4046 | 59.54% | GPSZRA214 | 0.1541 | 84.59% |
| GPSZRA002 | 0.4052 | 59.48% | GPSZRA215 | 0.1746 | 82.54% |
| GPSZRA003 | 0.4853 | 51.47% | GPSZRA216 | 0.2839 | 71.61% |
| GPSZRA004 | 0.2842 | 71.58% | GPSZRA217 | 0.1847 | 81.53% |
| GPSZRA005 | 0.186 | 81.40% | GPSZRA218 | 0.223 | 77.70% |
| GPSZRA006 | 0.3618 | 63.82% | GPSZRA219 | 0.3631 | 63.69% |
| GPSZRA007 | 0.2455 | 75.45% | GPSZRA220 | 0.2314 | 76.86% |
| GPSZRA008 | 0.1395 | 86.05% | GPSZRA221 | 0.2128 | 78.72% |
| GPSZRA009 | 0.1927 | 80.73% | GPSZRA222 | 0.1668 | 83.32% |
| GPSZRA010 | 0.1723 | 82.77% | GPSZRA223 | 0.1713 | 82.87% |
| GPSZRA011 | 0.2768 | 72.32% | GPSZRA224 | 0.2473 | 75.27% |
| GPSZRA012 | 0.2909 | 70.91% | GPSZRA225 | 0.2193 | 78.07% |
| GPSZRA013 | 0.4605 | 53.95% | GPSZRA226 | 0.1783 | 82.17% |
| GPSZRA014 | 0.5628 | 43.72% | GPSZRA227 | 0.1511 | 84.89% |
| GPSZRA015 | 0.5919 | 40.81% | GPSZRA228 | 0.1627 | 83.73% |
| GPSZRA016 | 0.5 | 50.00% | GPSZRA229 | 0.16 | 84.00% |
| GPSZRA017 | 0.3845 | 61.55% | GPSZRA230 | 0.2518 | 74.82% |
| GPSZRA018 | 0.3748 | 62.52% | GPSZRA231 | 0.1652 | 83.48% |
| GPSZRA019 | 0.2739 | 72.61% | GPSZRA232 | 0.1772 | 82.28% |
| GPSZRA020 | 0.1745 | 82.55% | GPSZRA233 | 0.1629 | 83.71% |
| GPSZRA021 | 0.1734 | 82.66% | GPSZRA234 | 0.1584 | 84.16% |
| GPSZRA022 | 0.2738 | 72.62% | GPSZRA235 | 0.188 | 81.20% |
| GPSZRA023 | 0.2966 | 70.34% | GPSZRA236 | 0.4404 | 55.96% |
| GPSZRA024 | 0.4347 | 56.53% | GPSZRA237 | 0.2132 | 78.68% |
| GPSZRA025 | 0.298 | 70.20% | GPSZRA238 | 0.2495 | 75.05% |
| GPSZRA026 | 0.5639 | 43.61% | GPSZRA239 | 0.1803 | 81.97% |
| GPSZRA027 | 0.5229 | 47.71% | GPSZRA240 | 0.2908 | 70.92% |
| GPSZRA028 | 0.4503 | 54.97% | GPSZRA241 | 0.3446 | 65.54% |
| GPSZRA029 | 0.2489 | 75.11% | GPSZRA242 | 0.3016 | 69.84% |
| GPSZRA030 | 0.3936 | 60.64% | GPSZRA243 | 0.3082 | 69.18% |
| GPSZRA031 | 0.5401 | 45.99% | GPSZRA244 | 0.3138 | 68.62% |
| GPSZRA032 | 0.487 | 51.30% | GPSZRA245 | 0.4358 | 56.42% |
| GPSZRA033 | 0.3372 | 66.28% | GPSZRA246 | 0.3337 | 66.63% |
| GPSZRA034 | 0.4028 | 59.72% | GPSZRA247 | 0.2585 | 74.15% |
| GPSZRA035 | 0.4303 | 56.97% | GPSZRA248 | 0.2695 | 73.05% |
| GPSZRA036 | 0.3323 | 66.77% | GPSZRA249 | 0.3636 | 63.64% |
| GPSZRA037 | 0.6487 | 35.13% | GPSZRA250 | 0.2191 | 78.09% |
| GPSZRA038 | 0.5376 | 46.24% | GPSZRA251 | 0.2152 | 78.48% |
| GPSZRA039 | 0.4898 | 51.02% | GPSZRA252 | 0.1928 | 80.72% |
| GPSZRA040 | 0.2172 | 78.28% | GPSZRA253 | 0.2976 | 70.24% |
| GPSZRA041 | 0.4016 | 59.84% | GPSZRA254 | 0.2048 | 79.52% |
| GPSZRA042 | 0.3104 | 68.96% | GPSZRA255 | 0.2266 | 77.34% |
| GPSZRA043 | 0.2633 | 73.67% | GPSZRA256 | 0.3231 | 67.69% |
| GPSZRA044 | 0.2948 | 70.52% | GPSZRA257 | 0.2431 | 75.69% |
| GPSZRA045 | 0.3365 | 66.35% | GPSZRA258 | 0.3109 | 68.91% |
| GPSZRA046 | 0.3848 | 61.52% | GPSZRA259 | 0.2618 | 73.82% |
| GPSZRA047 | 0.2999 | 70.01% | GPSZRA260 | 0.7018 | 29.82% |
| GPSZRA048 | 0.2944 | 70.56% | GPSZRA261 | 0.4811 | 51.89% |
| GPSZRA049 | 0.2474 | 75.26% | GPSZRA262 | 0.4925 | 50.75% |
| GPSZRA050 | 0.3313 | 66.87% | GPSZRA263 | 0.4844 | 51.56% |
| GPSZRA051 | 0.2603 | 73.97% | GPSZRA264 | 0.8836 | 11.64% |
| GPSZRA052 | 0.2626 | 73.74% | GPSZRA265 | 0.4791 | 52.09% |
| GPSZRA053 | 0.2188 | 78.12% | GPSZRA266 | 0.1627 | 83.73% |
| GPSZRA054 | 0.2416 | 75.84% | GPSZRA267 | 0.1331 | 86.69% |
| GPSZRA055 | 0.1594 | 84.06% | GPSZRA268 | 0.2658 | 73.42% |
| GPSZRA056 | 0.1678 | 83.22% | GPSZRA269 | 0.2224 | 77.76% |
| GPSZRA057 | 0.2121 | 78.79% | GPSZRA270 | 0.1622 | 83.78% |
| GPSZRA058 | 0.1742 | 82.58% | GPSZRA271 | 0.1264 | 87.36% |
| GPSZRA059 | 0.1776 | 82.24% | GPSZRA272 | 0.1347 | 86.53% |
| GPSZRA060 | 0.2541 | 74.59% | GPSZRA273 | 0.2483 | 75.17% |
| GPSZRA061 | 0.3442 | 65.58% | GPSZRA274 | 0.1691 | 83.09% |
| GPSZRA062 | 0.3043 | 69.57% | GPSZRA275 | 0.1843 | 81.57% |
| GPSZRA063 | 0.3521 | 64.79% | GPSZRA276 | 0.1513 | 84.87% |
| GPSZRA064 | 0.1641 | 83.59% | GPSZRA277 | 0.1524 | 84.76% |
| GPSZRA065 | 0.1733 | 82.67% | GPSZRA278 | 0.2994 | 70.06% |
| GPSZRA066 | 0.2094 | 79.06% | GPSZRA279 | 0.3838 | 61.62% |
| GPSZRA067 | 0.2152 | 78.48% | GPSZRA280 | 0.1719 | 82.81% |
| GPSZRA068 | 0.2152 | 78.48% | GPSZRA281 | 0.151 | 84.90% |
| GPSZRA069 | 0.1901 | 80.99% | GPSZRA282 | 0.1356 | 86.44% |
| GPSZRA070 | 0.2184 | 78.16% | GPSZRA283 | 0.1286 | 87.14% |
| GPSZRA071 | 0.4058 | 59.42% | GPSZRA284 | 0.1074 | 89.26% |
| GPSZRA072 | 0.438 | 56.20% | GPSZRA285 | 0.1398 | 86.02% |
| GPSZRA073 | 0.1789 | 82.11% | GPSZRA286 | 0.1403 | 85.97% |
| GPSZRA074 | 0.1785 | 82.15% | GPSZRA287 | 0.1578 | 84.22% |
| GPSZRA075 | 0.1754 | 82.46% | GPSZRA288 | 0.1248 | 87.52% |
| GPSZRA076 | 0.2165 | 78.35% | GPSZRA289 | 0.1468 | 85.32% |
| GPSZRA077 | 0.2322 | 76.78% | GPSZRA290 | 0.1475 | 85.25% |
| GPSZRA078 | 0.1384 | 86.16% | GPSZRA291 | 0.1752 | 82.48% |
| GPSZRA079 | 0.1568 | 84.32% | GPSZRA292 | 0.125 | 87.50% |
| GPSZRA080 | 0.1586 | 84.14% | GPSZRA293 | 0.3291 | 67.09% |
| GPSZRA081 | 0.2186 | 78.14% | GPSZRA294 | 0.2309 | 76.91% |
| GPSZRA082 | 0.192 | 80.80% | GPSZRA295 | 0.2612 | 73.88% |
| GPSZRA083 | 0.189 | 81.10% | GPSZRA296 | 0.1729 | 82.71% |
| GPSZRA084 | 0.1272 | 87.28% | GPSZRA297 | 0.1807 | 81.93% |
| GPSZRA085 | 0.1179 | 88.21% | GPSZRA298 | 0.1999 | 80.01% |
| GPSZRA086 | 0.1492 | 85.08% | GPSZRA299 | 0.2177 | 78.23% |
| GPSZRA087 | 0.1521 | 84.79% | GPSZRA300 | 0.2142 | 78.58% |
| GPSZRA088 | 0.1526 | 84.74% | GPSZRA301 | 0.162 | 83.80% |
| GPSZRA089 | 0.2173 | 78.27% | GPSZRA302 | 0.162 | 83.80% |
| GPSZRA090 | 0.1531 | 84.69% | GPSZRA303 | 0.1819 | 81.81% |
| GPSZRA091 | 0.212 | 78.80% | GPSZRA304 | 0.1603 | 83.97% |
| GPSZRA092 | 0.1385 | 86.15% | GPSZRA305 | 0.2138 | 78.62% |
| GPSZRA093 | 0.1715 | 82.85% | GPSZRA306 | 0.2115 | 78.85% |
| GPSZRA094 | 0.1917 | 80.83% | GPSZRA307 | 0.143 | 85.70% |
| GPSZRA095 | 0.2116 | 78.84% | GPSZRA308 | 0.1221 | 87.79% |
| GPSZRA096 | 0.2055 | 79.45% | GPSZRA309 | 0.2823 | 71.77% |
| GPSZRA097 | 0.1886 | 81.14% | GPSZRA310 | 0.3006 | 69.94% |
| GPSZRA098 | 0.2011 | 79.89% | GPSZRA311 | 0.1854 | 81.46% |
| GPSZRA099 | 0.2106 | 78.94% | GPSZRA312 | 0.1786 | 82.14% |
| GPSZRA100 | 0.1922 | 80.78% | GPSZRA313 | 0.1438 | 85.62% |
| GPSZRA101 | 0.1842 | 81.58% | GPSZRA314 | 0.1595 | 84.05% |
| GPSZRA102 | 0.2369 | 76.31% | GPSZRA315 | 0.1831 | 81.69% |
| GPSZRA103 | 0.1318 | 86.82% | GPSZRA316 | 0.1736 | 82.64% |
| GPSZRA104 | 0.1222 | 87.78% | GPSZRA317 | 0.1924 | 80.76% |
| GPSZRA105 | 0.1618 | 83.82% | GPSZRA318 | 0.1486 | 85.14% |
| GPSZRA106 | 0.2068 | 79.32% | GPSZRA319 | 0.2611 | 73.89% |
| GPSZRA107 | 0.196 | 80.40% | GPSZRA320 | 0.2258 | 77.42% |
| GPSZRA108 | 0.2053 | 79.47% | GPSZRA321 | 0.3091 | 69.09% |
| GPSZRA109 | 0.1747 | 82.53% | GPSZRA322 | 0.4212 | 57.88% |
| GPSZRA110 | 0.1554 | 84.46% | GPSZRA323 | 0.3269 | 67.31% |
| GPSZRA111 | 0.1676 | 83.24% | GPSZRA324 | 0.5462 | 45.38% |
| GPSZRA112 | 0.1298 | 87.02% | GPSZRA325 | 0.4312 | 56.88% |
| GPSZRA113 | 0.1551 | 84.49% | GPSZRA326 | 0.6027 | 39.73% |
| GPSZRA114 | 0.1501 | 84.99% | GPSZRA327 | 0.6194 | 38.06% |
| GPSZRA115 | 0.1757 | 82.43% | GPSZRA328 | 0.7224 | 27.76% |
| GPSZRA116 | 0.2285 | 77.15% | GPSZRA329 | 0.3663 | 63.37% |
| GPSZRA117 | 0.2097 | 79.03% | GPSZRA330 | 0.4111 | 58.89% |
| GPSZRA118 | 0.1926 | 80.74% | GPSZRA331 | 0.2787 | 72.13% |
| GPSZRA119 | 0.3447 | 65.53% | GPSZRA332 | 0.3129 | 68.71% |
| GPSZRA120 | 0.1918 | 80.82% | GPSZRA333 | 0.1886 | 81.14% |
| GPSZRA121 | 0.3653 | 63.47% | GPSZRA334 | 0.219 | 78.10% |
| GPSZRA122 | 0.4597 | 54.03% | GPSZRA335 | 0.2678 | 73.22% |
| GPSZRA123 | 0.5405 | 45.95% | GPSZRA336 | 0.2044 | 79.56% |
| GPSZRA124 | 0.4273 | 57.27% | GPSZRA337 | 0.2312 | 76.88% |
| GPSZRA125 | 0.4422 | 55.78% | GPSZRA338 | 0.4133 | 58.67% |
| GPSZRA126 | 0.4184 | 58.16% | GPSZRA339 | 0.2184 | 78.16% |
| GPSZRA127 | 0.6397 | 36.03% | GPSZRA340 | 0.2231 | 77.69% |
| GPSZRA128 | 0.7982 | 20.18% | GPSZRA341 | 0.2514 | 74.86% |
| GPSZRA129 | 0.8535 | 14.65% | GPSZRA342 | 0.2407 | 75.93% |
| GPSZRA130 | 0.6577 | 34.23% | GPSZRA343 | 0.2028 | 79.72% |
| GPSZRA131 | 0.7402 | 25.98% | GPSZRA344 | 0.1579 | 84.21% |
| GPSZRA132 | 0.6782 | 32.18% | GPSZRA345 | 0.1362 | 86.38% |
| GPSZRA133 | 0.6978 | 30.22% | GPSZRA346 | 0.2527 | 74.73% |
| GPSZRA134 | 0.6119 | 38.81% | GPSZRA347 | 0.2224 | 77.76% |
| GPSZRA135 | 0.7721 | 22.79% | GPSZRA348 | 0.22 | 78.00% |
| GPSZRA136 | 0.7502 | 24.98% | GPSZRA349 | 0.3571 | 64.29% |
| GPSZRA137 | 0.8548 | 14.52% | GPSZRA350 | 0.2666 | 73.34% |
| GPSZRA138 | 0.8801 | 11.99 | GPSZRA351 | 0.3134 | 68.66% |
| GPSZRA139 | 0.8531 | 14.69% | GPSZRA352 | 0.4174 | 58.26% |
| GPSZRA140 | 0.8025 | 19.75% | GPSZRA353 | 0.2129 | 78.71% |
| GPSZRA143 | 0.8887 | 11.13% | GPSZRA354 | 0.2226 | 77.74% |
| GPSZRA144 | 0.8698 | 13.02% | GPSZRA355 | 0.3698 | 63.02% |
| GPSZRA147 | 0.6459 | 35.41% | GPSZRA356 | 0.2935 | 70.65% |
| GPSZRA148 | 0.6059 | 39.41% | GPSZRA357 | 0.3572 | 64.28% |
| GPSZRA149 | 0.6649 | 33.51% | GPSZRA358 | 0.4907 | 50.93% |
| GPSZRA150 | 0.5918 | 40.82% | GPSZRA359 | 0.2563 | 74.37% |
| GPSZRA151 | 0.5406 | 45.94% | GPSZRA360 | 0.1279 | 87.21% |
| GPSZRA152 | 0.5113 | 48.87% | GPSZRA361 | 0.2505 | 74.95% |
| GPSZRA153 | 0.6521 | 34.79% | GPSZRA362 | 0.3329 | 66.71% |
| GPSZRA154 | 0.3913 | 60.87% | GPSZRA363 | 0.3454 | 65.46% |
| GPSZRA155 | 0.521 | 47.90% | GPSZRA364 | 0.2114 | 78.86% |
| GPSZRA156 | 0.338 | 66.20% | GPSZRA365 | 0.2839 | 71.61% |
| GPSZRA157 | 0.584 | 41.60% | GPSZRA366 | 0.1763 | 82.37% |
| GPSZRA158 | 0.4359 | 56.41% | GPSZRA367 | 0.2656 | 73.44% |
| GPSZRA159 | 0.5301 | 46.99% | GPSZRA368 | 0.4652 | 53.48% |
| GPSZRA160 | 0.4222 | 57.78% | GPSZRA369 | 0.5829 | 41.71% |
| GPSZRA161 | 0.3862 | 61.38% | GPSZRA370 | 0.8914 | 10.86% |
| GPSZRA162 | 0.4035 | 59.65% | GPSZRA371 | 0.6634 | 33.66% |
| GPSZRA163 | 0.4434 | 55.66% | GPSZRA372 | 0.6253 | 37.47% |
| GPSZRA164 | 0.2688 | 73.12% | GPSZRA373 | 0.4974 | 50.26% |
| GPSZRA165 | 0.1823 | 81.77% | GPSZRA374 | 0.332 | 66.80% |
| GPSZRA166 | 0.3468 | 65.32% | GPSZRA375 | 0.291 | 70.90% |
| GPSZRA167 | 0.3434 | 65.66% | GPSZRA376 | 0.4399 | 56.01% |
| GPSZRA168 | 0.4919 | 50.81% | GPSZRA377 | 0.2223 | 77.77% |
| GPSZRA169 | 0.2806 | 71.94% | GPSZRA378 | 0.1669 | 83.31% |
| GPSZRA170 | 0.4072 | 59.28% | GPSZRA379 | 0.1329 | 86.71% |
| GPSZRA171 | 0.3237 | 67.63% | GPSZRA380 | 0.2063 | 79.37% |
| GPSZRA172 | 0.5589 | 44.11% | GPSZRA381 | 0.1129 | 88.71% |
| GPSZRA173 | 0.3931 | 60.69% | GPSZRA382 | 0.1522 | 84.78% |
| GPSZRA174 | 0.3088 | 69.12% | GPSZRA383 | 0.2288 | 77.12% |
| GPSZRA175 | 0.3885 | 61.15% | GPSZRA384 | 0.2851 | 71.49% |
| GPSZRA176 | 0.2257 | 77.43% | GPSZRA385 | 0.4027 | 59.73% |
| GPSZRA177 | 0.3062 | 69.38% | GPSZRA386 | 0.2634 | 73.66% |
| GPSZRA178 | 0.289 | 71.10% | GPSZRA387 | 0.1866 | 81.34% |
| GPSZRA179 | 0.4929 | 50.71% | GPSZRA388 | 0.1755 | 82.45% |
| GPSZRA180 | 0.6131 | 38.69% | GPSZRA389 | 0.7006 | 29.94% |
| GPSZRA181 | 0.5225 | 47.75% | GPSZRA390 | 0.8507 | 14.93% |
| GPSZRA182 | 0.6133 | 38.67% | GPSZRA391 | 0.5901 | 40.99% |
| GPSZRA183 | 0.5194 | 48.06% | GPSZRA392 | 0.6921 | 30.79% |
| GPSZRA184 | 0.3485 | 65.15% | GPSZRA393 | 0.1632 | 83.68% |
| GPSZRA185 | 0.5359 | 46.41% | GPSZRA394 | 0.4629 | 53.71% |
| GPSZRA186 | 0.3462 | 65.38% | GPSZRA395 | 0.6319 | 36.81% |
| GPSZRA187 | 0.4728 | 52.72% | GPSZRA396 | 0.6969 | 30.31% |
| GPSZRA188 | 0.48 | 52.00% | GPSZRA397 | 0.7422 | 25.78% |
| GPSZRA189 | 0.7865 | 21.35% | GPSZRA398 | 0.842 | 15.80% |
| GPSZRA190 | 0.2627 | 73.73% | GPSZRA399 | 0.382 | 61.80% |
| GPSZRA191 | 0.2958 | 70.42% | GPSZRA400 | 0.1668 | 83.32% |
| GPSZRA192 | 0.4745 | 52.55% | GPSZRA401 | 0.1588 | 84.12% |
| GPSZRA193 | 0.5753 | 42.47% | GPSZRA402 | 0.1203 | 87.97% |
| GPSZRA194 | 0.3908 | 60.92% | GPSZRA403 | 0.2085 | 79.15% |
| GPSZRA195 | 0.3923 | 60.77% | GPSZRA404 | 0.188 | 81.20% |
| GPSZRA196 | 0.3618 | 63.82% | GPSZRA405 | 0.3308 | 66.92% |
| GPSZRA197 | 0.2401 | 75.99% | GPSZRA406 | 0.3588 | 64.12% |
| GPSZRA198 | 0.2911 | 70.89% | GPSZRA407 | 0.1977 | 80.23% |
| GPSZRA199 | 0.2403 | 75.97% | GPSZRA408 | 0.1792 | 82.08% |
| GPSZRA200 | 0.3162 | 68.38% | GPSZRA409 | 0.1396 | 86.04% |
| GPSZRA201 | 0.5073 | 49.27% | GPSZRA410 | 0.1458 | 85.42% |
| GPSZRA202 | 0.4747 | 52.53% | GPSZRA411 | 0.2032 | 79.68% |
| GPSZRA203 | 0.2577 | 74.23% | GPSZRA412 | 0.2758 | 72.42% |
| GPSZRA204 | 0.2271 | 77.29% | GPSZRA413 | 0.2153 | 78.47% |
| GPSZRA205 | 0.3164 | 68.36% | GPSZRA414 | 0.2545 | 74.55% |
| GPSZRA206 | 0.2052 | 79.48% | GPSZRA415 | 0.2963 | 70.37% |
| GPSZRA207 | 0.2238 | 77.62% | GPSZRA416 | 0.2009 | 79.91% |
| GPSZRA208 | 0.2641 | 73.59% | GPSZRA417 | 0.2729 | 72.71% |
| GPSZRA209 | 0.234 | 76.60% | GPSZRA418 | 0.2476 | 75.24% |
| GPSZRA210 | 0.2382 | 76.18% | GPSZRA419 | 0.2299 | 77.01% |
| GPSZRA211 | 0.224 | 77.60% | MOCK | 1.0469 | -4.69% |
| GPSZRA212 | 0.1765 | 82.35% | NC1 | 1 | 0.00% |
| GPSZRA213 | 0.2643 | 73.57% | | | |

MOCK was the blank control group containing only transfection reagent and plasmid; NC1 was the negative control siRNA, and the sequence information is shown in Table 7 below:

**Table 7: NC1 sequences**

| | SEQ ID NO: | Sense strand (5'-3') | SEQ ID NO: | Antisense strand (5'-3') |
|---|---|---|---|---|
| NC1 | 1422 | UUCUCCGAACGUGUCACGUUU | 1421 | AAACGUGACACGUUCGGAGAAUU |

Conclusion: The candidate naked sequences exhibited top-tier performance in the single-concentration psiCHECK assay. A total of 198 sequences that advanced to the next round of screening achieved a silencing efficiency of over 75%.

### Example 2: Detection of the on-target activity of unmodified siRNA for inhibiting RAGE

The sequences with high inhibition rates in Example 1 were subjected to on-target activity assays at siRNA final concentrations of 0.01 nM, 0.1 nM, and 1 nM. The experimental methods used were the same as those used in Example 1, except that the final concentration of siRNA was different. The detection results are presented in Table 7 below. The results demonstrate that these duplexes exhibit potent inhibitory activity against RAGE expression at different concentrations.

**Table 8: On-target activity assay of unmodified siRNA at different concentrations**

| Sequence name | 0.01 nM | | 0.1 nM | | 1 nM | |
|---|---|---|---|---|---|---|
| | Residual activity | Inhibition rate | Residual activity | Inhibition rate | Residual activity | Inhibition rate |
| GPSZRA005 | 0.9145 | 8.55% | 0.4257 | 57.43% | 0.2055 | 79.45% |
| GPSZRA008 | 0.9098 | 9.02% | 0.3902 | 60.98% | 0.1564 | 84.36% |
| GPSZRA020 | 0.871 | 12.90% | 0.5343 | 46.57% | 0.217 | 78.30% |
| GPSZRA021 | 0.9058 | 9.42% | 0.456 | 54.40% | 0.2261 | 77.39% |
| GPSZRA029 | 0.9575 | 4.25% | 0.5479 | 45.21% | 0.2912 | 70.88% |
| GPSZRA040 | 0.9105 | 8.95% | 0.5499 | 45.01% | 0.2772 | 72.28% |
| GPSZRA049 | 0.8768 | 12.32% | 0.4357 | 56.43% | 0.2261 | 77.39% |
| GPSZRA053 | 0.9809 | 1.91% | 0.5187 | 48.13% | 0.2423 | 75.77% |
| GPSZRA054 | 0.966 | 3.40% | 0.5726 | 42.74% | 0.2739 | 72.61% |
| GPSZRA055 | 0.8907 | 10.93% | 0.5302 | 46.98% | 0.1721 | 82.79% |
| GPSZRA056 | 0.9469 | 5.31% | 0.484 | 51.60% | 0.2073 | 79.27% |
| GPSZRA057 | 0.9844 | 1.56% | 0.6353 | 36.47% | 0.2655 | 73.45% |
| GPSZRA058 | 0.8054 | 19.46% | 0.3829 | 61.71% | 0.1964 | 80.36% |
| GPSZRA059 | 0.9924 | 0.76% | 0.3382 | 66.18% | 0.2146 | 78.54% |
| GPSZRA064 | 0.9611 | 3.89% | 0.5058 | 49.42% | 0.254 | 74.60% |
| GPSZRA069 | 0.9404 | 5.96% | 0.5096 | 49.04% | 0.2387 | 76.13% |
| GPSZRA074 | 0.9845 | 1.55% | 0.5547 | 44.53% | 0.2959 | 70.41% |
| GPSZRA075 | 0.9934 | 0.66% | 0.6165 | 38.35% | 0.3029 | 69.71% |
| GPSZRA078 | 0.9915 | 0.85% | 0.5723 | 42.77% | 0.2526 | 74.74% |
| GPSZRA080 | 0.9828 | 1.72% | 0.5578 | 44.22% | 0.2167 | 78.33% |
| GPSZRA081 | 0.9435 | 5.65% | 0.5173 | 48.27% | 0.241 | 75.90% |
| GPSZRA082 | 0.963 | 3.70 | 0.5316 | 46.84% | 0.2285 | 77.15% |
| GPSZRA083 | 0.936 | 6.40% | 0.4768 | 52.32% | 0.2188 | 78.12% |
| GPSZRA084 | 0.8776 | 12.24% | 0.2942 | 70.58% | 0.1397 | 86.03% |
| GPSZRA085 | 0.8865 | 11.35% | 0.3675 | 63.25% | 0.1366 | 86.34% |
| GPSZRA086 | 0.8865 | 11.35% | 0.3778 | 62.22% | 0.1396 | 86.04% |
| GPSZRA087 | 0.7138 | 28.62% | 0.259 | 74.10% | 0.1201 | 87.99% |
| GPSZRA088 | 0.8632 | 13.68% | 0.3643 | 63.57% | 0.1345 | 86.55% |
| GPSZRA089 | 0.8986 | 10.14% | 0.4383 | 56.17% | 0.1851 | 81.49% |
| GPSZRA090 | 0.8042 | 19.58% | 0.4428 | 55.72% | 0.1365 | 86.35% |
| GPSZRA091 | 0.8504 | 14.96% | 0.4438 | 55.62% | 0.1785 | 82.15% |
| GPSZRA092 | 0.7687 | 23.13% | 0.271 | 72.90% | 0.1097 | 89.03% |
| GPSZRA093 | 0.7267 | 27.33% | 0.2829 | 71.71% | 0.1486 | 85.14% |
| GPSZRA094 | 0.7524 | 24.76% | 0.2895 | 71.05% | 0.1628 | 83.72% |
| GPSZRA095 | 0.744 | 25.60% | 0.3064 | 69.36% | 0.1941 | 80.59% |
| GPSZRA096 | 0.807 | 19.30% | 0.3549 | 64.51% | 0.1763 | 82.37% |
| GPSZRA097 | 0.7561 | 24.39% | 0.3022 | 69.78% | 0.1727 | 82.73% |
| GPSZRA098 | 0.8449 | 15.51% | 0.3359 | 66.41% | 0.179 | 82.10% |
| GPSZRA099 | 0.8329 | 16.71% | 0.3081 | 69.19% | 0.1946 | 80.54% |
| GPSZRA100 | 0.8626 | 13.74% | 0.3201 | 67.99% | 0.1689 | 83.11% |
| GPSZRA101 | 0.8397 | 16.03% | 0.2857 | 71.43% | 0.2009 | 79.91% |
| GPSZRA102 | 0.9702 | 2.98% | 0.4044 | 59.56% | 0.2329 | 76.71% |
| GPSZRA103 | 0.7158 | 28.42% | 0.2486 | 75.14% | 0.1271 | 87.29% |
| GPSZRA104 | 0.6907 | 30.93% | 0.2117 | 78.83% | 0.1253 | 87.47% |
| GPSZRA105 | 0.8997 | 10.03% | 0.3321 | 66.79% | 0.141 | 85.90% |
| GPSZRA106 | 0.8481 | 15.19% | 0.4059 | 59.41% | 0.1947 | 80.53% |
| GPSZRA107 | 0.7277 | 27.23% | 0.3069 | 69.31% | 0.1854 | 81.46% |
| GPSZRA108 | 0.815 | 18.50% | 0.4414 | 55.86% | 0.2047 | 79.53% |
| GPSZRA109 | 0.7589 | 24.11% | 0.3509 | 64.91% | 0.2108 | 78.92% |
| GPSZRA110 | 0.7434 | 25.67% | 0.3233 | 67.67% | 0.1724 | 82.76% |
| GPSZRA111 | 0.8041 | 19.59% | 0.4181 | 58.19% | 0.2162 | 78.38% |
| GPSZRA112 | 0.6992 | 30.08% | 0.3274 | 67.26% | 0.1668 | 83.33% |
| GPSZRA113 | 0.7454 | 25.46% | 0.3496 | 65.04% | 0.18 | 82.00% |
| GPSZRA114 | 0.7376 | 26.24% | 0.3389 | 66.11% | 0.1675 | 83.25% |
| GPSZRA115 | 0.8324 | 16.76% | 0.3965 | 60.35% | 0.1697 | 83.03% |
| GPSZRA116 | 0.8318 | 16.82% | 0.3938 | 60.62% | 0.2195 | 78.05% |
| GPSZRA117 | 0.8853 | 11.47% | 0.4347 | 56.53% | 0.2124 | 78.76% |
| GPSZRA118 | 0.8711 | 12.89% | 0.3703 | 62.97% | 0.1796 | 82.04% |
| GPSZRA120 | 0.9424 | 5.76% | 0.4411 | 55.89% | 0.1545 | 84.55% |
| GPSZRA165 | 0.8774 | 12.26% | 0.3946 | 60.54% | 0.1224 | 87.76% |
| GPSZRA176 | 0.8336 | 16.64% | 0.3421 | 65.79% | 0.1736 | 82.64% |
| GPSZRA197 | 0.8295 | 17.05% | 0.3356 | 66.44% | 0.25 | 75.00% |
| GPSZRA199 | 0.7885 | 21.15% | 0.359 | 64.10% | 0.2146 | 78.54% |
| GPSZRA204 | 0.8422 | 15.78% | 0.3686 | 63.14% | 0.1722 | 82.78% |
| GPSZRA206 | 0.7915 | 20.85% | 0.3152 | 68.48% | 0.1904 | 80.96% |
| GPSZRA207 | 0.7433 | 25.67% | 0.3234 | 67.66% | 0.1943 | 80.57% |
| GPSZRA209 | 0.6783 | 32.17% | 0.2624 | 73.76% | 0.1918 | 80.82% |
| GPSZRA212 | 0.8775 | 12.25% | 0.286 | 71.40% | 0.1392 | 86.08% |
| GPSZRA214 | 0.8038 | 19.62% | 0.2273 | 77.27% | 0.1399 | 86.01% |
| GPSZRA215 | 0.8739 | 12.61% | 0.2437 | 75.63% | 0.1474 | 85.26% |
| GPSZRA217 | 0.8286 | 17.14% | 0.2409 | 75.91% | 0.1382 | 86.18% |
| GPSZRA218 | 0.9284 | 7.16% | 0.3057 | 69.43% | 0.1619 | 83.81% |
| GPSZRA220 | 0.8436 | 15.64% | 0.3182 | 68.18% | 0.1777 | 82.23% |
| GPSZRA221 | 0.8739 | 12.61% | 0.294 | 70.60% | 0.1936 | 80.64% |
| GPSZRA222 | 0.6937 | 30.63% | 0.2265 | 77.35% | 0.1579 | 84.21% |
| GPSZRA223 | 0.85 | 15.00% | 0.322 | 67.80% | 0.1908 | 80.92% |
| GPSZRA224 | 0.7294 | 27.06% | 0.3196 | 68.04% | 0.2332 | 76.68% |
| GPSZRA225 | 0.824 | 17.60% | 0.3685 | 63.15% | 0.2227 | 77.73% |
| GPSZRA226 | 0.7672 | 23.28% | 0.2744 | 72.56% | 0.1736 | 82.64% |
| GPSZRA227 | 0.6632 | 33.68% | 0.2244 | 77.56% | 0.1545 | 84.55% |
| GPSZRA228 | 0.6444 | 35.56% | 0.2115 | 78.85% | 0.1401 | 85.99% |
| GPSZRA229 | 0.6361 | 36.39% | 0.2323 | 76.77% | 0.1547 | 84.53% |
| GPSZRA231 | 0.6373 | 36.27% | 0.2077 | 79.23% | 0.1627 | 83.73% |
| GPSZRA232 | 0.6476 | 35.24% | 0.2474 | 75.26% | 0.1721 | 82.79% |
| GPSZRA233 | 0.4727 | 52.73% | 0.17 | 83.00% | 0.1467 | 85.33% |
| GPSZRA234 | 0.7245 | 27.55% | 0.258 | 74.20% | 0.169 | 83.10% |
| GPSZRA235 | 0.8702 | 12.98% | 0.3749 | 62.51% | 0.1952 | 80.48% |
| GPSZRA237 | 0.963 | 3.70 | 0.4511 | 54.89% | 0.145 | 85.50% |
| GPSZRA238 | 0.9151 | 8.49% | 0.4804 | 51.96% | 0.1964 | 80.36% |
| GPSZRA239 | 0.8157 | 18.43% | 0.3365 | 66.35% | 0.1331 | 86.69% |
| GPSZRA250 | 0.9163 | 8.37% | 0.4863 | 51.37% | 0.1865 | 81.35% |
| GPSZRA251 | 0.8742 | 12.58% | 0.4046 | 59.54% | 0.1716 | 82.84% |
| GPSZRA252 | 0.7607 | 23.93% | 0.2317 | 76.83% | 0.1327 | 86.73% |
| GPSZRA254 | 0.7044 | 29.56% | 0.2028 | 79.72% | 0.1263 | 87.37% |
| GPSZRA255 | 0.7648 | 23.52% | 0.2181 | 78.19% | 0.1196 | 88.04% |
| GPSZRA257 | 0.797 | 20.30% | 0.2458 | 75.42% | 0.1246 | 87.54% |
| GPSZRA266 | 0.5671 | 43.29% | 0.132 | 86.80% | 0.0815 | 91.85% |
| GPSZRA267 | 0.7278 | 27.22% | 0.1753 | 82.47% | 0.0694 | 93.06% |
| GPSZRA269 | 0.8151 | 18.49% | 0.3266 | 67.34% | 0.1383 | 86.17% |
| GPSZRA270 | 0.9208 | 7.92% | 0.416 | 58.40% | 0.1489 | 85.11% |
| GPSZRA271 | 0.7154 | 28.46% | 0.1647 | 83.53% | 0.0701 | 92.99% |
| GPSZRA272 | 0.7881 | 21.19% | 0.1963 | 80.37% | 0.0654 | 93.46% |
| GPSZRA273 | 0.813 | 18.70% | 0.2996 | 70.04% | 0.1595 | 84.05% |
| GPSZRA274 | 0.7707 | 22.93% | 0.2606 | 73.94% | 0.1137 | 88.63% |
| GPSZRA275 | 0.7804 | 21.96% | 0.269 | 73.10% | 0.1225 | 87.75% |
| GPSZRA276 | 0.7027 | 29.73% | 0.2332 | 76.68% | 0.0945 | 90.55% |
| GPSZRA277 | 0.795 | 20.50% | 0.2896 | 71.04% | 0.0939 | 90.61% |
| GPSZRA280 | 0.85 | 15.00% | 0.4405 | 55.95% | 0.1717 | 82.83% |
| GPSZRA281 | 0.8014 | 19.86% | 0.24 | 76.00% | 0.1251 | 87.49% |
| GPSZRA282 | 0.6862 | 31.38% | 0.1839 | 81.61% | 0.0975 | 90.25% |
| GPSZRA283 | 0.7635 | 23.65% | 0.2089 | 79.11% | 0.0903 | 90.97% |
| GPSZRA284 | 0.8459 | 15.41% | 0.145 | 85.50% | 0.0809 | 91.91% |
| GPSZRA285 | 0.7835 | 21.65% | 0.1917 | 80.83% | 0.0996 | 90.04% |
| GPSZRA286 | 0.7456 | 25.44% | 0.2021 | 79.79% | 0.108 | 89.20% |
| GPSZRA287 | 0.8485 | 15.15% | 0.2267 | 77.33% | 0.1293 | 87.07% |
| GPSZRA288 | 0.7231 | 27.69% | 0.1859 | 81.41% | 0.0961 | 90.39% |
| GPSZRA289 | 0.8555 | 14.45% | 0.3037 | 69.63% | 0.1141 | 88.59% |
| GPSZRA290 | 0.8005 | 19.95% | 0.2216 | 77.84% | 0.1129 | 88.71% |
| GPSZRA291 | 0.9002 | 9.98% | 0.3433 | 65.67% | 0.186 | 81.40% |
| GPSZRA292 | 0.759 | 24.10% | 0.2936 | 70.64% | 0.1441 | 85.59% |
| GPSZRA294 | 0.9778 | 2.22% | 0.5846 | 41.54% | 0.2699 | 73.01% |
| GPSZRA296 | 0.7605 | 23.95% | 0.3174 | 68.26% | 0.1787 | 82.13% |
| GPSZRA297 | 0.7888 | 21.12% | 0.3288 | 67.12% | 0.2048 | 79.52% |
| GPSZRA298 | 0.7493 | 25.07% | 0.3429 | 65.71% | 0.1886 | 81.14% |
| GPSZRA299 | 0.7403 | 25.97% | 0.4134 | 58.66% | 0.2691 | 73.09% |
| GPSZRA300 | 0.7575 | 24.25% | 0.3858 | 61.42% | 0.2459 | 75.41% |
| GPSZRA301 | 0.5295 | 47.05% | 0.2311 | 76.89% | 0.2008 | 79.92% |
| GPSZRA302 | 0.686 | 31.40% | 0.2519 | 74.81% | 0.1761 | 82.39% |
| GPSZRA303 | 0.7632 | 23.68% | 0.2783 | 72.17% | 0.1664 | 83.36% |
| GPSZRA304 | 0.6891 | 31.09% | 0.2108 | 78.92% | 0.1445 | 85.55% |
| GPSZRA305 | 0.7265 | 27.35% | 0.2507 | 74.93% | 0.1799 | 82.01% |
| GPSZRA306 | 0.8876 | 11.24% | 0.355 | 64.50% | 0.1786 | 82.14% |
| GPSZRA307 | 0.8762 | 12.38% | 0.3055 | 69.45% | 0.1105 | 88.95% |
| GPSZRA308 | 0.7011 | 29.89% | 0.2 | 80.00% | 0.0925 | 90.75% |
| GPSZRA311 | 0.6219 | 37.81% | 0.213 | 78.70% | 0.1589 | 84.11% |
| GPSZRA312 | 0.6691 | 33.09% | 0.2195 | 78.05% | 0.1631 | 83.69% |
| GPSZRA313 | 0.6544 | 34.56% | 0.2174 | 78.26% | 0.1444 | 85.56% |
| GPSZRA314 | 0.6218 | 37.82% | 0.2412 | 75.88% | 0.1507 | 84.93% |
| GPSZRA315 | 0.4997 | 50.03% | 0.184 | 81.60% | 0.1794 | 82.06% |
| GPSZRA316 | 0.6987 | 30.13% | 0.3335 | 66.65% | 0.16 | 84.00% |
| GPSZRA317 | 0.6871 | 31.29% | 0.3598 | 64.02% | 0.2103 | 78.97% |
| GPSZRA318 | 0.6752 | 32.48% | 0.2295 | 77.05% | 0.1247 | 87.53% |
| GPSZRA320 | 0.7 | 30.00% | 0.265 | 73.50% | 0.1669 | 83.31% |
| GPSZRA333 | 0.701 | 29.90% | 0.2532 | 74.68% | 0.1489 | 85.11% |
| GPSZRA334 | 0.7691 | 23.09% | 0.3067 | 69.33% | 0.1849 | 81.51% |
| GPSZRA336 | 0.7166 | 28.34% | 0.257 | 74.30% | 0.1829 | 81.71% |
| GPSZRA337 | 0.8571 | 14.29% | 0.3571 | 64.29% | 0.1759 | 82.41% |
| GPSZRA339 | 0.7562 | 24.38% | 0.3454 | 65.46% | 0.1678 | 83.22% |
| GPSZRA340 | 0.8204 | 17.96% | 0.3528 | 64.72% | 0.2049 | 79.51% |
| GPSZRA342 | 0.6995 | 30.05% | 0.3932 | 60.68% | 0.2615 | 73.85% |
| GPSZRA343 | 0.9654 | 3.46% | 0.4666 | 53.34% | 0.2345 | 76.55% |
| GPSZRA344 | 0.6753 | 32.47% | 0.2695 | 73.05% | 0.1739 | 82.61% |
| GPSZRA345 | 0.4497 | 55.03% | 0.2677 | 73.23% | 0.146 | 85.40% |
| GPSZRA347 | 0.788 | 21.20% | 0.5033 | 49.67% | 0.2924 | 70.76% |
| GPSZRA348 | 0.7027 | 29.73% | 0.4587 | 54.13% | 0.2626 | 73.74% |
| GPSZRA353 | 0.7461 | 25.39% | 0.5423 | 45.77% | 0.2376 | 76.24% |
| GPSZRA354 | 0.8594 | 14.06% | 0.485 | 51.50% | 0.2616 | 73.84% |
| GPSZRA360 | 0.8671 | 13.29% | 0.3072 | 69.28% | 0.093 | 90.70% |
| GPSZRA364 | 0.9893 | 1.07% | 0.5305 | 46.95% | 0.2977 | 70.23% |
| GPSZRA366 | 0.8774 | 12.26% | 0.3525 | 64.75% | 0.1774 | 82.26% |
| GPSZRA378 | 0.7417 | 25.83% | 0.2355 | 76.45% | 0.1331 | 86.69% |
| GPSZRA379 | 0.7201 | 27.99% | 0.2575 | 74.25% | 0.1213 | 87.87% |
| GPSZRA380 | 0.8637 | 13.63% | 0.2853 | 71.47% | 0.1371 | 86.29% |
| GPSZRA381 | 0.5831 | 41.69% | 0.1348 | 86.52% | 0.078 | 92.20% |
| GPSZRA382 | 0.8194 | 18.06% | 0.3102 | 68.98% | 0.1142 | 88.58% |
| GPSZRA383 | 0.9098 | 9.02% | 0.4756 | 52.44% | 0.183 | 81.70% |
| GPSZRA388 | 0.9645 | 3.55% | 0.4113 | 58.87% | 0.1543 | 84.57% |
| GPSZRA393 | 0.8752 | 12.48% | 0.4628 | 53.72% | 0.1609 | 83.91% |
| GPSZRA400 | 0.9781 | 2.19% | 0.4851 | 51.49% | 0.1694 | 83.06% |
| GPSZRA401 | 0.907 | 9.30% | 0.4661 | 53.39% | 0.1559 | 84.41% |
| GPSZRA402 | 0.7188 | 28.12% | 0.3439 | 65.61% | 0.1086 | 89.14% |
| GPSZRA403 | 0.9478 | 5.22% | 0.587 | 41.30% | 0.2731 | 72.69% |
| GPSZRA404 | 0.9108 | 8.92% | 0.5531 | 44.69% | 0.2326 | 76.74% |
| GPSZRA407 | 0.7846 | 21.54% | 0.3674 | 63.26% | 0.1516 | 84.84% |
| GPSZRA408 | 0.7575 | 24.25% | 0.3478 | 65.22% | 0.1226 | 87.74% |
| GPSZRA409 | 0.8282 | 17.18% | 0.4477 | 55.23% | 0.1193 | 88.07% |
| GPSZRA410 | 0.7063 | 29.37% | 0.291 | 70.90% | 0.1133 | 88.67% |
| GPSZRA411 | 0.7828 | 21.72% | 0.4 | 60.00% | 0.1598 | 84.02% |
| GPSZRA413 | 0.7714 | 22.86% | 0.6072 | 39.29% | 0.2623 | 73.77% |
| GPSZRA416 | 0.7497 | 25.03% | 0.5404 | 45.96% | 0.2308 | 76.92% |
| GPSZRA418 | 0.8511 | 14.89% | 0.3964 | 60.36% | 0.1804 | 81.96% |
| GPSZRA419 | 0.8481 | 15.19% | 0.3874 | 61.26% | 0.1593 | 84.07% |
| MOCK | 0.9813 | 1.87% | 1.0317 | -3.17% | 1.0597 | -5.97% |
| NC1 | 1.0000 | 0.00% | 1.0000 | 0.00% | 1.0000 | 0.00% |

MOCK was the blank control group containing only transfection reagent and plasmid; NC1 was the negative control siRNA, and the sequence information can be found in Table 7 in Example 1.

Conclusion: The candidate naked sequences demonstrated top-tier performance in the three-concentration psiCHECK assay. A total of 57 sequences were selected for the next round, which exhibited a silencing efficiency of over 30% at 0.01 nM, >75% at 0.1 nM, and >85% at 1 nM.

### Example 3: Detection of the on-target activity of modified siRNA for inhibiting RAGE

The sequences with high inhibition rates from Examples 1 and 2 were chemically modified at siRNA. The specific sequences are shown in Table 3, and the on-target activity assay was performed at the final siRNA concentrations of 0.1 nM and 1 nM. The experimental methods used were the same as those used in Example 1, except that the siRNAs were different. The detection results are presented in Table 9 below. The results demonstrate that these duplexes comprising chemical modifications exhibit potent inhibitory activity against RAGE expression at different concentrations.

**Table 9: On-target activity assay of modified siRNA at different concentrations**

| | 0.1 nM | | 1 nM | |
|---|---|---|---|---|
| Sequence name | Residual activity | Inhibition rate | Residual activity | Inhibition rate |
| GPSZRA084S1 | 0.8307 | 16.93% | 0.3455 | 65.46% |
| GPSZRA084S2 | 0.759 | 24.10% | 0.27 | 73.00% |
| GPSZRA084S3 | 0.7731 | 22.69% | 0.36 | 64.00% |
| GPSZRA084S4 | 0.7269 | 27.31% | 0.2237 | 77.63% |
| GPSZRA084S5 | 0.7729 | 22.71% | 0.3017 | 69.83% |
| GPSZRA087S1 | 0.6726 | 32.74% | 0.2552 | 74.48% |
| GPSZRA087S2 | 0.7511 | 24.89% | 0.441 | 55.90% |
| GPSZRA087S3 | 0.6731 | 32.69% | 0.3145 | 68.55% |
| GPSZRA087S4 | 0.6994 | 30.06% | 0.4078 | 59.22% |
| GPSZRA087S5 | 0.7317 | 26.83% | 0.4432 | 55.68% |
| GPSZRA092S1 | 0.5793 | 42.07% | 0.2168 | 78.32% |
| GPSZRA092S2 | 0.5957 | 40.43% | 0.1592 | 84.08% |
| GPSZRA092S3 | 0.5827 | 41.73% | 0.2052 | 79.48% |
| GPSZRA092S4 | 0.5232 | 47.68% | 0.1704 | 82.96% |
| GPSZRA092S5 | 0.6035 | 39.65% | 0.2232 | 77.68% |
| GPSZRA093S1 | 0.776 | 22.40% | 0.3252 | 67.48% |
| GPSZRA093S2 | 0.708 | 29.20% | 0.2585 | 74.15% |
| GPSZRA093S3 | 0.74 | 26.00% | 0.3994 | 60.06% |
| GPSZRA093S4 | 0.7822 | 21.78% | 0.3128 | 68.72% |
| GPSZRA093S5 | 0.8422 | 15.78% | 0.4779 | 52.21% |
| GPSZRA097S1 | 0.7219 | 27.81% | 0.2453 | 75.47% |
| GPSZRA097S2 | 0.7411 | 25.89% | 0.2657 | 73.43% |
| GPSZRA097S3 | 0.7831 | 21.69% | 0.4218 | 57.82% |
| GPSZRA097S4 | 0.7448 | 25.52% | 0.2753 | 72.47% |
| GPSZRA097S5 | 0.7477 | 25.23% | 0.3225 | 67.75% |
| GPSZRA118S1 | 0.9419 | 5.81% | 0.519 | 48.10% |
| GPSZRA118S2 | 0.8753 | 12.47% | 0.3804 | 61.97% |
| GPSZRA118S3 | 0.9018 | 9.82% | 0.4659 | 53.41% |
| GPSZRA118S4 | 0.9274 | 7.26% | 0.449 | 55.10% |
| GPSZRA118S5 | 0.9725 | 2.75% | 0.523 | 47.70% |
| GPSZRA217S1 | 0.83 | 17.00% | 0.4016 | 59.84% |
| GPSZRA217S2 | 0.7906 | 20.94% | 0.3239 | 67.61% |
| GPSZRA217S3 | 0.6537 | 34.63% | 0.3011 | 69.89% |
| GPSZRA217S4 | 0.7116 | 28.84% | 0.3229 | 67.71% |
| GPSZRA217S5 | 0.7149 | 28.51% | 0.3956 | 60.44% |
| GPSZRA222S1 | 0.3619 | 63.81% | 0.1402 | 85.98% |
| GPSZRA222S2 | 0.4253 | 57.47% | 0.2047 | 79.53% |
| GPSZRA222S3 | 0.437 | 56.30% | 0.1696 | 83.04% |
| GPSZRA222S4 | 0.5269 | 47.31% | 0.1899 | 81.01% |
| GPSZRA222S5 | 0.4875 | 51.25% | 0.2162 | 78.38% |
| GPSZRA227S1 | 0.4118 | 58.82% | 0.1609 | 83.91% |
| GPSZRA227S2 | 0.3171 | 68.29% | 0.1192 | 88.08% |
| GPSZRA227S3 | 0.3856 | 61.44% | 0.149 | 85.10% |
| GPSZRA227S4 | 0.3828 | 61.72% | 0.1481 | 85.19% |
| GPSZRA227S5 | 0.4003 | 59.97% | 0.1474 | 85.26% |
| GPSZRA228S1 | 0.5185 | 48.15% | 0.2959 | 70.41% |
| GPSZRA228S2 | 0.4085 | 59.15% | 0.2134 | 78.66% |
| GPSZRA228S3 | 0.4267 | 57.33% | 0.3135 | 68.65% |
| GPSZRA228S4 | 0.3438 | 65.62% | 0.1932 | 80.68% |
| GPSZRA228S5 | 0.2175 | 78.25% | 0.1971 | 80.29% |
| GPSZRA229S1 | 0.634 | 36.60% | 0.2377 | 76.23% |
| GPSZRA229S2 | 0.5036 | 49.64% | 0.182 | 81.80% |
| GPSZRA229S3 | 0.6006 | 39.94% | 0.2089 | 79.11% |
| GPSZRA229S4 | 0.5481 | 45.19% | 0.1784 | 82.16% |
| GPSZRA229S5 | 0.5975 | 40.25% | 0.1966 | 80.34% |
| GPSZRA231S1 | 0.3542 | 64.58% | 0.1173 | 88.27% |
| GPSZRA231S2 | 0.4164 | 58.36% | 0.1314 | 86.86% |
| GPSZRA231S3 | 0.3751 | 62.49% | 0.1327 | 86.73% |
| GPSZRA231S4 | 0.4068 | 59.32% | 0.1256 | 87.44% |
| GPSZRA231S5 | 0.336 | 66.40% | 0.1171 | 88.29% |
| GPSZRA232S1 | 0.7581 | 24.19% | 0.4011 | 59.89% |
| GPSZRA232S2 | 0.8593 | 14.07% | 0.4595 | 54.05% |
| GPSZRA232S3 | 0.868 | 13.20% | 0.5046 | 49.54% |
| GPSZRA232S4 | 0.8608 | 13.92% | 0.4025 | 59.75% |
| GPSZRA232S5 | 0.9187 | 8.13% | 0.4282 | 57.18% |
| GPSZRA233S1 | 0.2644 | 73.56% | 0.1298 | 87.02% |
| GPSZRA233S2 | 0.1846 | 81.54% | 0.1019 | 89.81% |
| GPSZRA233S3 | 0.2656 | 73.44% | 0.1726 | 82.74% |
| GPSZRA233S4 | 0.2525 | 74.75% | 0.1333 | 86.67% |
| GPSZRA233S5 | 0.2275 | 77.25% | 0.1165 | 88.35% |
| GPSZRA252S1 | 0.639 | 36.10% | 0.2948 | 70.52% |
| GPSZRA252S2 | 0.517 | 48.30% | 0.2598 | 74.02% |
| GPSZRA252S3 | 0.6382 | 36.18% | 0.2994 | 70.06% |
| GPSZRA252S4 | 0.66 | 34.00% | 0.2763 | 72.37% |
| GPSZRA252S5 | 0.6783 | 32.17% | 0.286 | 71.40% |
| GPSZRA257S1 | 0.7381 | 26.19% | 0.2482 | 75.18% |
| GPSZRA257S2 | 0.7998 | 20.02% | 0.3228 | 67.72% |
| GPSZRA257S3 | 0.7942 | 20.58% | 0.3273 | 67.27% |
| GPSZRA257S4 | 0.8109 | 18.91% | 0.3025 | 69.75% |
| GPSZRA257S5 | 0.7931 | 20.69% | 0.2978 | 70.22% |
| GPSZRA266S1 | 0.5809 | 41.91% | 0.2151 | 78.49% |
| GPSZRA266S2 | 0.6204 | 37.96% | 0.1946 | 80.54% |
| GPSZRA266S3 | 0.6815 | 31.85% | 0.2408 | 75.92% |
| GPSZRA266S4 | 0.6309 | 36.91% | 0.2043 | 79.57% |
| GPSZRA266S5 | 0.658 | 34.20% | 0.1958 | 80.42% |
| GPSZRA267S1 | 0.7913 | 20.87% | 0.2109 | 78.91% |
| GPSZRA267S2 | 0.6659 | 33.41% | 0.14 | 86.00% |
| GPSZRA267S3 | 0.7499 | 25.01% | 0.1965 | 80.35% |
| GPSZRA267S4 | 0.7602 | 23.98% | 0.1992 | 80.08% |
| GPSZRA267S5 | 0.6777 | 32.23% | 0.1568 | 84.32% |
| GPSZRA271S1 | 0.7684 | 23.16% | 0.1885 | 81.15% |
| GPSZRA271S2 | 0.5981 | 40.19% | 0.1248 | 87.52% |
| GPSZRA271S3 | 0.61 | 39.00% | 0.113 | 88.70% |
| GPSZRA271S4 | 0.6105 | 38.95% | 0.1218 | 87.82% |
| GPSZRA271S5 | 0.6669 | 33.31% | 0.1371 | 86.29% |
| GPSZRA272S1 | 0.5895 | 41.05% | 0.1255 | 87.45% |
| GPSZRA272S2 | 0.5733 | 42.67% | 0.1046 | 89.54% |
| GPSZRA272S3 | 0.7188 | 28.12% | 0.1392 | 86.08% |
| GPSZRA272S4 | 0.5454 | 45.46% | 0.1214 | 87.86% |
| GPSZRA272S5 | 0.6269 | 37.31% | 0.1108 | 88.92% |
| GPSZRA274S1 | 0.669 | 33.10% | 0.2249 | 77.51% |
| GPSZRA274S2 | 0.5553 | 44.47% | 0.222 | 77.80% |
| GPSZRA274S3 | 0.7023 | 29.77% | 0.4074 | 59.26% |
| GPSZRA274S4 | 0.6548 | 34.52% | 0.2276 | 77.24% |
| GPSZRA274S5 | 0.6196 | 38.04% | 0.2266 | 77.34% |
| GPSZRA275S1 | 0.4606 | 53.94% | 0.1461 | 85.39% |
| GPSZRA275S2 | 0.4979 | 50.21% | 0.2049 | 79.51% |
| GPSZRA275S3 | 0.5921 | 40.79% | 0.2936 | 70.64% |
| GPSZRA275S4 | 0.5696 | 43.04% | 0.3555 | 64.45% |
| GPSZRA275S5 | 0.6436 | 35.64% | 0.2528 | 74.72% |
| GPSZRA276S1 | 0.4175 | 58.25% | 0.1232 | 87.68% |
| GPSZRA276S2 | 0.6525 | 34.75% | 0.1818 | 81.82% |
| GPSZRA276S3 | 0.8308 | 16.92% | 0.349 | 65.10% |
| GPSZRA276S4 | 0.6747 | 32.53% | 0.2119 | 78.81% |
| GPSZRA276S5 | 0.7538 | 24.62% | 0.2269 | 77.31% |
| GPSZRA277S1 | 0.6775 | 32.25% | 0.215 | 78.50% |
| GPSZRA277S2 | 0.7406 | 25.94% | 0.2273 | 77.27% |
| GPSZRA277S3 | 0.7305 | 26.95% | 0.2624 | 73.76% |
| GPSZRA277S4 | 0.6456 | 35.44% | 0.1802 | 81.98% |
| GPSZRA277S5 | 0.7642 | 23.58% | 0.2248 | 77.52% |
| GPSZRA281S1 | 0.6039 | 39.61% | 0.1484 | 85.16% |
| GPSZRA281S2 | 0.5538 | 44.62% | 0.1427 | 85.73% |
| GPSZRA281S3 | 0.7541 | 24.59% | 0.2086 | 79.14% |
| GPSZRA281S4 | 0.4999 | 50.01% | 0.0933 | 90.67% |
| GPSZRA281S5 | 0.7557 | 24.43% | 0.2109 | 78.91% |
| GPSZRA282S1 | 0.456 | 54.40% | 0.1872 | 81.28% |
| GPSZRA282S2 | 0.5754 | 42.46% | 0.2061 | 79.39% |
| GPSZRA282S3 | 0.767 | 23.30% | 0.3885 | 61.15% |
| GPSZRA282S4 | 0.5526 | 44.74% | 0.1891 | 81.09% |
| GPSZRA282S5 | 0.6182 | 38.18% | 0.2658 | 73.42% |
| GPSZRA283S1 | 0.8504 | 14.96% | 0.273 | 72.70% |
| GPSZRA283S2 | 0.6937 | 30.63% | 0.1851 | 81.49% |
| GPSZRA283S3 | 0.721 | 27.90% | 0.2469 | 75.31% |
| GPSZRA283S4 | 0.7221 | 27.79% | 0.2062 | 79.38% |
| GPSZRA283S5 | 0.8046 | 19.54% | 0.299 | 70.10% |
| GPSZRA284S1 | 0.38 | 62.00% | 0.1266 | 87.34% |
| GPSZRA284S2 | 0.4545 | 54.55% | 0.1395 | 86.05% |
| GPSZRA284S3 | 0.3487 | 65.13% | 0.1046 | 89.54% |
| GPSZRA284S4 | 0.3379 | 66.21% | 0.0942 | 90.58% |
| GPSZRA284S5 | 0.4891 | 51.09% | 0.1357 | 86.43% |
| GPSZRA285S1 | 0.6417 | 35.83% | 0.1695 | 83.05% |
| GPSZRA285S2 | 0.4664 | 53.36% | 0.1255 | 87.45% |
| GPSZRA285S3 | 0.5674 | 43.26% | 0.1922 | 80.78% |
| GPSZRA285S4 | 0.5159 | 48.41% | 0.1596 | 84.04% |
| GPSZRA285S5 | 0.5733 | 42.67% | 0.2009 | 79.91% |
| GPSZRA286S1 | 0.8653 | 13.47% | 0.4569 | 54.31% |
| GPSZRA286S2 | 0.8812 | 11.88% | 0.4377 | 56.23% |
| GPSZRA286S3 | 0.8339 | 16.61% | 0.4482 | 55.18% |
| GPSZRA286S4 | 0.796 | 20.40% | 0.409 | 59.10% |
| GPSZRA286S5 | 0.7393 | 26.07% | 0.3605 | 63.95% |
| GPSZRA287S1 | 0.409 | 59.10% | 0.1419 | 85.81% |
| GPSZRA287S2 | 0.291 | 70.90% | 0.1163 | 88.37% |
| GPSZRA287S3 | 0.3053 | 69.47% | 0.1579 | 84.22% |
| GPSZRA287S4 | 0.2706 | 72.94% | 0.1132 | 88.68% |
| GPSZRA287S5 | 0.3272 | 67.28% | 0.1132 | 88.68% |
| GPSZRA288S1 | 0.7478 | 25.22% | 0.2515 | 74.85% |
| GPSZRA288S2 | 0.7809 | 21.91% | 0.2624 | 73.76% |
| GPSZRA288S3 | 0.8371 | 16.29% | 0.2994 | 70.06% |
| GPSZRA288S4 | 0.6937 | 30.63% | 0.1977 | 80.23% |
| GPSZRA288S5 | 0.7475 | 25.25% | 0.2699 | 73.01% |
| GPSZRA290S1 | 0.6468 | 35.32% | 0.1596 | 84.04% |
| GPSZRA290S2 | 0.651 | 34.90% | 0.1506 | 84.94% |
| GPSZRA290S3 | 0.6972 | 30.28% | 0.1821 | 81.79% |
| GPSZRA290S4 | 0.6822 | 31.78% | 0.178 | 82.20% |
| GPSZRA290S5 | 0.7248 | 27.52% | 0.1899 | 81.01% |
| GPSZRA292S1 | 0.4279 | 57.21% | 0.1412 | 85.89% |
| GPSZRA292S2 | 0.6643 | 33.57% | 0.2547 | 74.53% |
| GPSZRA292S3 | 0.6397 | 36.03% | 0.2819 | 71.81% |
| GPSZRA292S4 | 0.7126 | 28.74% | 0.267 | 73.30% |
| GPSZRA292S5 | 0.6873 | 31.27% | 0.2794 | 72.06% |
| GPSZRA301S1 | 0.5566 | 44.34% | 0.1817 | 81.83% |
| GPSZRA301S2 | 0.4199 | 58.01% | 0.1697 | 83.03% |
| GPSZRA301S3 | 0.6529 | 34.71% | 0.2251 | 77.49% |
| GPSZRA301S4 | 0.4099 | 59.01% | 0.1684 | 83.16% |
| GPSZRA301S5 | 0.538 | 46.20% | 0.1816 | 81.84% |
| GPSZRA308S1 | 0.7439 | 25.61% | 0.3158 | 68.42% |
| GPSZRA308S2 | 0.7644 | 23.56% | 0.274 | 72.60% |
| GPSZRA308S3 | 0.7495 | 25.05% | 0.4209 | 57.91% |
| GPSZRA308S4 | 0.6827 | 31.73% | 0.3864 | 61.36% |
| GPSZRA308S5 | 0.7241 | 27.59% | 0.3895 | 61.05% |
| GPSZRA311S1 | 0.7753 | 22.47% | 0.3176 | 68.24% |
| GPSZRA311S2 | 0.8006 | 19.94% | 0.3682 | 63.18% |
| GPSZRA311S3 | 0.7753 | 22.47% | 0.3763 | 62.37% |
| GPSZRA311S4 | 0.8402 | 15.98% | 0.4268 | 57.32% |
| GPSZRA311S5 | 0.9558 | 4.42% | 0.5423 | 45.77% |
| GPSZRA312S1 | 0.7365 | 26.35% | 0.3256 | 67.44% |
| GPSZRA312S2 | 0.7609 | 23.91% | 0.303 | 69.70% |
| GPSZRA312S3 | 0.7072 | 29.28% | 0.3318 | 66.82% |
| GPSZRA312S4 | 0.7347 | 26.53% | 0.2939 | 70.61% |
| GPSZRA312S5 | 0.749 | 25.10% | 0.3332 | 66.68% |
| GPSZRA313S1 | 0.699 | 30.10% | 0.2352 | 76.48% |
| GPSZRA313S2 | 0.7447 | 25.53% | 0.2834 | 71.66% |
| GPSZRA313S3 | 0.8192 | 18.08% | 0.4882 | 51.18% |
| GPSZRA313S4 | 0.7832 | 21.68% | 0.3075 | 69.25% |
| GPSZRA313S5 | 0.7696 | 23.04% | 0.3176 | 68.24% |
| GPSZRA314S1 | 0.2152 | 78.48% | 0.1077 | 89.23% |
| GPSZRA314S2 | 0.2945 | 70.55% | 0.1151 | 88.49% |
| GPSZRA314S3 | 0.2774 | 72.26% | 0.1097 | 89.03% |
| GPSZRA314S4 | 0.2992 | 70.08% | 0.1126 | 88.74% |
| GPSZRA314S5 | 0.2488 | 75.12% | 0.1155 | 88.45% |
| GPSZRA315S1 | 0.7315 | 26.85% | 0.289 | 71.10% |
| GPSZRA315S2 | 0.7167 | 28.33% | 0.2655 | 73.45% |
| GPSZRA315S3 | 0.6827 | 31.73% | 0.2771 | 72.29% |
| GPSZRA315S4 | 0.7178 | 28.22% | 0.2386 | 76.14% |
| GPSZRA315S5 | 0.8512 | 14.88% | 0.3779 | 62.21% |
| GPSZRA316S1 | 0.729 | 27.10% | 0.3059 | 69.41% |
| GPSZRA316S2 | 0.715 | 28.50% | 0.3767 | 62.33% |
| GPSZRA316S3 | 0.6463 | 35.37% | 0.2697 | 73.03% |
| GPSZRA316S4 | 0.6623 | 33.77% | 0.3013 | 69.87% |
| GPSZRA316S5 | 0.7213 | 27.87% | 0.3764 | 62.36% |
| GPSZRA317S1 | 0.6945 | 30.55% | 0.3838 | 61.62% |
| GPSZRA317S2 | 0.6927 | 30.73% | 0.4279 | 57.21% |
| GPSZRA317S3 | 0.7065 | 29.35% | 0.4437 | 55.63% |
| GPSZRA317S4 | 0.7398 | 26.02% | 0.4386 | 56.14% |
| GPSZRA317S5 | 0.7611 | 23.89% | 0.5487 | 45.13% |
| GPSZRA318S1 | 0.4395 | 56.05% | 0.0925 | 90.75% |
| GPSZRA318S2 | 0.4757 | 52.43% | 0.124 | 87.60% |
| GPSZRA318S3 | 0.5046 | 49.54% | 0.1282 | 87.18% |
| GPSZRA318S4 | 0.4777 | 52.23% | 0.1405 | 85.95% |
| GPSZRA318S5 | 0.6558 | 34.42% | 0.1928 | 80.72% |
| GPSZRA320S1 | 0.6384 | 36.16% | 0.2969 | 70.31% |
| GPSZRA320S2 | 0.6643 | 33.57% | 0.274 | 72.60% |
| GPSZRA320S3 | 0.7092 | 29.08% | 0.4574 | 54.26% |
| GPSZRA320S4 | 0.5909 | 40.91% | 0.3154 | 68.46% |
| GPSZRA320S5 | 0.6544 | 34.56% | 0.3117 | 68.83% |
| GPSZRA333S1 | 0.9036 | 9.64% | 0.4512 | 54.88% |
| GPSZRA333S2 | 0.8688 | 13.12% | 0.4381 | 56.19% |
| GPSZRA333S3 | 0.8264 | 17.36% | 0.5209 | 47.91% |
| GPSZRA333S4 | 0.9051 | 9.49% | 0.4061 | 59.39% |
| GPSZRA333S5 | 0.8887 | 11.13% | 0.4189 | 58.11% |
| GPSZRA342S1 | 0.734 | 26.60% | 0.321 | 67.90% |
| GPSZRA342S2 | 0.7811 | 21.89% | 0.3751 | 62.49% |
| GPSZRA342S3 | 0.8069 | 19.31% | 0.5388 | 46.12% |
| GPSZRA342S4 | 0.7623 | 23.77% | 0.3314 | 66.86% |
| GPSZRA342S5 | 0.8157 | 18.43% | 0.5039 | 49.61% |
| GPSZRA378S1 | 0.8745 | 12.55% | 0.4782 | 52.18% |
| GPSZRA378S2 | 0.8432 | 15.68% | 0.5683 | 43.17% |
| GPSZRA378S3 | 0.7684 | 23.16% | 0.4672 | 53.28% |
| GPSZRA378S4 | 0.7696 | 23.04% | 0.4402 | 55.98% |
| GPSZRA378S5 | 0.8584 | 14.16% | 0.4744 | 52.56% |
| GPSZRA379S1 | 0.5888 | 41.12% | 0.2011 | 79.89% |
| GPSZRA379S2 | 0.5918 | 40.82% | 0.1962 | 80.38% |
| GPSZRA379S3 | 0.6659 | 33.41% | 0.2178 | 78.22% |
| GPSZRA379S4 | 0.7647 | 23.53% | 0.2632 | 73.68% |
| GPSZRA379S5 | 0.7731 | 22.69% | 0.2709 | 72.91% |
| GPSZRA380S1 | 0.8037 | 19.63% | 0.3198 | 68.02% |
| GPSZRA380S2 | 0.9093 | 9.07% | 0.5112 | 48.88% |
| GPSZRA380S3 | 0.9454 | 5.46% | 0.5289 | 47.11% |
| GPSZRA380S4 | 0.8532 | 14.68% | 0.3955 | 60.45% |
| GPSZRA380S5 | 0.923 | 7.70% | 0.6162 | 38.38% |
| GPSZRA381S1 | 0.4644 | 53.56% | 0.1673 | 83.27% |
| GPSZRA381S2 | 0.4269 | 57.31% | 0.1491 | 85.09% |
| GPSZRA381S3 | 0.498 | 50.20% | 0.1802 | 81.98% |
| GPSZRA381S4 | 0.4657 | 53.43% | 0.2047 | 79.53% |
| GPSZRA381S5 | 0.5694 | 43.06% | 0.2079 | 79.21% |
| GPSZRA402S1 | 0.3177 | 68.23% | 0.1328 | 86.72% |
| GPSZRA402S2 | 0.2727 | 72.73% | 0.1206 | 87.94% |
| GPSZRA402S3 | 0.245 | 75.50% | 0.1062 | 89.38% |
| GPSZRA402S4 | 0.3158 | 68.42% | 0.1166 | 88.34% |
| GPSZRA402S5 | 0.2271 | 77.29% | 0.1054 | 89.46% |
| GPSZRA407S1 | 0.4767 | 52.33% | 0.1474 | 85.26% |
| GPSZRA407S2 | 0.554 | 44.60% | 0.1665 | 83.35% |
| GPSZRA407S3 | 0.6073 | 39.27% | 0.2183 | 78.17% |
| GPSZRA407S4 | 0.5088 | 49.12% | 0.177 | 82.30% |
| GPSZRA407S5 | 0.5946 | 40.54% | 0.2357 | 76.43% |
| GPSZRA408S1 | 0.8999 | 10.01% | 0.4585 | 54.15% |
| GPSZRA408S2 | 0.8696 | 13.04% | 0.365 | 63.50% |
| GPSZRA408S3 | 0.8078 | 19.22% | 0.2755 | 72.45% |
| GPSZRA408S4 | 0.7961 | 20.39% | 0.327 | 67.30% |
| GPSZRA408S5 | 0.8544 | 14.56% | 0.6066 | 39.34% |
| GPSZRA409S1 | 0.8822 | 11.78% | 0.4816 | 51.84% |
| GPSZRA409S2 | 0.927 | 7.30% | 0.4135 | 58.65% |
| GPSZRA409S3 | 0.8778 | 12.22% | 0.4168 | 58.32% |
| GPSZRA409S4 | 0.9037 | 9.63% | 0.4299 | 57.01% |
| GPSZRA409S5 | 0.924 | 7.60% | 0.5108 | 48.92% |
| GPSZRA410S1 | 0.6053 | 39.47% | 0.1495 | 85.05% |
| GPSZRA410S2 | 0.5645 | 43.55% | 0.1169 | 88.31% |
| GPSZRA410S3 | 0.6303 | 36.97% | 0.1422 | 85.78% |
| GPSZRA410S4 | 0.5899 | 41.01% | 0.1218 | 87.82% |
| GPSZRA410S5 | 0.7806 | 21.95% | 0.2529 | 74.71% |
| GPSZRA411S1 | 0.9682 | 3.18% | 0.5003 | 49.97% |
| GPSZRA411S2 | 0.6836 | 31.64% | 0.2397 | 76.03% |
| GPSZRA411S3 | 0.7664 | 23.36% | 0.287 | 71.30% |
| GPSZRA411S4 | 0.808 | 19.20% | 0.3185 | 68.15% |
| MOCK | 0.9954 | 0.46% | 0.9674 | 3.26% |
| NC1 | 1.0000 | 0.00% | 1.0000 | 0.00% |

MOCK was the blank control group containing only transfection reagent and plasmid; NC1 was the negative control siRNA, and the sequence information can be found in Table 19.

Conclusion: Each candidate sequence exhibited superior performance compared with other modification combinations except its own. A total of 102 sequences were selected for the next round, which satisfied the criteria of a silencing efficiency of over 60% at 0.1 nM and more than 80% at 1 nM.

### Example 4: Cellular-level verification of the inhibitory effect of siRNA on the target gene RAGE

### 4.1 Single-concentration assay

Experimental principle: siRNA is transfected into the selected cell line, and the intracellular siRNA interferes with and silences the target RAGE-mRNA. The relative expression level of the target RAGE protein gene in cells is determined by the relative quantification method; the activity of each sequence in silencing the endogenously expressed mRNA of the target RAGE protein gene is analyzed.

Experimental objective: To verify the inhibitory effect of siRNA on the target gene RAGE at the cellular level, and retain the sequences with superior efficacy.

The experimental steps are as follows:

### 1. Cell transfection

### (1) Experimental reagents, as shown in Table 10.

**Table 10: Experimental reagents used in the experiment**

| Reagent name | Manufacturer | Cat. No./Batch No. | Storage conditions |
|---|---|---|---|
| DEPC water | GenePharma | -/240088 | 4°C |
| Opti-MEM | Gibco | 31985-070/2537156 | 2-8°C |
| Lipofectamine RNAiMAX | ThermoFisher | 56532/CN2488104 | 4°C |
| F12K culture medium | GIBCO | 21127-022/2616994 | 4°C |
| DMEM | GIBCO | 11965-092/2646156 | 4°C |
| 0.25% trypsin | GIBCO | 25200-072/2468314 | 4°C |
| PBS | Sigma-Aldrich | 806552-500ML/RNBL3279 | 4°C |
| Buffer CRL | GenePharma | -/20220401 | Normal temperature |
| Buffer CRW0 | GenePharma | -/20220401 | Normal temperature |
| Buffer CRW1 | GenePharma | -/20220401 | Normal temperature |
| DNase I | GenePharma | -/20220401 | -20°C |
| Magnetic beads | Luoyang Giant-Bio | GNT-104/20220407 | Normal temperature |

### (2) Preparation before the experiment

Nanodrop was used to detect the purity and concentration of siRNA to ensure that the purity and concentration of siRNA meet the requirements for subsequent screening.

### (3) Cell preparation

The A549 cell line was obtained from the Cell Bank of the Chinese Academy of Sciences, while the BEAS-2B cell line was purchased from iCell Bioscience. A549 RAGE LV or BEAS-2B RAGE LV cells cultured in 10 cm culture dishes were routinely digested with trypsin 48 h after passage. The cells were resuspended in complete medium. A549 RAGE LV cells and BEAS-2B RAGE LV cells were seeded into a 96-well plate at a density of 2.8×10⁵/mL and 2.4×10⁵/mL, respectively, 50 µL/well, for subsequent experiments.

### (4) Cell transfection

A single concentration of 1 nM was set for siRNA; the concentration gradient for the three-concentration setting was 1 nM, 0.1 nM; for the IC50 assay, the concentration gradient was designed as 10 nM, 1 nM, 0.3 nM, 0.1 nM, 0.03 nM, 0.01 nM, 0.003 nM and 0.001 nM for transfection. The dosage of the transfection reagent Lipofectamine RNAiMAX was 0.25 µL /well. After accounting for reagent loss, the transfection complexes were prepared as follows: siRNA and Lipofectamine RNAiMAX transfection reagent were separately diluted in 25 µL of Opti-MEM^{®} per well. After incubation for 5 min, the two components were mixed thoroughly, followed by another incubation for 20 min.

When the incubation period expired, the transfection complexes were added to the corresponding wells at a volume of 50 µL/well, resulting in a final volume of 100 µL per well.

After transfection for approximately 12 h, the transfection complexes were aspirated and discarded, 100 µl of complete medium was added to each well and the plates were placed in the incubator for further culture.

At 48 h post-transfection, the transfected 96-well plate was taken out. The supernatant was discarded, and 100 µL of Buffer CRL was added to each well, followed by pipetting up and down to mix thoroughly, and then incubation at room temperature for 5 min.

### (5) RNA extraction

### 1) Sample loading to plates

Second plate: Lysed samples were added to each well, followed by the addition of 400 µL of isopropanol; the mixture was pipetted up and down to ensure thorough mixing, and then 20 µL of magnetic beads was added (the magnetic beads must be pipetted up and down to achieve homogenization to prevent aggregation and avoid reducing the bead adsorption efficiency);
Third plate: 900 µL of Buffer CRW 0 was added to each well;
First plate: 200 µL of DNase I reaction solution was added to each well;
Fourth plate: 900 µL of Buffer CRW 0 was added to each well;
Fifth plate: 900 µL of Buffer CRW 1 was added to each well;
Sixth plate: 100 µL of DEPC water was added to each well.

### 2) Instrument running protocol, as shown in Table 11.

**Table 11: Automated magnetic bead-based extraction instrument running protocol**

| 7 steps in total | Step 01 | Step 02 | Step 03 | Step 04 | Step 05 | Step 06 | Step 07 |
|---|---|---|---|---|---|---|---|
| Station (plate sequence) | 2 | 3 | 1 | 4 | 5 | 6 | 2 |
| Name | lysis | wash1 | DNaseI digestion | wash2 | wash3 | Elution | Discard |
| Volume (µL) | 900 | 900 | 100 | 900 | 900 | 100 | 200 |
| Mixing method | Medium speed | Medium speed | Customized | Medium speed | Medium speed | Medium speed | High Speed |
| Mixing time | 5 min | 2 min | 6 min x3 | 2 min | 2 min | 5 min 30 s | 15 s |
| Adsorption mode | High intensity | High intensity | High intensity | High intensity | High intensity | High intensity | |
| Adsorption time | 1 min 30 s | 35 s | 35 s | 30 s | 30 s | 30 s | |
| Temperature | | | 37°C | | | 60°C | |

3) The RNA was temporarily stored at 4°C and used for PCR experiments within one day. (For long-term storage, it should be placed at -80°C).

### 2. qRT-PCR assay

GAPDH was used as an internal reference for the analysis of relative expression levels.
(1) The primers used in the qRT-PCR assay are shown in Table 12, and the experimental reagents are shown in Table 13.

**Table 12: Primers used in qRT-PCR assay**

| Gene name | Primer name | Sequence | SEQ ID NO: |
|---|---|---|---|
| GAPDH (For internal reference) | HGAPDH-FO | CATGAGAAGTATGACAACAGCCT | 1439 |
| | HGAPDH-RE | AGTCCTTCCACGATACCAAAGT | 1440 |
| | HGAPDH-PR | CAATGCCTCCTGCACCACCAA | 1441 |
| RAGE | RAGE-F9 | GTGTCCTTCCCAACGGCTC | 1442 |
| | RAGE-R9 | ATTGCCTGGCACCGGAAAA | 1443 |
| | RAGE-P9.1 | CCGGCTGTCGGGATCCAGGAT | 1444 |

**Table 13: Reagents used and their sources**

| Reagent name | Manufacturer | Cat. No./Batch No. | Storage conditions |
|---|---|---|---|
| HiScript II U+ One Step qRT-PCR Probe Kit | Vazyme | Q223-01/017E2290JA | -20°C |
| H-RAGE-F | Svnbio Tech | / | -20°C |
| H-RAGE-R | Svnbio Tech | / | -20°C |
| H-RAGE-PR | GenePharma | / | -20°C |
| GAPDH-F | Svnbio Tech | / | -20°C |
| GAPDH-R | Svnbio Tech | / | -20°C |
| GAPDH-PR | GenePharma | / | -20°C |

### (2) gDNA removal, with the reaction system detailed in Table 14.

**Table 14: Reaction system**

| System | Volume per reaction |
|---|---|
| 10 x Buffer | 2µL |
| DNase 1 | 2µL |
| RNA template | 16µL |

20 µL of the reaction solution was incubated at 37°C for 15 min to remove genomic DNA, then 2 µL of EDTA was added.

### (3) PCR reaction system, as shown in Table 15

**Table 15: PCR reaction system**

| PCR reaction solution | 13ul system |
|---|---|
| 2 × Mix | 6.5 |
| Enzyme Mix | 0.65 |
| RAGE-9-F (10 µM) | 0.26 |
| RAGE-9-R (10 µM) | 0.26 |
| RAGE-9.1 PR(10 µM) JOE | 0.65 |
| HGAPDH-F (2 µM) | 0.325 |
| HGAPDH-R (2 µM) | 0.325 |
| HGAPDH-PR(2 µM)FAM | 0.325 |
| ddH2O | 0.455 |
| RNA template (40ng) | 3.25 |

### (4) PCR reaction procedure, as shown in Table 16.

**Table 16: PCR reaction procedure**

| Step | Temperature | Time | Cycle number |
|---|---|---|---|
| 1 | 50°C | 10 min | 1 |
| | 95°C | 30 s | |
| 2 | 95°C | 15 s | 40 |
| | 58°C | 30 s | |
| | 72°C | 30 s (fluorescence collection) | |

The experimental results are presented below:

**Table 17: Comparison of candidate sequences and different modifications**

| Test sequence | A549 RAGE LV | | BEAS-2B | |
|---|---|---|---|---|
| | 1nM inhibition rate | SD | 1nM inhibition rate | SD |
| GPSZRA222S1 | 93.77% | 0.0039 | 91.15% | 0.0066 |
| GPSZRA222S3 | 93.28% | 0.0082 | 89.98% | 0.0163 |
| GPSZRA222S4 | 93.07% | 0.0136 | 89.70% | 0.0205 |
| GPSZRA227S1 | 91.21% | 0.0186 | 88.45% | 0.0309 |
| GPSZRA227S2 | 91.98% | 0.0202 | 86.80% | 0.0230 |
| GPSZRA227S3 | 88.39% | 0.0306 | 71.37% | 0.1463 |
| GPSZRA227S4 | 89.39% | 0.0201 | 86.46% | 0.0184 |
| GPSZRA227S5 | 90.40% | 0.0097 | 82.34% | 0.0769 |
| GPSZRA267S2 | 88.02% | 0.0171 | 84.96% | 0.0292 |
| GPSZRA267S3 | 85.20% | 0.0177 | 78.43% | 0.0754 |
| GPSZRA267S4 | 83.02% | 0.0325 | 70.68% | 0.0463 |
| GPSZRA267S5 | 87.68% | 0.0042 | 81.35% | 0.0363 |
| GPSZRA284S1 | 89.74% | 0.0035 | 66.34% | 0.1353 |
| GPSZRA284S2 | 85.07% | 0.0192 | 73.48% | 0.0847 |
| GPSZRA284S3 | 90.61% | 0.0063 | 76.70% | 0.0597 |
| GPSZRA284S4 | 90.13% | 0.0080 | 73.03% | 0.1239 |
| GPSZRA284S5 | 89.61% | 0.0157 | 77.40% | 0.0720 |
| GPSZRA314S1 | 89.63% | 0.0157 | 78.94% | 0.0201 |
| GPSZRA314S2 | 90.95% | 0.0223 | 78.41% | 0.0217 |
| GPSZRA314S3 | 91.52% | 0.0058 | 76.40% | 0.0240 |
| GPSZRA314S4 | 91.56% | 0.0013 | 78.82% | 0.0262 |
| GPSZRA314S5 | 91.04% | 0.0076 | 76.37% | 0.0209 |
| GPSZRA318S1 | 89.68% | 0.0093 | 68.65% | 0.1816 |
| GPSZRA318S2 | 90.31% | 0.0044 | 77.33% | 0.0083 |
| GPSZRA318S3 | 89.82% | 0.0022 | 71.45% | 0.0773 |
| GPSZRA318S4 | 90.79% | 0.0146 | 79.59% | 0.0809 |
| GPSZRA318S5 | 89.81% | 0.0220 | 80.07% | 0.0654 |
| GPSZRA402S1 | 89.02% | 0.0119 | 81.80% | 0.0714 |
| GPSZRA402S2 | 90.34% | 0.0057 | 83.65% | 0.0538 |
| GPSZRA402S3 | 90.55% | 0.0090 | 80.72% | 0.0540 |
| GPSZRA402S4 | 90.62% | 0.0108 | 78.92% | 0.0392 |
| GPSZRA402S5 | 89.89% | 0.0081 | 80.67% | 0.0628 |

### Summary of all sequences:

**Table 18: Summary of all sequences**

| Test sequence | A549 RAGE LV | | BEAS-2B RAGE LV | |
|---|---|---|---|---|
| | 1nM inhibition rate | SD | 1nM inhibition rate | SD |
| GPSZRA92S2 | 73.97% | 0.0151 | 63.76% | 0.0251 |
| GPSZRA92S4 | 79.60% | 0.0158 | 65.46% | 0.0626 |
| GPSZRA222S1 | 93.77% | 0.0039 | 91.15% | 0.0066 |
| GPSZRA222S3 | 93.28% | 0.0082 | 89.98% | 0.0163 |
| GPSZRA222S4 | 93.07% | 0.0136 | 89.70% | 0.0205 |
| GPSZRA227S1 | 91.21% | 0.0186 | 88.45% | 0.0309 |
| GPSZRA227S2 | 91.98% | 0.0202 | 86.80% | 0.0230 |
| GPSZRA227S3 | 88.39% | 0.0306 | 71.37% | 0.1463 |
| GPSZRA227S4 | 89.39% | 0.0201 | 86.46% | 0.0184 |
| GPSZRA227S5 | 90.40% | 0.0097 | 82.34% | 0.0769 |
| GPSZRA228S4 | 76.78% | 0.0307 | 56.86% | 0.1827 |
| GPSZRA228S5 | 84.52% | 0.0166 | 59.50% | 0.1327 |
| GPSZRA229S2 | 81.68% | 0.0441 | 72.58% | 0.0737 |
| GPSZRA229S4 | 78.91% | 0.0558 | 71.02% | 0.0896 |
| GPSZRA229S5 | 73.90% | 0.0651 | 59.92% | 0.1496 |
| GPSZRA231S1 | 86.47% | 0.0308 | 81.66% | 0.0405 |
| GPSZRA231S2 | 84.81% | 0.0236 | 83.54% | 0.0200 |
| GPSZRA231S3 | 77.61% | 0.0280 | 78.55% | 0.0340 |
| GPSZRA231S4 | 85.39% | 0.0623 | 80.82% | 0.0437 |
| GPSZRA231S5 | 87.19% | 0.0081 | 85.41% | 0.0472 |
| GPSZRA233S1 | 85.42% | 0.0286 | 87.65% | 0.0503 |
| GPSZRA233S2 | 87.64% | 0.0481 | 85.44% | 0.0244 |
| GPSZRA233S3 | 90.83% | 0.0157 | 87.59% | 0.0233 |
| GPSZRA233S4 | 90.30% | 0.0064 | 83.58% | 0.0555 |
| GPSZRA233S5 | 90.57% | 0.0347 | 80.64% | 0.0560 |
| GPSZRA266S2 | 79.78% | 0.0266 | 74.83% | 0.0137 |
| GPSZRA266S5 | 76.05% | 0.0554 | 68.75% | 0.0572 |
| GPSZRA267S2 | 88.02% | 0.0171 | 84.96% | 0.0292 |
| GPSZRA267S3 | 85.20% | 0.0177 | 78.43% | 0.0754 |
| GPSZRA267S4 | 83.02% | 0.0325 | 70.68% | 0.0463 |
| GPSZRA267S5 | 87.68% | 0.0042 | 81.35% | 0.0363 |
| GPSZRA271S1 | 82.56% | 0.0148 | 74.01% | 0.0290 |
| GPSZRA271S2 | 85.52% | 0.0416 | 81.82% | 0.0486 |
| GPSZRA271S3 | 85.00% | 0.0188 | 75.89% | 0.0495 |
| GPSZRA271S4 | 85.24% | 0.0179 | 78.42% | 0.0450 |
| GPSZRA271S5 | 87.25% | 0.0243 | 78.86% | 0.0689 |
| GPSZRA272S1 | 82.36% | 0.0212 | 65.89% | 0.1120 |
| GPSZRA272S2 | 81.86% | 0.0113 | 59.71% | 0.1174 |
| GPSZRA272S3 | 82.92% | 0.0184 | 54.99% | 0.1357 |
| GPSZRA272S4 | 82.86% | 0.0209 | 61.18% | 0.1395 |
| GPSZRA272S5 | 82.03% | 0.0065 | 53.01% | 0.1638 |
| GPSZRA275S1 | 73.11% | 0.0239 | 46.81% | 0.1406 |
| GPSZRA276S1 | 91.07% | 0.0775 | 62.02% | 0.0561 |
| GPSZRA276S2 | 75.60% | 0.0414 | 46.62% | 0.0507 |
| GPSZRA277S4 | 79.32% | 0.0201 | 57.62% | 0.1230 |
| GPSZRA281S1 | 80.76% | 0.0038 | 70.56% | 0.0995 |
| GPSZRA281S2 | 80.60% | 0.0184 | 65.22% | 0.1087 |
| GPSZRA281S3 | 74.89% | 0.0135 | 39.81% | 0.1253 |
| GPSZRA282S1 | 73.66% | 0.0517 | 50.00% | 0.0969 |
| GPSZRA282S4 | 80.46% | 0.0493 | 65.74% | 0.0907 |
| GPSZRA283S2 | 78.40% | 0.0340 | 53.00% | 0.1197 |
| GPSZRA284S1 | 89.74% | 0.0035 | 66.34% | 0.1353 |
| GPSZRA284S2 | 85.07% | 0.0192 | 73.48% | 0.0847 |
| GPSZRA284S3 | 90.61% | 0.0063 | 76.70% | 0.0597 |
| GPSZRA284S4 | 90.13% | 0.0080 | 73.03% | 0.1239 |
| GPSZRA284S5 | 89.61% | 0.0157 | 77.40% | 0.0720 |
| GPSZRA285S1 | 64.91% | 0.0651 | 41.05% | 0.2506 |
| GPSZRA285S2 | 75.79% | 0.0295 | 37.69% | 0.1755 |
| GPSZRA285S3 | 74.14% | 0.0086 | 24.54% | 0.1842 |
| GPSZRA285S4 | 70.90% | 0.0103 | 44.62% | 0.0966 |
| GPSZRA287S1 | 80.38% | 0.0122 | 56.35% | 0.0955 |
| GPSZRA287S2 | 88.06% | 0.0103 | 68.09% | 0.1131 |
| GPSZRA287S3 | 88.42% | 0.0017 | 69.21% | 0.0338 |
| GPSZRA287S4 | 88.41% | 0.0151 | 76.81% | 0.0376 |
| GPSZRA287S5 | 86.00% | 0.0165 | 71.49% | 0.0752 |
| GPSZRA288S4 | 86.32% | 0.0239 | 69.66% | 0.0668 |
| GPSZRA290S1 | 82.69% | 0.0095 | 61.34% | 0.1368 |
| GPSZRA290S2 | 81.38% | 0.0242 | 48.73% | 0.1863 |
| GPSZRA290S3 | 77.99% | 0.0126 | 25.01% | 0.0697 |
| GPSZRA290S4 | 68.51% | 0.1169 | 15.52% | 0.0991 |
| GPSZRA290S5 | 81.42% | 0.0054 | 52.86% | 0.1411 |
| GPSZRA292S1 | 84.52% | 0.0169 | 69.58% | 0.0724 |
| GPSZRA301S1 | 86.70% | 0.0131 | 66.99% | 0.0614 |
| GPSZRA301S2 | 90.84% | 0.0057 | 65.58% | 0.0170 |
| GPSZRA301S4 | 89.30% | 0.0053 | 67.67% | 0.0354 |
| GPSZRA301S5 | 88.47% | 0.0100 | 61.16% | 0.1199 |
| GPSZRA314S1 | 89.63% | 0.0157 | 78.94% | 0.0201 |
| GPSZRA314S2 | 90.95% | 0.0223 | 78.41% | 0.0217 |
| GPSZRA314S3 | 91.52% | 0.0058 | 76.40% | 0.0240 |
| GPSZRA314S4 | 91.56% | 0.0013 | 78.82% | 0.0262 |
| GPSZRA314S5 | 91.04% | 0.0076 | 76.37% | 0.0209 |
| GPSZRA318S1 | 89.68% | 0.0093 | 68.65% | 0.1816 |
| GPSZRA318S2 | 90.31% | 0.0044 | 77.33% | 0.0083 |
| GPSZRA318S3 | 89.82% | 0.0022 | 71.45% | 0.0773 |
| GPSZRA318S4 | 90.79% | 0.0146 | 79.59% | 0.0809 |
| GPSZRA318S5 | 89.81% | 0.0220 | 80.07% | 0.0654 |
| GPSZRA379S2 | 81.33% | 0.0347 | 77.57% | 0.0811 |
| GPSZRA381S1 | 89.13% | 0.0064 | 82.38% | 0.0558 |
| GPSZRA381S2 | 89.44% | 0.0056 | 78.48% | 0.0751 |
| GPSZRA381S3 | 87.60% | 0.0344 | 76.85% | 0.0620 |
| GPSZRA402S1 | 89.02% | 0.0119 | 81.80% | 0.0714 |
| GPSZRA402S2 | 90.34% | 0.0057 | 83.65% | 0.0538 |
| GPSZRA402S3 | 90.55% | 0.0090 | 80.72% | 0.0540 |
| GPSZRA402S4 | 90.62% | 0.0108 | 78.92% | 0.0392 |
| GPSZRA402S5 | 89.89% | 0.0081 | 80.67% | 0.0628 |
| GPSZRA407S1 | 87.57% | 0.0156 | 75.64% | 0.0605 |
| GPSZRA407S2 | 84.64% | 0.0172 | 76.02% | 0.0691 |
| GPSZRA407S3 | 87.13% | 0.0056 | 73.28% | 0.0703 |
| GPSZRA410S1 | 89.04% | 0.0061 | 78.23% | 0.0154 |
| GPSZRA410S2 | 89.02% | 0.0054 | 73.61% | 0.0671 |
| GPSZRA410S3 | 86.10% | 0.0082 | 69.32% | 0.0875 |
| GPSZRA410S4 | 88.72% | 0.0148 | 79.52% | 0.0714 |
| NC5 | 8.39% | 0.1234 | -6.41% | 0.3357 |
| MOCK | -0.34% | 0.1005 | -4.03% | 0.3287 |
| BLANK | 8.41% | 0.0906 | -3.45% | 0.2104 |

MOCK was the blank control group containing only transfection reagent and plasmid; NC5 was the negative control siRNA, the sequence information can be found in Table 4, BLANK: no substances were added.

Note: NC1 is a meaningless naked sequence, serving as the negative control for naked sequences in the psiCHECK assay, and has been consistently used in subsequent screening of modified psiCHECK sequences. NC5 is a meaningless sequence with modifications. Given that all screening sequences in the cell line are modified, NC5 is used as the negative control in all cell experiments. The sequences of NC1 and NC5 are shown in Table 19.

**Table 19: NC series sequences**

| | SEQ ID NO: | Sense strand (5'-3') | SEQ ID NO: | Antisense strand (5'-3') |
|---|---|---|---|---|
| NC1 | 1422 | UUCUCCGAACGUGUCACGUUU | 1421 | AAACGUGACACGUUCGGAGAAUU |
| NC5 | 1446 | CfsAmsCfUmUfAmCfGmCfUmGfA mGfUmAfCmUfUmCfGmAf | 1445 | UmsCfsGmAfAmGfUmAfCmUfCmAfGmCf GmUfAmAfGmUfGmsAfsUm |

Conclusion: A total of 59 sequences were selected for the next round, which exhibited a RAGE LV silencing efficiency of over 90% in A549 cells and over 80% in BEAS-2B cells: GPSZRA222S1, GPSZRA222S3, GPSZRA222S4, GPSZRA227S1, GPSZRA227S2, GPSZRA227S3, GPSZRA227S4, GPSZRA227S5, GPSZRA228S5, GPSZRA231S1, GPSZRA231S2, GPSZRA231S3, GPSZRA231S4, GPSZRA231S5, GPSZRA233S1, GPSZRA233S2, GPSZRA233S3, GPSZRA233S4, GPSZRA233S5, GPSZRA267S2, GPSZRA267S3, GPSZRA267S5, GPSZRA271S1, GPSZRA271S2, GPSZRA271S3, GPSZRA271S4, GPSZRA271S5, GPSZRA282S4, GPSZRA284S2, GPSZRA284S3, GPSZRA284S4, GPSZRA284S5, GPSZRA287S4, GPSZRA287S5, GPSZRA301S2, GPSZRA314S1, GPSZRA314S2, GPSZRA314S3, GPSZRA314S4, GPSZRA314S5, GPSZRA318S2, GPSZRA318S3, GPSZRA318S4, GPSZRA318S5, GPSZRA381S1, GPSZRA381S2, GPSZRA381S3, GPSZRA402S1, GPSZRA402S2, GPSZRA402S3, GPSZRA402S4, GPSZRA402S5, GPSZRA407S1, GPSZRA407S2, GPSZRA407S4, GPSZRA410S1, GPSZRA410S2, GPSZRA410S3, GPSZRA410S4.

### 4.2 Two-concentration assay

The 59 sequences obtained from screening in 4.1 were then screened in the next round. The experimental steps were the same as in 4.1, except that the screening was carried out at two concentrations: 0.1 nM and 1 nM.

The experimental results are shown in Table 19.

**Table 19: Summary of sequences from the two-concentration assay**

| Test sequence | A549 RAGE LV | | | | BEAS-2B RAGE LV | | | |
|---|---|---|---|---|---|---|---|---|
| | 0.1nM Inhibition rate | SD | 1nM Inhibition rate | SD | 0.1nM Inhibition rate | SD | 1nM Inhibition rate | SD |
| GPSZRA222S1 | 82.17% | 0.0118 | 84.17% | 0.0582 | 68.08% | 0.0618 | 90.97% | 0.0125 |
| GPSZRA222S3 | 84.28% | 0.0193 | 91.91% | 0.0199 | 51.32% | 0.2023 | 88.58% | 0.0394 |
| GPSZRA222S4 | 73.84% | 0.0624 | 93.37% | 0.0164 | 67.74% | 0.0352 | 83.06% | 0.1078 |
| GPSZRA227S1 | 69.58% | 0.0392 | 92.05% | 0.0226 | 51.33% | 0.0936 | 87.82% | 0.0322 |
| GPSZRA227S2 | 42.12% | 0.2530 | 92.18% | 0.0173 | 54.89% | 0.0963 | 87.75% | 0.0641 |
| GPSZRA227S4 | 65.41% | 0.1085 | 91.42% | 0.0217 | 56.03% | 0.0878 | 86.57% | 0.0433 |
| GPSZRA227S5 | 63.58% | 0.1450 | 91.06% | 0.0321 | 38.49% | 0.1726 | 88.65% | 0.1000 |
| GPSZRA231S1 | 10.90% | 0.6498 | 84.31% | 0.0147 | 1.31% | 0.1942 | 73.39% | 0.0414 |
| GPSZRA231S2 | 39.32% | 0.1299 | 83.48% | 0.0532 | 40.91% | 0.0956 | 66.56% | 0.1499 |
| GPSZRA231S4 | 46.12% | 0.0540 | 85.51% | 0.0386 | -0.44% | 0.2452 | 81.30% | 0.0094 |
| GPSZRA231S5 | 56.00% | 0.1588 | 87.23% | 0.0299 | 35.93% | 0.1011 | 87.51% | 0.1105 |
| GPSZRA233S1 | 42.78% | 0.1645 | 87.07% | 0.0326 | 10.53% | 0.1246 | 87.19% | 0.1153 |
| GPSZRA233S2 | 56.92% | 0.0261 | 85.76% | 0.0359 | 40.25% | 0.1156 | 86.36% | 0.0170 |
| GPSZRA233S3 | 75.07% | 0.0102 | 76.73% | 0.1382 | 53.05% | 0.1149 | 85.37% | 0.0381 |
| GPSZRA233S4 | 62.75% | 0.3666 | 88.19% | 0.0312 | 45.10% | 0.1995 | 84.91% | 0.0319 |
| GPSZRA233S5 | 62.12% | 0.0703 | 86.80% | 0.0310 | 33.85% | 0.1904 | 83.09% | 0.0161 |
| GPSZRA271S2 | 47.88% | 0.0990 | 82.60% | 0.0287 | 40.51% | 0.2648 | 82.29% | 0.0399 |
| GPSZRA267S2 | 54.12% | 0.0690 | 85.60% | 0.0291 | 38.03% | 0.1233 | 75.54% | 0.0450 |
| GPSZRA267S5 | 35.10% | 0.1559 | 84.76% | 0.0273 | 25.86% | 0.1055 | 74.35% | 0.0490 |
| GPSZRA284S3 | 73.43% | 0.0203 | 86.89% | 0.0256 | 49.04% | 0.0736 | 70.78% | 0.0250 |
| GPSZRA284S4 | 61.35% | 0.1035 | 83.00% | 0.0579 | 42.72% | 0.0432 | 79.38% | 0.0330 |
| GPSZRA301S2 | 56.81% | 0.0457 | 77.27% | 0.0880 | 21.78% | 0.1178 | 63.14% | 0.0615 |
| GPSZRA314S2 | 69.42% | 0.0218 | 82.03% | 0.0534 | 63.28% | 0.0975 | 83.18% | 0.0307 |
| GPSZRA314S3 | 66.02% | 0.0735 | 85.25% | 0.0087 | 59.86% | 0.0313 | 78.59% | 0.0574 |
| GPSZRA314S4 | 63.05% | 0.0441 | 82.20% | 0.0458 | 53.53% | 0.1930 | 84.98% | 0.0292 |
| GPSZRA314S5 | 67.94% | 0.1530 | 86.47% | 0.0356 | 62.50% | 0.0733 | 86.87% | 0.0171 |
| GPSZRA318S2 | 64.69% | 0.1536 | 86.43% | 0.0369 | 64.24% | 0.0497 | 83.73% | 0.0208 |
| GPSZRA318S4 | 60.84% | 0.1335 | 84.43% | 0.0541 | 60.15% | 0.0932 | 84.33% | 0.0421 |
| GPSZRA318S5 | 48.75% | 0.0764 | 77.90% | 0.0574 | 44.52% | 0.0080 | 80.42% | 0.0342 |
| GPSZRA381S1 | 67.89% | 0.0901 | 83.36% | 0.0263 | 46.86% | 0.1287 | 79.11% | 0.0624 |
| GPSZRA402S1 | 59.59% | 0.0307 | 85.60% | 0.0401 | 50.26% | 0.1252 | 79.89% | 0.0470 |
| GPSZRA402S2 | 73.40% | 0.0406 | 76.67% | 0.1662 | 64.83% | 0.0477 | 80.14% | 0.0149 |
| GPSZRA402S3 | 60.73% | 0.0968 | 81.50% | 0.0204 | 51.27% | 0.0643 | 80.65% | 0.0303 |
| GPSZRA402S4 | 70.67% | 0.0856 | 84.46% | 0.0550 | 67.84% | 0.0494 | 82.46% | 0.0148 |
| GPSZRA227S3 | 30.32% | 0.2013 | 67.58% | 0.0332 | 38.95% | 0.2120 | 82.20% | 0.0634 |
| GPSZRA228S5 | 11.34% | 0.1743 | 59.02% | 0.0766 | 17.94% | 0.2440 | 76.75% | 0.0380 |
| GPSZRA231S3 | 32.67% | 0.0738 | 61.50% | 0.0570 | 42.05% | 0.1385 | 81.92% | 0.0565 |
| GPSZRA267S3 | 15.73% | 0.1349 | 69.07% | 0.0825 | 35.46% | 0.2069 | 79.21% | 0.0702 |
| GPSZRA271S1 | 18.34% | 0.1400 | 62.73% | 0.0175 | 17.30% | 0.2817 | 77.42% | 0.0820 |
| GPSZRA271S3 | -10.16% | 0.2384 | 69.53% | 0.1066 | 43.15% | 0.2277 | 79.99% | 0.0274 |
| GPSZRA271S4 | 2.51% | 0.5129 | 74.34% | 0.0432 | -10.75% | 0.4277 | 83.48% | 0.0485 |
| GPSZRA271S5 | 39.80% | 0.1400 | 68.26% | 0.0484 | 11.71% | 0.7649 | 81.52% | 0.0602 |
| GPSZRA282S4 | -15.07% | 0.2850 | 54.59% | 0.1868 | -7.67% | 0.4797 | 76.39% | 0.0485 |
| GPSZRA284S2 | 32.45% | 0.3461 | 69.75% | 0.1411 | -3.67% | 0.4591 | 79.57% | 0.0403 |
| GPSZRA284S5 | 43.79% | 0.0391 | 81.02% | 0.0146 | 18.72% | 0.3635 | 85.92% | 0.0578 |
| GPSZRA287S4 | 39.71% | 0.0869 | 80.33% | 0.0401 | 39.08% | 0.0090 | 84.06% | 0.0712 |
| GPSZRA287S5 | 13.51% | 0.1336 | 70.15% | 0.0361 | 12.61% | 0.4940 | 82.68% | 0.0582 |
| GPSZRA314S1 | 46.46% | 0.0899 | 80.72% | 0.0500 | 4.82% | 0.4194 | 89.69% | 0.0339 |
| GPSZRA318S3 | 55.52% | 0.0233 | 80.65% | 0.0276 | 65.06% | 0.1255 | 86.74% | 0.0608 |
| GPSZRA381S2 | 46.54% | 0.0630 | 78.80% | 0.0223 | 72.23% | 0.1010 | 89.29% | 0.0270 |
| GPSZRA381S3 | 39.80% | 0.1028 | 82.99% | 0.0345 | 73.63% | 0.0871 | 85.99% | 0.0524 |
| GPSZRA402S5 | 65.48% | 0.1223 | 84.50% | 0.0284 | 80.57% | 0.0613 | 85.96% | 0.0520 |
| GPSZRA407S1 | 34.36% | 0.1575 | 76.17% | 0.1295 | 57.34% | 0.2350 | 83.12% | 0.0375 |
| GPSZRA407S2 | 59.46% | 0.0515 | 76.36% | 0.0776 | 68.38% | 0.0378 | 84.63% | 0.0496 |
| GPSZRA407S4 | 44.27% | 0.2028 | 75.82% | 0.0827 | 62.09% | 0.0836 | 85.95% | 0.0324 |
| GPSZRA410S1 | 48.35% | 0.0668 | 77.27% | 0.0532 | 74.31% | 0.0432 | 86.41% | 0.0229 |
| GPSZRA410S2 | 40.59% | 0.1033 | 83.59% | 0.0662 | 65.82% | 0.0924 | 85.72% | 0.0435 |
| GPSZRA410S3 | 33.13% | 0.1292 | 74.10% | 0.0407 | 54.69% | 0.0871 | 82.05% | 0.0586 |
| GPSZRA410S4 | 58.14% | 0.0283 | 84.19% | 0.0219 | 67.28% | 0.0651 | 87.33% | 0.0259 |
| NC5 | 9.91% | 0.4060 | 0.16% | 0.3012 | 17.58% | 0.0614 | 1.74% | 0.1459 |
| MOCK | -2.74% | 0.2765 | -2.74% | 0.2765 | -2.88% | 0.2860 | -2.88% | 0.2860 |
| BLANK | -16.14% | 0.2357 | -16.14% | 0.2357 | 1.77% | 0.0713 | 1.77% | 0.0713 |

MOCK was the blank control group containing only transfection reagent and plasmid; NC5 was the negative control siRNA, the sequence information can be found in Table 19, BLANK: no substances were added.

Conclusion: Sequences ranking in the top 50% in terms of efficacy at these concentrations were selected for the next round: GPSZRA222S1, GPSZRA222S3, GPSZRA222S4, GPSZRA227S1, GPSZRA227S4, GPSZRA227S5, GPSZRA233S4, GPSZRA267S2, GPSZRA284S3, GPSZRA287S4, GPSZRA314S1, GPSZRA314S2, GPSZRA314S3, GPSZRA314S4, GPSZRA314S5, GPSZRA314S5, GPSZRA318S2, GPSZRA318S3, GPSZRA318S4, GPSZRA381S2, GPSZRA381S3, GPSZRA402S1, GPSZRA402S2, GPSZRA402S4, GPSZRA402S5, GPSZRA407S2, GPSZRA407S4, GPSZRA410S1, GPSZRA410S2, GPSZRA410S4, amounting to 29 sequences in total.

### Example 5: IC50 assay

To perform IC50 assay on cell lines, and determine the final candidate sequences based on the absolute IC50 values.

Experimental principle: Using GrapdhPad software, set the X-axis as log (concentration) and the Y-axis as inhibition rate. Fit the IC50 curve by means of nonlinear regression, with the curve equation expressed as "Y=Bottom + (Top-Bottom)/(1+10^{((LogIC50-X)*HillSlope)})". In the formula, Bottom represents the minimum inhibition rate, Top represents the maximum inhibition rate, HillSlope represents the slope of the curve, and LogIC50 refers to the logarithm of IC50 (half-maximal inhibitory concentration).

Experimental objective: To perform IC50 assay on cell lines, and determine the final candidate sequences based on the absolute IC50 values.

The test results are shown in Tables 20 and 21.

**Table 20: Naked sequences of siRNA sequences involved in the IC50 test**

| Sequence ID | sense seq (5'-3') | antisense seq (5'-3') |
|---|---|---|
| GPSZRA222 | UCUUCACACUGCAGUCGGA | UCCGACUGCAGUGUGAAGAGC |
| GPSZRA227 | ACACUGCAGUCGGAGCUAA | UUAGCUCCGACUGCAGUGUGA |
| GPSZRA233 | CAGUCGGAGCUAAUGGUGA | UCACCAUUAGCUCCGACUGCA |
| GPSZRA267 | GCAGUAGCUCCUGGUGGAA | UUCCACCAGGAGCUACUGCUC |
| GPSZRA284 | CCGUAACCCUGACCUGUGA | UCACAGGUCAGGGUUACGGUU |
| GPSZRA287 | UAACCCUGACCUGUGAAGU | ACUUCACAGGUCAGGGUUACG |
| GPSZRA314 | ACUGGAUGAAGGAUGGUGU | ACACCAUCCUUCAUCCAGUGG |
| GPSZRA318 | GAUGAAGGAUGGUGUGCCC | GGGCACACCAUCCUUCAUCCA |
| GPSZRA381 | AUCAGUCGGAGGAACCUGA | UCAGGUUCCUCCGACUGAUUC |
| GPSZRA402 | CCAACUCUCUCCUGUAUAA | UUAUACAGGAGAGAGUUGGUC |
| GPSZRA407 | CCUGUAUAACCCCACCUUG | CAAGGUGGGGUUAUACAGGAG |
| GPSZRA410 | GUAUAACCCCACCUUGCCA | UGGCAAGGUGGGGUUAUACAG |

**Table 21: IC50 results**

| Test sequence | IC50(pM) | |
|---|---|---|
| | A549 RAGE LV | BEAS-2B-RAGE-LV |
| GPSZRA222S1 | 83 | 43 |
| GPSZRA222S3 | 24 | 50 |
| GPSZRA222S4 | 29 | 35 |
| GPSZRA227S1 | 83 | 58 |
| GPSZRA227S4 | 60 | 74 |
| GPSZRA227S5 | 109 | 121 |
| GPSZRA233S4 | 179 | 122 |
| GPSZRA267S2 | 118 | 51 |
| GPSZRA284S3 | 23 | 96 |
| GPSZRA287S4 | 137 | 112 |
| GPSZRA314S1 | 23 | 37 |
| GPSZRA314S2 | 85 | 64 |
| GPSZRA314S3 | 31 | 43 |
| GPSZRA314S4 | 99 | 67 |
| GPSZRA314S5 | 11 | 40 |
| GPSZRA318S2 | 41 | 40 |
| GPSZRA318S3 | 183 | 70 |
| GPSZRA318S4 | 124 | 81 |
| GPSZRA381S2 | 232 | 149 |
| GPSZRA381S3 | 158 | 120 |
| GPSZRA402S1 | 147 | 90 |
| GPSZRA402S2 | 65 | 69 |
| GPSZRA402S4 | 121 | 54 |
| GPSZRA402S5 | 121 | 43 |
| GPSZRA407S2 | 110 | 221 |
| GPSZRA407S4 | 122 | 109 |
| GPSZRA410S1 | 165 | 91 |
| GPSZRA410S2 | 126 | 86 |
| GPSZRA410S4 | 93 | 76 |

Results: Sequences with IC50 values less than 30 in A549 RAGE LV or less than 65 in BEAS-2B RAGE LV were selected and retained: GPSZRA222S1, GPSZRA222S3, GPSZRA222S4, GPSZRA227S1, GPSZRA267S2, GPSZRA284S3, GPSZRA314S1, GPSZRA314S2, GPSZRA314S3, GPSZRA314S5, GPSZRA318S2, GPSZRA402S4, GPSZRA402S5, totaling 13 sequences.

### Example 6: Stability assay

Experimental principle: After entering the human body, siRNA will pass through the blood and various tissues and organs, and finally enter the cells to exert a silencing effect. During this transport process, many environmental factors will affect the stability of siRNA, such as human serum and ubiquitous RNase, as well as metabolites and enzymes in various tissues. Stability directly affects its activity in vivo. This experiment investigates the stability of siRNA by simulating three environments in vitro: RNase, serum, and tissue metabolic enzymes (S9).

Experimental objective: To test the stability of candidate sequences in RNase, human serum, and Human Lung S9 in vitro.

### Experimental steps:

(1) Preparation of RNase A solution: The solutions and reagents listed in Table 22 were added sequentially, diluted to a final volume of 10 mL with ultrapure water to obtain a stock solution with a concentration of 10 mg/mL, and stored at 4°C for later use.

**Table 22: Preparation of RNase A solution**

| Reagent | Volume or mass |
|---|---|
| RNase A | 100 mg |
| NaCl | 0.009 g |
| 1M Tris (pH 8.0) | 100 µL |

5 µL of siRNA (10 µM) was aspirated, followed by the addition of 1 µl of RNase A (0.1 mg/mL) and 4 µl of water, and 6 reaction tubes were prepared for each group, which were then treated in a water bath at 37°C for different time periods of 1 min, 15 min, 30 min, 60 min, 120 min and 240 min (the actual time gradient was subject to the experimental result figures). After the treatment, the tubes were snap-frozen in liquid nitrogen and stored at -80°C.

### (2) Serum stability

Reaction systems were prepared according to Table 23 below, and 5 reaction tubes were prepared for each group and then treated in a 37°C water bath for different time periods of 1 min, 3 h, 6 h, 24 h and 48 h (the actual time gradient was subject to the experimental result figures). After the treatment, the tubes were snap-frozen in liquid nitrogen and stored at -80°C.

**Table 23: Addition details for serum stability assay**

| Group | Volume of siRNA (10 µ M) added (µl) | Volume of serum added (µl) | Total volume (µl) | Gel Electrophores is dilution factor |
|---|---|---|---|---|
| 50% serum | 5 | 5 | 10 | 4 |

### (3) S9 stability

5 µL of siRNA (10 µM) was aspirated, followed by the addition of 1 µL of Human Lung S9 (5 mg/mL) and 4 µL of incubation solution (125 mM Tris·HCl, 7.5 mM MgCl2, 2.5 mM EDTA; with the formula detailed in Table 24 below), 6 reaction tubes were prepared for each group and then treated in a water bath at 37°C for different time periods of 1 h, 3 h, 6 h, 24 h, 48 h and 72 h (the actual time gradient was subject to the experimental result figures). After the treatment, the tubes were snap-frozen in liquid nitrogen and stored at -80°C.

**Table 24: S9 incubation solution formula (100 mL)**

| | Component | Molar mass g/mol | Weigh Mass (g) | DEPC water | |
|---|---|---|---|---|---|
| Composite solution (100mL) | 125 mM Tris-HCl | 121.14 | 1.5143 | First, dissolve Tirs in 75 mL of DEPC water, then adjust the PH to 7.4 with HCl, and then add MgCl2 and EDTA | Make the volume up to 100 mL with DEPC water |
| | 7.5 mM MgCl2 | 95.21 | 0.0714 | | |
| | 2.5 mM EDTA | 372.24 | 0.0931 | | |

### (4) siRNA gel electrophoresis analysis

Using 20% PAGE gel, the sample was run for 55 min at 200 V and 400 A before being photographed and analyzed. When processing serum samples, they were diluted with DEPC water according to the proportions in the table above before loading.

### (5) Experimental results

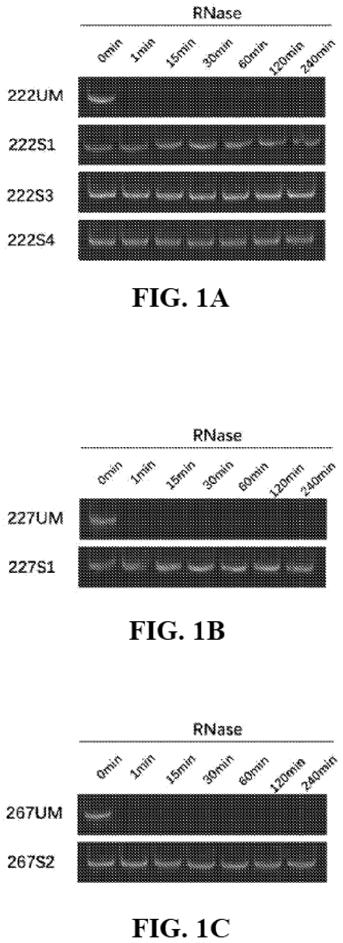

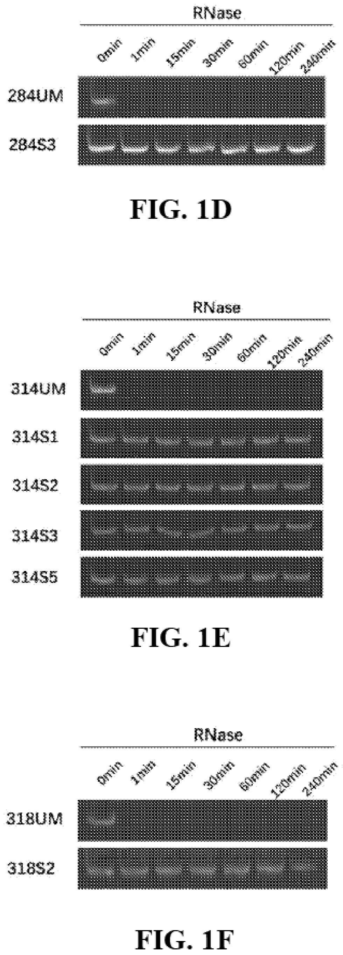

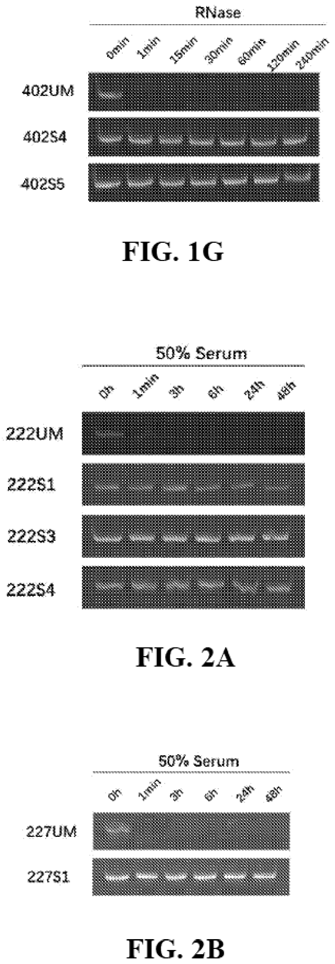

1) The RNase experimental results are shown in FIG. 1A to FIG. 1G and Table 25.

**Table 25: Grayscale results of RNase stability assay using gel electrophoresis**

| Sample information | Grayscale (%) | | | | | | |
|---|---|---|---|---|---|---|---|
| | 0min | 1min | 15min | 30min | 60min | 120min | 240min |
| GPSZRA222UM | 92.88 | 0.00 | 0.00 | 0.00 | 0.00 | 0.00 | 0.00 |
| GPSZRA222S1 | 95.11 | 92.93 | 96.65 | 92.20 | 95.60 | 94.49 | 93.87 |
| GPSZRA222S3 | 94.70 | 96.15 | 95.50 | 95.97 | 94.25 | 92.82 | 95.55 |
| GPSZRA222S4 | 92.34 | 92.14 | 96.15 | 95.75 | 91.39 | 94.12 | 95.57 |
| GPSZRA227UM | 92.18 | 0.00 | 0.00 | 0.00 | 0.00 | 0.00 | 0.00 |
| GPSZRA227S1 | 92.99 | 91.31 | 93.16 | 92.71 | 93.09 | 91.03 | 93.73 |
| GPSZRA267UM | 93.90 | 0.00 | 0.00 | 0.00 | 0.00 | 0.00 | 0.00 |
| GPSZRA267S2 | 94.73 | 93.73 | 92.94 | 95.78 | 92.27 | 93.05 | 93.97 |
| GPSZRA402UM | 94.85 | 0.00 | 0.00 | 0.00 | 0.00 | 0.00 | 0.00 |
| GPSZRA402S4 | 93.51 | 95.02 | 95.49 | 94.11 | 94.64 | 93.52 | 94.13 |
| GPSZRA402S5 | 94.99 | 93.24 | 92.85 | 93.76 | 91.79 | 92.11 | 93.59 |
| GPSZRA314UM | 95.50 | 0.00 | 0.00 | 0.00 | 0.00 | 0.00 | 0.00 |
| GPSZRA314S1 | 93.70 | 94.47 | 93.30 | 93.79 | 92.39 | 92.44 | 92.82 |
| GPSZRA314S2 | 96.00 | 94.66 | 94.84 | 94.86 | 92.86 | 94.02 | 93.14 |
| GPSZRA314S3 | 93.52 | 92.11 | 95.29 | 93.26 | 93.68 | 91.42 | 95.88 |
| GPSZRA314S5 | 90.61 | 94.16 | 95.62 | 94.46 | 95.02 | 94.35 | 95.23 |
| GPSZRA284UM | 95.65 | 0.00 | 0.00 | 0.00 | 0.00 | 0.00 | 0.00 |
| GPSZRA284S3 | 94.76 | 90.50 | 92.07 | 92.82 | 92.86 | 90.19 | 91.76 |
| GPSZRA318UM | 94.46 | 0.00 | 0.00 | 0.00 | 0.00 | 0.00 | 0.00 |
| GPSZRA318S2 | 93.48 | 95.22 | 92.26 | 93.32 | 95.02 | 95.02 | 92.57 |

The suffix "UM" attached to the sequence number denotes an unmodified sequence.

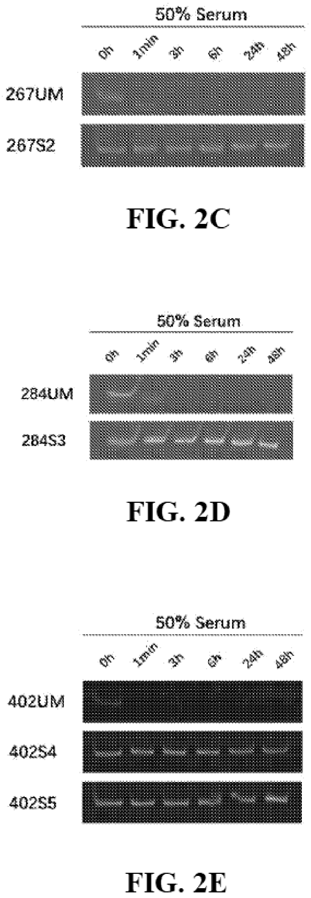

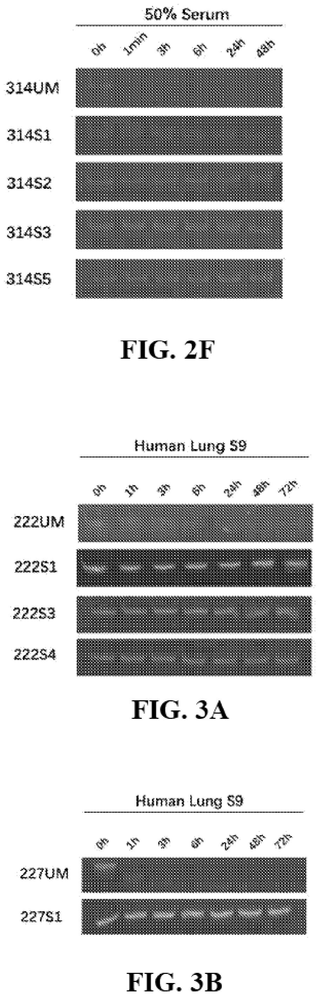

2) Serum stability: the experimental results are shown in Fig. 2A to Fig. 2F and Table 26.

**Table 26: Grayscale results of serum stability assay using gel electrophoresis**

| Sample information | Grayscale (%) | | | | | |
|---|---|---|---|---|---|---|
| | 0min | 1min | 3h | 6h | 24h | 48h |
| GPSZRA222UM | 96.74 | 40.01 | 0.00 | 0.00 | 0.00 | 0.00 |
| GPSZRA222S1 | 96.42 | 96.37 | 96.97 | 96.80 | 94.71 | 90.73 |
| GPSZRA222S3 | 97.10 | 98.43 | 97.82 | 99.05 | 96.14 | 98.35 |
| GPSZRA222S4 | 90.94 | 92.89 | 94.58 | 91.63 | 88.25 | 94.17 |
| GPSZRA227UM | 86.62 | 0.00 | 0.00 | 0.00 | 0.00 | 0.00 |
| GPSZRA227S1 | 96.95 | 97.94 | 97.87 | 95.73 | 94.68 | 97.42 |
| GPSZRA267UM | 87.22 | 0.00 | 0.00 | 0.00 | 0.00 | 0.00 |
| GPSZRA267S2 | 93.48 | 95.89 | 95.10 | 95.05 | 95.73 | 92.75 |
| GPSZRA284UM | 92.91 | 66.32 | 0.00 | 0.00 | 0.00 | 0.00 |
| GPSZRA284S3 | 96.63 | 97.85 | 98.44 | 96.87 | 95.96 | 97.56 |
| GPSZRA402UM | 92.84 | 0.00 | 0.00 | 0.00 | 0.00 | 0.00 |
| GPSZRA402S4 | 96.31 | 98.04 | 96.27 | 96.37 | 94.91 | 93.05 |
| GPSZRA402S5 | 97.01 | 98.10 | 97.20 | 97.06 | 96.75 | 95.29 |
| GPSZRA314UM | 90.78 | 0.00 | 0.00 | 0.00 | 0.00 | 0.00 |
| GPSZRA314S1 | 90.40 | 92.26 | 91.47 | 93.95 | 89.61 | 92.42 |
| GPSZRA314S2 | 91.28 | 96.61 | 91.57 | 91.83 | 88.67 | 90.38 |
| GPSZRA314S3 | 97.22 | 97.43 | 94.63 | 91.82 | 94.71 | 92.63 |
| GPSZRA314S5 | 94.77 | 94.86 | 95.90 | 94.67 | 92.69 | 94.62 |
| GPSZRA318UM | 92.54 | 0.00 | 0.00 | 0.00 | 0.00 | 0.00 |
| GPSZRA318S2 | 95.88 | 92.34 | 94.24 | 93.38 | 93.25 | 92.48 |

The suffix "UM" attached to the sequence number denotes an unmodified sequence.

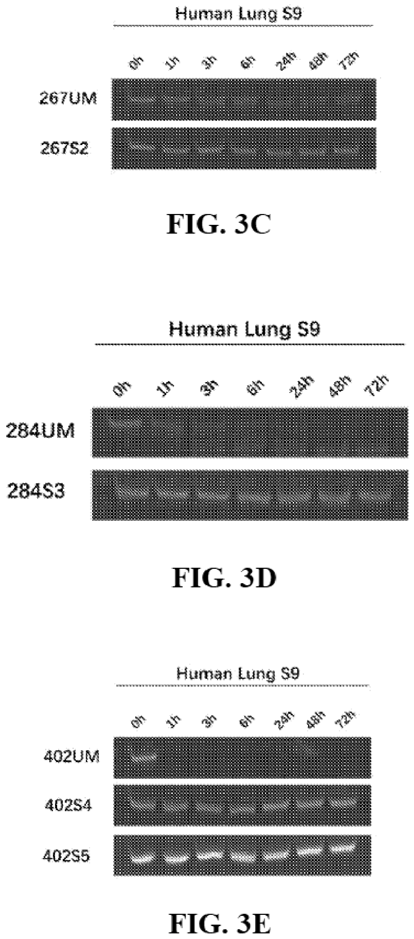

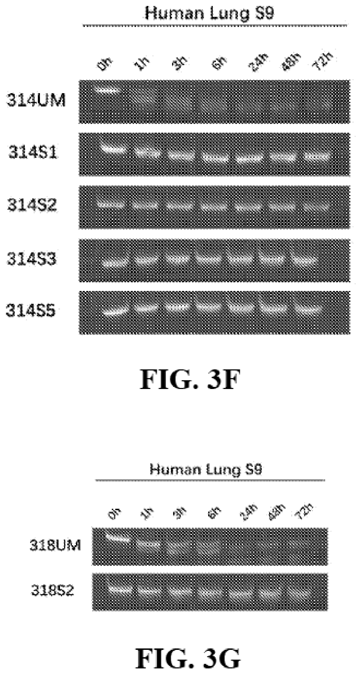

3) S9 stability: the experimental results are shown in Fig. 3A to Fig. 3G and Table 27.

**Table 27: Grayscale results of S9 stability assay using gel electrophoresis**

| Sample information | Grayscale (%) | | | | | | |
|---|---|---|---|---|---|---|---|
| | 0h | 1h | 3h | 6h | 24h | 48h | 72h |
| GPSZRA222UM | 80.53 | 47.75 | 50.47 | 42.97 | 34.20 | 35.22 | 25.49 |
| GPSZRA222S1 | 91.02 | 91.49 | 91.36 | 91.31 | 90.44 | 94.75 | 92.34 |
| GPSZRA222S3 | 98.24 | 97.83 | 97.62 | 95.91 | 97.58 | 96.61 | 96.44 |
| GPSZRA222S4 | 98.76 | 98.09 | 97.93 | 97.88 | 97.44 | 96.89 | 96.40 |
| GPSZRA227UM | 91.56 | 50.98 | 44.08 | 39.12 | 42.13 | 37.62 | 31.88 |
| GPSZRA227S1 | 98.06 | 97.81 | 98.07 | 97.07 | 97.33 | 98.07 | 97.77 |
| GPSZRA267UM | 97.89 | 95.46 | 92.84 | 85.25 | 74.24 | 77.74 | 80.40 |
| GPSZRA267S2 | 98.02 | 98.26 | 96.09 | 98.48 | 98.60 | 97.81 | 98.75 |
| GPSZRA284UM | 89.37 | 76.51 | 52.67 | 47.41 | 44.01 | 24.34 | 17.31 |
| GPSZRA284S3 | 99.23 | 98.61 | 98.96 | 96.78 | 98.78 | 97.38 | 98.68 |
| GPSZRA402UM | 95.51 | 0.00 | 0.00 | 0.00 | 0.00 | 0.00 | 0.00 |
| GPSZRA402S4 | 94.40 | 93.45 | 94.71 | 95.04 | 95.45 | 94.44 | 96.16 |
| GPSZRA402S5 | 95.04 | 94.65 | 95.69 | 92.98 | 93.35 | 96.25 | 95.33 |
| GPSZRA314UM | 96.28 | 28.90 | 12.41 | 7.72 | 6.61 | 10.16 | 5.36 |
| GPSZRA314S1 | 98.57 | 98.74 | 98.38 | 98.74 | 98.49 | 98.84 | 99.17 |
| GPSZRA314S2 | 97.35 | 98.37 | 98.68 | 99.33 | 98.70 | 99.09 | 99.13 |
| GPSZRA314S3 | 98.79 | 97.02 | 98.41 | 98.34 | 98.44 | 98.77 | 99.10 |
| GPSZRA314S5 | 98.83 | 98.11 | 98.32 | 98.24 | 98.41 | 99.19 | 98.94 |
| GPSZRA318UM | 94.86 | 82.43 | 58.83 | 47.93 | 50.90 | 54.13 | 54.12 |
| GPSZRA318S2 | 98.89 | 97.33 | 97.93 | 98.14 | 97.66 | 98.44 | 98.53 |

The suffix "UM" attached to the sequence number denotes an unmodified sequence.

Conclusion: The stability of siRNA is indicated by the decrease in grayscale over time; the slower the grayscale decreases over time, the more stable the siRNA. The results showed that a total of 13 test sequences, namely GPSZRA222S1, GPSZRA222S3, GPSZRA222S4, GPSZRA227S1, GPSZRA267S2, GPSZRA284S3, GPSZRA314S1, GPSZRA314S2, GPSZRA314S3, GPSZRA314S5, GPSZRA318S2, GPSZRA402S4 and GPSZRA402S5, exhibited favorable stability against RNase, serum and S9 in vitro compared with their corresponding unmodified sequences.

### Example 7: IC50 assay with VP modification

To further enhance the inhibitory activity of siRNA, we modified the siRNA obtained in Example 6 with VP. Also, the IC50 after modification was tested.

The experimental steps and methods are as described in Example 5.

The experimental results are shown in Table 28 and Table 29.

**Table 28: Naked siRNA sequences used in this experiment**

| Sequence ID | sense seq (5'-3') | antisense seq (5'-3') |
|---|---|---|
| GPSZRA222 | UCUUCACACUGCAGUCGGA | UCCGACUGCAGUGUGAAGAGC |
| GPSZRA227 | ACACUGCAGUCGGAGCUAA | UUAGCUCCGACUGCAGUGUGA |
| GPSZRA267 | GCAGUAGCUCCUGGUGGAA | UUCCACCAGGAGCUACUGCUC |
| GPSZRA284 | CCGUAACCCUGACCUGUGA | UCACAGGUCAGGGUUACGGUU |
| GPSZRA314 | ACUGGAUGAAGGAUGGUGU | ACACCAUCCUUCAUCCAGUGG |
| GPSZRA318 | GAUGAAGGAUGGUGUGCCC | GGGCACACCAUCCUUCAUCCA |
| GPSZRA402 | CCAACUCUCUCCUGUAUAA | UUAUACAGGAGAGAGUUGGUC |

**Table 29: Inhibitory activity of VP-modified siRNA**

| Test sequence | IC50(pM) | |
|---|---|---|
| | A549 RAGE LV | BEAS-2B-RAGE-LV |
| GPSZRA222S1-VP | 11 | 23 |
| GPSZRA222S3-VP | 13 | 21 |
| GPSZRA222S4-VP | 9 | 18 |
| GPSZRA227S1-VP | 32 | 53 |
| GPSZRA267S2-VP | 14 | 21 |
| GPSZRA284S3-VP | 12 | 17 |
| GPSZRA314S1-VP | 10 | 24 |
| GPSZRA314S2-VP | 5 | 26 |
| GPSZRA314S3-VP | 8 | 44 |
| GPSZRA314S5-VP | 3 | 25 |
| GPSZRA318S2-VP | 8 | 55 |
| GPSZRA402S4-VP | 2 | 14 |
| GPSZRA402S5-VP | 10 | 17 |

Results: After VP modification, the inhibitory activity of siRNA was significantly enhanced.

### Example 8: ELISA assay to verify the in vitro inhibitory effect of siRNA on RAGE protein

Experimental principle: The expression of target proteins is positively correlated with their mRNA levels. The expression levels of intracellular and extracellular proteins in RAGE cells after transfection with siRNA are detected by an ELISA kit, which directly reflects its in vitro efficacy.

Experimental objective: To verify the in vitro inhibitory effect of siRNA sequence on RAGE protein. The supernatant contains sRAGE (RAGE protein free outside the cell), and the cell lysate contains fRAGE (RAGE protein located on the cell membrane).

### Experimental steps:

### (1) Experimental reagents, as shown in Table 30

**Table 30: Experimental reagents used in ELISA**

| Reagent name | Manufacturer | Cat. No./Batch No. | Storage conditions |
|---|---|---|---|
| M-PER^{™} Mammalian Protein Extraction Reagent | Thermo | 78501/XJ355179 | RT |
| Halt^{™} Protease Inhibitor Cocktail (100×) | Thermo | 1862209/YB367196 | 4°C |
| Human RAGE ELISA Assay kit | R&D | SRG00/P365782 | 4°C |

### (2) Sample preparation

Supernatant: The supernatant was directly aspirated 48 h after cell transfection and stored frozen in a -80°C refrigerator.

Lysis buffer: 48 h after cell transfection, the cells were washed twice with 100 µL of PBS, followed by the addition of 100 µL of M-PER lysis buffer (containing 1× Halt^{™} Protease Inhibitor Cocktail). The mixture was pipetted up and down five times and then stored frozen in a -80°C refrigerator.

### (3) Preparation of solutions required for the experiment

Washing buffer: The wash buffer concentrate was allowed to warm up to room temperature and gently mixed until the crystals dissolved completely, then the wash buffer concentrate was diluted with deionized water or distilled water at a ratio of 1:25.

Substrate solution: Color reagents A and B should be mixed together in equal volumes within 15 min of use. The system should be kept away from light.

Preparation of standards: After the standard powder in the kit was allowed to warm up to room temperature, it was reconstituted with deionized water or distilled water according to the instructions on the vial label, mixed gently and allowed to dissolve completely for 15 min before use. This reconstitution yielded a stock solution with a concentration of 50000 pg/mL, which should be stored at 4°C.

900 µL of calibrator diluent RD5-5 was transferred into the tube containing the 5000 pg/mL solution. 500 µL of calibrator diluent was transferred into the remaining tubes. 100 µL of the standard stock solution was added to the 5000 pg/mL tube and mixed thoroughly. Subsequently, 500 µL of the mixture was transferred to the 2500 pg/mL tube and mixed thoroughly. The above steps were repeated to obtain a series of standard solutions with concentrations of 5000 pg/mL, 2500 pg/mL, 1250 pg/mL, 625 pg/mL, 313 pg/mL, 156 pg/mL and 78 pg/mL.

### (4) Assay procedure

1) The unused microplate strips were removed from the microplate, placed back into the foil pouch containing desiccant, and resealed tightly.
2) 100 µL of the assay diluent RD1-60 was added per well.
3) The sample to be tested was diluted with diluent RD5-5 according to the set dilution ratio, and 50 µL of standard, control or sample was added per well according to the layout. The plate was sealed with the provided adhesive strips. It was incubated at room temperature for 2 h.
4) Each well was aspirated and washed with 400 µL of wash buffer per well for a total of 4 washes. After the final wash, the plate was inverted and blotted dry with clean absorbent paper.
5) 200 µL of Human RAGE Conjugate was added to each well. The plate was sealed with new adhesive strips. It was incubated at room temperature for 2 h.
6) The above steps were repeated for washing.
7) 200 µL of substrate solution was added to each well and incubation was performed at room temperature for 30 min, taking care to avoid direct light.
8) 50 µL of stop solution was added to each well, the plate was gently shaken to mix the liquid thoroughly, and measured immediately thereafter.
9) The absorbance value of each well was measured immediately using a microplate reader set at a wavelength of 450 nm.
10) The absorbance value of each well was measured immediately using a microplate reader set at a wavelength of 570 nm.

The experimental result is as shown in Table 31 to Table 34.

**Table 31: Inhibition rate of siRNA on A549 RAGE LV supernatant**

| Test sequence | Supernatant | | | | | |
|---|---|---|---|---|---|---|
| | A549 RAGE LV | | | | | |
| | 0.01nM Inhibition rate | SD | 0.1nM Inhibition rate | SD | 1nM Inhibition rate | SD |
| GPSZRA402S4-VP | 38.14% | 0.0858 | 73.77% | 0.0184 | 79.97% | 0.0158 |
| GPSZRA402S5-VP | 36.15% | 0.1025 | 72.48% | 0.0104 | 79.11% | 0.0247 |
| GPSZRA314S1-VP | 32.01% | 0.0341 | 79.72% | 0.0317 | 89.24% | 0.0011 |
| GPSZRA314S2-VP | 23.04% | 0.0376 | 76.05% | 0.0438 | 88.91% | 0.0116 |
| GPSZRA314S3-VP | 35.06% | 0.0174 | 75.79% | 0.0024 | 89.52% | 0.0081 |
| GPSZRA314S5-VP | 36.13% | 0.0395 | 78.33% | 0.0429 | 91.23% | 0.0012 |
| GPSZRA222S3-VP | 41.29% | 0.0345 | 81.83% | 0.0120 | 88.77% | 0.0044 |
| GPSZRA222S4-VP | 43.31% | 0.0201 | 83.14% | 0.0076 | 88.14% | 0.0110 |
| GPSZRA318S2-VP | 22.38% | 0.0807 | 77.07% | 0.0156 | 90.31% | 0.0046 |

**Table 32: Inhibition rate of siRNA on A549 RAGE LV lysis buffer**

| Test sequence | Lysis buffer | | | | | |
|---|---|---|---|---|---|---|
| | A549 RAGE LV | | | | | |
| | 0.01nM Inhibition rate | SD | 0.1nM Inhibition rate | SD | 1nM Inhibition rate | SD |
| GPSZRA402S4-VP | 41.11% | 0.0714 | 84.11% | 0.0294 | 93.26% | 0.0081 |
| GPSZRA402S5-VP | 38.44% | 0.0910 | 83.92% | 0.0350 | 94.28% | 0.0180 |
| GPSZRA314S1-VP | 40.35% | 0.0620 | 86.71% | 0.0312 | 95.63% | 0.0078 |
| GPSZRA314S2-VP | 37.61% | 0.0768 | 83.15% | 0.0454 | 95.34% | 0.0081 |
| GPSZRA314S3-VP | 40.84% | 0.0235 | 75.44% | 0.0667 | 94.09% | 0.0085 |
| GPSZRA314S5-VP | 32.28% | 0.0350 | 83.76% | 0.0330 | 95.85% | 0.0041 |
| GPSZRA222S3-VP | 58.96% | 0.0570 | 94.06% | 0.0064 | 98.68% | 0.0011 |
| GPSZRA222S4-VP | 60.33% | 0.0531 | 96.21% | 0.0022 | 98.74% | 0.0025 |
| GPSZRA318S2-VP | 52.61% | 0.0046 | 90.92% | 0.0182 | 98.13% | 0.0039 |

**Table 33: Inhibition rate of siRNA on BEAS-2B RAGE LV supernatant**

| Test sequence | Supernatant | | | | | |
|---|---|---|---|---|---|---|
| | BEAS-2B RAGE LV | | | | | |
| | 0.01nM Inhibition rate | SD | 0.1nM Inhibition rate | SD | 1nM Inhibition rate | SD |
| GPSZRA402S4-VP | 28.52% | 0.0731 | 65.17% | 0.0729 | 81.42% | 0.0052 |
| GPSZRA402S5-VP | 38.59% | 0.0685 | 67.76% | 0.0154 | 84.58% | 0.0048 |
| GPSZRA314S1-VP | 37.52% | 0.1336 | 69.66% | 0.0300 | 87.59% | 0.0009 |
| GPSZRA314S2-VP | 27.00% | 0.2437 | 69.63% | 0.0380 | 85.42% | 0.0135 |
| GPSZRA314S3-VP | 35.31% | 0.0423 | 59.49% | 0.0232 | 83.81% | 0.0161 |
| GPSZRA314S5-VP | 23.00% | 0.0646 | 64.26% | 0.0263 | 85.93% | 0.0131 |
| GPSZRA222S3-VP | 37.89% | 0.0202 | 83.77% | 0.0210 | 92.86% | 0.0114 |
| GPSZRA222S4-VP | 38.72% | 0.0485 | 87.18% | 0.0064 | 92.51% | 0.0013 |
| GPSZRA318S2-VP | 30.58% | 0.0488 | 80.43% | 0.0132 | 90.93% | 0.0060 |

**Table 34: Inhibition rate of siRNA on BEAS-2B RAGE LV lysis buffer**

| Test sequence | Lysis buffer | | | | | |
|---|---|---|---|---|---|---|
| | BEAS-2B RAGE LV | | | | | |
| | 0.01nM inhibition rate | SD | 0.1nM Inhibition rate | SD | 1nM Inhibition rate | SD |
| GPSZRA402S4 -VP | 30.27% | 0.0520 | 69.42% | 0.0331 | 91.02% | 0.0142 |
| GPSZRA402S5 -VP | 17.95% | 0.1185 | 66.75% | 0.0178 | 92.11% | 0.0184 |
| GPSZRA314S1 -VP | 27.16% | 0.0496 | 77.41% | 0.0165 | 97.57% | 0.0070 |
| GPSZRA314S2 -VP | 17.41% | 0.0625 | 74.54% | 0.0307 | 97.03% | 0.0026 |
| GPSZRA314S3 -VP | 23.78% | 0.0486 | 70.91% | 0.0161 | 95.26% | 0.0105 |
| GPSZRA314S5 -VP | 24.57% | 0.0980 | 75.35% | 0.0223 | 98.17% | 0.0034 |
| GPSZRA222S3 -VP | 32.64% | 0.0224 | 89.92% | 0.0096 | 98.83% | 0.0005 |
| GPSZRA222S4 -VP | 43.79% | 0.0258 | 94.07% | 0.0018 | 98.82% | 0.0045 |
| GPSZRA318S2 -VP | 50.04% | 0.0249 | 90.29% | 0.0008 | 97.62% | 0.0076 |

Results: The modified siRNA in the present application has a very strong inhibitory effect on both sRAGE and fRAGE proteins.

Summary: The inhibition data of the screening sequences with superior activity are summarized in Table 35.

**Table 35: Summary of inhibition data for screening sequences with superior activity**

| Sequence name | psiCHECK Single concentration (%) | psiCHECK Dual concentrations (%) | | Cell line single concentration (%) | | Cell line dual concentration (%) | | | | Cell line IC50 (pM) | |
|---|---|---|---|---|---|---|---|---|---|---|---|
| | 1nM | 0.1nM | 1nM | A549 (1nM) | BEAS-2B (1nM) | A549 (0.1nM) | A549 (1nM) | BEAS-2B (0.1 nM) | BEAS-2B (1nM) | A549 | BE AS -2B |
| GPSZRA2 22 | 83.32% | 77.35% | 84.21 % | | | | | | | | |
| GPSZRA2 22S1 | | 63.81% | 85.98% | 93.77% | 91.15% | 82.17% | 84.17% | 68.08% | 90.97% | 83 | 43 |
| GPSZRA2 22S1-VP | | | | | | | | | | 11 | 23 |
| GPSZRA2 22S3 | | 56.30% | 83.04% | 93.28% | 89.98% | 84.28% | 91.91% | 51.32% | 88.58% | 24 | 50 |
| GPSZRA2 22S3-VP | | | | | | | | | | 13 | 21 |
| GPSZRA2 22S4 | | 47.31% | 81.01% | 93.07% | 89.70% | 73.84% | 93.37% | 67.74% | 83.06% | 29 | 35 |
| GPSZRA2 22S4-VP | | | | | | | | | | 9 | 18 |
| GPSZRA2 27 | 84.89% | 77.56% | 84.55% | | | | | | | | |
| GPSZRA2 27S1 | | 58.82% | 83.91% | 91.21% | 88.45% | 69.58% | 92.05% | 51.33% | 87.82% | 83 | 58 |
| GPSZRA2 27S1-VP | | | | | | | | | | 32 | 53 |
| GPSZRA267 | 86.69% | 82.47% | 93.06% | | | | | | | | |
| GPSZRA267S2 | | 33.41% | 86.00% | 88.02% | 84.96% | 54.12% | 85.60% | 38.03% | 75.54% | 118 | 51 |
| GPSZRA2 67S2-VP | | | | | | | | | | 14 | 21 |
| GPSZRA2 84 | 89.26% | 85.50% | 91.91 % | | | | | | | | |
| GPSZRA2 84S3 | | 65.13% | 89.54 % | 90.61 % | 76.70 % | 73.43% | 86.89 % | 49.04 % | 70.78 % | 23 | 96 |
| GPSZRA2 84S3-VP | | | | | | | | | | 12 | 17 |
| GPSZRA3 14 | 84.05% | 75.88% | 84.93% | | | | | | | | |
| GPSZRA3 14S1 | | 78.48% | 89.23% | 89.63% | 78.94% | 46.46% | 80.72% | 4.82% | 89.69% | 23 | 37 |
| GPSZRA3 14S1-VP | | | | | | | | | | 10 | 24 |
| GPSZRA3 14S2 | | 70.55% | 88.49 % | 90.95 % | 78.41 % | 69.42% | 82.03 % | 63.28 % | 83.18 % | 85 | 64 |
| GPSZRA3 14S2-VP | | | | | | | | | | 5 | 26 |
| GPSZRA3 14S3 | | 72.26% | 89.03 % | 91.52 % | 76.40 % | 66.02% | 85.25 % | 59.86 % | 78.59 % | 31 | 43 |
| GPSZRA3 14S3-VP | | | | | | | | | | 8 | 44 |
| GPSZRA3 14S5 | | 75.12% | 88.45 % | 91.04 % | 76.37 % | 67.94% | 86.47 % | 62.50 % | 86.87 % | 11 | 40 |
| GPSZRA3 14S5-VP | | | | | | | | | | 3 | 25 |
| GPSZRA3 18 | 85.14% | 77.05% | 87.53 % | | | | | | | | |
| GPSZRA3 18S2 | | 52.43% | 87.60 % | 90.31 % | 77.33 % | 64.69% | 86.43 % | 64.24 % | 83.73 % | 41 | 40 |
| GPSZRA3 18S2-VP | | | | | | | | | | 8 | 55 |
| GPSZRA4 02 | 87.97% | 65.61% | 89.14 % | | | | | | | | |
| GPSZRA4 02S4 | | 68.42% | 88.34 % | 90.62 % | 78.92 % | 70.67% | 84.46 % | 67.84 % | 82.46 % | 121 | 54 |
| GPSZRA4 02S4-VP | | | | | | | | | | 2 | 14 |
| GPSZRA4 02S5 | | 77.29% | 89.46 % | 89.89 % | 80.67 % | 65.48% | 84.50 % | 80.57 % | 85.96 % | 121 | 43 |
| GPSZRA4 02S5-VP | | | | | | | | | | 10 | 17 |

Results: As shown in Table 35, a total of 13 sequences, namely GPSZRA222S1, GPSZRA222S3, GPSZRA222S4, GPSZRA227S1, GPSZRA267S2, GPSZRA284S3, GPSZRA314S1, GPSZRA314S2, GPSZRA314S3, GPSZRA314S5, GPSZRA318S2, GPSZRA402S4 and GPSZRA402S5, along with their corresponding 13 VP-modified derivatives (GPSZRA222S1-VP, GPSZRA222S3-VP, GPSZRA222S4-VP, GPSZRA227S1-VP, GPSZRA267S2-VP, GPSZRA284S3-VP, GPSZRA314S1-VP, GPSZRA314S2-VP, GPSZRA314S3-VP, GPSZRA314S5-VP, GPSZRA318S2-VP, GPSZRA402S4-VP, GPSZRA402S5-VP), and the siRNAs of their corresponding naked sequences (GPSZRA222, GPSZRA267, GPSZRA284, GPSZRA314, GPSZRA318, GPSZRA402), exhibited outstanding on-target activity. They exerted highly potent inhibitory effects on the target gene RAGE in both A549 RAGE LV cells and BEAS-2B RAGE LV cells. Furthermore, the activity was further enhanced with an increase in the degree of modification.

### Example 9: Stability assay

The purpose of this experiment is to test the stability of VP-modified siRNA. The experimental steps are as described in Example 6.

### Experimental results:

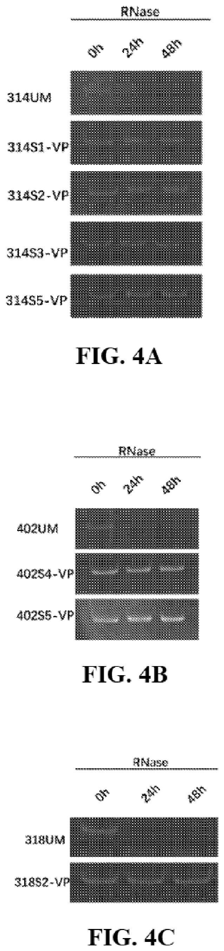

(1) RNase stability: the experimental results are shown in FIG. 4A to FIG. 4C and Table 36.

**Table 36: Grayscale results of RNase stability assay using gel electrophoresis**

| Sample information | Grayscale (%) | | |
|---|---|---|---|
| | 0 | 24 h | 48 h |
| GPSZRA314UM | 89.66 | 24.45 | 11.33 |
| GPSZRA314S1-VP | 93.85 | 96.58 | 95.05 |
| GPSZRA314S2-VP | 95.75 | 95.31 | 93.88 |
| GPSZRA314S3-VP | 95.46 | 91.97 | 94.80 |
| GPSZRA314S5-VP | 97.28 | 97.28 | 96.81 |
| GPSZRA402UM | 96.22 | 8.26 | 6.11 |
| GPSZRA402S4-VP | 99.06 | 97.92 | 98.86 |
| GPSZRA402S5-VP | 95.69 | 95.20 | 96.99 |
| GPSZRA318UM | 93.39 | 22.68 | 5.13 |
| GPSZRA318S2-VP | 94.25 | 96.37 | 95.34 |

The suffix "UM" attached to the sequence denotes an unmodified siRNA sequence.

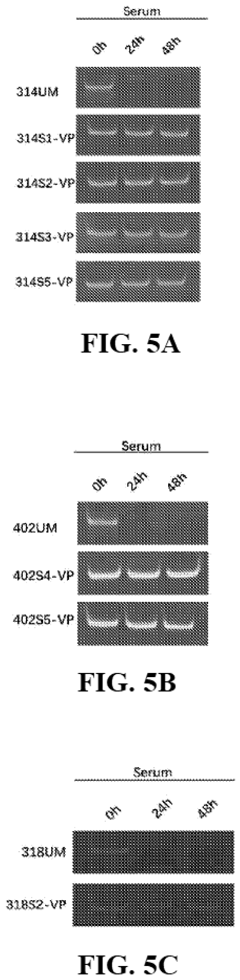

(2) Serum stability: the experimental results are shown in Fig. 5A to Fig. 5C and Table 37.

**Table 37: Grayscale results of serum stability assay using gel electrophoresis**

| Sample information | Grayscale (%) | | |
|---|---|---|---|
| | 0 | 24 h | 48 h |
| GPSZRA314UM | 85.34 | 8.03 | 3.98 |
| GPSZRA314S1-VP | 95.76 | 96.22 | 93.22 |
| GPSZRA314S2-VP | 95.90 | 95.91 | 95.34 |
| GPSZRA314S3-VP | 91.69 | 85.92 | 86.93 |
| GPSZRA314S5-VP | 94.70 | 92.91 | 91.24 |
| GPSZRA402UM | 96.22 | 17.92 | 7.99 |
| GPSZRA402S4-VP | 98.85 | 97.34 | 97.64 |
| GPSZRA402S5-VP | 99.62 | 98.31 | 96.59 |
| GPSZRA318UM | 93.84 | 13.74 | 6.13 |
| GPSZRA318S2-VP | 87.59 | 87.29 | 87.57 |

The suffix "UM" attached to the sequence denotes an unmodified siRNA sequence.

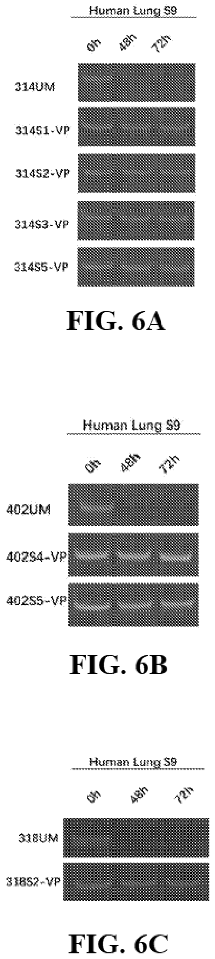

(3) S9 stability: the experimental results are shown in Fig. 6A to Fig. 6C and Table 38.

**Table 38: Grayscale results of S9 stability assay using gel electrophoresis**

| Sample information | Grayscale (%) | | |
|---|---|---|---|
| | 0 | 48 h | 72 h |
| GPSZRA314UM | 92.32 | 1.81 | 1.00 |
| GPSZRA314S1-VP | 96.33 | 93.13 | 91.12 |
| GPSZRA314S2-VP | 94.65 | 92.17 | 96.00 |
| GPSZRA314S3-VP | 91.95 | 92.88 | 91.47 |
| GPSZRA314S5-VP | 93.16 | 96.09 | 94.70 |
| GPSZRA402UM | 88.90 | 5.35 | 6.03 |
| GPSZRA402S4-VP | 96.09 | 97.27 | 98.52 |
| GPSZRA402S5-VP | 97.30 | 96.49 | 97.23 |
| GPSZRA318UM | 88.39 | 6.29 | 3.07 |
| GPSZRA318S2-VP | 94.73 | 95.44 | 88.30 |

The suffix "UM" attached to the sequence denotes an unmodified siRNA sequence.

Conclusion: The stability of siRNA is indicated by the decrease in grayscale over time; the slower the grayscale decreases over time, the more stable the siRNA. The results showed that siRNA sequences with different modification patterns, after further VP modification, still exhibited excellent RNase stability, serum stability, and human lung S9 stability.

### Example 10: Cytotoxicity

Experimental principle: The CCK-8 assay is a method for detecting cell proliferation and cytotoxicity. Its principle involves using dehydrogenases in the mitochondria of cells to reduce WST-8 in the CCK-8 reagent to a colored formazan product. The content of dehydrogenases is measured by determining the optical density (OD) value at 450 nm, which allows for the indirect assessment of the proliferative activity of cells at the corresponding time point. The CCK-8 assay following siRNA transfection can assess whether siRNA has proliferative toxicity to cells.

Experimental objective: To test the cytotoxicity of modified siRNA against A549 RAGE LV (A549 cells overexpressing RAGE) or BEAS-2B RAGE LV (BEAS-2B cells overexpressing RAGE).

The cytotoxicity of the sequences is verified by using CCK8 cell proliferation curves.

### Experimental steps:

### (1) Experimental reagents

**Table 39: Sources of experimental reagents used**

| Reagent name | Manufacturer | Cat. No./Batch No. | Storage conditions |
|---|---|---|---|
| CCK8 assay kit | DOJINDO | CK04 | 4°C |

### (2) Cell preparation

A549 RAGE LV cells (A549 cells overexpressing RAGE) or BEAS-2B RAGE LV cells (BEAS-2B overexpressing RAGE) cultured in 10 cm culture dishes were routinely digested with trypsin 48 h after passage. The cells were resuspended in complete medium, then seeded into a 96-well plate at a density of 1.4×10⁴ cells/well or 1.2×10⁴ cells/well with a volume of 50 µL per well (the transfection complexes were added immediately after cell plating). The surrounding wells were filled with 100 µL of PBS to eliminate the edge effect, and then the plate was ready for subsequent experiments.

### (3) Cell transfection

A total of 9 sequences were used, with NC1, MOCK, BLANK, and complete culture medium as controls. The MOCK group was the group treated with transfection reagent alone without any sequence added; the BLANK group was the group containing only cells. NC1 was the negative control siRNA, and the sequence information can be found in Table 7 in Example 1.

siRNA concentration gradients were set: 10 nM, 1 nM, 0.1 nM, and 0.01 nM for transfection. The transfection reagent lipofectamine RNAiMAX was used at a volume of 0.25 µL/well. After accounting for reagent loss, the transfection complexes were prepared as follows: siRNA and Lipofectamine RNAiMAX transfection reagent were separately diluted in 25 µL of Opti-MEM^{®} per well. After incubation for 5 min, the two components were mixed thoroughly, followed by another incubation for 20 min.

When the incubation period expired, the transfection complexes were added to the corresponding wells at a volume of 50 µL/well, resulting in a final volume of 100 µL per well.

Approximately 6 h after transfection, the transfection complexes were aspirated and discarded, and the absorbance values at 0 h were measured.

For the other time points, 100 µL of complete medium was added to each well, and the plates were placed in the incubator for further cultivation.

### (4) Cell analysis on instrument

The cell plates were removed at 24 h and 48 h, and a mixture of 10 µl of CCK-8 solution and 90 µl of blank culture medium was added to each well. The CCK8 readings of the blank wells in the complete culture medium of each 96-well plate were used as the background.

After the culture plates were incubated in the incubator for 4 h, the absorbance at 450 nm was measured using a microplate reader.

### Experimental results:

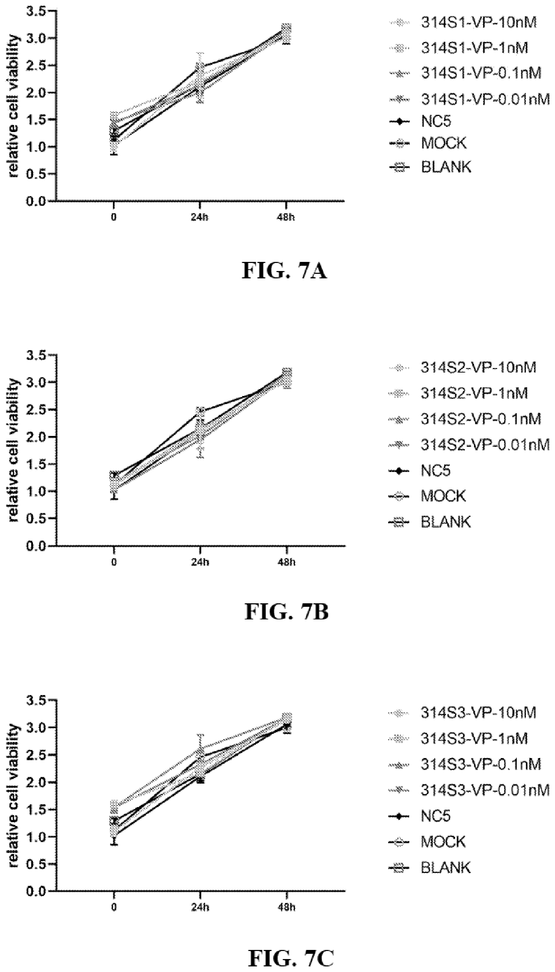

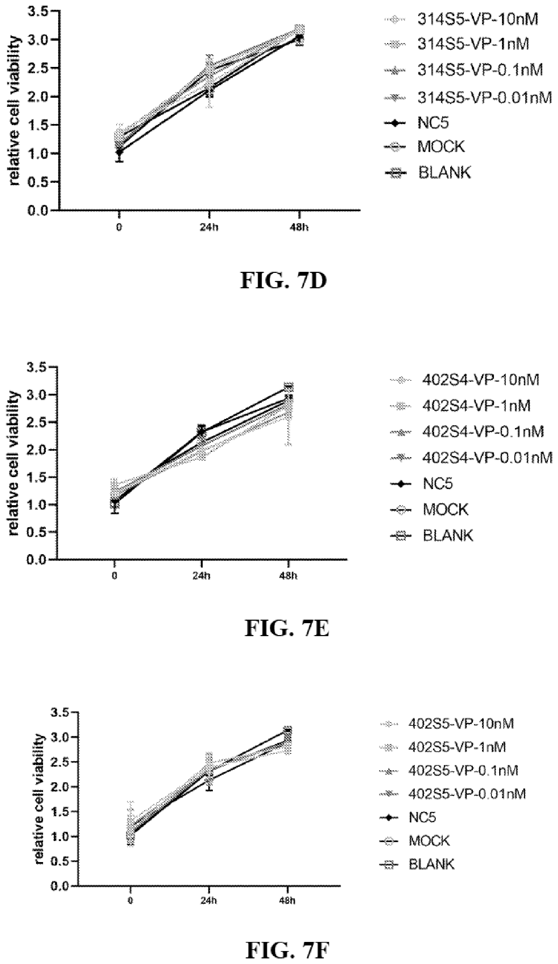

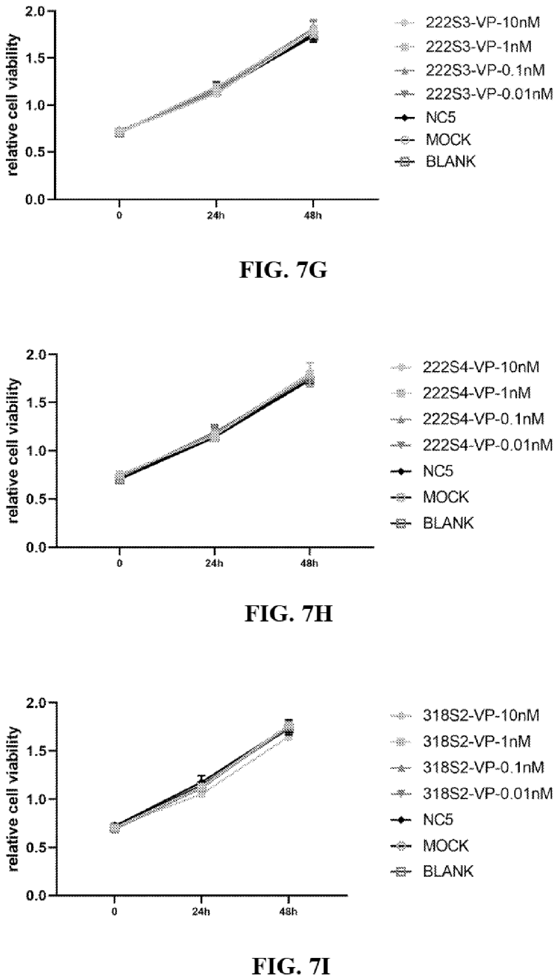

(1) Cytotoxicity of modified siRNAs against A549 RAGE LV cells, with the experimental results presented in FIG. 7A to FIG. 7I and Table 40.

**Table 40: Cytotoxicity of modified siRNAs against A549 RAGE LV**

| Test sequence | Time (h) | 10 nM | | 1 nM | | 0.1 nM | | 0.01 nM | |
|---|---|---|---|---|---|---|---|---|---|
| | | Cell Vitality | SD | Cell Vitality | SD | Cell Vitality | SD | Cell Vitality | SD |
| GPSZRA314S1-VP | 0 | 1.00 | 0.08 | 1.58 | 0.0 7 | 1.45 | 0.06 | 1.43 | 0.07 |
| | 24 | 2.31 | 0.42 | 2.20 | 0.1 1 | 1.99 | 0.17 | 2.14 | 0.33 |
| | 48 | 3.01 | 0.08 | 3.10 | 0.1 4 | 3.11 | 0.12 | 3.14 | 0.08 |
| GPSZRA314S2-VP | 0 | 1.11 | 0.04 | 1.13 | 0.1 0 | 1.17 | 0.05 | 1.02 | 0.04 |
| | 24 | 2.10 | 0.33 | 2.14 | 0.2 6 | 2.02 | 0.23 | 1.95 | 0.33 |
| | 48 | 3.05 | 0.05 | 3.04 | 0.1 3 | 3.15 | 0.03 | 3.09 | 0.04 |
| GPSZRA314S3-VP | 0 | 1.10 | 0.09 | 1.59 | 0.0 6 | 1.54 | 0.08 | 1.54 | 0.10 |
| | 24 | 2.27 | 0.08 | 2.17 | 0.0 2 | 2.61 | 0.25 | 2.33 | 0.23 |
| | 48 | 3.14 | 0.06 | 3.18 | 0.0 2 | 3.19 | 0.01 | 3.18 | 0.01 |
| GPSZRA314S5-VP | 0 | 1.37 | 0.14 | 1.33 | 0.0 7 | 1.33 | 0.05 | 1.16 | 0.02 |
| | 24 | 2.23 | 0.42 | 2.47 | 0.1 7 | 2.36 | 0.22 | 2.54 | 0.19 |
| | 48 | 3.17 | 0.01 | 3.17 | 0.0 2 | 3.19 | 0.03 | 3.19 | 0.02 |
| GPSZRA402S4-VP | 0 | 1.20 | 0.06 | 1.36 | 0.1 1 | 1.18 | 0.07 | 1.23 | 0.10 |
| | 24 | 1.99 | 0.03 | 1.86 | 0.0 5 | 1.97 | 0.11 | 2.07 | 0.15 |
| | 48 | 2.60 | 0.51 | 2.81 | 0.1 0 | 2.68 | 0.06 | 2.82 | 0.08 |
| GPSZRA402S5-VP | 0 | 1.31 | 0.39 | 1.11 | 0.3 0 | 1.21 | 0.33 | 1.08 | 0.04 |
| | 24 | 2.39 | 0.20 | 2.48 | 0.2 0 | 2.34 | 0.31 | 2.35 | 0.22 |
| | 48 | 2.73 | 0.07 | 2.81 | 0.0 8 | 2.89 | 0.11 | 2.90 | 0.13 |
| GPSZRA222S3-VP | 0 | 1.81 | 0.08 | 1.89 | 0.1 0 | 1.95 | 0.15 | 1.96 | 0.10 |
| | 24 | 3.07 | 0.05 | 3.12 | 0.0 2 | 3.10 | 0.04 | 3.12 | 0.06 |
| | 48 | 3.39 | 0.04 | 3.41 | 0.0 0 | 3.40 | 0.01 | 3.39 | 0.00 |
| GPSZRA222S4-VP | 0 | 1.92 | 0.08 | 1.92 | 0.0 9 | 1.91 | 0.07 | 1.89 | 0.06 |
| | 24 | 3.09 | 0.06 | 3.09 | 0.0 3 | 3.12 | 0.08 | 3.08 | 0.07 |
| | 48 | 3.38 | 0.01 | 3.40 | 0.0 1 | 3.39 | 0.03 | 3.41 | 0.00 |
| GPSZRA318S2-VP | 0 | 1.79 | 0.03 | 1.88 | 0.0 1 | 1.90 | 0.04 | 1.88 | 0.05 |
| | 24 | 3.06 | 0.02 | 3.14 | 0.0 4 | 3.11 | 0.04 | 3.09 | 0.05 |
| | 48 | 3.39 | 0.03 | 3.43 | 0.0 1 | 3.40 | 0.01 | 3.37 | 0.02 |
| NC5 | 0 | 1.93 | 0.05 | | | | | | |
| | 24 | 3.07 | 0.05 | | | | | | |
| | 48 | 3.38 | 0.02 | | | | | | |
| MOCK | 0 | 1.84 | 0.05 | | | | | | |
| | 24 | 3.05 | 0.02 | | | | | | |
| | 48 | 3.31 | 0.01 | | | | | | |
| BLANK | 0 | 1.92 | 0.02 | | | | | | |
| | 24 | 3.07 | 0.03 | | | | | | |
| | 48 | 3.34 | 0.03 | | | | | | |

Among them, the MOCK group was the group treated with transfection reagent alone without any sequence added; the BLANK group was the group containing only cells. NC5 was the negative control siRNA, and the sequence information can be found in Table 19 in Example 4.

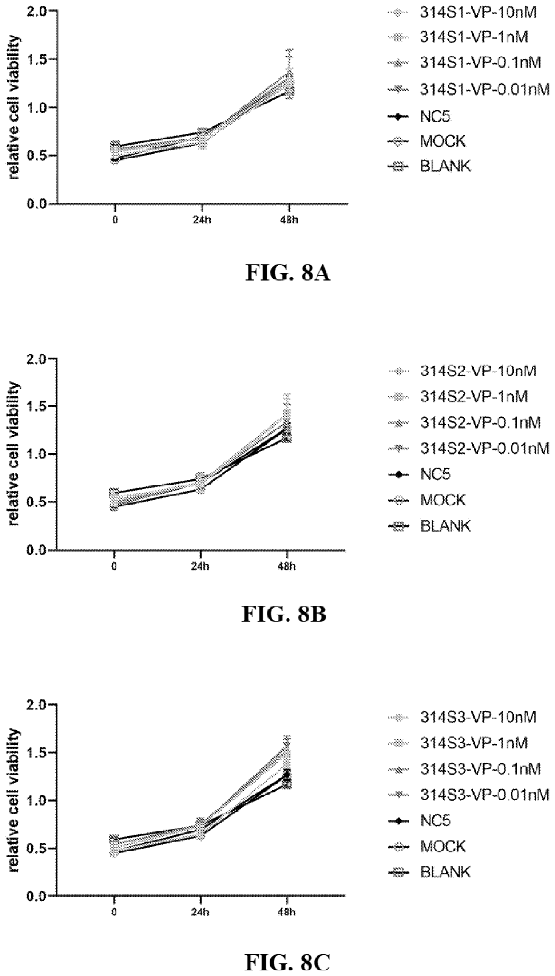

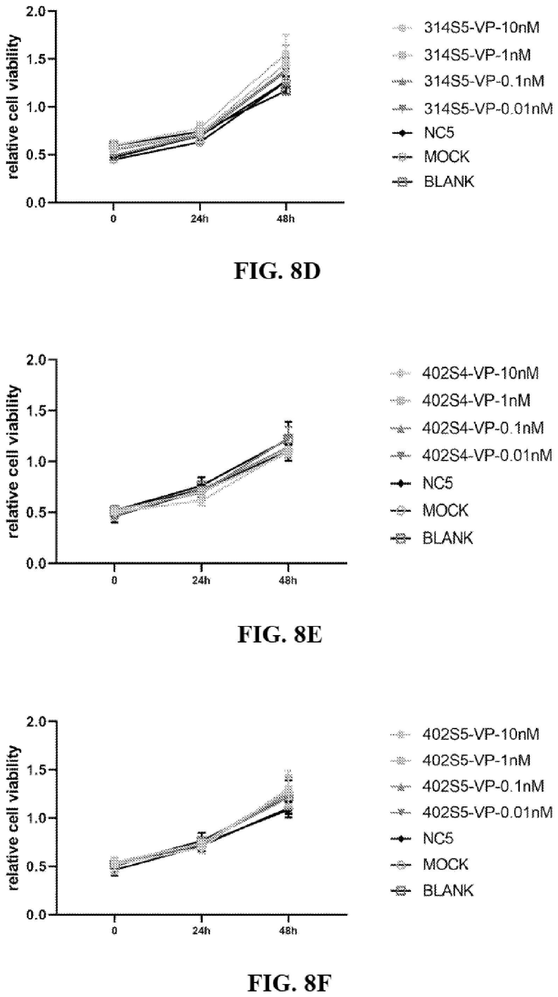

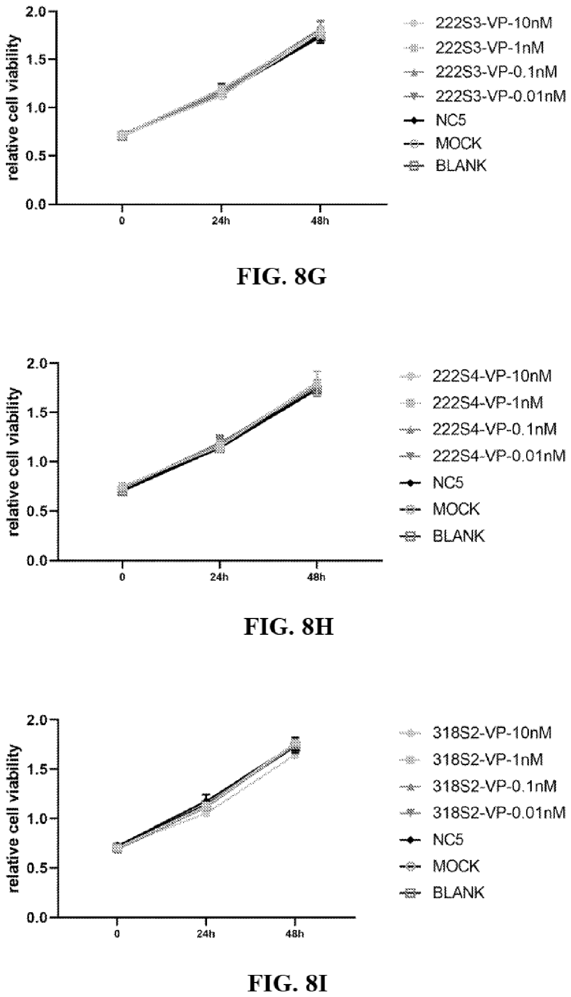

(2) Cytotoxicity of modified siRNAs against BEAS-2B RAGE LV cells, with the experimental results presented in FIG. 8A to FIG. 8I and Table 41.

**Table 41: Cytotoxicity of modified siRNAs against BEAS-2B RAGE LV**

| Test sequence | Time (h) | 10 nM | | 1 nM | | 0.1 nM | | 0.01 nM | |
|---|---|---|---|---|---|---|---|---|---|
| | | Cell vitality | SD | Cell vitality | SD | Cell vitality | SD | Cell vitality | SD |
| GPSZRA31 4S1-VP | 0 | 0.50 | 0.02 | 0.54 | 0.05 | 0.56 | 0.07 | 0.56 | 0.08 |
| | 24 | 0.64 | 0.06 | 0.68 | 0.08 | 0.67 | 0.08 | 0.68 | 0.09 |
| | 48 | 1.24 | 0.06 | 1.26 | 0.14 | 1.37 | 0.22 | 1.31 | 0.22 |
| GPSZRA31 4S2-VP | 0 | 0.55 | 0.07 | 0.52 | 0.05 | 0.50 | 0.04 | 0.47 | 0.04 |
| | 24 | 0.68 | 0.08 | 0.70 | 0.09 | 0.70 | 0.10 | 0.71 | 0.08 |
| | 48 | 1.40 | 0.22 | 1.42 | 0.16 | 1.41 | 0.11 | 1.34 | 0.08 |
| GPSZRA31 4S3-VP | 0 | 0.47 | 0.02 | 0.50 | 0.05 | 0.54 | 0.09 | 0.54 | 0.08 |
| | 24 | 0.65 | 0.03 | 0.73 | 0.02 | 0.75 | 0.05 | 0.74 | 0.08 |
| | 48 | 1.38 | 0.03 | 1.49 | 0.06 | 1.52 | 0.15 | 1.57 | 0.07 |
| GPSZRA31 4S5-VP | 0 | 0.60 | 0.05 | 0.56 | 0.04 | 0.55 | 0.06 | 0.49 | 0.05 |
| | 24 | 0.78 | 0.07 | 0.73 | 0.09 | 0.71 | 0.09 | 0.70 | 0.09 |
| | 48 | 1.56 | 0.19 | 1.45 | 0.19 | 1.39 | 0.15 | 1.37 | 0.14 |
| GPSZRA40 2S4-VP | 0 | 0.50 | 0.02 | 0.52 | 0.02 | 0.47 | 0.02 | 0.52 | 0.04 |
| | 24 | 0.62 | 0.05 | 0.70 | 0.03 | 0.73 | 0.03 | 0.70 | 0.05 |
| | 48 | 1.11 | 0.04 | 1.08 | 0.03 | 1.14 | 0.10 | 1.23 | 0.11 |
| GPSZRA40 2S5-VP | 0 | 0.52 | 0.06 | 0.54 | 0.05 | 0.53 | 0.02 | 0.53 | 0.00 |
| | 24 | 0.70 | 0.06 | 0.74 | 0.03 | 0.73 | 0.05 | 0.74 | 0.04 |
| | 48 | 1.31 | 0.18 | 1.28 | 0.16 | 1.26 | 0.16 | 1.22 | 0.11 |
| GPSZRA22 2S3-VP | 0 | 0.71 | 0.02 | 0.72 | 0.01 | 0.72 | 0.01 | 0.72 | 0.02 |
| | 24 | 1.12 | 0.02 | 1.20 | 0.00 | 1.15 | 0.03 | 1.17 | 0.04 |
| | 48 | 1.78 | 0.06 | 1.78 | 0.02 | 1.82 | 0.08 | 1.82 | 0.09 |
| GPSZRA22 2S4-VP | 0 | 0.73 | 0.02 | 0.75 | 0.01 | 0.74 | 0.03 | 0.73 | 0.02 |
| | 24 | 1.17 | 0.07 | 1.16 | 0.05 | 1.18 | 0.06 | 1.19 | 0.08 |
| | 48 | 1.81 | 0.10 | 1.80 | 0.12 | 1.78 | 0.02 | 1.76 | 0.04 |
| GPSZRA31 8S2-VP | 0 | 0.72 | 0.02 | 0.71 | 0.02 | 0.69 | 0.01 | 0.69 | 0.01 |
| | 24 | 1.05 | 0.02 | 1.13 | 0.02 | 1.12 | 0.03 | 1.12 | 0.01 |
| | 48 | 1.65 | 0.02 | 1.77 | 0.02 | 1.75 | 0.02 | 1.76 | 0.03 |
| NC5 | 0 | 0.72 | 0.02 | | | | | | |
| | 24 | 1.18 | 0.07 | | | | | | |
| | 48 | 1.76 | 0.06 | | | | | | |
| MOCK | 0 | 0.72 | 0.02 | | | | | | |
| | 24 | 1.14 | 0.03 | | | | | | |
| | 48 | 1.75 | 0.08 | | | | | | |
| BLANK | 0 | 0.70 | 0.01 | | | | | | |
| | 24 | 1.14 | 0.02 | | | | | | |
| | 48 | 1.73 | 0.01 | | | | | | |

Among them, the MOCK group was the group treated with transfection reagent alone without any sequence added; the BLANK group was the group containing only cells. NC5 was the negative control siRNA, and the sequence information can be found in Table 19 in Example 4.

Conclusion: A total of 9 sequences, namely GPSZRA314S1-VP, GPSZRA314S2-VP, GPSZRA314S3-VP, GPSZRA314S5-VP, GPSZRA402S4-VP, GPSZRA402S5-VP, GPSZRA222S3-VP, GPSZRA222S4-VP and GPSZRA318S2-VP, exhibited no significant differences in cytotoxicity against A549 RAGE LV and BEAS-2B RAGE LV cells compared with BLANK, MOCK group and negative control. Therefore, these sequences showed no cytotoxicity toward the above-mentioned cells.

### Example 11: Immunotoxicity

Experimental principle: PBMCs are a mixture of various immune cells that are key components of the innate and adaptive immune systems. When PBMCs are stimulated by external pathogens, they induce a series of immune responses, ultimately expressing interferons and cytokines. By co-incubating siRNA with PBMCs and detecting the expression of representative downstream cytokines and interferons, it is possible to determine whether siRNA has immunotoxicity.

Experimental objective: To transfect PBMC cells with a sequence, detect downstream immune response-related molecules, and verify whether the sequence has immunotoxicity.

In this experiment, PHA (phytohemagglutinin) was used as a plant-derived hemagglutinin, which can serve as a positive control agent to stimulate immune cells to mount an immune response and induce the expression of cytokines. Poly IC is a double-stranded RNA analog that can induce the expression of interferons and inflammatory factors. Donor refers to the provider of PBMC cells; Donor 1, Donor 2 and Donor 3 represent three different healthy human subjects. The experiment was performed as follows: first, PBMCs were isolated from the donors, then the isolated PBMCs were transfected with modified siRNAs, followed by the detection of downstream molecules related to immune response, so as to verify whether the sequences possessed immunotoxicity.

### Experimental steps:

### (1) Experimental reagents, as shown in Table 42.

**Table 42: Sources of experimental reagents**

| Reagent name | Manufacturer | Cat. No./Batch No. | Storage conditions |
|---|---|---|---|
| PBS | GIBCO | 10010-031/2492791 | 4°C |
| Human peripheral blood lymphocyte separation medium | Solarbio | P8610 | Room temperature |
| 1640 medium | GIBCO | 11875-093/2561359 | 4°C |
| Poly IC | MCE | HY-107202A | -20°C |
| PHA | Autobio | TB-IGRA | 4°C |
| GenePORTER 2 Transfection reagent | amsbio | AMS.T202015 | 4°C |
| Opti-MEM culture medium | GIBCO | 31985-070/2537156 | 4°C |
| Cytokine combined detection kit | Jiangxi Cellgenebio | 12-Plex | 4°C |
| 96 Well Plate | Corning | 3590/25019022 | Room temperature |

### (2) Cell preparation

### Isolation of PBMC cells:

Three blood samples from donors were prepared. Three 15 mL centrifuge tubes (labeled with serial numbers) were taken, into which blood (about 2 mL) was poured. Subsequently, 2 mL of PBS was added, and the mixture was inverted repeatedly for thorough mixing;
Another three 15 mL centrifuge tubes were prepared, and 3 mL of lymphocyte separation medium was added. The mixed blood samples were slowly transferred into the tubes using a pipette, followed by centrifugation in a low-temperature centrifuge with the parameters set as follows: 2000 rpm for 20 min at 4°C, with acceleration set to 9 and deceleration set to 6;
Another three 15 mL centrifuge tubes were prepared, and 4 mL of pbs was added. The membrane (i.e., the cells) between the upper yellow layer and the middle white layer after centrifugation was aspirated (2 mL), and then subjected to low-temperature centrifugation in a centrifuge with the parameters set as follows: 1500 rpm, 10 min, 4°C, with acceleration set to 9 and deceleration set to 9;
The supernatant was discarded, and 2 mL of PBS was added for washing. The pellet was not required to be resuspended, and centrifugation was performed again under the same parameters as above;
The supernatant was discarded, and 300 µL of 1640 medium was added to resuspend the pellet. Three 1.5 mL EP tubes were taken, with 180 µL of PBS added to each tube; subsequently, 20 µL of the cell suspension was transferred into the tubes and mixed thoroughly (for a 10-fold dilution), followed by cell counting using a cell counter;
The cells were then seeded into a 96-well plate at 2×10⁶ cells/mL with a volume of 50 µL/well for subsequent transfection experiments.

### (3) Cell transfection

A total of 7 sequences were used, with PHA, Poly IC, Inclisiran, MOCK, and complete culture medium as controls. The MOCK group was the group treated with transfection reagent alone without any sequence added.

siRNA final concentration: 100 nM; poly IC final concentration: 2 µg/ml.
Preparation of siRNA Diluent: 1 µL of 10 µM siRNA stock solution was added to 12.5 µL of DNA Diluent B and mixed thoroughly;
Preparation of Poly IC Diluent: 1 µL of 0.2 µg/µL Poly IC solution was added to 12.5 µL of DNA Diluent B and mixed thoroughly;
Preparation of GenePORTER2 Diluent: 1.75 µL of GenePORTER2 was dissolved in 7 µL of Opti-MEM;
Preparation of transfection complexes: The siRNA diluent or Poly IC diluent was mixed with the GenePORTER2 diluent, respectively, and incubated at room temperature for 5-10 min;
Upon completion of the incubation period, 22.25 µL of the transfection complex was added to each corresponding well, followed by the addition of the respective siRNA. Culture medium was then supplemented to achieve a final volume of 100 µL per well;
Note: PHA does not require transfection reagent; 15 µL of PHA was directly mixed with the cell suspension, and culture medium was supplemented to a final volume of 100 µL.

Approximately 48 h after transfection, the cell supernatant was collected for subsequent immunogenicity assays.

### (4) Immunogenicity assay

2 mL of calibrator was placed into a centrifuge tube and labeled as the highest concentration;
Subsequently, 2 mL of sample diluent was added, and the mixture was incubated at room temperature for 15 min (to be used within 4 h);
The mixture was gently pipetted up and down for 30 s; violent shaking and bubble formation were avoided;
Eleven loading tubes were prepared and labeled as 1:2, 1:4, 1:8, 1:16, 1:32, 1:64, 1:128, 1:256, 1:512, 1:1024, and 1:2048, and 300 µL of sample diluent was added to each tube (to be used within 4 h);
300 µL of the highest-concentration calibrator was transferred to the 1:2 tube, pipetted gently to mix thoroughly. Then 300 µL of the mixture from the 1:2 tube was transferred to the 1:4 tube, and the serial dilution was repeated sequentially until the 1:2048 tube;
The total number of samples was calculated (33 test samples, 11 calibrators, and 1 negative control). The required volume of capture microsphere cocktail was determined based on the total sample number (25 µL per sample);
The capture microsphere cocktail was pipetted up and down for 30 s. The required volume of the cocktail was aspirated and centrifuged at 200g for 5 min using a low-speed centrifuge. The supernatant was carefully aspirated and discarded. An equal volume of microsphere buffer was added to resuspend the pellet, which was then incubated for 15-30 min in the dark after gentle pipetting;
25 µL of fluorescence detection reagent was added to each assay tube;
25 µL of the serially diluted calibrator was added to the calibrator tubes, and 25 µL of the test sample was added to the sample tubes, followed by vortexing to mix thoroughly;
The incubated capture microsphere cocktail was pipetted up and down for 30 s, 25 µL of the cocktail was added to each assay tube, and the tubes were shaken;
After the above operations, the tubes were incubated at room temperature for 2.5-3 h in the dark;
1 mL of wash buffer was added to each assay tube, which was then centrifuged at 200g for 5 min. The liquid in the tubes was discarded, and any residual liquid at the tube mouth was blotted and removed with absorbent paper;
200 µL of wash buffer was added to each assay tube, which was then placed in the dark awaiting detection;
The assay tubes were loaded into the detection instrument in the following order: calibrators (from the lowest to the highest concentration), negative control (25 µL of microspheres + 300 µL of wash buffer), and test samples. Each assay tube was vortexed for 3-5 s and immediately loaded for detection.

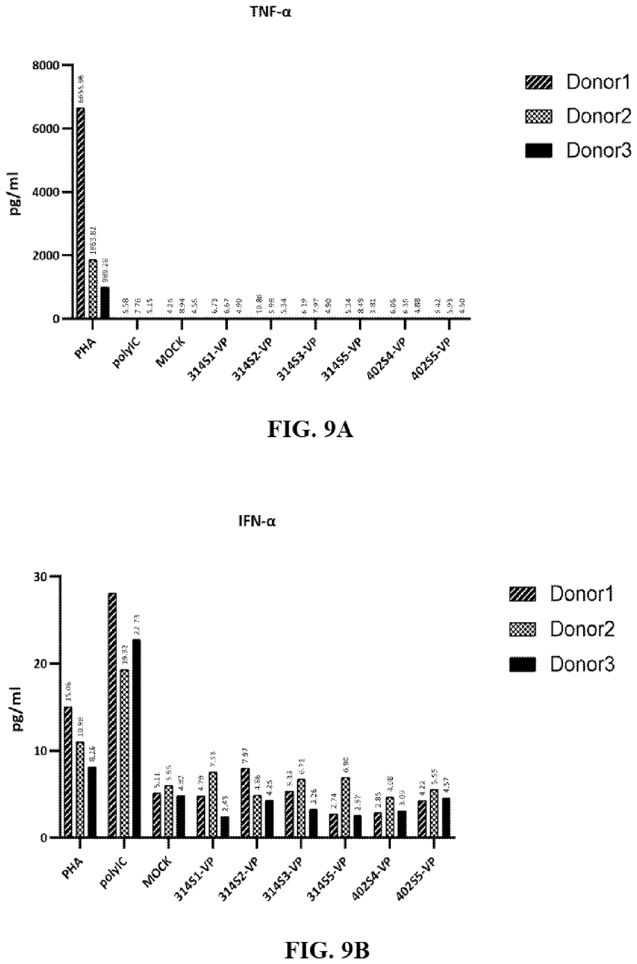

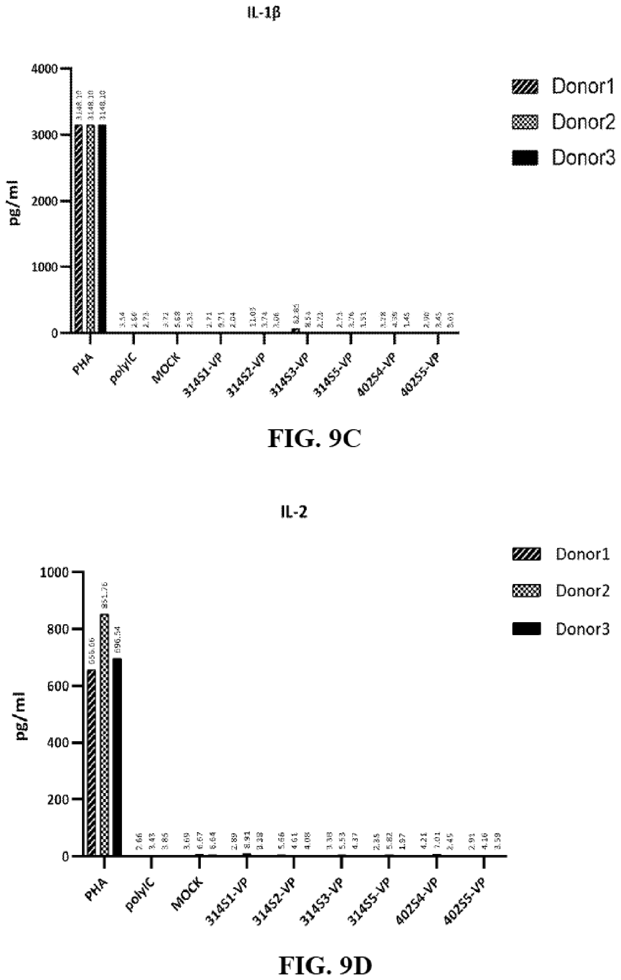

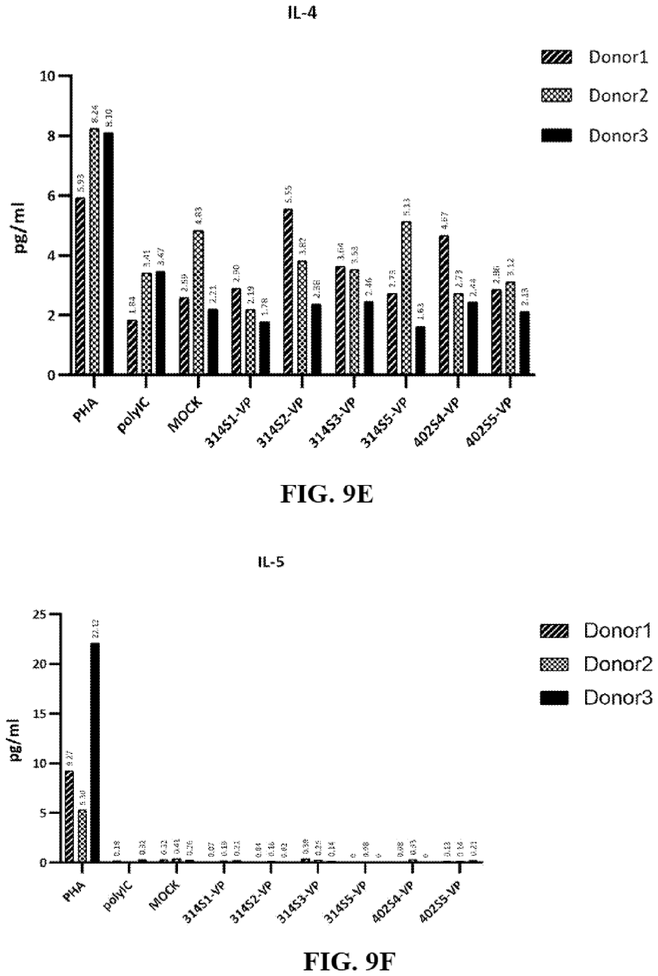

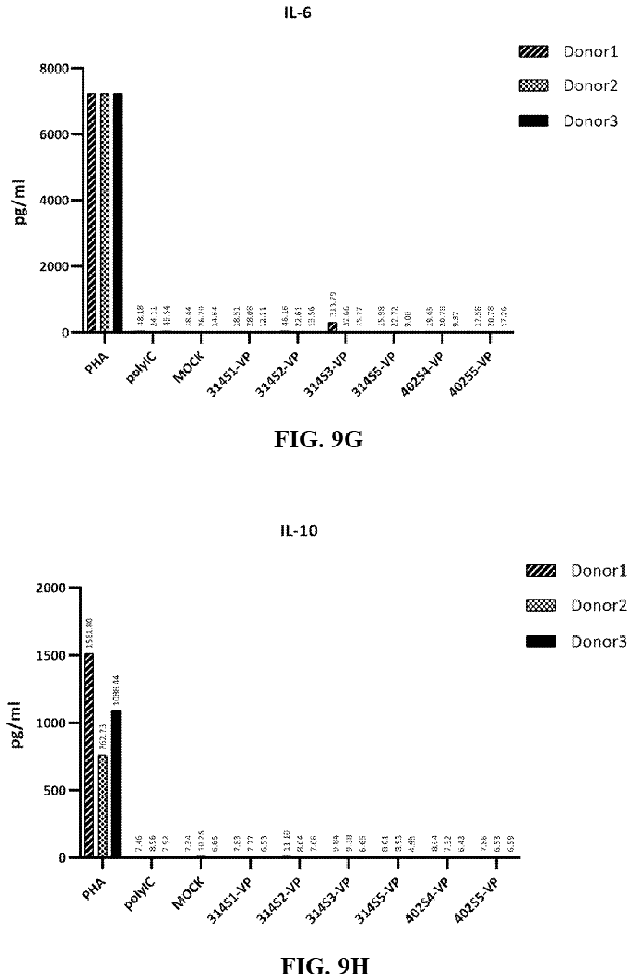

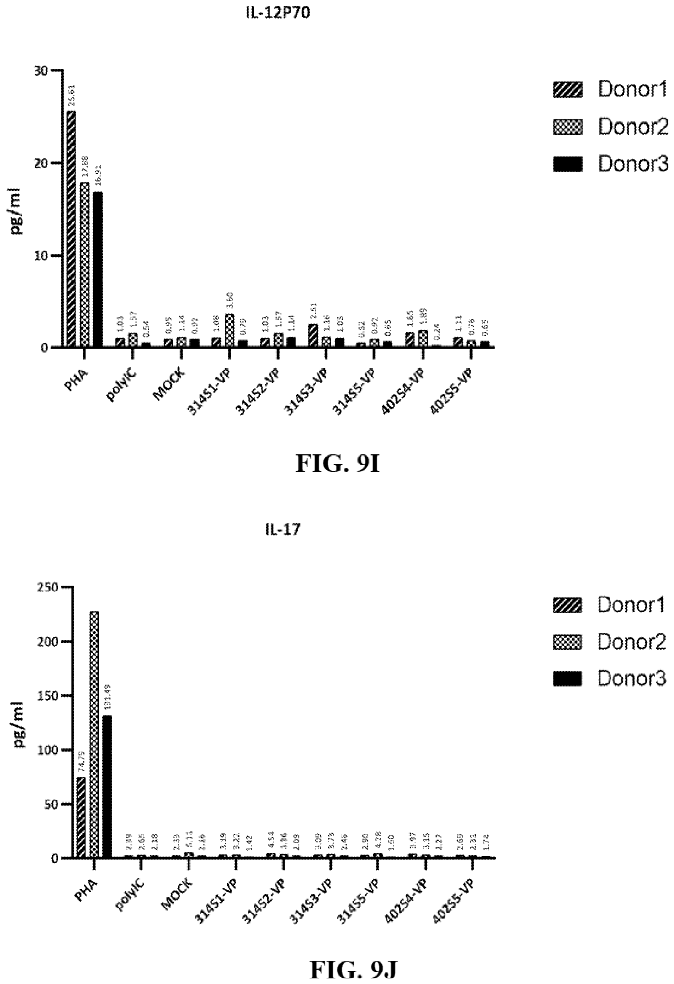

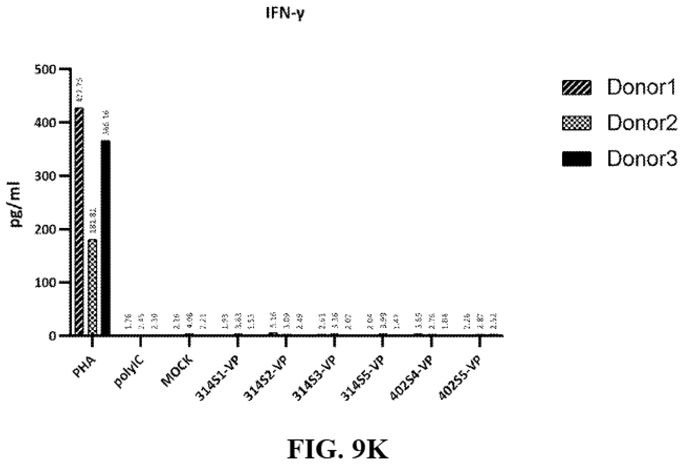

The experimental results are presented in FIG. 9A to FIG. 9K and Tables 43 and 44 below. Compared with the MOCK group, PHA activated the production of cytokines including TNF-α, IFN-α, IL-1β, IL-2, IL-4, IL-5, IL-6, IL-10, IL-12P70, IL-17, and IFN-γ, whereas the test sequences did not induce a significant upregulation of these cytokines.

Tables 43 and 44 summarize the experimental results by calculating the average of the 3 donor values.

**Table 43: Cellular immune responses induced by modified siRNAs**

| Test object | IFN-α | | IL-1β | | IL-2 | | IL-4 | | IL-5 | | IL-6 | |
|---|---|---|---|---|---|---|---|---|---|---|---|---|
| | pg/m l | SD | pg/ml | SD | pg/ml | SD | pg/m l | SD | pg/m l | SD | pg/ml | SD |
| PHA | 11.4 0 | 3.4 7 | 3148.1 0 | 0.00 | 734.9 9 | 103.0 8 | 7.42 | 1.3 0 | 12.2 3 | 8.7 9 | 7251.0 5 | 0.00 |
| polyIC | 23.3 7 | 4.4 1 | 2.96 | 0.51 | 3.31 | 0.60 | 2.91 | 0.9 2 | 0.25 | 0.1 0 | 39.28 | 13.20 |
| MOCK | 5.29 | 0.5 9 | 3.91 | 1.68 | 5.67 | 1.71 | 3.21 | 1.4 2 | 0.33 | 0.0 8 | 19.93 | 6.17 |
| GPSZRA314S 1-VP | 4.92 | 2.5 5 | 4.82 | 4.25 | 5.06 | 3.34 | 2.29 | 0.5 7 | 0.16 | 0.0 8 | 19.57 | 8.04 |
| GPSZRA314S 2-VP | 5.69 | 2.0 0 | 5.94 | 4.42 | 4.78 | 0.80 | 3.92 | 1.5 9 | 0.07 | 0.0 8 | 27.44 | 16.83 |
| GPSZRA314S 3-VP | 5.10 | 1.7 4 | 24.71 | 33.1 6 | 4.43 | 1.08 | 3.21 | 0.6 5 | 0.26 | 0.1 3 | 120.74 | 167.4 0 |
| GPSZRA314S 5-VP | 4.07 | 2.4 5 | 2.67 | 1.13 | 3.38 | 2.12 | 3.16 | 1.7 9 | 0.03 | 0.0 5 | 15.90 | 6.86 |
| GPSZRA402S 4-VP | 3.54 | 0.9 9 | 3.04 | 1.48 | 4.56 | 2.30 | 3.28 | 1.2 1 | 0.14 | 0.1 7 | 16.73 | 5.89 |
| GPSZRA402S 5-VP | 4.78 | 0.6 9 | 3.12 | 0.29 | 3.55 | 0.63 | 2.70 | 0.5 1 | 0.16 | 0.0 4 | 18.54 | 1.95 |

As shown in Table 43, compared with the MOCK group, the secretion of IFN-α, IL-1β, IL-2, IL-4, IL-5, and IL-6 was increased by the stimulation of PHA or Poly IC, while the siRNA treatment group showed no significant effect.

**Table 44: Cellular immune responses induced by modified siRNAs**

| Test object | IL-10 | | IL-12P70 | | IL-17 | | INF-γ | | TNF-α | |
|---|---|---|---|---|---|---|---|---|---|---|
| | pg/ml | SD | pg/ml | SD | pg/ml | SD | pg/ml | SD | pg/ml | SD |
| PHA | 1120.99 | 375.5 9 | 20.13 | 4.77 | 144.52 | 77.08 | 325.24 | 127.97 | 3169.69 | 3050.70 |
| polyIC | 8.11 | 0.77 | 1.05 | 0.52 | 2.41 | 0.24 | 2.17 | 0.36 | 6.16 | 1.40 |
| MOCK | 8.08 | 1.91 | 1.00 | 0.12 | 3.20 | 1.66 | 2.84 | 1.05 | 5.91 | 2.63 |
| GPSZRA 314S1-VP | 7.21 | 0.65 | 1.82 | 1.55 | 2.61 | 1.03 | 2.36 | 1.12 | 6.10 | 1.04 |
| GPSZRA 314S2-VP | 8.73 | 2.11 | 1.25 | 0.29 | 3.33 | 1.23 | 3.58 | 1.40 | 7.39 | 3.02 |
| GPSZRA 314S3-VP | 8.62 | 1.72 | 1.57 | 0.82 | 3.09 | 0.64 | 2.66 | 0.67 | 6.35 | 1.54 |
| GPSZRA 314S5-VP | 7.29 | 2.09 | 0.70 | 0.20 | 2.93 | 1.34 | 2.50 | 1.32 | 5.88 | 2.39 |
| GPSZRA 402S4-VP | 7.53 | 1.11 | 1.26 | 0.89 | 3.13 | 0.85 | 2.75 | 0.91 | 5.76 | 0.78 |
| GPSZRA 402S5-VP | 6.99 | 0.75 | 0.84 | 0.24 | 2.25 | 0.48 | 2.55 | 0.31 | 5.28 | 0.72 |

As shown in Table 44, compared with the MOCK group, the secretion of IL-10, IL-12P70, IL-17, IFN-γ, and TNF-α increased with the stimulation of PHA, while the siRNA treatment group did not lead to an increase in the secretion of these factors.

Conclusion: All 6 sequences, GPSZRA314S1-VP, GPSZRA314S2-VP, GPSZRA314S3-VP, GPSZRA314S5-VP, GPSZRA402S4-VP, and GPSZRA402S5-VP, showed no immunotoxicity.

### Example 12: PSICHECK off-target assay

Experimental principle: In some cases, siRNA may downregulate non-target genes whose 3'-untranslated region of certain transcripts is complementary to the seed region (nucleotides 2-8) of the guide strand. This off-target effect, whose regulatory mechanism is similar to that of miRNA target silencing, is called miRNA-like off-target. In addition, other types of off-target effects of siRNA may be due to the loading of the sense strand into the RNA-induced silencing complex, namely sense strand off-target. To simultaneously investigate whether siRNA has miRNA-like off-target risk, sense strand off-target risk, and sense strand seed region off-target risk, we designed three types of off-target plasmids: GSSM (antisense strand seed region off-target plasmid), PSCM (sense strand off-target plasmid), PSSM (sense strand seed region off-target plasmid), as well as the target-positive plasmid (GPSZRA) which were cotransfected with siRNA into 293T cells. The presence of off-target risk was evaluated by the level of silencing efficiency.

Experimental objective: To evaluate the off-target risk of candidate sequences and VP-modified sequences in vitro using the psiCHECK system, including the off-target risk of the antisense strand seed region, the off-target risk of the sense strand, and the off-target risk of the sense strand seed region.

### Experimental steps:

(1) The experimental reagents are shown in Table 45.

**Table 45: Experimental reagents used in the experiment**

| Reagent name | Manufacturer | Cat. No./Batch No. | Storage conditions |
|---|---|---|---|
| DEPC water | GenePharma | -/159297 | 4°C |
| Dual-luciferase assay kit (main) | Promega | E2940/0000500057 | -20°C |
| Opti-MEM | Gibco | 31985-070/2152782 | 2-8°C |
| Lipofectamine 2000 | ThermoFisher | 11668-019/2398586 | 4°C |
| DMEM medium | Transgen Biotech | FI101-01/Q20506 | 4°C |
| Trypsin | GIBCO | 25200-072/2185857 | 4°C |
| PBS | Hyclone | SH30256.01/AF29483105 | 4°C |
| 96-well cell culture plate | Corning | 3599/28921006 | RT |
| 96-well cell assay plate | Corning | 353296/- | RT |

### (2) Cell preparation

Conventionally cultured 293T cells were digested with trypsin. The cells were resuspended in complete culture medium and the cell concentration was adjusted to 1×10⁶ cells/mL for subsequent experiments.

### (3) Cell transfection

### 1) Dilution of transfection reagent

Based on the actual number of wells transfected and taking into account losses, the transfection reagent was diluted with opti-MEM and then added to the 96-well plate (transfection reagent + RNA plate) using a multi-channel pipette, so that each well contained 130 µL opti-MEM and 1.2 µL Lipofectamine 2000 transfection reagent. This was the transfection reagent dilution plate.

### 2) Dilution of siRNA (RNA oligo)

siRNA was dissolved: 1 OD + 250 µL DEPC water. The siRNA concentration at this point was 10 µM, which can be stored at -20°C for later use. siRNA was prepared according to the predetermined siRNA concentration. If 10 nM siRNA was needed, 4 µL of 10 µM siRNA was aspirated to 196 µL of opti-MEM water. At this point, the siRNA concentration was 200 nM for later use. Then the 200 nM siRNA was diluted to 20 nM. The prepared siRNA was added to a 96-well plate (transfection reagent + RNA plate) at a rate of 20 µL/well, according to the siRNA layout on the plate.

### 3) Plasmid dilution

Based on the siRNA layout on the plate, the corresponding plasmid was diluted with opti-MEM according to the number of wells and considering loss, mixed well and then added to a 96-well plate (plated according to the corresponding siRNA wells), so that each well contained 80 ng of plasmid in 50 µL of opti-MEM. This was the plasmid dilution plate.

### 4) Transfection

A multi-channel pipette was used to plate the plasmids according to the "plasmid plate". 50 µL of plasmids was aspirated from each well and added to the corresponding "transfection reagent + RNA plate", yielding transfection complexes at 200 µL/ well, which were then incubated for approximately 20 min. A fully automated liquid handling workstation operation program was used to pipette 50 µL of the transfection complex (200 µL per well) from each well and dispensed into 3 replicate cell plates. Subsequently, 50 µL of the cells prepared in 6.1 was added to each well, and the plates were placed in an incubator.

### 5) Cell lysis detection

The cell plates were removed from the carbon dioxide incubator, and 20 µL of the original cell culture medium was aspirated and discarded using a liquid handling workstation.

L from the kit was added to a new 96-well plate. A fully automated liquid handling workstation was then operated with a program to add 75 µL of Dual-Glo^{®} Luciferase Reagent to each well of all cell plates. The plates were incubated at room temperature for 20 min, after which the program of the fully automated liquid handling workstation run again to transfer 75 µL of the lysate to a white plate. A microplate reader was used to detect Firefly luciferase, and the Firefly luminescence intensity was measured with a multimode microplate reader.

A fully automated liquid handling workstation was operated with a program to add 75 µL of Dual-Glo^{®} Stop & Glo^{®} Reagent to each well of the white plate. Renilla luciferase was detected using a microplate reader.

The results are shown in Table 46 to Table 49 below.

**Table 46: Inhibitory efficiency of the target-positive plasmid (GPSZRA) for siRNA sequences**

| Test sequence | Target-positive plasmid (GPSZRA) | | | | | |
|---|---|---|---|---|---|---|
| | 0.1 nM | SD | 1 nM | SD | 10 nM | SD |
| GPSZRA222S1 | 62.08% | 0.0592 | 82.64% | 0.0298 | 85.31% | 0.0217 |
| GPSZRA222S3 | 53.69% | 0.0995 | 80.90% | 0.0311 | 85.94% | 0.0258 |
| GPSZRA222S4 | 63.44% | 0.0374 | 81.55% | 0.0263 | 85.63% | 0.0089 |
| GPSZRA222S3-VP | 80.18% | 0.0391 | 84.89% | 0.0212 | 86.81% | 0.0196 |
| GPSZRA222S4-VP | 78.37% | 0.0437 | 85.78% | 0.0210 | 88.41% | 0.0080 |
| GPSZRA267S2 | 39.35% | 0.0884 | 87.95% | 0.0163 | 94.34% | 0.0088 |
| GPSZRA267S2-VP | 81.22% | 0.0139 | 94.22% | 0.0039 | 95.77% | 0.0031 |
| GPSZRA318S2 | 31.58% | 0.0137 | 86.32% | 0.0056 | 93.63% | 0.0013 |
| GPSZRA402S4 | 70.23% | 0.1298 | 89.74% | 0.0468 | 88.39% | 0.0458 |
| GPSZRA402S5 | 74.26% | 0.0775 | 89.68% | 0.0352 | 90.87% | 0.0191 |
| GPSZRA402S4-VP | 82.29% | 0.0380 | 90.79% | 0.0161 | 89.91% | 0.0124 |
| GPSZRA402S5-VP | 84.03% | 0.0317 | 91.31% | 0.0037 | 91.56% | 0.0049 |
| GPSZRA314S1 | 77.51% | 0.0200 | 88.47% | 0.0125 | 90.63% | 0.0171 |
| GPSZRA314S2 | 76.82% | 0.0358 | 89.53% | 0.0094 | 90.25% | 0.0189 |
| GPSZRA314S3 | 71.23% | 0.0288 | 88.54% | 0.0080 | 90.46% | 0.0100 |
| GPSZRA314S5 | 73.01% | 0.0081 | 89.93% | 0.0071 | 91.14% | 0.0087 |
| GPSZRA314S1-VP | 86.79% | 0.0094 | 89.62% | 0.0072 | 92.10% | 0.0085 |
| GPSZRA314S2-VP | 86.24% | 0.0095 | 89.83% | 0.0080 | 91.54% | 0.0079 |
| GPSZRA314S3-VP | 84.57% | 0.0210 | 90.10% | 0.0142 | 91.68% | 0.0170 |
| GPSZRA314S5-VP | 87.15% | 0.0148 | 90.02% | 0.0084 | 91.90% | 0.0091 |
| NC1 | 0.00% | 0.1075 | 0.00% | 0.1081 | 0.00% | 0.0912 |

**Table 47: Inhibition efficiency of siRNA sequences on antisense strand seed region off-target plasmid**

| Test sequence | Antisense strand seed region off-target plasmid (GSSM) | | | |
|---|---|---|---|---|
| | 0.1 nM | SD | 1 nM | SD |
| GPSZRA222S1 | 10.99% | 0.0832 | 31.18% | 0.0795 |
| GPSZRA222S3 | 9.56% | 0.0656 | 12.04% | 0.1995 |
| GPSZRA222S4 | 3.68% | 0.0510 | 10.20% | 0.0912 |
| GPSZRA222S3-VP | 12.59% | 0.0761 | 33.52% | 0.0858 |
| GPSZRA222S4-VP | 6.97% | 0.0786 | 25.15% | 0.0845 |
| GPSZRA267S2 | -4.41% | 0.0399 | 19.22% | 0.1166 |
| GPSZRA267S2-VP | 10.16% | 0.0527 | 36.15% | 0.0714 |
| GPSZRA318S2 | 3.11% | 0.0523 | 12.59% | 0.0139 |
| GPSZRA402S4 | 4.75% | 0.2148 | 13.35% | 0.1976 |
| GPSZRA402S5 | 9.08% | 0.1364 | 10.06% | 0.0598 |
| GPSZRA402S4-VP | 13.47% | 0.1425 | 12.23% | 0.1321 |
| GPSZRA402S5-VP | 9.73% | 0.1139 | 14.47% | 0.0983 |
| GPSZRA314S1 | 4.14% | 0.0625 | 6.32% | 0.0888 |
| GPSZRA314S2 | 3.87% | 0.0453 | 10.88% | 0.0218 |
| GPSZRA314S3 | 3.33% | 0.0910 | 22.23% | 0.0252 |
| GPSZRA314S5 | -0.15% | 0.0672 | 3.40% | 0.0311 |
| GPSZRA314S1-VP | 3.11% | 0.0422 | 6.00% | 0.0310 |
| GPSZRA314S2-VP | 5.55% | 0.0018 | 14.63% | 0.0652 |
| GPSZRA314S3-VP | 7.73% | 0.0217 | 22.42% | 0.0477 |
| GPSZRA314S5-VP | 6.29% | 0.0355 | 16.43% | 0.0331 |
| NC1 | 0.00% | 0.0369 | 0.00% | 0.0797 |

**Table 48: Inhibition efficiency of siRNA sequences on sense strand off-target plasmid**

| Test sequence | Sense strand off-target plasmid (PSCM) | | | | | |
|---|---|---|---|---|---|---|
| | 0.1 nM | SD | 1 nM | SD | 10 nM | SD |
| GPSZRA222S1 | 1.85% | 0.0335 | 14.03% | 0.0347 | 31.22% | 0.0451 |
| GPSZRA222S3 | 1.39% | 0.0354 | 7.23% | 0.1070 | 6.37% | 0.0266 |
| GPSZRA222S4 | 0.54% | 0.0783 | 2.86% | 0.0563 | 9.41% | 0.0351 |
| GPSZRA222S3-VP | 0.48% | 0.1026 | 6.27% | 0.0250 | 3.35% | 0.0720 |
| GPSZRA222S4-VP | -1.06% | 0.1247 | -4.59% | 0.0347 | 10.28% | 0.0484 |
| GPSZRA267S2 | -1.06% | 0.0106 | 1.40% | 0.0644 | -0.83% | 0.0642 |
| GPSZRA267S2-VP | -4.91% | 0.0223 | -8.50% | 0.0196 | 5.26% | 0.0286 |
| GPSZRA318S2 | 1.98% | 0.0164 | 15.66% | 0.0282 | 19.67% | 0.0524 |
| GPSZRA402S4 | 19.53% | 0.0462 | 9.91% | 0.2081 | 2.52% | 0.0994 |
| GPSZRA402S5 | 26.66% | 0.0415 | 16.85% | 0.0658 | -8.58% | 0.0502 |
| GPSZRA402S4-VP | 30.00% | 0.1230 | 1.78% | 0.0864 | -15.95% | 0.1465 |
| GPSZRA402S5-VP | 19.60% | 0.2220 | 8.88% | 0.2008 | -5.78% | 0.1388 |
| GPSZRA314S1 | 3.64% | 0.0392 | -21.96% | 0.0456 | 0.92% | 0.0859 |
| GPSZRA314S2 | 3.05% | 0.0343 | -15.16% | 0.0686 | -6.06% | 0.1231 |
| GPSZRA314S3 | 8.36% | 0.1101 | -26.24% | 0.0628 | 2.84% | 0.1559 |
| GPSZRA314S5 | 4.72% | 0.0713 | -14.54% | 0.0545 | -3.29% | 0.1012 |
| GPSZRA314S1-VP | -8.00% | 0.1685 | -25.24% | 0.0930 | 5.20% | 0.1217 |
| GPSZRA314S2-VP | -8.00% | 0.0859 | -31.81% | 0.1622 | 2.88 | 0.1220 |
| GPSZRA314S3-VP | -6.11% | 0.0248 | -20.06% | 0.0572 | -16.14% | 0.1907 |
| GPSZRA314S5-VP | 12.49% | 0.0475 | -20.37% | 0.0824 | -10.47% | 0.1533 |
| NC1 | 0.00% | 0.0690 | 0.00% | 0.0494 | 0.00% | 0.1166 |

**Table 49: Inhibition efficiency of siRNA sequences on sense strand seed region off-target plasmid**

| Test sequence | Sense strand seed region off-target plasmid (PSSM) | | | | | |
|---|---|---|---|---|---|---|
| | 0.1 nM | SD | 1 nM | SD | 10 nM | SD |
| GPSZRA222S1 | -6.79% | 0.1117 | 2.46% | 0.0444 | 18.98% | 0.0599 |
| GPSZRA222S3 | 4.61% | 0.0535 | 5.82% | 0.0568 | 15.96% | 0.0348 |
| GPSZRA222S4 | -0.59% | 0.0632 | -5.29% | 0.1618 | 12.56% | 0.0909 |
| GPSZRA222S3-VP | 1.13% | 0.0715 | -8.42% | 0.0823 | 5.42% | 0.0216 |
| GPSZRA222S4-VP | 1.95% | 0.0590 | 0.36% | 0.0547 | 7.93% | 0.0330 |
| GPSZRA267S2 | -3.62% | 0.0654 | -1.66% | 0.0100 | 5.35% | 0.0256 |
| GPSZRA267S2-VP | 0.76% | 0.0680 | -5.11% | 0.0134 | 6.78% | 0.0242 |
| GPSZRA318S2 | -5.96% | 0.0492 | -2.00% | 0.0111 | 8.68% | 0.0202 |
| GPSZRA402S4 | 1.65% | 0.1588 | 18.66% | 0.1607 | 3.69% | 0.0763 |
| GPSZRA402S5 | -7.14% | 0.0428 | 26.06% | 0.1284 | 7.78% | 0.0097 |
| GPSZRA402S4-VP | -4.59% | 0.0670 | 17.65% | 0.0946 | -0.05% | 0.1086 |
| GPSZRA402S5-VP | -5.41% | 0.0377 | 14.99% | 0.2679 | -19.02% | 0.2453 |
| GPSZRA314S1 | -1.92% | 0.0223 | -10.44% | 0.0548 | -4.83% | 0.0873 |
| GPSZRA314S2 | 6.54% | 0.0173 | 7.81% | 0.1477 | -8.05% | 0.0533 |
| GPSZRA314S3 | 6.58% | 0.0316 | 1.15% | 0.0429 | -12.53% | 0.0279 |
| GPSZRA314S5 | 6.56% | 0.0300 | -4.84% | 0.0715 | -14.12% | 0.0649 |
| GPSZRA314S1-VP | 1.69% | 0.0840 | -17.92% | 0.0978 | -10.25% | 0.1163 |
| GPSZRA314S2-VP | -1.33% | 0.0298 | -25.41% | 0.0717 | -17.06% | 0.0578 |
| GPSZRA314S3-VP | -3.67% | 0.0653 | -21.18% | 0.0734 | -20.09% | 0.0573 |
| GPSZRA314S5-VP | -5.57% | 0.0727 | -19.07% | 0.0728 | -19.50% | 0.0457 |
| NC1 | 0.00% | 0.0721 | 0.00% | 0.1027 | 0.00% | 0.0544 |

Conclusion: The modified siRNAs exhibited an extremely high on-target rate with an off-target rate within the acceptable range, making them suitable candidate molecules for drug development.

### Example 13: In vivo activity assay

This experiment was conducted to determine the efficiency of siRNA in knocking out RAGE mRNA and protein in mice.

In vivo activity assay in RAGE humanized (CDS region) homozygous/RAGE humanized (CDS+3'UTR region) heterozygous mice
(1) In vivo administration: commercial RAGE homozygous/heterozygous mice. In the in vivo experiment, each group contained 3-4 mice, which were male and 6-8 weeks old. Candidate siRNA molecules at a dose of 3mg/kg were delivered to the lungs via aerosol needle airway administration, with an aerosol volume of 50ul each time. Mice were anesthetized and sacrificed after 11 or 15 days of treatment. First, bronchoalveolar lavage fluid (BALF) was collected with pre-cooled PBS, centrifuged to obtain the supernatant for detecting RAGE protein expression level. After perfusion, lung tissues were collected and frozen for detecting RAGE expression at mRNA and protein levels.
(2) RNA expression detection: After lung tissue was removed from the body, it was quickly frozen in liquid nitrogen and then stored in a -80°C freezer for long-term preservation. Fresh or ultra-low temperature frozen samples were cut into 20-40mg pieces with a disposable blade, added to 1.5 ml sterile tubes, and quickly placed in liquid nitrogen. According to the instructions of TaKaRa MiniBEST Universal RNA Extraction Kit (Takara: 9767), the prepared tissue lysis buffer was added, and magnetic beads were added, followed by homogenization on a tissue homogenizer. After homogenization, the subsequent RNA extraction steps were performed according to the instructions. Extended gene expression detection was performed using the One Step PrimeScript^{™} RT-PCR Kit (Takara: RR064A). Real-time fluorescence PCR was performed using the ΔΔCt assay in the ABI QS5 real-time fluorescence PCR system. Three replicate wells were used for each mouse's lung tissue sample. The testing results of the in vivo activity of duplexes are shown in Table 50 to Table 54 below.

TRiM delivery is the name given by arrowhead for the siRNA delivery system it developed. siRNA has a large molecular weight and strong hydrophilicity, making it difficult to penetrate into cells. In order to deliver siRNA to specific tissue cells, a specific small molecule target is coupled to the sense strand end of the siRNA through a linker with a specific structure. Small molecule targets can specifically bind to specific receptors (integrin αvβ6) on target tissue cells and mediate the endocytosis of siRNA coupled with the small molecule targets into the cells, thereby initiating the RNA interference process and exerting the drug effect.

(3) Protein expression detection: After anesthetizing mice, bronchoalveolar lavage fluid was obtained by perfusion with pre-cooled PBS. The sample was centrifuged at 12,000 rpm for 15 min in a centrifuge at 4°C, and then the supernatant was taken for protein quantification (Pierce^{™} BCA Protein Assay Kits (Thermo-23225)). The obtained lung tissue was cut into small pieces, and 30-50 mg/tube was weighed on a balance and washed twice with pre-cooled PBS. RIPA lysis buffer (containing PMSF) was added, and magnetic beads were added followed by vortex oscillation for 3 times. The mixture was then centrifuged at 14000 g for 5 min at 4°C to precipitate tissue or cell debris. The supernatant was transferred as total tissue protein, aliquoted and stored at -80°C. RAGE protein expression was detected using the Human RAGE ELISA KIT (R&D-DRG00). Two replicate wells were used for each mouse's lung tissue sample. The testing results of the in vivo activity of duplexes are shown in Table 50 to Table 54 below.

**Table 50: Knockdown of RAGE mRNA and protein activity in homozygous RAGE humanized (CDS region) mice at 15 days after aerosol airway administration of GPSZRA314S1-VP, GPSZRA314S2-VP and GPSZRA314S3-VP (TRiM delivery)**

| Duplex number | 3 mg/kg (day 15) | | | | | |
|---|---|---|---|---|---|---|
| | Avg expression | Std Dev | Lung RAGE protein | Std Dev | Total BALF RAGE protein (ng/ml) | Std Dev |
| | (Lung RNA) * | (+/-) | (ng /ml) | (+/-) | | (+/-) |
| Saline | 1.048 | 0.3485 | 1759537 | 370579 | 239902 | 208641 |
| GPSZRA314S1-VP | 0.135 | 0.04435 | 750767 | 219289 | 2347 | 748.1 |
| GPSZRA314S2-VP | 0.338 | 0.113 | 841008 | 328840 | 2109 | 698.6 |
| GPSZRA314S3-VP | 0.345 | 0.2445 | 1186798 | 272215 | 3174 | 1998 |
| *: This value is the relative expression level of the RAGE mRNA compared with the saline group | | | | | | |

**Table 51: Knockdown of RAGE mRNA and protein activity in homozygous RAGE humanized (CDS region) mice at 15 days after aerosol airway administration of 314S5-VP (TRiM delivery)**

| Duplex number | 3 mg/kg (day 15) | | | | | |
|---|---|---|---|---|---|---|
| | Avg expression | Std Dev | Lung RAGE protein | Std Dev | Total BALF RAGE protein (ng/ml) | Std Dev |
| | (Lung RNA) * | (+/-) | (ng /ml) | (+/-) | | (+/-) |
| Saline | 0.735 | 0.07778 | 3737739 | 771841 | NA | NA |
| GPSZRA314S5-VP | 0.38 | 0.2739 | 756350 | 310559 | NA | NA |
| | | | | | | |
| *: This value is the relative expression level of the RAGE mRNA compared with the saline group | | | | | | |

**Table 52: Knockdown of RAGE mRNA and protein activity in homozygous RAGE humanized (CDS region) mice at 15 days after aerosol airway administration of GPSZRA222S4-VP, GPSZRA318S2-VP (TRiM delivery)**

| Duplex number | 3 mg/kg (day 15) | | | | | |
|---|---|---|---|---|---|---|
| | Avg expression | Std Dev | Lung RAGE protein | Std Dev | Total BALF RAGE protein (ng/ml) | Std Dev |
| | (Lung RNA) * | (+/-) | (ng /ml) | (+/-) | | (+/-) |
| Saline | 1.22 | 0.8174 | 2263442 | 437588 | 3298 | 588.8 |
| GPSZRA222S4-VP | 2.08 | 0.4728 | 1170762 | 288891 | 2369 | 881.4 |
| GPSZRA318S2-VP | 0.5475 | 0.2732 | 1186007 | 326795 | 2199 | 515 |
| *: This value is the relative expression level of the RAGE mRNA compared with the Saline group | | | | | | |

**Table 53: Knockdown of RAGE mRNA and protein activity in homozygous RAGE humanized (CDS region) mice at 15 days after aerosol airway administration of GPSZRA233S4-VP, GPSZRA222S3-VP (TRiM delivery)**

| Duplex number | 3 mg/kg (day 11) | | | | | |
|---|---|---|---|---|---|---|
| | Avg expression | Std Dev | Lung RAGE protein | Std Dev | Total BALF RAGE protein (ng/ml) | Std Dev |
| | (Lung RNA) * | (+/-) | (ng /ml) | (+/-) | | (+/-) |
| Saline | 1.005 | 0.1173 | 3464072 | 389893 | 2976 | 382.4 |
| GPSZRA233S4-VP | 0.314 | 0.07603 | 1809950 | 471233 | 2896 | 861.6 |
| GPSZRA222S3-VP | 0.285 | 0.2572 | 1909336 | 272295 | 2667 | 817.3 |
| *: This value is the relative expression level of the RAGE mRNA compared with the saline group | | | | | | |

**Table 54: Knockdown of RAGE mRNA and protein activity in heterozygous RAGE humanized (CDS+3'UTR regions) mice at 11 days after aerosol airway administration of 402S4-VP (TRiM delivery)**

| Duplex number | 3 mg/kg (day 11) | | | | | |
|---|---|---|---|---|---|---|
| | Avg expression | Std Dev | Lung RAGE protein | Std Dev | Total BALF RAGE protein (ng/ml) | Std Dev |
| | (Lung RNA) * | (+/-) | (ng /ml) | (+/-) | | (+/-) |
| Saline | 1 | 0 | 1238080 | 0 | 2616 | 0 |
| GPSZRA402S4-VP | 0.025 | 0.007071 | 390571 | 315306 | 1709 | 0 |
| *: This value is the relative expression level of the RAGE mRNA compared with the saline group | | | | | | |

Results: This in vivo experiment showed that VP-modified siRNA could effectively reduce RAGE mRNA and protein expression in mice.

### Example 14: In vivo activity detection in cynomolgus monkeys

This experiment was conducted to determine the efficiency of siRNA in knocking out RAGE protein in cynomolgus monkeys.

### Experimental method:

(1) Animal enrollment: A physical examination was conducted on the fur, limbs, mouth and nose of one male and one female cynomolgus monkey randomly selected, and their basic physiological functions were examined. After the inspection was completed and no abnormalities were found, the animals were put into cages and a two-week acclimatization period began.
(2) In vivo administration: The two cynomolgus monkeys were fasted for more than 3 h before administration. After anesthetizing the animals with Sutacetin (5 mg/kg), tracheal intubation was performed. The animal remained in a supine position, and the nozzle of the high-pressure sprayer was inserted into the trachea through the tracheal tube. The test sample siRNA molecules were injected into the trachea at a concentration of 1.2 mg/kg. After administration, the animal should remain in a semi-reclining position until it regained consciousness, at which point it can be returned to its cage.

The siRNA was also delivered using TRiM, as detailed in Example 13.

(3) BALF sample collection: Animals must fast for more than 3 h before BALF sample collection. After the animals were anesthetized with Zoletil (5 mg/kg), 5 mL of normal saline was instilled into the right lower lung via a bronchoscope. The animal's posture was gently adjusted to allow bronchoalveolar lavage with the normal saline, and then the instilled normal saline was aspirated as much as possible. The BALF supernatant and cells were separated by centrifugation (2000×g, 4°C, 5 min). The supernatant was collected as the BALF sample, stored at -80°C, and the cells were discarded. The end time of tracheal administration was defined as Day 0 (D0), 0h. The BALF collection time points were before D0 administration, D7, D14, D21, D28, D35, D42, D49, and D56. All samples were stored at -80°C.

(4) Detection of protein expression in BALF samples: RAGE protein expression in BALF supernatant was detected using Monkey RAGE ELISA KIT (Animaluni Cat No: LV50407M). Two replicate wells were used for each monkey's sample. Two monkeys, one male and one female, were enrolled. The RAGE expression in the BALF of the female monkey was too low to be detected by the kit. Subsequently, only the male monkey's BALF was tested for RAGE protein knockdown. The testing results of the in vivo activity of duplexes are shown in Table 55 below.

**Table 55: Inhibition of RAGE protein activity in monkeys at different time points after nebulized airway administration of 402S4-VP (TRiM delivery)**

| Duplex number | 1.2 mg/kg BALF protein inhibition efficiency (%) | | | | | |
|---|---|---|---|---|---|---|
| GPSZRA402S4-VP | Day 0 | Day 7 | Day 14 | Day 21 | Day 28 | Day 35 |
| | 0 | 34.2 | 50.6 | 48.7 | 62 | 68 |

Results: This in vivo experiment showed that VP-modified GPSZRA402S4-VP could effectively reduce RAGE protein expression in monkeys.

Although the specific embodiments of the present invention have been described in detail, those skilled in the art will understand that various modifications and changes may be made to the details based on all the teachings published, and these changes are all within the scope of protection of the present invention. The entire scope of the present invention is defined by the appended claims and any equivalents thereof.

## Claims

1. A small interfering RNA (siRNA) for inhibiting the expression of the receptor for advanced glycation end products (RAGE) gene, wherein the siRNA comprises a sense strand and an antisense strand, the antisense strand comprises at least 14 consecutive nucleotides that differ from the nucleotide sequence set forth in any one of SEQ ID NO: 1 to SEQ ID NO: 419 by no more than 4 nucleotides (e.g., 0, 1, 2, 3, or 4 nucleotides), and the sense strand is at least partially complementary to the antisense strand.

2. The siRNA according to claim 1, wherein the antisense strand comprises at least 17 consecutive nucleotides that differ from the nucleotide sequence set forth in any one of SEQ ID NO: 1 to SEQ ID NO: 419 by no more than 2 nucleotides (e.g., 0, 1 or 2 nucleotides).

3. The siRNA according to claim 1 or 2, wherein the sense strand comprises at least 14 consecutive nucleotides that differ from the nucleotide sequence set forth in any one of SEQ ID NO: 420 to SEQ ID NO: 838 by no more than 4 nucleotides (e.g., 0, 1, 2, 3, or 4 nucleotides);
preferably, the sense strand has a region within the 14 consecutive nucleotides that is at least 85% complementary to the antisense strand;
preferably, the sense strand comprises at least 17 consecutive nucleotides that differ from the nucleotide sequence set forth in any one of SEQ ID NO: 420 to SEQ ID NO: 838 by no more than 2 nucleotides (e.g., 0, 1 or 2 nucleotides).

4. The siRNA according to any one of claims 1-3, wherein the siRNA comprises a blunt end and/or an overhang of 1-4 nucleotides;
preferably, the siRNA comprises an overhang of 1 or 2 nucleotides;
preferably, the overhang is present at the 5' end and/or the 3' end of the antisense strand and/or the sense strand; and
preferably, the 3' end of the antisense strand of the siRNA comprises an overhang of 2 nucleotides.

5. The siRNA according to any one of claims 1-4, wherein each of the antisense strand and the sense strand independently has a length of 14-30 nucleotides; preferably, each of the antisense strand and the sense strand independently has a length of 17-30 nucleotides; preferably, the antisense strand has a length of 19-27 nucleotides; and preferably, the sense strand has a length of 17-25 nucleotides.

6. The siRNA according to any one of claims 1-4, wherein the antisense strand has a length of 21-23 nucleotides, and the sense strand has a length of 19-21 nucleotides.

7. The siRNA according to any one of claims 1-6, wherein there are no more than 4 nucleotide mismatches (e.g., 0, 1, 2, 3, or 4 mismatches) between the sense strand and the antisense strand;
preferably, there are no more than 2 nucleotide mismatches (e.g., 0, 1, or 2 mismatches) between the sense strand and the antisense strand.

8. The siRNA according to any one of claims 1-7, wherein the sequences of the sense strand and the antisense strand of the siRNA are selected from the sense strand and antisense strand sequences of any one of the duplexes GPSZRA001 - GPSZRA419 provided in Table 1;
preferably, the sequences of the sense strand and the antisense strand of the siRNA are selected from the sense strand and antisense strand sequences of any one of the duplexes provided in Table 7.

9. The siRNA according to any one of claims 1-8, wherein the siRNA at least comprises one modified nucleotide.

10. The siRNA according to claim 9, wherein all nucleotides in the sense strand and/or antisense strand of the siRNA are modified nucleotides or nucleotide analogs;
preferably, all nucleotides in the sense strand of the siRNA are modified nucleotides or nucleotide analogs, and all nucleotides in the antisense strand of the siRNA are modified nucleotides or nucleotide analogs.

11. The siRNA according to claim 9 or 10, wherein the modified nucleotides or nucleotide analogs are selected from 2'-methoxynucleotides, 2'-fluoronucleotides, 5'-(E)-vinylphosphonate-modified nucleotides, 2'-deoxyribonucleotides, 2',3'-seco-nucleotide analogs, 2'-fluoroarabinonucleotides, 2'-methoxyethyl nucleotides, 2'-amino-modified nucleotides, 2'-alkyl-modified nucleotides, 3'-methoxynucleotides, 2'-allyl-modified nucleotides, phosphorothioate group-containing nucleotides, methylphosphonate group-containing nucleotides, 5'-phosphatecontaining nucleotides, 5'-phosphate mimic-containing nucleotides, diol-modified nucleotides, abasic nucleotides, morpholino nucleotides, locked nucleic acids, unlocked nucleic acids, or glycerol nucleotides.

12. The siRNA according to any one of claims 9-11, wherein the nucleotides in the sense strand of the siRNA are selected from at least 2 of 2'-methoxynucleotides, 2'-fluoronucleotides or locked nucleic acids; and/or the nucleotides in the antisense strand of the siRNA are selected from 2'-methoxynucleotides and 2'-fluoronucleotides; preferably, the first nucleotide of the antisense strand further comprises a 5'-(E)-vinylphosphonate modification.

13. The siRNA according to any one of claims 9-12, wherein the sense strand and/or antisense strand of the siRNA comprises a modified internucleoside linkage;
preferably, each of the 5' end and/or 3' end of the sense strand independently comprises 1 or 2 phosphorothioate linkages; and/or each of the/or 5' end and 3' end of the antisense strand independently comprises 1 or 2 phosphorothioate linkages.

14. The siRNA according to any one of claims 9-13, wherein the antisense strand of the siRNA comprises the following modification patterns:
(1) 5'-NmsNfsNmNfNmNfNmNfNmNfNmNmNmNfNmNfNmNfNmsNmsNm-3' (SEQ ID NO: 1409);
(2) 5'-NmsNfsNmNmNmNfNmNfNmNmNmNmNmNfNmNfNmNmNmsNmsNm-3' (SEQ ID NO: 1410);
(3) 5'- NmsNfsNmNmNmNfNmNmNmNmNmNmNmNfNmNfNmNmNmsNmsNm-3' (SEQ ID NO: 1411);
(4) 5'-VPNmsNfsNmNfNmNfNmNfNmNfNmNmNmNfNmNfNmNfNmsNmsNm-3' (SEQ ID NO: 1423);
(5) 5'-VPNmsNfsNmNmNmNfNmNfNmNmNmNmNmNfNmNfNmNmNmsNmsNm-3' (SEQ ID NO: 1424); or
(6) 5'-VPNmsNfsNmNmNmNfNmNmNmNmNmNmNmNfNmNfNmNmNmsNmsNm-3' (SEQ ID NO: 1425);
wherein Nm represents a methoxy-modified nucleotide, Nf represents a fluoro-modified nucleotide, VP represents a 5'-(E)-vinylphosphonate-modified nucleotide, and s represents a phosphorothioate linkage.

15. The siRNA according to any one of claims 9-14, wherein the sense strand of the siRNA comprises the following modification patterns:
(1) 5'- NmsNmNfNmNfNmNfNfNfNmNfNmNfNmNfNmNfNmsNf-3' (SEQ ID NO: 1412);
(2) 5'- NmsNmNfNmNmNmNfNfNfNmNfNmNfNmNfNmNmNmsNm-3' (SEQ ID NO: 1413);
(3) 5'- NmsNmNmNmNmNmNfNfNfNmNmNmNmNmNmNmNmNmsNm-3' (SEQ ID NO: 1414);
(4) 5'- N(LNA)sNmNfNmNmNmNfNfNfNmNfNmNfNmNfNmNmNmsNm-3' (SEQ ID NO: 1415); or
(5) 5'- NmsNmNmNmNfNmNfNfNfNmNmNmNmNmNfNmNmNmsNm-3' (SEQ ID NO: 1416);
wherein Nm represents a methoxy-modified nucleotide, Nf represents a fluoro-modified nucleotide, N(LNA) represents a locked nucleic acid-modified nucleotide, and s represents a phosphorothioate linkage.

16. The siRNA according to any one of claims 9-15, wherein the antisense strand comprises the nucleotide sequence set forth in any one of SEQ ID NOs: 839-1123, 1426-1438.

17. The siRNA according to any one of claims 9-16, wherein the sense strand comprises the nucleotide sequence set forth in any one of SEQ ID NOs: 1124-1408.

18. The siRNA according to any one of claims 9-17, wherein the sequences of the sense strand and the antisense strand of the siRNA are selected from the sense strand and antisense strand sequences of any one of the duplexes GPSZRA084S1 - GPSZRA402S5-VP provided in Table 4;
preferably, the antisense strand comprises the nucleotide sequence set forth in any one of SEQ ID NOs: 846, 960, 1017, 847, 904, 961, 1018, 1075, 1076, 850, 907, 964, 1021, 1078, 852, 909, 966, 1023, 1080, 913, 970, 1084, 857, 914, 971, 1028, 1085, 1035, 923, 980, 1037, 1094, 1040, 1097, 930, 878, 935, 992, 1049, 1106, 939, 996, 1053, 1110, 889, 946, 1003, 890, 947, 1004, 1061, 1118, 891, 948, 1062, 894, 951, 1008, 1065;
preferably, the sense strand comprises the nucleotide sequence set forth in any one of SEQ ID NOs: 1131, 1245, 1302, 1132, 1189, 1246, 1303, 1360, 1361, 1135, 1192, 1249, 1306, 1363, 1137, 1194, 1251, 1308, 1365, 1198, 1255, 1369, 1142, 1199, 1256, 1313, 1370, 1320, 1208, 1265, 1322, 1379, 1325, 1382, 1215, 1163, 1220, 1277, 1334, 1391, 1224, 1281, 1338, 1395, 1174, 1231, 1288, 1175, 1232, 1289, 1346, 1403, 1176, 1233, 1347, 1179, 1236, 1293, 1350;
more preferably, the sequences of the sense strand and the antisense strand of the siRNA are selected from the sequences of the sense strand and the antisense strand of any one of the duplexes provided in Table 4: GPSZRA222S1, GPSZRA222S3, GPSZRA222S4, GPSZRA227S1, GPSZRA227S2, GPSZRA227S3, GPSZRA227S4, GPSZRA227S5, GPSZRA228S5, GPSZRA231S1, GPSZRA231S2, GPSZRA231S3, GPSZRA231S4, GPSZRA231S5, GPSZRA233S1, GPSZRA233S2, GPSZRA233S3, GPSZRA233S4, GPSZRA233S5, GPSZRA267S2, GPSZRA267S3, GPSZRA267S5, GPSZRA271S1, GPSZRA271S2, GPSZRA271S3, GPSZRA271S4, GPSZRA271S5, GPSZRA282S4, GPSZRA284S2, GPSZRA284S3, GPSZRA284S4, GPSZRA284S5, GPSZRA287S4, GPSZRA287S5, GPSZRA301S2, GPSZRA314S1, GPSZRA314S2, GPSZRA314S3, GPSZRA314S4, GPSZRA314S5, GPSZRA318S2, GPSZRA318S3, GPSZRA318S4, GPSZRA318S5, GPSZRA381S1, GPSZRA381S2, GPSZRA381S3, GPSZRA402S1, GPSZRA402S2, GPSZRA402S3, GPSZRA402S4, GPSZRA402S5, GPSZRA407S1, GPSZRA407S2, GPSZRA407S4, GPSZRA410S1, GPSZRA410S2, GPSZRA410S3, or GPSZRA410S4.

19. The siRNA according to any one of claims 9-17, wherein the antisense strand comprises the nucleotide sequence set forth in any one of SEQ ID NOs: 846, 960, 1017, 847, 1018, 1075, 1023, 913, 980, 1040, 878, 935, 992, 1049, 1106, 939, 996, 1053, 946, 1003, 890, 947, 1061, 1118, 948, 1062, 894, 951, 1065;
preferably, the sense strand comprises the nucleotide sequence set forth in any one of SEQ ID NOs: 1131, 1245, 1302, 1132, 1303, 1360, 1308, 1198, 1265, 1325, 1163, 1220, 1277, 1334, 1391, 1224, 1281, 1338, 1231, 1288, 1175, 1232, 1346, 1403, 1233, 1347, 1179, 1236, 1350;
more preferably, the sequences of the sense strand and the antisense strand of the siRNA are selected from the sequences of the sense strand and the antisense strand of any one of the duplexes provided in Table 4: GPSZRA222S1, GPSZRA222S3, GPSZRA222S4, GPSZRA227S1, GPSZRA227S4, GPSZRA227S5, GPSZRA233S4, GPSZRA267S2, GPSZRA284S3, GPSZRA287S4, GPSZRA314S1, GPSZRA314S2, GPSZRA314S3, GPSZRA314S4, GPSZRA314S5, GPSZRA318S2, GPSZRA318S3, GPSZRA318S4, GPSZRA381S2, GPSZRA381S3, GPSZRA402S1, GPSZRA402S2, GPSZRA402S4, GPSZRA402S5, GPSZRA407S2, GPSZRA407S4, GPSZRA410S1, GPSZRA410S2, or GPSZRA410S4.

20. The siRNA according to any one of claims 9-17, wherein the antisense strand comprises the nucleotide sequence set forth in any one of SEQ ID NOs: 846, 960, 1017, 847, 913, 980, 878, 935, 992, 1106, 939, 1061, 1118;
preferably, the sense strand comprises the nucleotide sequence set forth in any one of SEQ ID NOs: 1131, 1245, 1302, 1132, 1198, 1265, 1163, 1220, 1277, 1391, 1224, 1346, 1403;
more preferably, the sequences of the sense strand and the antisense strand of the siRNA are selected from the sequences of the sense strand and the antisense strand of any one of the duplexes provided in Table 4: GPSZRA222S1, GPSZRA222S3, GPSZRA222S4, GPSZRA227S1, GPSZRA267S2, GPSZRA284S3, GPSZRA314S1, GPSZRA314S2, GPSZRA314S3, GPSZRA314S5, GPSZRA318S2, GPSZRA402S4, or GPSZRA402S5.

21. The siRNA according to any one of claims 9-17, wherein the antisense strand comprises the nucleotide sequence set forth in any one of SEQ ID NOs: 1426-1438;
preferably, the sense strand comprises the nucleotide sequence set forth in any one of SEQ ID NOs: 1131, 1245, 1302, 1132, 1198, 1265, 1163, 1220, 1277, 1391, 1224, 1346, 1403;
more preferably, the sequences of the sense strand and the antisense strand of the siRNA are selected from the sequences of the sense strand and the antisense strand of any one of the duplexes provided in Table 4: GPSZRA222S1-VP, GPSZRA222S3-VP, GPSZRA222S4-VP, GPSZRA227S1-VP, GPSZRA267S2-VP, GPSZRA284S3-VP, GPSZRA314S1-VP, GPSZRA314S2-VP, GPSZRA314S3-VP, GPSZRA314S5-VP, GPSZRA318S2-VP, GPSZRA402S4-VP, GPSZRA402S5-VP.

22. A conjugate, wherein the conjugate comprises the siRNA according to any one of claims 1-21 and a pharmaceutically acceptable targeting molecule.

23. A pharmaceutical composition, wherein the pharmaceutical composition comprises the siRNA according to any one of claims 1-21 or the conjugate according to claim 22, and a pharmaceutically acceptable carrier and/or excipient.

24. The pharmaceutical composition according to claim 23, wherein the pharmaceutically acceptable carrier is a delivery vector; preferably, the siRNA is encapsulated by the delivery vector.

25. Use of the siRNA according to any one of claims 1-21, the conjugate according to claim 22, or the pharmaceutical composition according to claim 23 or 24, in the manufacture of a medicament for treating and/or preventing an RAGE-related disease;
wherein preferably, the siRNA, the conjugate, or the pharmaceutical composition is used alone or in combination with an additional pharmaceutically active agent (e.g., an siRNA targeting a different target sequence in the RAGE gene or an siRNA targeting another target).

26. The use according to claim 25, wherein the RAGE-related disease is a respiratory disease, such as cystic fibrosis, pneumonia, chronic bronchitis, noncystic fibrotic bronchiectasis, chronic obstructive pulmonary disease (COPD), asthma, respiratory infection, primary ciliary dyskinesia, or lung carcinoma cystic fibrosis; preferably, the respiratory disease is asthma or chronic obstructive pulmonary disease (COPD).

27. The use according to claim 25, wherein the RAGE-related disease is a neurological disease, such as Alzheimer's disease or stroke.

28. The use according to claim 25, wherein the RAGE-related disease is a urological disease, such as diabetic nephropathy.

29. The use according to claim 25, wherein the RAGE-related disease is an inflammatory disease or a disease involving inflammation.

30. A method for inhibiting RAGE expression in cells, comprising: introducing into cells the siRNA according to any one of claims 1-18, the conjugate according to claim 22, and/or the pharmaceutical composition according to claim 23 or 24;
wherein preferably, the siRNA, the conjugate, or the pharmaceutical composition is used alone or in combination with an additional pharmaceutically active agent (e.g., an siRNA targeting a different target sequence in the RAGE gene or an siRNA targeting another target).

31. A method for preventing and/or treating an RAGE-related disease in a subject, comprising administering an effective amount of the siRNA according to any one of claims 1-21, the conjugate according to claim 22, or the pharmaceutical composition according to claim 23 or 24 to a subject in need thereof;
preferably, the RAGE-related disease is a respiratory disease, such as cystic fibrosis, pneumonia, chronic bronchitis, noncystic fibrotic bronchiectasis, chronic obstructive pulmonary disease (COPD), asthma, respiratory infection, primary ciliary dyskinesia, or lung carcinoma cystic fibrosis;
preferably, the respiratory disease is chronic obstructive pulmonary disease (COPD);
preferably, the RAGE-related disease is a neurological disease, such as Alzheimer's disease or stroke;
preferably, the RAGE-related disease is a urological disease, such as diabetic nephropathy;
preferably, the RAGE-related disease is an inflammatory disease or a disease involving inflammation;
preferably, the subject is a mammal, such as a human;
wherein preferably, the siRNA, the conjugate, or the pharmaceutical composition is used alone or in combination with an additional pharmaceutically active agent (e.g., an siRNA targeting a different target sequence in the RAGE gene or an siRNA targeting another target).
